# EUROPEAN PATENT SPECIFICATION

(11) **EP 1 296 996 B1**
(45) Date of publication and mention of the grant of the patent: **26.05.2004**
(21) Application number: 01938490.8
(22) Date of filing: 14.06.2001
(51) Int. Cl.: C07H 19/16, A61K 31/7076, A61P 11/00, A61P 29/00

(54) **PURINE DERIVATIVES**
PURINDERIVATE
DERIVES DE PURINE

(30) Priority: 27.06.2000 GB 0015727
(43) Date of publication of application: 02.04.2003
(73) Proprietor: Pfizer Limited, Sandwich, Kent CT13 9NJ (GB); PFIZER INC., New York, N.Y. 10017 (US)
(72) Inventor: MANTELL, Simon John, Sandwich Kent CT13 9NJ (GB); MONAGHAN, Sandra Marina, Sandwich Kent CT13 9NJ (GB); STEPHENSON, Peter Thomas, Sandwich Kent CT13 9NJ (GB)
(74) Representative: Dekker, Henrike
(86) International application number: PCT/IB2001/001064
(87) International publication number: WO 2002/000676

(56) References cited:
- WO-A-00/23457
- WO-A-00/77018
- WO-A-01/27130
- WO-A-01/27131
- WO-A-88/03147
- WO-A-94/07905
- WO-A-99/24451
- WO-A-99/34804
- PIER GIOVANNI BARALDI ET AL.: "Synthesis and Biological Activity of a New Series of N6-Arylcarbamoyl,2-(Ar)alkynyl-N6-arylcarb amoyl, and N6-carboxamido Derivatives of Adenosine-5'-N-ethyluronamide as A1 and A3 Adenosine Receptor Agonists." JOURNAL OF MEDICINAL CHEMISTRY, vol. 41, no. 17, 1998, pages 3174-3185, XP002149470

## Description

This invention relates to purine derivatives. More particularly, this invention relates to 2-aminoalkyl-9-(tetrahydro-2-furanyl)-9H-purine derivatives and to processes for the preparation of, intermediates used in the preparation of, compositions containing and the uses of, such derivatives.

These derivatives are selective, functional agonists of the human adenosine A2a receptor and may be used as anti-inflammatory agents in the treatment of, *inter* alia, diseases of the respiratory tract.

Adenosine is a ubiquitous molecule having a central role in mammalian intermediary metabolism. Independently, adenosine acts on multiple surface receptors to produce a variety of responses. Adenosine receptor classification has revealed the presence of at least four subtypes: A1, A2a, A2b and A3. Stimulation of adenosine A2 receptors on the surface of human neutrophils has been reported to potently inhibit a range of neutrophil functions. Activated neutrophils can damage lung tissue by release of reactive oxygen species, for example, superoxide anion radicals (O₂^{-.}), and granule products, for example, human neutrophil elastase (HNE), amongst other inflammatory mediators. In addition, activated neutrophils perform both de novo synthesis and release of arachidonate products such as leukotriene B₄ (LTB₄). LTB₄ is a potent chemoattractant that recruits additional neutrophils to the inflammatory focus, whereas released O₂^{-.} and HNE adversely affect the pulmonary extracellular matrix. The A2 receptor subtype mediating many of these responses (O₂^{-.} and LTB₄/HNE release and cell adhesion) is established as A2a. The A2 subtype (A2a or A2b) mediating the other effects remains to be established.

Selective agonist activity at the A2a receptor is considered to offer greater therapeutic benefit than the use of non-selective adenosine receptor agonists because interaction with other subtypes is associated with detrimental effects in the lung in animal models and human tissue studies. For example, asthmatics, but not non-asthmatics, bronchoconstrict when challenged with inhaled adenosine. This response is at least in part due to the activation of the A1 receptor subtype. Activation of A1 receptors also promotes neutrophil chemotaxis and adherence to endothelial cells, thus promoting lung injury. Furthermore, many patients with respiratory disease will be co-prescribed β₂-agonists, and negative interaction has been shown in animal studies between isoprenaline and adenosine receptors negatively coupled to adenylate cyclase. Degranulation of human mast cells is promoted by activation of adenosine A2b receptors, thus selectivity over the A2b receptor is also advantageous.

We have now surprisingly found the present purine derivatives inhibit neutrophil function and are selective agonists of the adenosine A2a receptor. They may also have antagonist activity at the adenosine A3 receptor. The present compounds may be used to treat any disease for which an adenosine A2a receptor agonist is indicated. They can be used to treat a disease where leukocyte (e.g. neutrophil, eosinophil, basophil, lymphocyte, macrophage)-induced tissue damage is implicated. They are useful as anti-inflammatory agents in the treatment of diseases of the respiratory tract such as adult respiratory distress syndrome (ARDS), bronchitis, chronic bronchitis, chronic obstructive pulmonary disease, cystic fibrosis, asthma, emphysema, bronchiectasis, chronic sinusitis and rhinitis. The present compounds may also be used in the treatment of septic shock, male erectile dysfunction, male factor infertility, female factor infertility, hypertension, stroke, epilepsy, cerebral ischaemia, peripheral vascular disease, post-ischaemic reperfusion injury, diabetes, rheumatoid arthritis, multiple sclerosis, psoriasis, dermatitis, allergic dermatitis, eczema, ulcerative colitis, Crohns disease, inflammatory bowel disease, *Heliobacter pylori* gastritis, non-*Heliobacter pylori* gastritis, non-steroidal anti-inflammatory drug-induced damage to the gastro-intestinal tract or a psychotic disorder, or for wound healing.

Accordingly, the present invention provides a compound of the formula 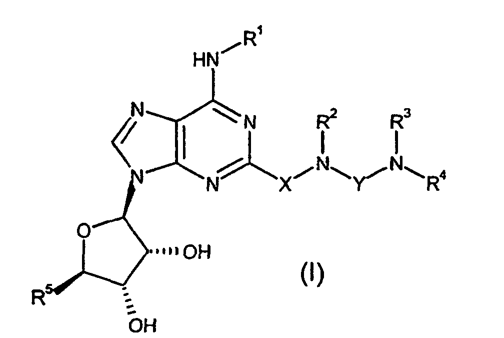 or a pharmaceutically acceptable salt or solvate thereof, wherein
R¹ is (i) H, (ii) C₁-C₆ alkyl optionally substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano, or (iii) fluorenyl;
R² is H or C₁-C₆ alkyl;
either, R³ and R⁴, taken together with the nitrogen atom to which they are attached, represent azetidinyl, pyrrolidinyl, piperidinyl, piperazinyl, homopiperidinyl or homopiperazinyl, each being optionally substituted on a ring nitrogen or carbon atom by C₁-C₆ alkyl or C₃-C₈ cycloalkyl and optionally substituted on a ring carbon atom not adjacent to a ring nitrogen atom by -NR⁶R⁷ or -OR⁹,
or, R³ is H, C₁-C₆ alkyl, C₃-C₈ cycloalkyl or benzyl, said C₁-C₆ alkyl being optionally substituted by C₃-C₈ cycloalkyl, and R⁴ is
   (a) C₁-C₆ alkyl, C₃-C₈ cycloalkyl or R¹⁵, said C₁-C₆ alkyl being optionally substituted by R¹⁵, or
   (b) -(C₂-C₆ alkylene)-R⁸, or
   (c) -(C₁-C₆ alkylene)-R¹³;
R⁵ is -CH₂OH or -CONR¹⁴R¹⁴;
R⁶ and R⁷ are either each independently H or C₁-C₆ alkyl or, taken together with the nitrogen atom to which they are attached, represent azetidinyl, pyrrolidinyl or piperidinyl, said azetidinyl, pyrrolidinyl and piperidinyl being optionally substituted by C₁-C₆ alkyl;
R⁸ is
   (i) azetidin-1-yl, pyrrolidin-1-yl, piperidin-1-yl, morpholin-4-yl, piperazin-1-yl, homopiperidin-1-yl, homopiperazin-1-yl or tetrahydroisoquinolin-1-yl, each being optionally substituted on a ring carbon atom by C₁-C₆ alkyl, C₃-C₈ cycloalkyl, phenyl, C₁-C₆ alkoxy-(C₁-C₆)-alkyl, R⁹R⁹N-(C₁-C₆)-alkyl, fluoro-(C₁-C₆)-alkyl, -CONR⁹R⁹, -COOR⁹ or C₂-C₅ alkanoyl and optionally substituted on a ring carbon atom not adjacent to a ring nitrogen atom by fluoro-(C₁-C₆)-alkoxy, halo, -OR⁹, cyano, -S(O)ₘR¹⁰, -NR⁹R⁹, -SO₂NR⁹R⁹, -NR⁹COR¹⁰ or -NR⁹SO₂R¹⁰ and said piperazin-1-yl and homopiperazin-1-yl being optionally substituted on the ring nitrogen atom not attached to the C₂-C₆ alkylene group by C₁-C₆ alkyl, phenyl, C₁-C₆ alkoxy-(C₂-C₆)-alkyl, R⁹R⁹N-(C₂-C₆)-alkyl, fluoro-(C₁-C₆)-alkyl, C₂-C₅ alkanoyl, -COOR¹⁰, C₃-C₈ cycloalkyl, -SO₂R¹⁰, -SO₂NR⁹R⁹ or -CONR⁹R⁹, or
   (ii) -NR¹¹R¹²;
R⁹ is H, C₁-C₆ alkyl, C₃-C₈ cycloalkyl or phenyl;
R¹⁰ is C₁-C₆ alkyl, C₃-C₈ cycloalkyl or phenyl;
R¹¹ is C₁-C₆ alkyl, C₃-C₈ cycloalkyl or benzyl;
R¹² is C₁-C₆ alkyl, C₃-C₈ cycloalkyl, phenyl, benzyl, fluoro-(C₁-C₆)-alkyl, -CONR⁹R⁹, -COOR¹⁰, -COR¹⁰, -SO₂R¹⁰ or -SO₂NR⁹R⁹, said C₁-C₆ alkyl being optionally substituted by phenyl;
R¹³ is phenyl, pyridin-2-yl, pyridin-3-yl or pyridin-4-yl, each being optionally substituted by C₁-C₆ alkyl, C₁-C₆ alkoxy, halo or cyano;
R¹⁴ is H or C₁-C₆ alkyl optionally substituted by cyclopropyl;
R¹⁵ is azetidin-3-yl, pyrrolidin-3-yl, piperidin-3-yl, piperidin-4-yl, homopiperidin-3-yl or homopiperidin-4-yl, each being optionally substituted by R¹³, C₁-C₆ alkyl, C₃-C₈ cycloalkyl or benzyl;
m is 0, 1 or 2;
X is -CH₂- or -CH₂CH₂-; and
Y is CO, CS, SO₂ or C=N(CN).

In the above definitions, halo means fluoro, chloro, bromo or iodo and alkyl, alkylene, alkanoyl and alkoxy groups containing the requisite number of carbon atoms can be unbranched or branched chain. Examples of alkyl include methyl, ethyl, n-propyl, i-propyl, n-butyl, i-butyl, sec-butyl and t-butyl. Examples of alkoxy include methoxy, ethoxy, n-propoxy, i-propoxy, n-butoxy, i-butoxy, sec-butoxy and t-butoxy. Examples of alkanoyl include acetyl and propanoyl. Examples of alkylene include methylene, 1,1-ethylene, 1,2-ethylene, 1,1-propylene, 1,2-propylene, 1,3-propylene and 2,2-propylene. Examples of cycloalkyl include cyclopropyl, cyclobutyl, cyclopentyl, cyclohexyl and cycloheptyl.

The pharmaceutically acceptable salts of the compounds of the formula (I) include the acid addition and the base salts thereof.

Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, para-toluenesulphonate and pamoate salts.

Suitable base salts are formed from bases which form non-toxic salts and examples are the sodium, potassium, aluminium, calcium, magnesium, zinc and diethanolamine salts.

For a review on suitable salts see Berge *et al*, *J. Pharm. Sci.,* **66**, 1-19, 1977.

The pharmaceutically acceptable solvates of the compounds of the formula (I) include the hydrates thereof.

Also included within the present scope of the compounds of the formula (I) are polymorphs thereof.

A compound of the formula (I) may contain one or more additional asymmetric carbon atoms and therefore exist in two or more stereoisomeric forms. The present invention includes the individual stereoisomers of the compounds of the formula (I) together with, where appropriate, the individual tautomers thereof, and mixtures thereof.

Separation of diastereoisomers may be achieved by conventional techniques, e.g. by fractional crystallisation, chromatography or H.P.L.C. of a stereoisomeric mixture of a compound of the formula (I) or a suitable salt or derivative thereof. An individual enantiomer of a compound of the formula (I) may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate.

Preferably, R¹ is C₁-C₆ alkyl optionally substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano.
Preferably, R¹ is C₁-C₆ alkyl substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano.
Preferably, R¹ is C₁-C₄ alkyl substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano.
Preferably, R¹ is methyl or ethyl substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano.
Preferably, R¹ is methyl or ethyl substituted by 1 or 2 substituents each independently selected from phenyl, naphthyl and fluorenyl, said phenyl, naphthyl and fluorenyl being optionally substituted by C₁-C₆ alkyl or halo.
Preferably, R¹ is diphenylethyl, di(chlorophenyl)ethyl, di(methylphenyl)ethyl, naphthylmethyl or fluorenylmethyl.
Preferably, R¹ is 2,2-diphenyleth-1-yl, 2,2-di(4-chlorophenyl)eth-1-yl, 2,2-di(3-chlorophenyl)eth-1-yl, 2,2-di(4-methylphenyl)eth-1-yl, 2,2-di(3-methylphenyl)eth-1-yl, naphth-1-ylmethyl or fluoren-9-ylmethyl.
Preferably, R¹ is 2,2-diphenyleth-1-yl.

Preferably, R² is H or C₁-C₄ alkyl.
Preferably, R² is H or C₁-C₂ alkyl.
Preferably, R² is H or methyl.
Preferably, R² is H.

Preferably R³ and R⁴ do not form part of the same cyclic structure.

Preferably, R³ is H or C₁-C₆ alkyl.
Preferably, R³ is H or C₁-C₄ alkyl.
Preferably, R³ is H or C₁-C₂ alkyl.
Preferably, R³ is H or methyl.
Preferably, R³ is H.

Preferably, R⁴ is (a) C₁-C₄ alkyl substituted by -R¹⁵, C₃-C₆ cycloalkyl or -R¹⁵; or (b) -(C₂-C₄ alkylene)-R⁸, or (c) -(C₁-C₄ alkylene)-R¹³.
Preferably, R⁴ is (a) C₁-C₂ alkyl substituted by -R¹⁵, C₅-C₆ cycloalkyl or -R¹⁵; or (b) -(ethylene)-R⁸, or (c) -(C₁-C₂ alkylene)-R¹³.
Preferably, R⁴ is -CH₂R¹⁵, cyclohexyl, -R¹⁵, -CH₂CH₂R⁸, -CH₂R¹³ or -CH₂CH₂R¹³.
Preferably, R⁴ is 2-diisopropylaminoeth-1-yl or 2-piperidin-1-yleth-1-yl.

Preferably, R⁵ is -CH₂OH or -CONH(C₁-C₆ alkyl).
Preferably, R⁵ is -CH₂OH or -CONH(C₁-C₄ alkyl).
Preferably, R⁵ is -CH₂OH or -CONH(C₁-C₂ alkyl).
Preferably, R⁵ is -CH₂OH or -CONHCH₂CH₃.

Preferably, R⁸ is (i) piperidin-1-yl or 1,2,3,4-tetrahydroisoquinolin-2-yl, each being optionally substituted on a ring carbon atom by C₁-C₆ alkyl, C₃-C₈ cycloalkyl, phenyl, C₁-C₆ alkoxy-(C₁-C₆)-alkyl, R⁹R⁹N-(C₁-C₆)-alkyl, fluoro-(C₁-C₆)-alkyl, -CONR⁹R⁹, -COOR⁹ or C₂-C₅ alkanoyl and optionally substituted on a ring carbon atom not adjacent to a ring nitrogen atom by fluoro-(C₁-C₆)-alkoxy, halo, -OR⁹, cyano, -S(O)ₘR¹⁰, -NR⁹R⁹, -SO₂NR⁹R⁹, -NR⁹COR¹⁰ or -NR⁹SO₂R¹⁰, or (ii) -NR¹¹R¹².
Preferably, R⁸ is (i) piperidin-1-yl or 1,2,3,4-tetrahydroisoquinolin-2-yl, each being optionally substituted on a ring carbon atom by C₁-C₆ alkyl, or (ii) -NR¹¹R¹².
Preferably, R⁸ is (i) piperidin-1-yl or 1,2,3,4-tetrahydroisoquinolin-2-yl, each being optionally substituted on a ring carbon atom by C₁-C₃ alkyl, or (ii) -NR¹¹R¹².
Preferably, R⁸ is (i) piperidin-1-yl or 1,2,3,4-tetrahydroisoquinolin-2-yl, each being optionally substituted on a ring carbon atom by methyl or propyl, or (ii) -NR¹¹R¹².
Preferably, R⁸ is piperidin-1-yl, 4-(2-propyl)piperidin-1-yl, 2,2,6,6-tetramethylpiperidin-1-yl, 1,2,3,4-tetrahydroisoquinolin-2-yl or -NR¹¹R¹².

Preferably, R¹¹ is C₁-C₈ alkyl or C₃-C₈ cycloalkyl.
Preferably, R¹¹ is C₁-C₅ alkyl or C₃-C₆ cycloalkyl.
Preferably, R¹¹ is propyl, butyl, pentyl, cyclohexyl or cyclopentyl.
Preferably, R¹¹ is -CH(CH₃)₂, -CH₂CH₂CH₂CH₃, -CH₂CH(CH₃)₂, -C(CH₃)₃, -CH(CH₂CH₃)₂, cyclohexyl or cyclopentyl.
Preferably, R¹² is C₁-C₆ alkyl, C₃-C₈ cycloalkyl, -COR¹⁰ or -SO₂R¹⁰ said C₁-C₆ alkyl being optionally substituted by phenyl.
Preferably, R¹² is C₁-C₅ alkyl, C₃-C₆ cycloalkyl, -COR¹⁰ or -SO₂R¹⁰ said C₁-C₆ alkyl being optionally substituted by phenyl.
Preferably, R¹² is propyl, butyl, pentyl, cyclohexyl, cyclopentyl, phenylbutyl, -COPh or -SO₂Ph.
Preferably, R¹² is -CH(CH₃)₂, -CH₂CH₂CH₂CH₃, -CH₂CH(CH₃)₂, -C(CH₃)₃, -CH(CH₂CH₃)₂, -C(CH₃)₂Ph, -SO₂Ph, -COPh, cyclohexyl or cyclopentyl.

Preferably, R¹³ is phenyl or pyridin-2-yl, each being optionally substituted by C₁-C₆ alkyl, C₁-C₆ alkoxy, halo or cyano.
Preferably, R¹³ is phenyl or pyridin-2-yl.

Preferably, R¹⁵ is pyrrolidin-3-yl or piperidin-4-yl, each being optionally substituted by R¹³, C₁-C₆ alkyl, C₃-C₈ cycloalkyl or benzyl.
Preferably, R¹⁵ is pyrrolidin-3-yl or piperidin-4-yl, each being optionally substituted by R¹³ or benzyl.
Preferably, R¹⁵ is 1-benzyl-piperidin-4-yl, 1-(2-pyridinyl)piperidin-4-yl, or 1-benzyl-pyrrolidin-3-yl.

Preferably, X is -CH₂-.

Preferably, Y is CO or C=N(CN).
Preferably, Y is CO.

Preferred individual compounds of the formula (I) include Examples 1-40 listed below and pharmaceutically acceptable salts and solvates thereof.

All of the compounds of the formula (I) can be prepared by conventional routes such as by the procedures described in the general methods presented below or by the specific methods described in the Examples section, or by similar methods thereto. The present invention also encompasses any one or more of these processes for preparing the compounds of formula (I), in addition to any novel intermediates used therein.

In the following general methods, R¹, R², R³, R⁴, R⁵, X and Y are as previously defined for a compound of the formula (I) unless otherwise stated.
1. Compounds of the formula (I) in which R⁵ is -CONR¹⁴R¹⁴ may be prepared by the deprotection of a compound of the formula 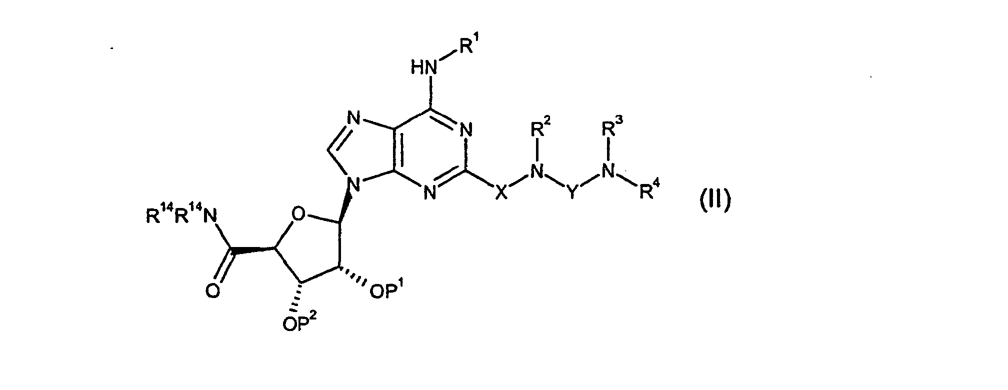 wherein P¹ and P² are suitable protecting groups which may be the same or different and may optionally form part of the same protecting group. Compounds of the formula (I) in which R⁵ is -CH₂OH may be prepared by the deprotection of a compound of the formula 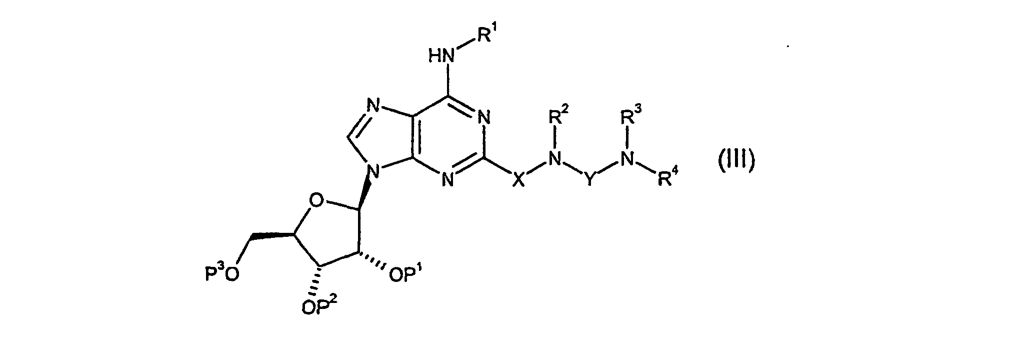 wherein P¹, P² and P³ are suitable protecting groups which may be the same or different, P¹ and P² optionally forming part of the same protecting group. When deprotecting a compound of the formula (II) or a compound of the formula (III), the relevant protecting groups may be removed separately, progressing through one or more semi-protected intermediates, or together, or in any combination. Examples of suitable protecting groups will be apparent to the skilled person [see, for instance, 'Protecting groups in Organic Synthesis (Second Edition)', Theodora W. Green and Peter G. M. Wuts, John Wiley and Sons, 1991]. Preferred individual protecting groups are alkanoyl and aroyl. Preferred protecting groups where P¹ and P² form part of the same protecting group are where P¹ and P² taken together are C₁-C₆ alkylene. Particularly preferred individual protecting groups are acetyl and benzoyl. A particularly preferred protecting group where P¹and P² form part of the same protecting group is where P¹ and P² taken together are dimethylmethylene. Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, a solution of a compound of the formula (III), wherein P¹, P² and P³ are each acetyl, in a suitable solvent, such as methanol, is treated with a nucleophilic reagent, such as ammonia or a primary amine, or a base such as potassium carbonate, typically at room temperature. In another typical procedure, a solution of a compound of the formula (II), wherein P¹and P² are each benzoyl, in a suitable solvent, such as methanol, is treated with a nucleophilic reagent, such as ammonia or a primary amine, or a base such as potassium carbonate, typically at a temperature from room temperature to 60 °C.

Compounds of the formula (II) in which X is -CH₂- (i.e. compounds of the formula (IIA)) and compounds of the formula (III) in which X is -CH₂- (i.e. compounds of the formula (IIIA)) may be prepared according to the route shown in Scheme 1 wherein P⁴ represents a suitable protecting group.

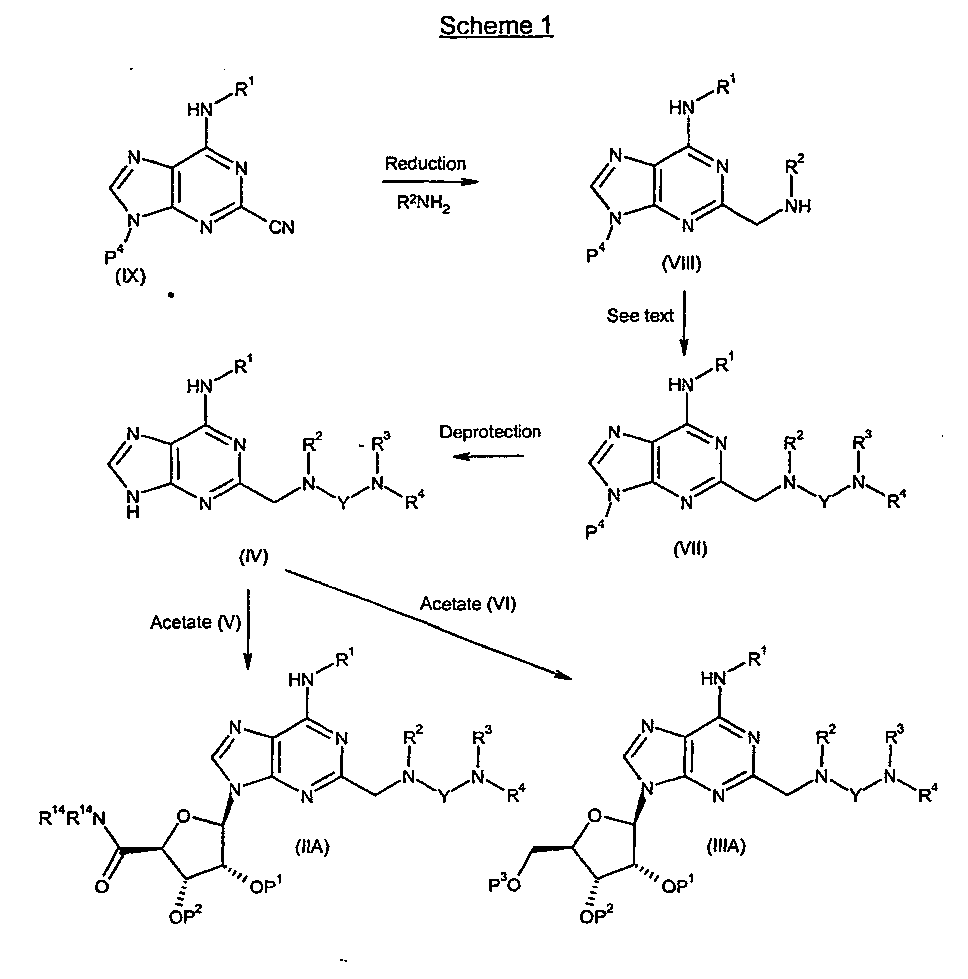

As shown in Scheme 1, compounds of the formula (IIA) may be prepared by the reaction of a compound of the formula 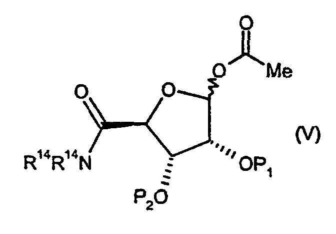 (in which P¹ and P² are as defined above) with trimethylsilyl trifluoromethanesulfonate and a compound of the formula (IV) which has been derivatised with N,O-bis(trimethylsilyl)acetamide. In a typical procedure, the compound of the formula (IV) is heated in the presence of a suitable solvent, such as 1,1,1-trichloroethane, with N,O-bis(trimethylsilyl)acetamide at an elevated temperature, preferably at about 50 °C. The mixture is then allowed to cool and the solvent is evaporated. A solution of the residue in a suitable solvent, such as toluene, is treated with the compound of the formula (V) and trimethylsilyl trifluoromethanesulfonate and the mixture is heated, preferably under reflux, under a nitrogen atmosphere, to give the compound of the formula (IIA).

Compounds of the formula (IIIA) may be prepared by the reaction of a compound of the formula 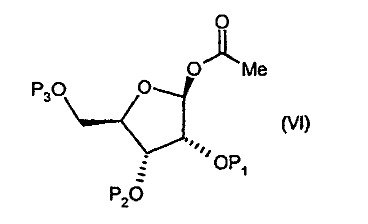 (in which P¹, P² and P³ are as defined above) with trimethylsilyl trifluoromethanesulfonate and a compound of the formula (IV) which has been derivatised with N,O-bis(trimethylsilyl)acetamide. In a typical procedure, the compound of the formula (IV) is heated in the presence of a suitable solvent, such as 1,1,1-trichloroethane, with N,O-bis(trimethylsilyl)acetamide at an elevated temperature, preferably at 50 °C. The mixture is then allowed to cool and the solvent is removed. A solution of the residue in a suitable solvent such as toluene is treated with the compound of the formula (VI) and trimethylsilyl trifluoromethanesulfonate and the mixture is heated, preferably under reflux, under a nitrogen atmosphere, to give the compound of the formula (IIIA).

Compounds of the formula (IV) may be prepared by the deprotection of a compound of the formula (VII) wherein P⁴ is a suitable protecting group. Examples of suitable protecting groups will be apparent to the skilled person [see, for instance, 'Protecting groups in Organic Synthesis (Second Edition)', Theodora W. Green and Peter G. M. Wuts, John Wiley and Sons, 1991]. A preferred protecting group is tetrahydropyran-2-yl. Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, where P⁴ is tetrahydropyran-2-yl, the protecting group is removed by treating a solution of the compound of the formula (VII) in a suitable solvent, such as methanol, with an acid such as hydrochloric acid, preferably 2M aqueous hydrochloric acid.

Compounds of the formula (VII) in which Y is CO may be prepared by the reaction of a compound of the formula 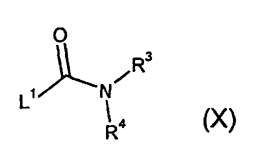 in which L¹ is a suitable leaving group, with a compound of the formula (VIII) in a suitable solvent, such as a mixture of toluene and isopropanol, typically at an elevated temperature, preferably under reflux. The leaving group L¹ is preferably halo (e.g. chloro) or imidazol-1-yl, most preferably imidazol-1-yl. Compounds of the formula (X) wherein L¹ is imidazol-1-yl may be prepared by the reaction of a compound of the formula

R³R⁴NH (XI)

with 1,1'-carbonyldiimidazole. In a typical reaction a compound of the formula (XI) is added to a solution of 1,1'-carbonyldiimidazole in a suitable solvent such as dichloromethane. Compounds of the formula (XI) are either commercially available or may be prepared by standard techniques well known to persons skilled in the art. Other compounds of the formula (X) are either commercially available or easily prepared by methods well known to the person skilled in the art.

Compounds of the formula (VII) in which Y is CO may also be prepared by the reaction of a compound of the formula (VIII) with a compound of the formula

L²COL³ (XII)

in which L² and L³ are suitable leaving groups, to form an intermediate of the formula 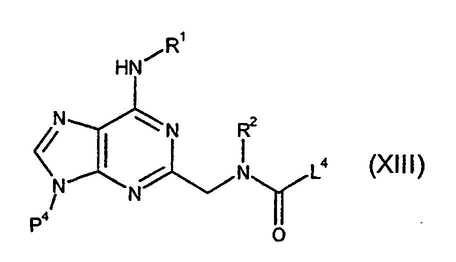 in which L⁴ represents either of the leaving groups L² or L³ and P⁴ is as defined above, followed by the addition of a compound of the formula (XI) to the reaction mixture. Preferably, L² and L³ are each halo or imidazol-1-yl. Most preferably, L² and L³ are each imidazol-1-yl. In a typical example, where L² and L³ are each imidazol-1-yl, a solution of the compound of the formula (VIII) in a suitable solvent, such as dichloromethane, is treated with 1,1'-carbonyldiimidazole. The reaction mixture is stirred, preferably at room temperature, until thin layer chromatography (TLC) indicates a substantially complete reaction has occurred and then a compound of the formula (XI) is added to give the compound of the formula (VII).

Compounds of the formula (VII) in which Y is CS may be prepared by the reaction of a compound of the formula

L⁵L⁶C=S (XIV)

in which L⁵ and L⁶ are suitable leaving groups, with a compound of the formula (VIII), to form an intermediate of the formula 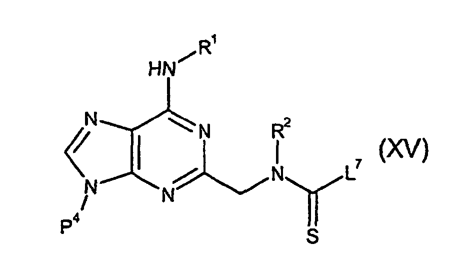 in which L⁷ represents either of the leaving groups L⁵ or L⁶, followed by the addition of a compound of the formula (XI). The leaving groups L⁵ and L⁶ may be the same or different and are typically selected from -S(C₁-C₆ alkyl) or imidazol-1-yl. Preferably, L⁵ and L⁶ are each methylthio or imidazol-1-yl. In a typical procedure, a solution of the compound of the formula (XIV) in a suitable solvent, such as ethanol, is treated with the compound of the formula (VIII), preferably at an elevated temperature, most preferably under reflux. When analysis by thin layer chromatography shows that a substantially complete reaction has occurred, a compound of the formula (XI) is added and the reaction mixture is preferably heated, most preferably under reflux

Alternatively, compounds of the formula (VII) in which Y is CS may be prepared by the reaction of a compound of the formula (XIV) in which L⁵ and L⁶ are as defined above, with a compound of the formula (XI), to form an intermediate of the formula 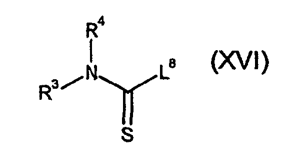 in which L⁸ represents either of the leaving groups L⁵ or L⁶, followed by the addition of a compound of the formula (VIII). In a typical procedure, a solution of the compound of the formula (XIV) in a suitable solvent, such as ethanol, is treated with the compound of the formula (XI), preferably at an elevated temperature, most preferably under reflux. When analysis by thin layer chromatography shows that a substantially complete reaction has occurred, a compound of the formula (VIII) is added and the reaction mixture is preferably heated, most preferably under reflux.

Compounds of the formula (VII) in which Y is SO₂ may be prepared by the reaction of a compound of the formula

R³R⁴NSO₂L⁹ (XVII)

in which L⁹ is a suitable leaving group, typically halo, with a compound of the formula (VIII), optionally in the presence of an acid acceptor. Preferably, L⁹ is chloro. In a typical example, a solution of the compound of the formula (VIII) in a suitable solvent, such as pyridine, is treated with the compound of the formula (XVII) and preferably heated, most preferably at 90°C. Compounds of the formula (XVII) may be prepared by treating a compound of the formula

R³R⁴NSO₃H (XVIII)

with an activating agent. In a typical example, where L⁹ is chloro, a solution of a compound of the formula (XVIII), in a suitable solvent such as toluene, is treated with PCI₅ and heated, preferably under reflux. Compounds of the formula (XVIII) may be prepared by treating a compound of the formula (XI) with chlorosulphonic acid. In a typical procedure, a solution of the compound of the formula (XI) in a suitable solvent, such as dichloromethane, is treated with chlorosulphonic acid, optionally in the presence of a proton acceptor such as triethylamine.

Compounds of the formula (VII) in which Y is C=N(CN) may be prepared by the reaction of a compound of the formula

L¹⁰L¹¹C=N(CN) (XIX)

in which L¹⁰ and L¹¹ are suitable leaving groups, with a compound of the formula (VIII), to form an intermediate of the formula 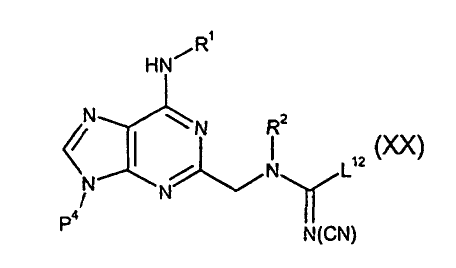 in which L¹² represents either of leaving groups L¹⁰ or L¹¹, followed by the addition of a compound of the formula (XI). The leaving groups L¹⁰ and L¹¹ may be the same or different and are typically selected from halo and -S(C₁-C₆ alkyl). Preferably, L¹⁰ and L¹¹ are each methythio. In a typical procedure, where L¹⁰ and L¹¹ are each methylthio, a solution of a compound of the formula (VIII) in a suitable solvent, such as ethanol, is treated with dimethylcyanothioimidocarbamate, preferably at room temperature. When a substantially complete reaction is indicated by thin layer chromatography (TLC), a compound of the formula (XI) is added and the reaction mixture is preferably heated, most preferably under reflux.

Alternatively, compounds of the formula (VII) in which Y is C=N(CN) may be prepared by the reaction of a compound of the formula (XIX) in which L¹⁰ and L¹¹ are as defined above, with a compound of the formula (XI), to form an intermediate of the formula 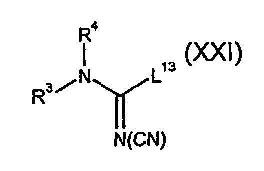 in which L¹³ represents either of the leaving groups L¹⁰ or L¹¹, followed by the addition of a compound of the formula (VIII). In a typical procedure, where L¹⁰ and L¹¹ are each methylthio, a solution of a compound of the formula (XI) in a suitable solvent, such as ethanol, is treated with dimethylcyanothioimidocarbamate, preferably at room temperature. When a substantially complete reaction is indicated by thin layer chromatography (TLC), a compound of the formula (VIII) is added and the reaction mixture is preferably heated, most preferably under reflux.

Compounds of the formula (VIII) may be prepared by the reduction of a compound of the formula (IX) with a suitable reducing agent, preferably a palladium catalyst and hydrogen gas, in the presence of a compound of the formula

R²NH₂ (XXIA).

In a typical procedure, where R² = H, a compound of the formula (IX) is dissolved in a suitable solvent, such as ethanol, which has been saturated with ammonia gas, a palladium catalyst such as 10% w/w palladium on carbon is added and the reaction mixture is stirred under an atmosphere of hydrogen gas, typically at a pressure of 414kPa (60psi). Compounds of the formula (IX) are known in the art (see, for example, WO-A-00/23457). Compounds of the formula (XXIA) are either commercially available or readily prepared by methods well known to those skilled in the art.

Compounds of the formula (III) in which X is -CH₂CH₂- (i.e. compounds of the formula (IIIB)) may be prepared according to the route shown in Scheme 2 wherein A represents an activating group and P¹, P² and P³ represent suitable protecting groups. P¹, P² and P³ may be the same or different, P¹ and P² optionally forming part of the same protecting group. Examples of suitable protecting groups will be apparent to the skilled person [see, for instance, 'Protecting groups in Organic Synthesis (Second Edition)', Theodora W. Green and Peter G. M. Wuts, John Wiley and Sons, 1991]. Preferred individual protecting groups are tri(C₁-C₆)alkylsilyl, di(C₁-C₆)alkylphenylsilyl and (C₁-C₆)alkyldiphenylsilyl. Preferred protecting groups where P¹ and P² form part of the same protecting group are where P¹ and P² taken together are C₁-C₆ alkylene. Particularly preferred individual protecting groups are tert-butyldimethylsilyl and triethylsilyl. A particularly preferred protecting group where P¹ and P² form part of the same protecting group is where P¹ and P² taken together are dimethylmethylene.

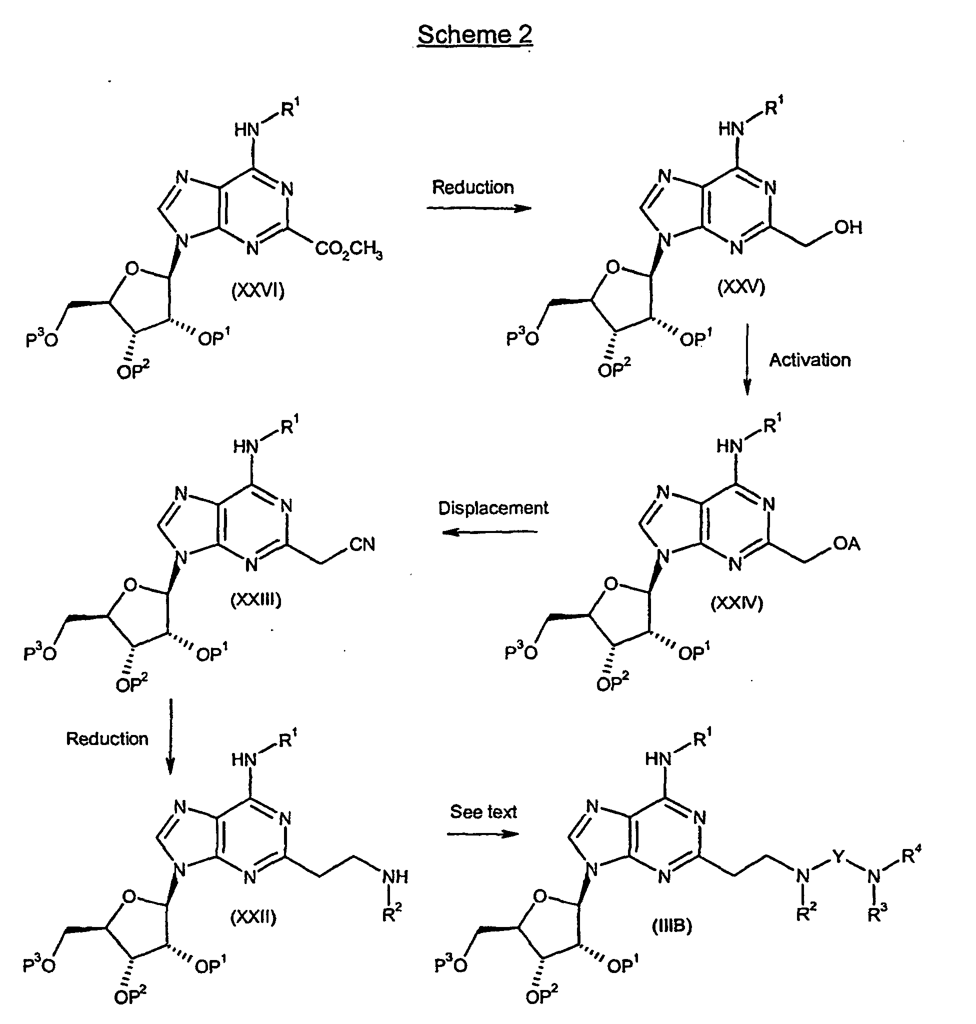

In Scheme 2, the compounds of the formula (IIIB) in which Y is CO may be prepared by the reaction of compound of the formula (X), in which L¹ is as defined above, with a compound of the formula (XXII) in a suitable solvent, such as a mixture of toluene and isopropanol, typically at an elevated temperature, preferably under reflux.

Alternatively, compounds of the formula (IIIB) in which Y is CO may be prepared by the reaction of a compound of the formula (XXII) with a compound of the formula (XII), in which L² and L³ are as defined above, to form an intermediate of the formula 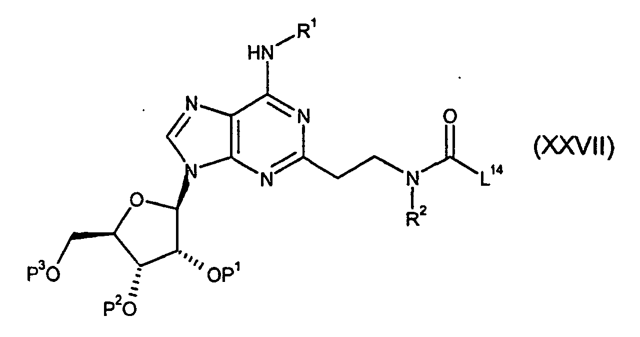 in which L¹⁴ represents either of leaving groups L² or L³. The intermediate (XXVII) is reacted with a compound of the formula (XI) to provide a compound of the formula (IIIB). In a typical example, where L² and L³ are each imidazol-1-yl, a solution of the compound of the formula (XXII) in a suitable solvent, such as dichloromethane, is treated with 1,1'-carbonyldiimidazole. The reaction mixture is stirred, preferably at room temperature, until thin layer chromatography (TLC) indicates a substantially complete reaction has occurred and then a compound of the formula (XI) is added to give the compound of the formula (IIIB).

Compounds of the formula (I) in which Y is CS may be prepared by the reaction of a compound of the formula (XIV) in which L⁵ and L⁶ are as defined above, with a compound of the formula (XXII), to form an intermediate of the formula 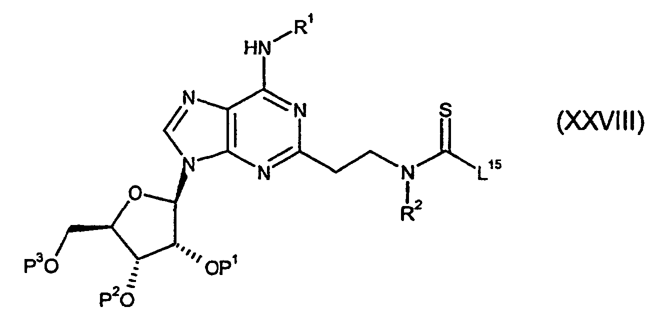 in which L¹⁵ represents either of leaving groups L⁵ or L⁶. The intermediate of the formula (XXVIII) is reacted with a compound of the formula (XI) to provide a compound of the formula (IIIB). In a typical procedure, a solution of the compound of the formula (XIV) in a suitable solvent, such as ethanol, is treated with the compound of the formula (XXII), preferably at an elevated temperature, most preferably under reflux. When analysis by thin layer chromatography shows that a substantially complete reaction has occurred, the compound of the formula (XI) is added and the reaction mixture is preferably heated, most preferably under reflux.

Alternatively, compounds of the formula (IIIB) in which Y is CS may be prepared by the reaction of a compound of the formula (XVI), in which L⁸ is as defined above, with a compound of the formula (XXII). In a typical procedure, a solution of the compound of the formula (XVI) in a suitable solvent, such as ethanol, is treated with the compound of the formula (XXII) and preferably heated, most preferably under reflux.

Compounds of the formula (IIIB) in which Y is SO₂ may be prepared by the reaction of a compound of the formula (XVII), in which L⁹ is as defined above, with a compound of formula (XXII), optionally in the presence of an acid acceptor. In a typical procedure, a solution of the compound of the formula (XVII) in a suitable solvent, such as pyridine, is treated with the compound of the formula (XXII) and heated, typically at 90°C.

Compounds of the formula (IIIB) in which Y is C=N(CN) may be prepared by the reaction of a compound of the formula (XIX) in which L¹⁰ and L¹¹ are as defined above, with a compound of the formula (XXII), to form an intermediate of the formula 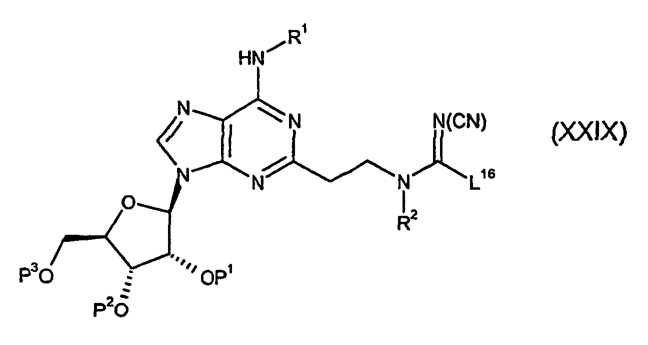 in which L¹⁶ represents either of leaving groups L¹⁰ or L¹¹. The intermediate of the formula (XXIX) is reacted with a compound of the formula (XI) to provide a compound of the formula (IIIB). In a typical procedure, where L¹⁰ and L¹¹ are each methylthio, a solution of a compound of the formula (XXII) in a suitable solvent, such as ethanol, is treated with dimethylcyanothioimidocarbamate, preferably at room temperature. When a substantially complete reaction is indicated by thin layer chromatography (TLC), a compound of the formula (XI) is added and the reaction mixture is preferably heated, most preferably under reflux.

Alternatively, compounds of the formula (IIIB) in which Y is C=N(CN) may be prepared by the reaction of a compound of the formula (XXI), in which L¹³ is as defined above, with a compound of the formula (XXII). In a typical procedure, a solution of the compound of the formula (XXI) in a suitable solvent, such as ethanol, is treated with the compound of the formula (XXII) and preferably heated, most preferably under reflux.

Compounds of the formula (XXII) may be prepared by the reduction of a compound of the formula (XXIII) with a suitable reducing agent in the presence of a compound of the formula (XXIA). A preferred reducing agent is Raney nickel, optionally in the presence of hydrogen gas. In a typical procedure, where R² = H, the compound of the formula (XXIII) is dissolved in a suitable solvent, such as ethanol, which has been saturated with ammonia gas, Raney nickel is added and the reaction mixture is shaken, preferably at room temperature.

Compounds of the formula (XXIII) may be prepared by the displacement of a leaving group 'OA', in which A is an activating group, from a compound of the formula (XXIV) with cyanide anion. In a typical example, a solution of the compound of the formula (XXIV) in a suitable solvent, such. as N,N-dimethylformamide, is treated with a source of cyanide ion, such as potassium cyanide to give the compound of the formula (XXIII). Examples of suitable choices for A will be apparent to the skilled man [see for example 'Advanced Organic Chemistry (Third Edition)', Jerry March, Wiley-Interscience, 1985]. Preferably, A is (C₁-C₆)alkylsulphonyl, phenylsulphonyl or ((C₁-C₆)alkylphenyl)sulphonyl. Most preferably, A is methylsulphonyl.

Compounds of the formula (XXIV) may be prepared by the activation of the free hydroxyl in a compound of the formula (XXV). In a typical example, where A is methylsulphonyl, a solution of the compound of the formula (XXV) in a suitable solvent, such as dichloromethane, is treated with methanesulfonyl chloride in the presence of a proton acceptor such as triethylamine.

Compounds of the formula (XXV) may be prepared by the reduction of an ester of the formula (XXVI) with a suitable reducing agent, such as lithium borohydride, in a suitable solvent, such as tetrahydrofuran.

Compounds of the formula (II) in which X is -CH₂CH₂- (i.e. compounds of the formula (IIB)) may be prepared according to the route shown in Scheme 3 wherein A represents an activating group, as defined above, and P¹ and P² represent suitable protecting groups. P¹ and P² may be the same or different and optionally form part of the same protecting group. Examples of suitable protecting groups will be apparent to the skilled person [see, for instance, 'Protecting groups in Organic Synthesis (Second Edition)', Theodora W. Green and Peter G. M. Wuts, John Wiley and Sons, 1991]. Preferred individual protecting groups are tri(C₁-C₆)alkylsilyl, di(C₁-C₆)alkylphenylsilyl and (C₁-C₆)alkyldiphenylsilyl. Preferred protecting groups where P¹ and P² form part of the same protecting group are where P¹ and P² taken together are C₁-C₆ alkylene. Particularly preferred individual protecting groups are tert-butyldimethylsilyl and triethylsilyl. A particularly preferred protecting group where P¹ and P² form part of the same protecting group is where P¹ and P² taken together are dimethylmethylene.

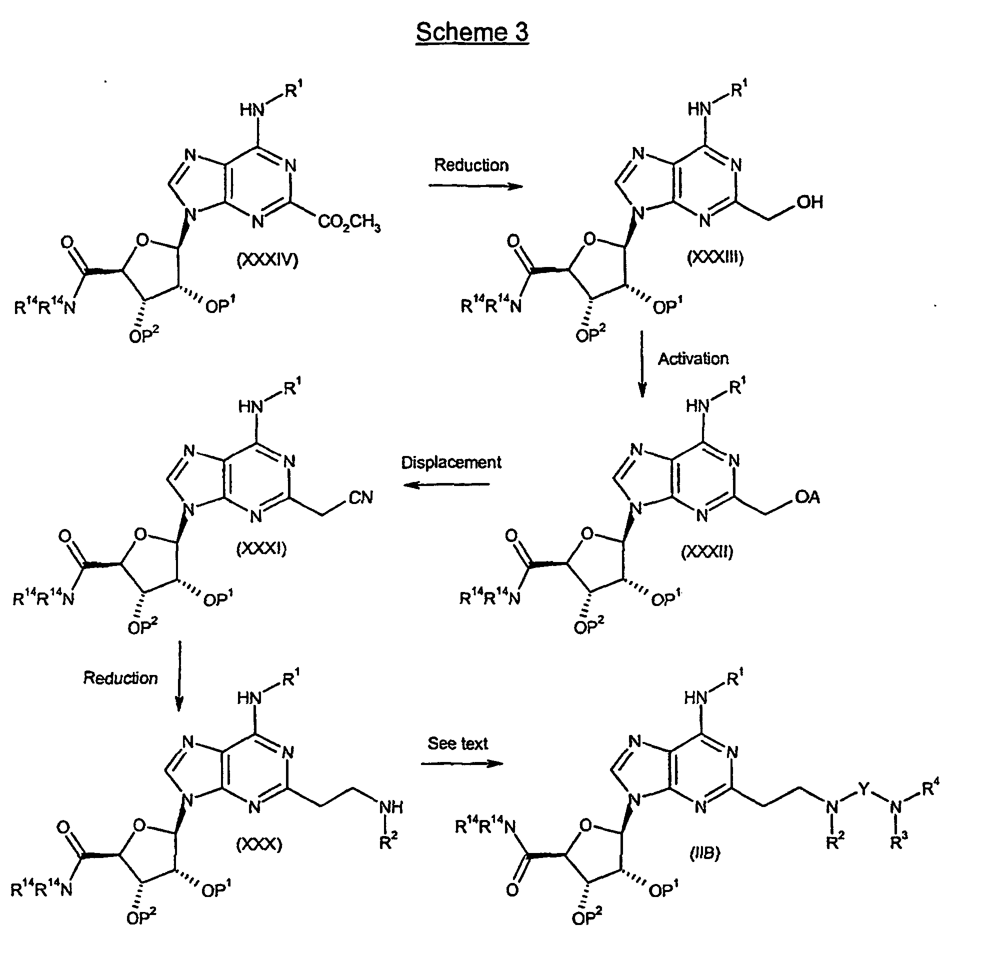

In Scheme 3, the compounds of the formula (IIB) in which Y is CO may be prepared by the reaction of a compound of the formula (X), in which L¹ is as defined above, with a compound of the formula (XXX) in a suitable solvent, such as a mixture of toluene and isopropanol, typically at an elevated temperature, preferably under reflux.

Alternatively, compounds of the formula (IIB) in which Y is CO may be prepared by the reaction of a compound of the formula (XXX) with a compound of the formula (XII), in which L² and L³ are as defined above, to form an intermediate of the formula 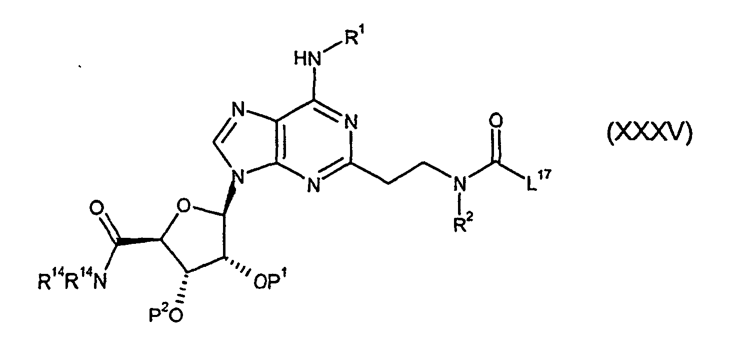 in which L¹⁷ represents either of leaving groups L² or L³. The intermediate of the formula (XXXV) is reacted with a compound of the formula (XI) to form a compound of the formula (IIB). In a typical example, a solution of the compound of the formula (XXX) in a suitable solvent, such as dichloromethane, is treated with the compound of the formula (XII). The reaction mixture is stirred, preferably at room temperature, until thin layer chromatography (TLC) indicates a substantially complete reaction has occurred and then a compound of the formula (XI) is added to give the compound of the formula (IIB).

Compounds of the formula (IIB) in which Y is CS may be prepared by the reaction of a compound of the formula (XIV) in which L⁵ and L⁶ are as defined above, with a compound of the formula (XXX), to form an intermediate of the formula 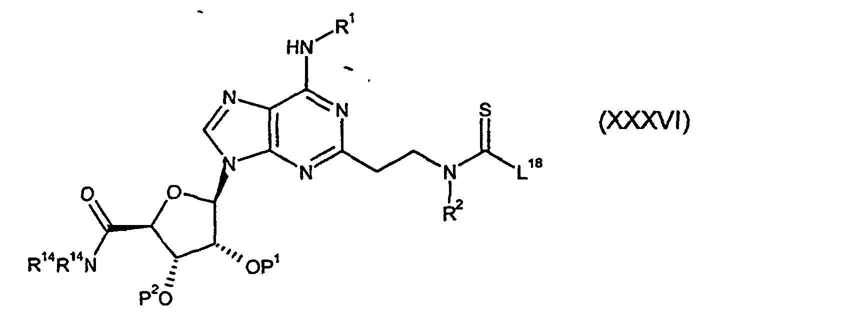 in which L¹⁸ represents either of leaving groups L⁵ or L⁶. The intermediate of the formula (XXXVI) is reacted with a compound of the formula (XI) to provide a compound of the formula (IIB). In a typical procedure, a solution of the compound of the formula (XIV) in a suitable solvent, such as ethanol, is treated with the compound of the formula (XXX), typically at an elevated temperature. Preferably, the reaction mixture is heated under reflux. When analysis by thin layer chromatography shows that a substantially complete reaction has occurred, the compound of the formula (XI) is added and the reaction mixture is preferably heated, most preferably under reflux.

Alternatively, compounds of the formula (IIB) in which Y is CS may be prepared by the reaction of a compound of the formula (XVI), in which L⁸ is as defined above, with a compound of the formula (XXX). In a typical procedure, a solution of the compound of the formula (XVI) in a suitable solvent, such as ethanol, is treated with the compound of the formula (XXX), typically at an elevated temperature. Preferably, the reaction mixture is heated under reflux.

Compounds of the formula (IIB) in which Y is SO₂ may be prepared by the reaction of a compound of the formula (XVII) in which L⁹ is as defined above with a compound of the formula (XXX). In a typical example, a solution of the compound of the formula (XXX) in a suitable solvent, such as pyridine, is treated with the compound of the formula (XVII) and heated, typically at 90°C.

Compounds of the formula (IIB) in which Y is C=N(CN) may be prepared by the reaction of a compound of the formula (XIX) in which L¹⁰ and L¹¹ are as defined above, with a compound of the formula (XXX), to form an intermediate of the formula 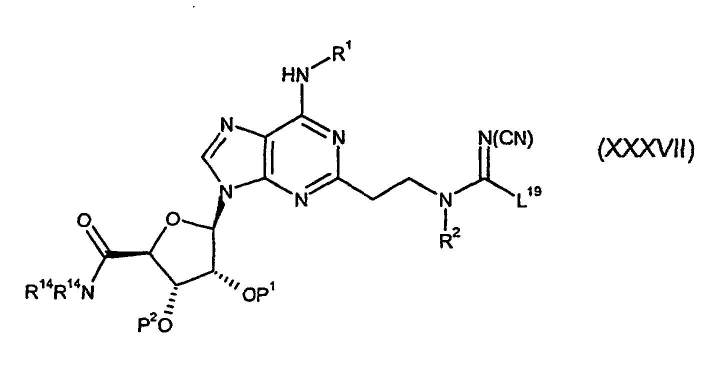 in which L¹⁹ represents either of leaving groups L¹⁰ or L¹¹. The intermediate of the formula (XXXVII) is reacted with a compound of the formula (XI) to provide a compound of the formula (IIB). In a typical procedure, where L¹⁰ and L¹¹ are each methylthio, a solution of a compound of the formula (XXX) in a suitable solvent, such as ethanol, is treated with dimethylcyanothioimidocarbamate, preferably at room temperature. When a substantially complete reaction is indicated by thin layer chromatography (TLC), a compound of the formula (XI) is added and the reaction mixture is preferably heated, most preferably under reflux.

Alternatively, compounds of the formula (IIB) in which Y is C=N(CN) may be prepared by the reaction of a compound of the formula (XXI), in which L¹³ is as defined above, with a compound of the formula (XXX). In a typical procedure, a solution of the compound of the formula (XXI) in a suitable solvent, such as ethanol, is treated with the compound of the formula (XXX) and preferably heated, most preferably under reflux.

Compounds of the formula (XXX) may be prepared by the reduction of a compound of the formula (XXXI) with a suitable reducing agent in the presence of a compound of the formula (XXIA). A preferred reducing agent is Raney nickel, optionally in the presence of hydrogen gas. In a typical example, where R² = H, a compound of the formula (XXXI) is dissolved in a suitable solvent, such as ethanol, which has been saturated with ammonia gas, Raney nickel is added and the reaction mixture is shaken, preferably at room temperature.

Compounds of the formula (XXXI) may be prepared by the displacement of a leaving group 'OA', from a compound of the formula (XXXII) with cyanide anion. In a typical example, a solution of the compound of the formula (XXXII) in a suitable solvent, such as N,N-dimethylformamide, is treated with a source of cyanide ion, such as potassium cyanide, to give the compound of the formula (XXXI).

Compounds of the formula (XXXII) may be prepared by the activation of the free hydroxyl in a compound of the formula (XXXIII). In a typical example, where A is methylsulphonyl, a solution of the compound of the formula (XXXIII) in a suitable solvent, such as dichloromethane, is treated with methanesulphonyl chloride in the presence of a proton acceptor such as triethylamine.

Compounds of the formula (XXXIII) may be prepared by the reduction of an ester of the formula (XXXIV) with a suitable reducing agent, such as lithium borohydride, in a suitable solvent, such as tetrahydrofuran.

2. Compounds of the formula (I) may also be prepared by the derivatisation of a compound of the formula 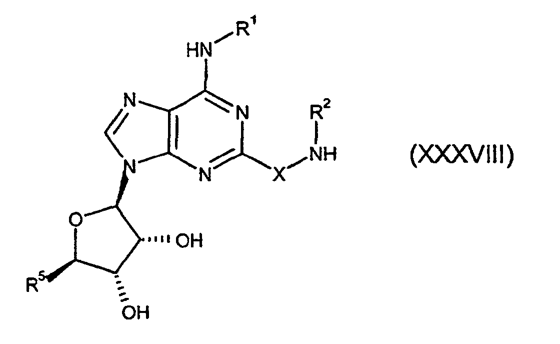 as described below.

Compounds of the formula (I) in which Y is CO may be prepared by the reaction of a compound of the formula (X), in which L¹ is as defined above, with a compound of the formula (XXXVIII) in a suitable solvent, such as a mixture of toluene and isopropanol, preferably at an elevated temperature, most preferably under reflux.

Alternatively, compounds of the formula (I) in which Y is CO may be prepared by the reaction of a compound of the formula (XXXVIII) with a compound of the formula (XII), in which L² and L³ are as defined above, to form an intermediate of the formula 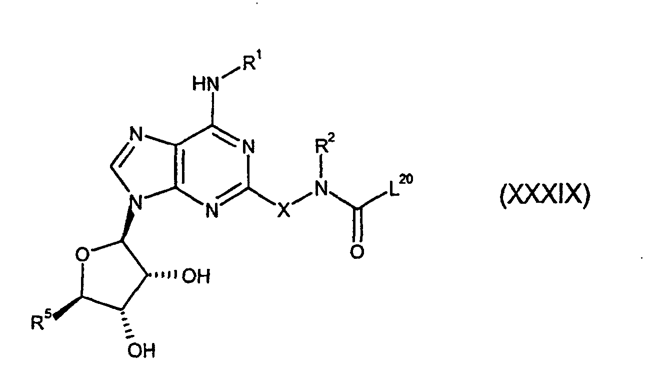 in which L²⁰ represents either of leaving groups L² or L³. The intermediate (XXXIX) is reacted with a compound of the formula (XI) to provide a compound of the formula (I). In a typical example, where L² and L³ are each imidazol-1-yl, a solution of the compound of the formula (XXXVIII) in a suitable solvent, such as dichloromethane, is treated with 1,1'-carbonyldiimidazole. The reaction mixture is stirred, preferably at room temperature, until thin layer chromatography (TLC) indicates a substantially complete reaction has occurred and then a compound of the formula (XI) is added to give the compound of the formula (I).

Compounds of the formula (I) in which Y is CS may be prepared by the reaction of a compound of the formula (XIV) in which L⁵ and L⁶ are as defined above, with a compound of the formula (XXXVIII), to form an intermediate of the formula 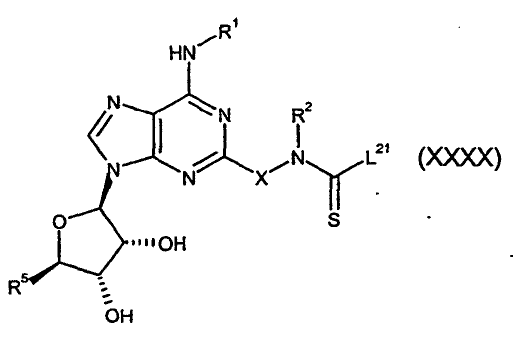 in which L²¹ represents either of the leaving groups L⁵ or L⁶. The intermediate of the formula (XXXX) is reacted with a compound of the formula (XI) to provide a compound of the formula (I). In a typical procedure, a solution of the compound of the formula (XXXVIII) in a suitable solvent, such as ethanol, is treated with the compound of the formula (XIV), preferably at an elevated temperature, most preferably under reflux. When analysis by thin layer chromatography shows that a substantially complete reaction has occurred, a compound of the formula (XI) is added and the reaction mixture is preferably heated, most preferably under reflux.

Alternatively, compound of the formula (I) in which Y is CS may be prepared by the reaction of a compound of the formula (XVI) in which L⁸ is as defined above with a compound of the formula (XXXVIII). In a typical procedure, a solution of the compound of the formula (XVI) in a suitable solvent, such as ethanol, is treated with the compound of the formula (XXXVIII) and preferably heated, most preferably under reflux.

Compounds of the formula (I) in which Y is SO₂ may be prepared by the reaction of a compound of the formula (XVII), in which L⁹ is as defined above, with a compound of the formula (XXXVIII), optionally in the presence of an acid acceptor. In a typical procedure, a solution of the compound of the formula (XXXVIII) in a suitable solvent, such as pyridine, is treated with the compound of the formula (XVII) and heated, typically at 90°C.

Compounds of the formula (I) in which Y is C=N(CN) may be prepared by the reaction of a compound of the formula (XIX) in which L¹⁰ and L¹¹ are as defined above, with a compound of the formula (XXXVIII), to form an intermediate of the formula 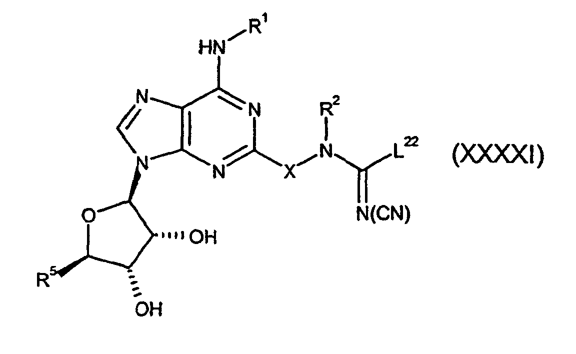 in which L²² represents either of L¹⁰ or L¹¹. The intermediate of the formula (XXXXI) is reacted with a compound of the formula (XI) to give a compound of the formula (I). In a typical procedure, where L¹⁰ and L¹¹ are each methylthio, a solution of a compound of the formula (XXXVIII) in a suitable solvent, such as ethanol, is treated with dimethylcyanothioimidocarbamate, preferably at room temperature. When a substantially complete reaction is indicated by thin layer chromatography (TLC), a compound of the formula (XI) is added and the reaction mixture is preferably heated, most preferably under reflux, to give the compound of the formula (I).

Alternatively, compounds of the formula (I) in which Y is C=N(CN) may be prepared by the reaction of a compound of the formula (XXI), in which L¹³ is as defined above, with a compound of the formula (XXXVIII). In a typical procedure, a solution of the compound of the formula (XXI) in a suitable solvent, such as ethanol, is treated with the compound of the formula (XXXVIII) and preferably heated, most preferably under reflux.

Compounds of the formula (XXXVIII) may be prepared according to the route shown in Scheme 4, wherein P¹, P² and P³ are as defined above.

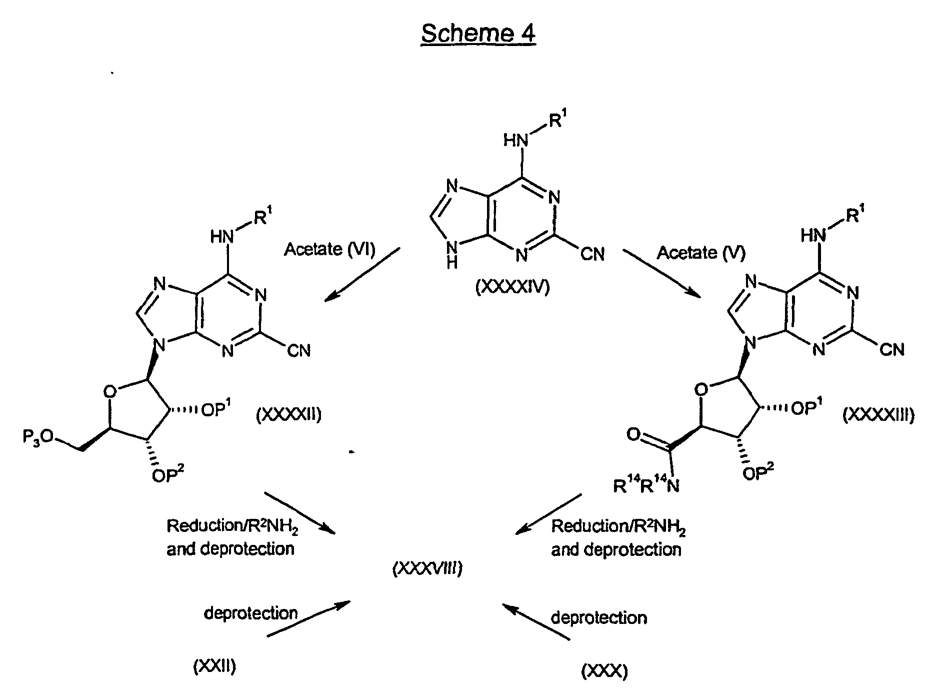

Compounds of the formula (XXXVIII) in which R⁵ is -CH₂OH and X is -CH₂- may be prepared by reducing a compound of the formula (XXXXII) in the presence of a compound of the formula (XXIA) to give a compound of the formula 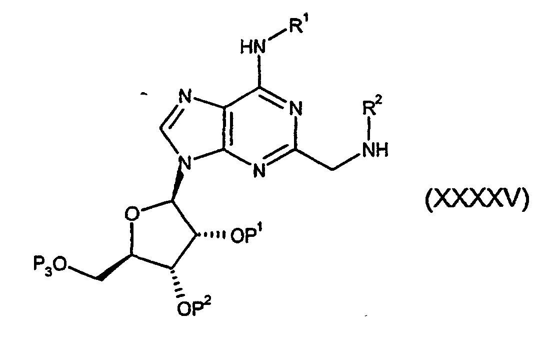 in which P¹, P² and P³ are as defined above, and deprotecting the compound of the formula (XXXXV). Alternatively, if the protecting groups employed are readily removed by the conditions chosen for the reduction, then the reducing and deprotecting steps will usually be performed together to give a compound of the formula (XXXVIII) directly from a compound of the formula (XXXXII). The reduction is carried out using a suitable reducing agent, such as a palladium catalyst and hydrogen gas, in the presence of a compound of formula (XXIA). Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, where R² is H, P¹, P² and P³ are each acetyl and the reducing and deprotecting steps are carried out together, a compound of the formula (XXXXII) is dissolved in a suitable solvent, such as ethanol, which has been saturated with ammonia gas, a palladium catalyst, such as 10% w/w palladium on carbon, is added and the reaction is stirred under an atmosphere of hydrogen gas, typically at a pressure of 414kPa (60psi).

Compounds of the formula (XXXVIII) in which R⁵ is -CONR¹⁴R¹⁴ and X is -CH₂-may be prepared by reducing a compound of the formula (XXXXIII) in the presence of a compound of the formula (XXIA) to give a compound of the formula 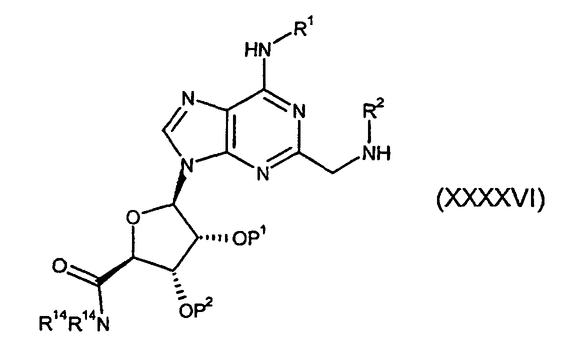 wherein P¹ and P² are as defined above and deprotecting the compound of the formula (XXXXVI). Alternatively, if the protecting groups employed are readily removed by the conditions chosen for the reduction, then the reducing and deprotecting steps will usually be performed together to give a compound of the formula (XXXVIII) directly from a compound of the formula (XXXXIII). The reduction is carried out using a suitable reducing agent, such as a palladium catalyst and hydrogen gas, in the presence of a compound of formula (XXIA). Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, where R² is H, P¹ and P² are each benzoyl and the reducing and deprotecting are carried out together, a compound of the formula (XXXXIII) is dissolved in a suitable solvent, such as ethanol, which has been saturated with ammonia gas, a palladium catalyst, such as 10% w/w palladium on carbon, is added and the reaction is stirred under an atmosphere of hydrogen gas, typically at a pressure of 414kPa (60psi).

Compounds of the formula (XXXXII) may be prepared by the reaction of an acetate of the formula (VI) with trimethylsilyl trifluoromethanesulfonate and a compound of the formula (XXXXIV) which has been derivatised with N,O-bis(trimethylsilyl)acetamide. In a typical procedure, a compound of the formula (XXXXIV) is heated, in the presence of a suitable solvent, such as 1,1,1-trichloroethane, with N,O-bis(trimethylsilyl)acetamide, preferably under reflux. The mixture is then allowed to cool and the solvent is removed. A solution of the residue in a suitable solvent, such as toluene, is treated with the acetate of the formula (VI) and trimethylsilyl trifluoromethanesulfonate. The mixture so formed is preferably heated, most preferably under reflux, under a nitrogen atmosphere, to give the compound of the formula (XXXXII).

Compounds of the formula (XXXXIII) may be prepared by the reaction of an acetate of the formula (V) with a compound of the formula (XXXXIV) and iodine. In a typical example, a compound of the formula (XXXXIV), a compound of the formula (V) and iodine are heated together, preferably at 150°C, under reduced pressure, preferably at 7 kPa (1 psi).

Compounds of the formula (XXXXIV) are known in the art (see, for example, WO-A-00/23457).

Compounds of the formula (XXXVIII) in which R⁵ is -CH₂OH and X is -CH₂CH₂-may be prepared by the deprotection of a compound of the formula (XXII), in which protecting groups P¹, P² and P³ are as defined above. Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, a solution of a compound of the formula (XXII), wherein P¹, P² and P³ are each tert-butyldimethylsilyl, in a suitable solvent, such as methanol, is treated with an acid such as hydrochloric acid, typically at room temperature.

Compounds of the formula (XXXVIII) in which R⁵ is -CONR¹⁴R¹⁴ and X is -CH₂CH₂- may be prepared by the deprotection of a compound of the formula (XXX) in which protecting groups P¹ and P² are as defined above. Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, a solution of a compound of the formula (XXX), wherein P¹ and P² are each tert-butyldimethylsilyl, in a suitable solvent, such as methanol, is treated with an acid such as hydrochloric acid, typically at room temperature.

Scheme 5, wherein P¹, P², P³ and P⁴ are as defined above, illustrates the preparation of compounds of the formula (XXVI) and compounds of the formula (XXXIV) used in Schemes 2 and 3 respectively.

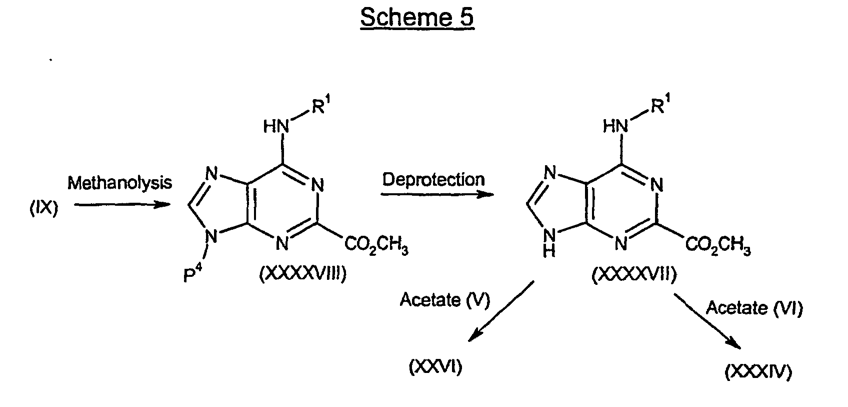

In Scheme 5, compounds of the formula (XXVI) may be prepared by the reaction of a compound of the formula (V) with trimethylsilyl trifluoromethanesulfonate and a compound of the formula (XXXXVII) which has been derivatised with N,O-bis(trimethylsilyl)acetamide. In a typical procedure, the compound of the formula (XXXXVII) is heated in the presence of a suitable solvent, such as 1,1,1-trichloroethane, with N,O-bis(trimethylsilyl)acetamide at an elevated temperature, preferably under reflux. The mixture is then allowed to cool and the solvent is removed. A solution of the residue in a suitable solvent, such as toluene, is treated with the compound of the formula (V) and trimethylsilyl trifluoromethanesulfonate and the mixture is heated, preferably under reflux, under a nitrogen atmosphere, to give the compound of the formula (XXVI).

Compounds of the formula (XXXIV) may be prepared by the reaction of a compound of the formula (VI) with trimethylsilyl trifluoromethanesulfonate and a compound of the formula (XXXXVII) which has been derivatised with N,O-bis(trimethylsilyl)acetamide. In a typical procedure, the compound of the formula (XXXXVII) is heated in the presence of a suitable solvent, such as 1,1,1-trichloroethane, with N,O-bis(trimethylsilyl)acetamide at an elevated temperature, preferably under reflux. The mixture is then allowed to cool and the solvent is removed. A solution of the residue in a suitable solvent, such as toluene, is treated with the compound of the formula (VI) and trimethylsilyl trifluoromethanesulfonate and the mixture is heated, preferably under reflux, under a nitrogen atmosphere, to give the compound of the formula (XXXIV).

It may be desirable in certain cases, having regard to the conditions used in future steps, to change the protecting groups in compounds of the formula (XXVI) or (XXXIV) prepared in this way. Suitable conditions for both the deprotecting step are well known to the skilled person [see, for instance, 'Protecting groups in Organic Synthesis (Second Edition)', Theodora W. Green and Peter G. M. Wuts, John Wiley and Sons, 1991]. In a typical procedure, where P¹, P² and, where appropriate, P³ are each acetyl, a solution of the compound of the formula (XXVI) or the compound of the formula (XXXIV), as the case may be, in a suitable solvent, such as methanol, is treated with a nucleophile such as ammonia or a primary amine, or a base such as potassium carbonate, typically at room temperature. Suitable conditions for the subsequent protecting step are also well known to the skilled person [see, for instance, 'Protecting groups in Organic Synthesis (Second Edition)', Theodora W. Green and Peter G. M. Wuts, John Wiley and Sons, 1991]. In a typical example, where the new protecting groups are to be each tert-butyldimethylsilyl, a solution of the deprotected intermediate, in a suitable solvent such as N,N-dimethylformamide, is treated with tert-butyldimethylsilylchloride and a suitable proton acceptor such as imidazole.

Compounds of the formula (XXXXVII) may be prepared by the deprotection of a compound of the formula (XXXVIII). Suitable conditions for the deprotection are well known in the art [see, for instance, 'Protecting groups in Organic Synthesis (Second Edition)', Theodora W. Green and Peter G. M. Wuts, John Wiley and Sons, 1991]. In a typical procedure, where P⁴ is tetrahydropyran-2-yl, the protecting group may be removed by treating a solution of the compound of the formula (XXXXVIII) in a suitable solvent, such as ethanol, with an acid such as hydrochloric acid.

Compounds of the formula (XXXXVIII) may be prepared by the methanolysis of a compound of the formula (IX). In a typical procedure, a solution of a compound of the formula (IX) in methanol is treated with an alkali metal methoxide, preferably sodium methoxide, and heated under reflux. The resulting mixture is cooled, evaporated, dissolved in a suitable solvent such as tetrahydrofuran and treated with an acid, such as hydrochloric acid, preferably 2N hydrochloric acid, to give the compound of the formula (XXXXVIII).

Compounds of the formula (V), as used in Schemes 1, 4 and 5, may be prepared as shown in Scheme 6, wherein P¹ and P² are as defined above.

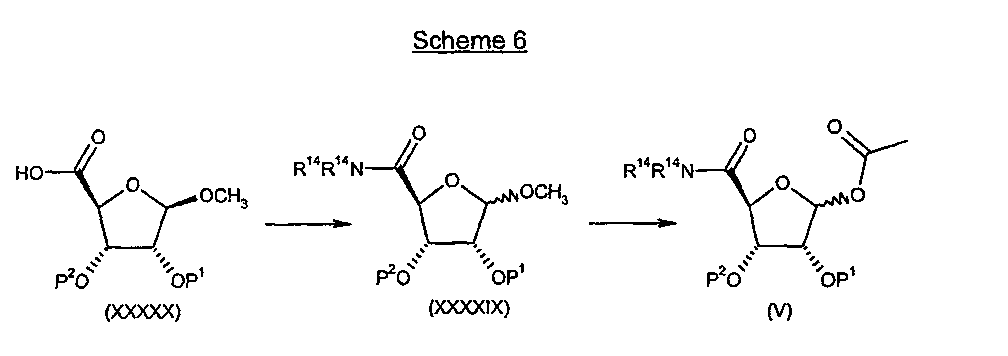

In Scheme 6, compounds of the formula (V) may be prepared by the treatment of a compound of the formula (XXXXIX) with a mixture of acetic acid, acetic anhydride and a strong acid such as hydrochloric or sulphuric acid with cooling (typically to -10 °C). A compound of formula (XXXXIX) may be prepared from an acid of the formula (XXXXX) by activation of the acid as, for example, an acid chloride and treatment of this activated intermediate with a compound of the formula

R¹⁴R¹⁴NH (XXXXXI).

In a typical procedure, a compound of formula (XXXXX) is dissolved in a suitable inert solvent (e.g. dichloromethane) and treated with oxalyl chloride and a catalytic amount of N,N-dimethylformamide. After removal of excess solvent and reagent by evaporation under reduced pressure, the residue is dissolved in anhydrous dichloromethane and treated with a compound of the formula (XXXXXI). With regard to the conditions employed in later steps, it may be appropriate to change the protecting groups P¹ and P² in compounds of the formula (XXXXIX). Alternative, suitable protecting groups are well-known to the skilled person [e.g. 'Protecting Groups in Organic Synthesis (Second Edition)', Theodora W. Green and Peter G. M. Wuts, John Wiley and Sons, 1991]. In a typical case, a solution of the compound of formula (XXXXIX) wherein P¹ and P² taken together are dimethylmethylene in a suitable solvent such as methanol may treated with an acid such as pyridinium para-toluenesulphonate to give a compound of formula (XXXXIX) wherein P¹ and P² are both replaced by H which may be subsequently reprotected with other functionality. For instance, the compound of formula (XXXXIX) wherein P¹ and P² are both replaced by H may be dissolved in a suitable solvent such as dichloromethane and the resulting solution may be treated with an acid acceptor, such as pyridine, and benzoyl chloride to give a compound of formula (XXXXIX) wherein P¹ and P² are each benzoyl. Compounds of the formula (XXXXX) are known in the art (see, for example, *J. Am. Chem. Soc*., 1958, **80**, 5168).

Compounds of the formula (XXXXXI) are either commercially available or easily prepared by methods well known to the person skilled in the art.

Compounds of the formula (VI), as used in Schemes 1, 4 and 5, are either commercially available or easily prepared by methods well known to the person skilled in the art.

3. Compounds of the formula (I) in which R⁴ is -(C₂-C₆ alkylene)-NR¹¹R^{a}, wherein R^{a} is -CONR⁹R⁹, -COOR¹⁰, -COR¹⁰, -SO₂R¹⁰ or -SO₂NR⁹R⁹, may be prepared by the derivatisation of an amine of the formula 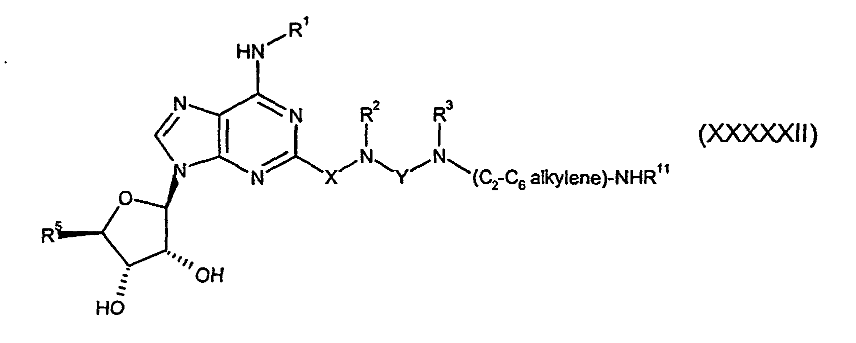 with a suitable acylating or sulphonylating agent. For example, compounds of the formula (I) in which R⁴ is -(C₂-C₆ alkylene)-NR¹¹COR¹⁰ may be prepared by the reaction of a compound of the formula (XXXXXII) with an acid chloride of the formula

R¹⁰COCl (XXXXXIII).

In a typical procedure, a solution of the compound of the formula (XXXXXII) in a suitable solvent, such as a mixture of ethyl acetate and N-methylpyrrolidinone, is treated with a suitable base, preferably a trialkylamine base such as triethylamine, and the compound of the formula (XXXXXIII). As a further example, compounds of the formula (I) in which R⁴ is -(C₂-C₆ alkylene)-NR¹¹SO₂R¹⁰ may be prepared by the reaction of a compound of the formula (XXXXXII) with a compound of the formula

R¹⁰SO₂Cl (XXXXXIV).

In a typical procedure, a solution of the compound of the formula (XXXXXII) in a suitable solvent, such as a mixture of ethyl acetate and N-methylpyrrolidinone, is treated with a suitable base, preferably a trialkylamine base such as triethylamine, and the compound of the formula (XXXXXIV).

Compounds of the formula (XXXXXII) may be prepared by analogy with the methods presented above for the preparation of compounds of the formula (I).

Compounds of the formula (XXXXXIII) or (XXXXXIV) are either commercially available or are easily prepared by methods well known to the skilled man.

A pharmaceutically acceptable salt of a compound of the formula (I) may be readily prepared by mixing together solutions of a compound of the formula (I) and the desired acid or base, as appropriate. The salt may precipitate from solution and be collected by filtration or may be recovered by evaporation of the solvent.

The anti-inflammatory properties of the compounds of the formula (I) are demonstrated by their ability to inhibit neutrophil function which indicates A2a receptor agonist activity. This was evaluated by determining the compound profile in an assay where superoxide production was measured from neutrophils activated by fMLP. Neutrophils were isolated from human peripheral blood using dextran sedimentation followed by centrifugation through Ficoll-Hypaque solution. Any contaminating erythrocytes in the granulocyte pellet were removed by lysis with ice-cold distilled water. Superoxide production from the neutrophils was induced by fMLP in the presence of a priming concentration of cytochalasin B. Adenosine deaminase was included in the assay to remove any endogenously produced adenosine that might suppress superoxide production. The effect of the compound on the fMLP-induced response was monitored colorometrically from the reduction of cytochrome C within the assay buffer. The potency of the compounds was assessed by the concentration giving 50% inhibition (IC₅₀) compared to the control response to fMLP.

The compounds of the formula (I) can be administered alone but will generally be administered in admixture with a suitable pharmaceutical excipient, diluent or carrier selected with regard to the intended route of administration and standard pharmaceutical practice.

For example, the compounds of the formula (I) can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the formula (I) may also be administered as fast-dispersing or fast-dissolving dosage forms or in the form of a high energy dispersion or as coated particles. Suitable formulations of the compounds of the formula (I) may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably com, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain between about 0.01 mg and 500mg of active compound whilst tablet fill weights may range from 50mg to 1000mg. An example of a formulation for a 10mg tablet is illustrated below:

| Ingredient | %w/w |
|---|---|
| Compound of the formula (I) or salt | 10.000* |
| Lactose | 64.125 |
| Starch | 21.375 |
| Croscarmellose sodium | 3.000 |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the formula (I) may be combined with various sweetening or flavouring agents, colouring matter or dyes, with emulsifying and/or suspending agents and with diluents such as water, ethanol, propylene glycol and glycerin, and combinations thereof.

The compounds of the formula (I) can also be administered parenterally, for example, intravenously, intra-arterially, intraperitoneally, intrathecally, intraventricularly, intraurethrally, intrastemally, intracranially, intramuscularly or subcutaneously, or they may be administered by infusion or needleless injection techniques. For such parenteral administration they are best used in the form of a sterile aqueous solution which may contain other substances, for example, a co-solvent and/or enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the formula (I) will usually be from 0.00001 to 100 mg/kg, preferably from 0.0001 to 100 mg/kg (in single or divided doses).

Thus tablets or capsules of the compound of the formula (I) may contain from 0.01 to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The compounds of formula (I) can also be administered intranasally or by inhalation and are conveniently delivered in the form of a dry powder inhaler or an aerosol spray presentation from a pressurised container, pump, spray, atomiser (preferably an atomiser using electrohydrodynamics to produce a fine mist) or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide, a further perfluorinated hydrocarbon such as Perflubron (trade mark) or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol (optionally aqueous ethanol) or a suitable agent for dispersing, solubilising or extending release and the propellant as the solvent, which may additionally contain a lubricant, e.g. sorbitan trioleate. Capsules, blisters and cartridges (made, for example, from gelatin or HPMC) for use in an inhaler or insulator may be formulated to contain a powder mix of a compound of the formula (I) and a suitable powder base such as lactose or starch and a performance modifier such as L-leucine, manitol or magnesium stearate.

A suitable solution formulation for use in an atomiser using electrohydrodynamics to produce a fine mist may contain from 1 µg to 10 mg of a compound of the formula (I), or a salt thereof, and the actuation volume may vary from 1 to 100 µl. A typical formulation may comprise a compound of the formula (I) or salt thereof, propylene glycol, sterile water, ethanol and sodium chloride.

Aerosol or dry powder formulations are preferably arranged so that each metered dose or "puff" contains from 1 to 4000 µg of a compound of the formula (I) for delivery to the patient. The overall daily dose with an aerosol will be in the range of from 1 µg to 20 mg which may be administered in a single dose or, more usually, in divided doses throughout the day.

Alternatively, the compounds of the formula (I) can be administered in the form of a suppository or pessary, or they may be applied topically in the form of a lotion, solution, cream, ointment or dusting powder. The compounds of the formula (I) may also be dermally or transdermally administered, for example, by the use of a skin patch. They may also be administered by the pulmonary, vaginal or rectal routes.

For application topically to the skin, the compounds of the formula (I) can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the formula (I) may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

It is to be appreciated that all references herein to treatment include curative, palliative and prophylactic treatment.

Thus the invention provides:
(i) a compound of the formula (I) or a pharmaceutically acceptable salt or solvate thereof;
(ii) a process for the preparation of a compound of the formula (I) or a pharmaceutically acceptable salt or solvate thereof;
(iii) a pharmaceutical composition including a compound of the formula (I) or a pharmaceutically acceptable salt or solvate thereof, together with a pharmaceutically acceptable excipient, diluent or carrier;
(iv) a compound of the formula (I) or a pharmaceutically acceptable salt, solvate or composition thereof, for use as a medicament;
(v) the use of a compound of the formula (I) or of a pharmaceutically acceptable salt, solvate or composition thereof, for the manufacture of a medicament having A2a receptor agonist activity;
(vi) the use of a compound of the formula (I) or of a pharmaceutically acceptable salt, solvate or composition thereof, for the manufacture of an anti-inflammatory agent;
(vii) the use of a compound of the formula (I) or of a pharmaceutically acceptable salt, solvate or composition thereof, for the manufacture of a medicament for the treatment of a respiratory disease;
(viii) use as in (vii) where the disease is selected from the group consisting of adult respiratory distress syndrome (ARDS), bronchitis, chronic bronchitis, chronic obstructive pulmonary disease, cystic fibrosis, asthma, emphysema, bronchiectasis, chronic sinusitis and rhinitis;
(ix) the use of a compound of the formula (I) or of a pharmaceutically acceptable salt, solvate or composition thereof, for the manufacture of a medicament for the treatment of septic shock, male erectile dysfunction, male factor infertility, female factor infertility, hypertension, stroke, epilepsy, cerebral ischaemia, peripheral vascular disease, post-ischaemic reperfusion injury, diabetes, rheumatoid arthritis, multiple sclerosis, psoriasis, dermatitis, allergic dermatitis, eczema, ulcerative colitis, Crohns disease, inflammatory bowel disease, *Heliobacter pylori* gastritis, non-*Heliobacter pylori* gastritis, non-steroidal anti-inflammatory drug-induced damage to the gastro-intestinal tract or a psychotic disorder, or for wound healing;
(x) a method of treatment of a mammal, including a human being, with a A2a receptor agonist including treating said mammal with an effective amount of a compound of the formula (I) or with a pharmaceutically acceptable salt, solvate or composition thereof;
(xi) a method of treatment of a mammal, including a human being, to treat an inflammatory disease including treating said mammal with an effective amount of a compound of the formula (I) or with a pharmaceutically acceptable salt, solvate or composition thereof;
(xii) a method of treatment of a mammal, including a human being, to treat a respiratory disease including treating said mammal with an effective amount of a compound of the formula (I) or with a pharmaceutically acceptable salt, solvate or composition thereof;
(xiii) a method as in (xii) where the disease is selected from the group consisting of adult respiratory distress syndrome (ARDS), bronchitis, chronic bronchitis, chronic obstructive pulmonary disease, cystic fibrosis, asthma, emphysema, bronchiectasis, chronic sinusitis and rhinitis;
(xiv) a method of treatment of a mammal, including a human being, to treat septic shock, male erectile dysfunction, male factor infertility, female factor infertility, hypertension, stroke, epilepsy, cerebral ischaemia, peripheral vascular disease, post-ischaemic reperfusion injury, diabetes, rheumatoid arthritis, multiple sclerosis, psoriasis, dermatitis, allergic dermatitis, eczema, ulcerative colitis, Crohns disease, inflammatory bowel disease, *Heliobacter pylori* gastritis, non-*Heliobacter pylori* gastritis, non-steroidal anti-inflammatory drug-induced damage to the gastro-intestinal tract or a psychotic disorder, or for wound healing, including treating said mammal with an effective amount of a compound of the formula (I) or with a pharmaceutically acceptable salt, solvate or composition thereof; and
(xv) certain novel intermediates disclosed herein.

The following Examples illustrate the preparation of the compounds of the formula (I).

¹H Nuclear magnetic resonance (NMR) spectra were in all cases consistent with the proposed structures. Characteristic chemical shifts (δ) are given in parts-per-million downfield from tetramethylsilane using conventional abbreviations for designation of major peaks: e.g. s, singlet; d, doublet; t, triplet; q, quartet; m, multiplet; br, broad. The mass spectra (m/z) were recorded in either thermospray or electrospray ionisation mode. The following abbreviations have been used for common solvents: CDCl₃, deuterochloroform; D₆DMSO, deuterodimethylsulphoxide; CD₃OD, deuteromethanol; THF, tetrahydrofuran. The reagent '0.88 concentrated aqueous ammonia' is a concentrated solution of ammonia in water possessing a specific gravity of 0.88. Where thin layer chromatography (TLC) has been used it refers to silica gel TLC using silica gel 60 F₂₅₄ plates, R_{f} is the distance travelled by a compound divided by the distance travelled by the solvent front on a TLC plate.

### EXAMPLE 1

### N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(diisopropylamino)ethyl]urea

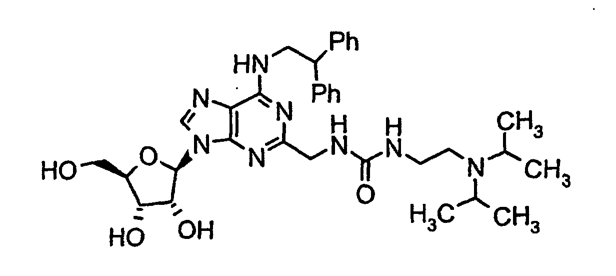

*N*-[2-(Diisopropylamino)ethyl]-1*H*-imidazole-1-carboxamide (84mg, 0.35mmol) (Preparation 27) was added to a stirred solution of (2*R*,3*R*,4*S*,5*R*)-2-{2-(aminomethyl)-6-[(2,2-diphenylethyl)amino]-9*H*-purin-9-yl}-5-(hydroxymethyl)tetrahydro-3,4-furandiol (150mg, 0.35mmol) (Preparation 2) in dichloromethane (5ml) at room temperature. The reaction was heated under reflux for 1 hour and then toluene (5ml) and isopropanol (2ml) were added. The dichloromethane was boiled off and the reaction was then heated under reflux for 1 hour. The reaction mixture was allowed to cool to room temperature and the solvent was removed under reduced pressure. The residue was purified by column chromatography on silica gel eluting with dichloromethane : methanol : 0.88 concentrated aqueous ammonia (95 : 5 : 0.5 by volume increasing to 80 : 20 : 2 by volume). This gave the title compound as a foam (60mg).

δ_{H} (400MHz; CD₃OD): 8.05 (1H, s), 7.35-7.20 (8H, m), 7.15-7.10 (2H, m), 5.90-5.85 (1H, m), 4.75-4.70 (1H, m), 4.50-4.45 (1H, m), 4.40-4.20 (5H, m), 4.15-4.10 (1H, m), 3.90-3.80 (1H, m), 3.70-3.65 (1H, m), 3.10-3.00 (2H, m), 3.00-2.90 (2H, m), 2.50-2.40 (2H, m), 1.00-0.90 (12H, m).

### EXAMPLE 2

### N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(1-piperidinyl)ethyl]urea

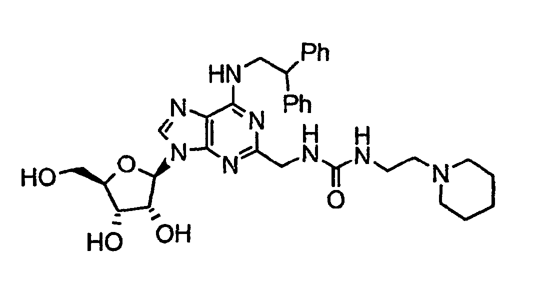

A solution of (2*R*,3*R*,4*R*,5*R*)-4-(acetyloxy)-2-[(acetyloxy)methyl]-5-(6-[(2,2-diphenylethyl)amino]-2-{[({[2-(1-piperidinyl)ethyl]amino}carbonyl)amino] methyl}-9*H*-purin-9-yl)tetrahydro-3-furanyl acetate (100mg, 0.13mmol) (Preparation 6) in methanol (50ml) was saturated with ammonia gas and then left to stand for 3 hours. The solvent was removed under reduced pressure to give a residue that was purified by elution through a plug of silica gel with dichloromethane : methanol : 0.88 concentrated aqueous ammonia (90 : 10 : 1 by volume) to give the title compound as a foam (45mg).
m/z: MH⁺ 631.
δ_{H} (400MHz; CD₃OD): 8.15 (1H, s), 7.40-7.15 (10H, m), 6.00-5.90 (1H, m), 4.90-4.70 (signal obscured by HOD in CD₃OD), 4.60-4.10 (7H, m), 3.90-3.80 (1H, m), 3.80-3.70 (1H, m), 3.30-3.20 (2H, m), 2.55-2.35 (6H, m), 1.65-1.40 (6H, m).

### EXAMPLE 3

### (2S,3S,4R,5R)-5-{2-{[({[2-(Diisopropylamino)ethyl]amino}carbonyl)amino] methyl}-6-[(2,2-diphenylethyl)amino]-9H-purin-9-yl}-N-ethyl-3,4-dihydroxytetrahydro-2-furancarboxamide

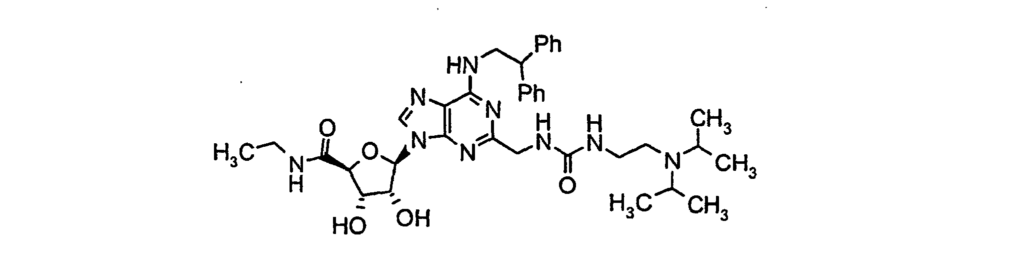

*N*-[2-(Diisopropylamino)ethyl]-1*H*-imidazole-1-carboxamide (84mg, 0.35mmol) (Preparation 27) was added to a stirred suspension of (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (100mg, 0.23mmol) (Preparation 11) in dichloromethane (5ml) at room temperature. The reaction was then heated to reflux and a drop of isopropanol was added to help dissolve the reagents. The reaction mixture was heated under reflux for 20 minutes and then toluene (5ml) was added. The dichloromethane was boiled off and the reaction was then heated under reflux for 30 minutes. The reaction mixture was allowed to cool to room temperature and the solvent was removed under reduced pressure. The residue was purified by column chromatography on silica gel eluting with dichloromethane : methanol : 0.88 concentrated aqueous ammonia (95 : 5 : 0.5 by volume increasing to 80 : 20 : 2 by volume). The solvent was removed under reduced pressure to give a yellow oil. The oil was dissolved in dichloromethane (2ml) and diethylether was added to induce crystallisation. Filtration gave the title compound as a white solid (70mg).
δ_{H} (400MHz; CD₃OD): 8.20 (1H, s), 7.40-7.10 (10H, m), 6.10-6.00 (1H, m), 4.90-4.80 (1H obscured by HOD in MeOH), 4.55-4.20 (7H, m), 3.40-3.10 (6H, m), 2.75-2.60 (2H, m), 1.20-1.00 (15H, m).

### EXAMPLE 4

### (2S,3S,4R,5R)-5-(6-[(2,2-Diphenylethyl)amino]-2-{[({[2-(1-piperidinyl)ethyl] amino}carbonyl)amino]methyl}-9H-purin-9-yl)-N-ethyl-3,4-dihydroxytetrahydro-2-furancarboxamide

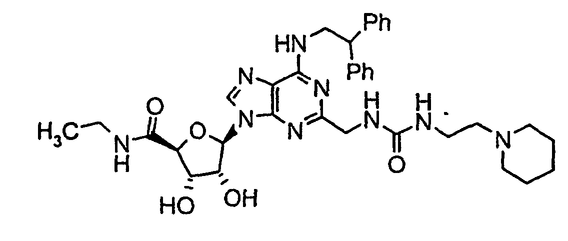

Potassium carbonate (20mg, 0.14mmol) was added to a solution of (2*S*,3*S*,4*R*,5*R*)-4-(benzoyloxy)-5-(6-[(2,2-diphenylethyl)amino]-2-{[({[2-(1-piperidinyl)ethyl]amino}carbonyl)amino]methyl}-9*H*-purin-9-yl)-2-[(ethylamino)carbonyl]tetrahydro-3-furanyl benzoate (100mg, 0.13mmol) (Preparation 14) in methanol (10ml). The reaction mixture was stirred at room temperature for 2 hours. More potassium carbonate (20mg, 0.14mmol) was then added and the reaction mixture was heated to 60°C for 2 hours. The solvent was removed under reduced pressure to give a residue that was slurried with acetone and filtered. The filtrate was evaporated under reduced pressure and the residue was partially purified by column chromatography on silica gel eluting with dichloromethane : methanol : 0.88 concentrated aqueous ammonia (90 : 10 : 1 by volume). The residue after solvent evaporation under reduced pressure was repurified by more column chromatography on silica gel eluting with dichloromethane : methanol : 0.88 concentrated aqueous ammonia (90 : 10 : 1 by volume). The solvent was removed by evaporation under reduced pressure to give the title compound as a foam (17mg).
m/z: MH⁺ 673.
δ_{H} (400MHz; CD₃OD): 8.20 (1H, s), 7.40-7.15 (10H, m), 6.05-6.00 (1H, m), 4.90-4.80 (1H obscured by HOD in MeOH), 4.55-4.20 (7H, m), 3.40-3.20 (4H, m), 2.55-2.40 (6H, m), 1.70-1.50 (4H, m), 1.50-1.40 (2H, m), 1.15-1.05 (3H, m).

### EXAMPLE 5

### (2S,3S,4R,5R)-5-{2-{[((E)-(Cyanoimino){[2-(1-piperidinyl)ethyl]amino} methyl)amino]methyl}-6-[(2,2-diphenylethyl)amino]-9H-purin-9-yl}-N-ethyl-3,4-dihydroxytetrahydro-2-furancarboxamide

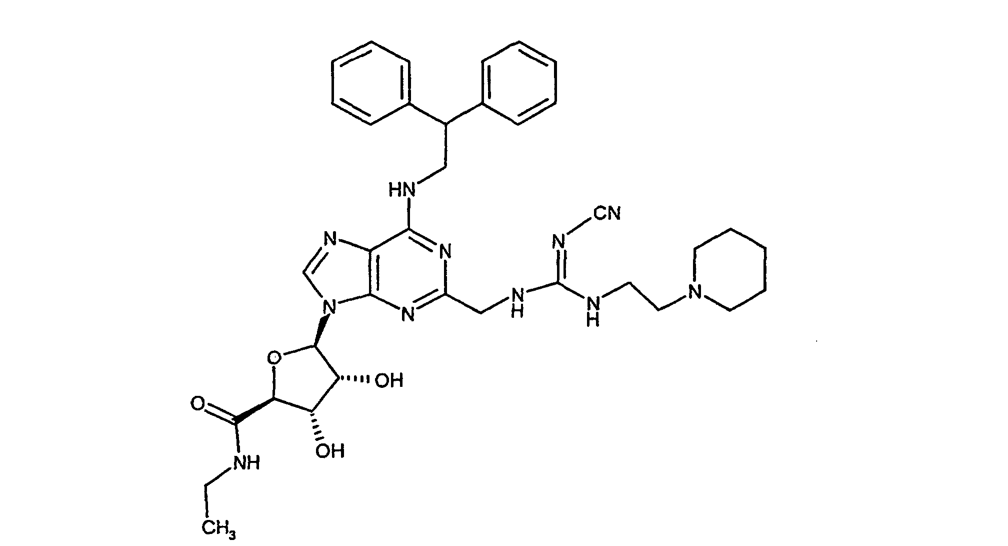

Dimethyl cyanodithioimidocarbonate (77mg, 0.48mmol) was added to a solution of (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (250mg, 0.48mmol) (Preparation 11) in ethanol (10ml). The reaction mixture was stirred for 3 hours at room temperature and then 2-aminoethylpiperidine (88µl, 0.68mmol) was added. The reaction mixture was heated under reflux for 2 hours, more 2-aminoethylpiperidine (0.17ml, 1.2mmol) was added and then the reaction mixture was heated under reflux for a further 4 hours. The reaction was cooled and evaporated to dryness and the residue was purified by flash chromatography on silica gel eluting with dichloromethane : methanol (98 : 2 by volume) increasing in polarity to dichloromethane : methanol : 0.88 concentrated aqueous ammonia (90 : 10 : 1 by volume) to give the title compound (64mg) a foam.
m/z MH⁺ 696.
δ_{H} (400MHz; CDCl₃): 8.30 (1H, s), 8.20 (1H, s), 7.85 (1H, s), 7.35-7.05 (10H, m), 6.95 (1H, bs), 5.95-5.85 (1H, m), 5.60-5.55 (1H, m), 5.45-5.40 (1H, m), 4.60-4.45 (2H, m), 4.40-4.35 (1H, m), 4.25-4.20 (1H, m), 4.15-4.00 (3H, m), 3.35-3.05 (4H, m signal partially obscured by HOD in DMSO), 2.40-2.15 (6H, m), 1.40-1.20 (6H, m).

### EXAMPLE 6

### (2S,3S,4R,5R)-5-{2-({[(Benzylamino)carbonyl]amino}methyl)-6-[(2,2-diphenylethyl)amino]-9H-purin-9-yl}-N-ethyl-3,4-dihydroxytetrahydro-2-furancarboxamide

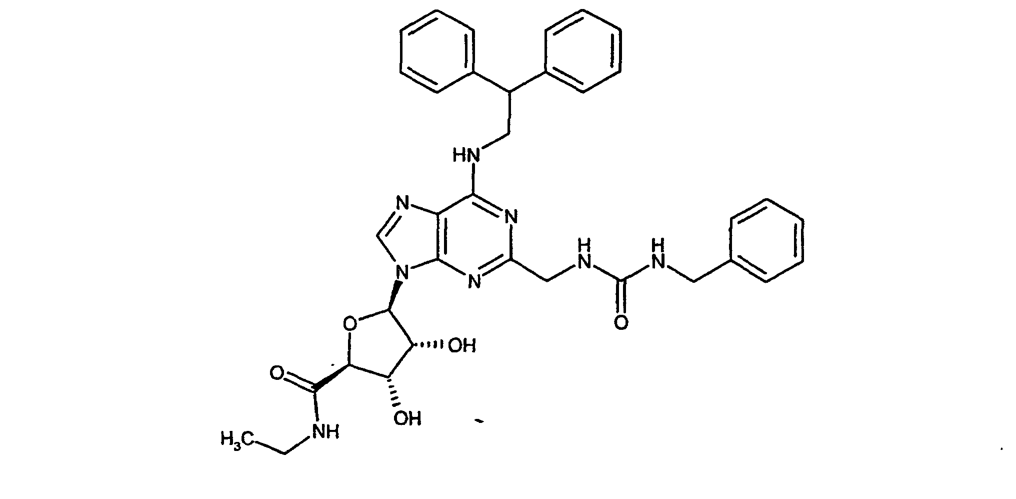

Benzylisocyanate (26mg, 0.30 mmol) was added to a solution of (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (Preparation 11) in dichloromethane (2ml). The reaction mixture was stirred for 16 hours at room temperature and allowed to evaporate. Ethanol (2ml) was added and then aqueous hydrochloric acid (1M, 1ml) was added. The reaction mixture was stirred at 60°C for 6 hours, then allowed to cool to room temperature and left for a further 16h. More aqueous hydrochloric acid (1M, 0.5ml) was added and the reaction mixture was stirred at room temperature for a further 4h. The solvent was removed under reduced pressure and the residue was purified by column chromatography on silica gel eluting with dichloromethane : methanol (95 : 5 by volume) increasing in polarity to dichloromethane : methanol (90 : 10 by volume). The material obtained was impure and hence was repurified by column chromatography on silica gel eluting with dichloromethane : methanol (95 : 5 by volume) increasing in polarity to dichloromethane : methanol (90 : 10 by volume) to give the title compound as a solid (85mg).
δ_{H} (300MHz; D₆DMSO): 8.35-8.25 (2H, m), 7.80-7.75 (1H, m), 7.40-7.10 (15H, m), 6.80-6.70 (1H, m), 6.40-6.30 (1H, m), 6.00-5.90 (1H, m), 5.65-5.60 (1H, m), 5.50-5.40 (1H, m), 4.65-4.55 (2H, m), 4.35-4.05 (8H, m), 3.25-3.05 (2H, m), 1.05-0.95 (3H, m).

### EXAMPLE 7

### (2S,3S,4R,5R)-5-{2-({[(Cyclohexylamino)carbonyl]amino}methyl)-6-[(2,2-diphenylethyl)amino]-9H-purin-9-yl}-N-ethyl-3,4-dihydroxytetrahydro-2-furancarboxamide

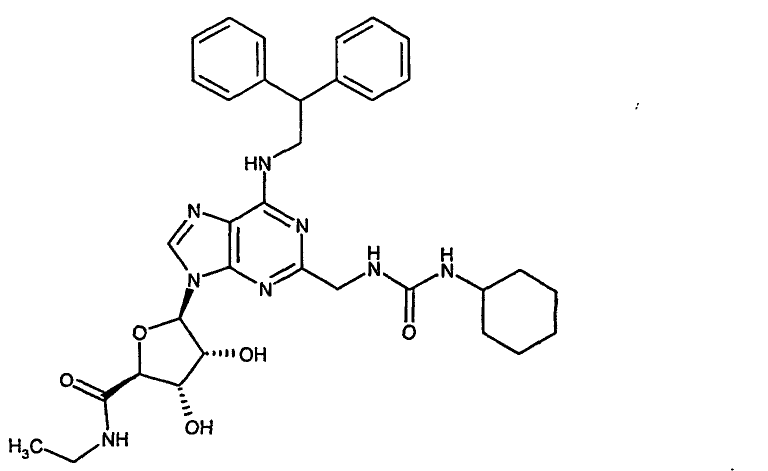

The compound was prepared from cyclohexylisocyanate and (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (Preparation 11) according to the procedure used in Example 6.
m/z MH⁺ 644.
δ_{H} (300MHz; d₆DMSO): 8.40-8.20 (2H, m), 7.80-7.70 (1H, m), 7.40-7.05 (10H, m), 6.25-5.85 (3H, m), 5.65-5.55 (1H, m), 5.55-5.40 (1H, m), 4.70-4.45 (2H, m), 4.35-4.00 (5H, m), 3.50-3.00 (3H, m), 1.85-1.40 (5H, m), 1.30-1.00 (8H, m).

### EXAMPLE 8

### (2S,3S,4R,5R)-5-{2-({[({2-[Benzoyl(isopropyl)amino]ethyl}amino)carbonyl] amino}methyl)-6-[(2,2-diphenylethyl)amino]-9H-purin-9-yl}-N-ethyl-3,4-dihydroxytetrahydro-2-furancarboxamide

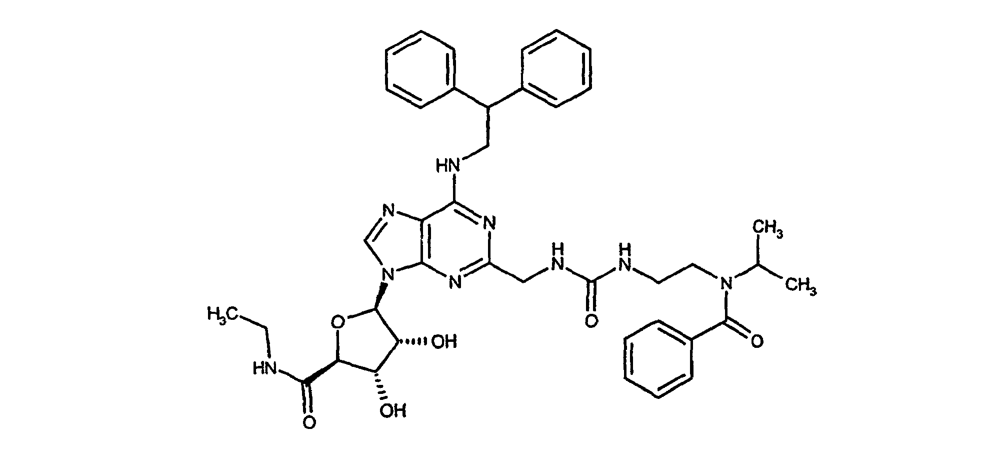

Benzoyl chloride (19mg, 0.14mmol) was added to a stirred solution of (2*S*,3*S*,4*R*,5*R*)-5-(6-[(2,2-diphenylethyl)amino]-2-{[({[2-(isopropylamino)ethyl]amino}carbonyl)amino]methyl}-9*H*-purin-9-yl)-*N*-ethyl-3,4-dihydroxytetrahydro-2-furancarboxamide (80mg, 0.12mmol) (Preparation 12) and triethylamine (0.034ml, 0.25mmol) in ethyl acetate (5ml) and N'-methylpyrrolidinone (0.2ml) at room temperature. The reaction mixture was stirred for 96 hours, washed with water (2ml) and evaporated under reduced pressure. The residue was purified by column chromatography on silica gel eluting with dichloromethane : methanol : 0.88 concentrated aqueous ammonia (90 : 10 : 1 by volume) increasing in polarity to dichloromethane : methanol : 0.88 concentrated aqueous ammonia (80 : 20 :3 by volume) to give the title compound as a foam (35mg).
m/z MH⁺ 787.
δ_{H} (400MHz; CD₃OD): 8.20 (1H, s), 7.50-7.15 (15H, m), 6.05-6.00 (1H, m), 4.90-4.80 (1H, m), 4.55-4.20 (7H, m), 3.95-3.85 (1H, m), 3.50-3.20 (6H, m), 1.20-1.00 (9H, m).

### EXAMPLE 9

### (2S,3S,4R,5R)-5-[6-[(2,2-Diphenylethyl)amino]-2-({[({2-[isopropyl(phenylsulfonyl)amino]ethyl}amino)carbonyl]amino}methyl)-9H-purin-9-yl]-N-ethyl-3,4-dihydroxytetrahydro-2-furancarboxamide

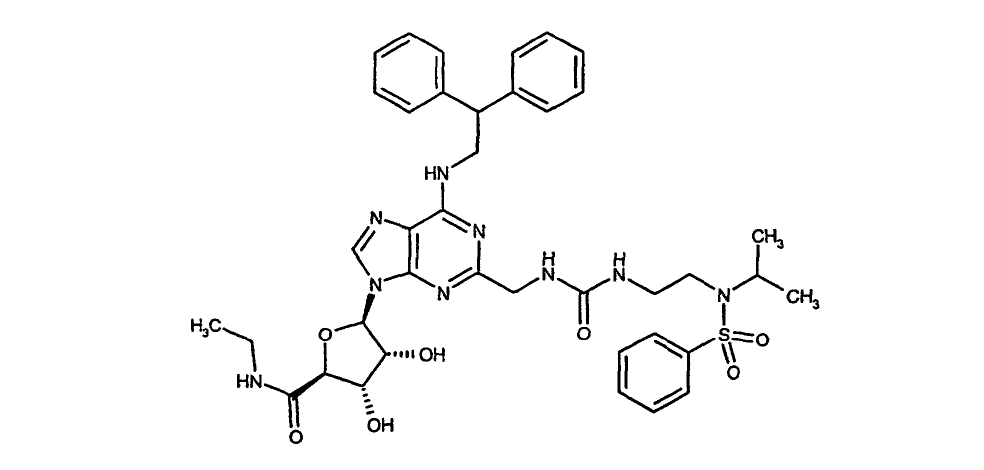

The title compound was prepared from (2*S*,3*S*,4*R*,5*R*)-5-(6-[(2,2-diphenylethyl)amino]-2-{[({[2-(isopropylamino)ethyl]amino}carbonyl)amino] methyl}-9*H*-purin-9-yl)-*N*-ethyl-3,4-dihydroxytetrahydro-2-furancarboxamide (80mg, 0.12mmol) (Preparation 12) and benzenesulphonyl chloride (0.0017ml, 0.14mmol) by a similar method to that of Example 8.
m/z MH⁺ 787.
δ_{H} (400MHz; CD₃OD): 8.15 (1H, s), 7.80-7.75 (2H, m), 7.60-7.50 (1H, m), 7.50-7.40 (2H, m), 7.30-7.15 (8H, m), 7.15-7.05 (2H, m), 6.05-5.95 (1H, m), 4.50-4.30 (5H, m), 4.30-4.20 (2H, m), 4.05-3.95 (1H, m), 3.40-3.20 (4H, m), 3.20-3.10 (2H, m), 1.10-1.00 (3H, m), 1.00-0.90 (6H, m).

### EXAMPLE 10

### N'-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N-methyl-N-[2-(2-pyridinyl)ethyl]urea

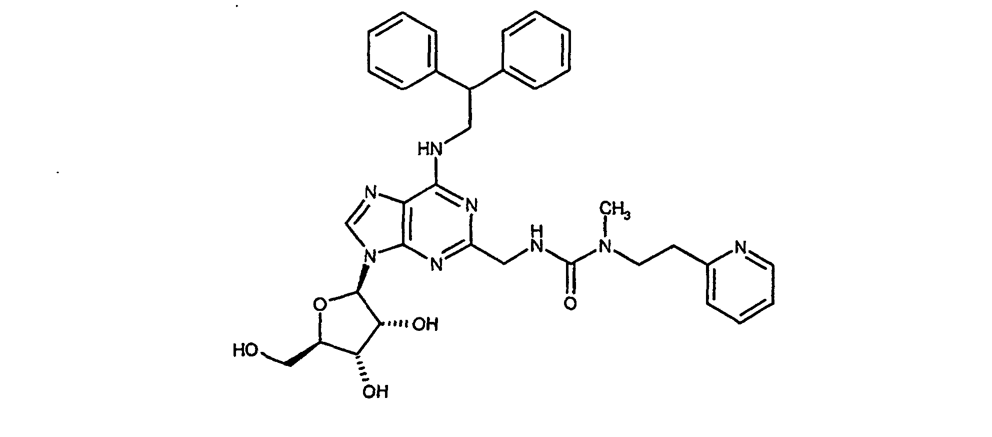

*N*,*O*-Bistrimethylsilylacetamide (0.5ml, 2.02mmol) was added to a suspension of *N*'-({6-[(2,2-diphenylethyl)amino]-9*H*-purin-2-yl}methyl)-*N*-methyl-*N*-[2-(2-pyridinyl)ethyl]urea (0.16g, 0.31mol) (Preparation 26) in 1,1,1-trichloroethane (15ml). The suspension was heated to reflux. When all suspended solid had dissolved the reaction mixture was allowed to cool to room temperature and the solvent was removed under reduced pressure. The residue was twice dissolved in toluene (50ml) and the solvent was removed under reduced pressure. The residue was then dissolved in toluene (15ml) and (2*R*,3*R*,4*R*,5*S*)-4,5-bis(acetyloxy)-2-[(acetyloxy)methyl]tetrahydro-3-furanyl acetate (0.112g, 0.36mol) was added. The solution was stirred at room temperature and trimethylsilyltrifluoromethanesulphonate (0.17ml, 0.94mmol) was added. The resulting solution was heated under reflux for 2 hours and then allowed to cool to room temperature. The reaction mixture was diluted by the addition of ethyl acetate (75ml) and then washed with saturated aqueous sodium hydrogen carbonate solution (two portions of 30ml) and saturated aqueous sodium chloride solution (30ml). The organic layer was dried over anhydrous magnesium sulphate. The solvent was removed under reduced pressure to give a solid that was purified by column chromatography on silica gel eluting with dichloromethane : methanol : 0.88 concentrated aqueous ammonia (97 : 3 : 0.5 by volume). This material was dissolved in methanol (50ml) and a stream of ammonia gas was passed through the solution until it was saturated. The solution was allowed to stand at room temperature for 4 hours. The solvent was removed under reduced pressure to give a residue that was purified by flash chromatography on silica gel eluting with dichloromethane : methanol : 0.88 concentrated aqueous ammonia (90 : 10 : 1 by volume) to give the title compound (0.05g) as a white solid.
m/z MH⁺ 639.
δ_{H} (300MHz; CD₃OD): 8.45-8.40 (1H, m), 8.10 (1H, s), 7.75-7.65 (1H, m), 7.35-7.10 (11H, m), 5.95-5.90 (1H, m), 4.80-4.75 (1H, m), 4.50-4.40 (3H, m), 4.40-4.20 (3H, m), 4.20-4.15 (1H, m), 3.90-3.85 (1H, m), 3.75-3.70 (1H, m), 3.60-3.45 (2H, m), 3.00-2.90 (2H, m), 2.80 (3H, s).

### EXAMPLES 11-28

The following Examples were prepared by a similar method to that of Example 3 using the stated starting materials.

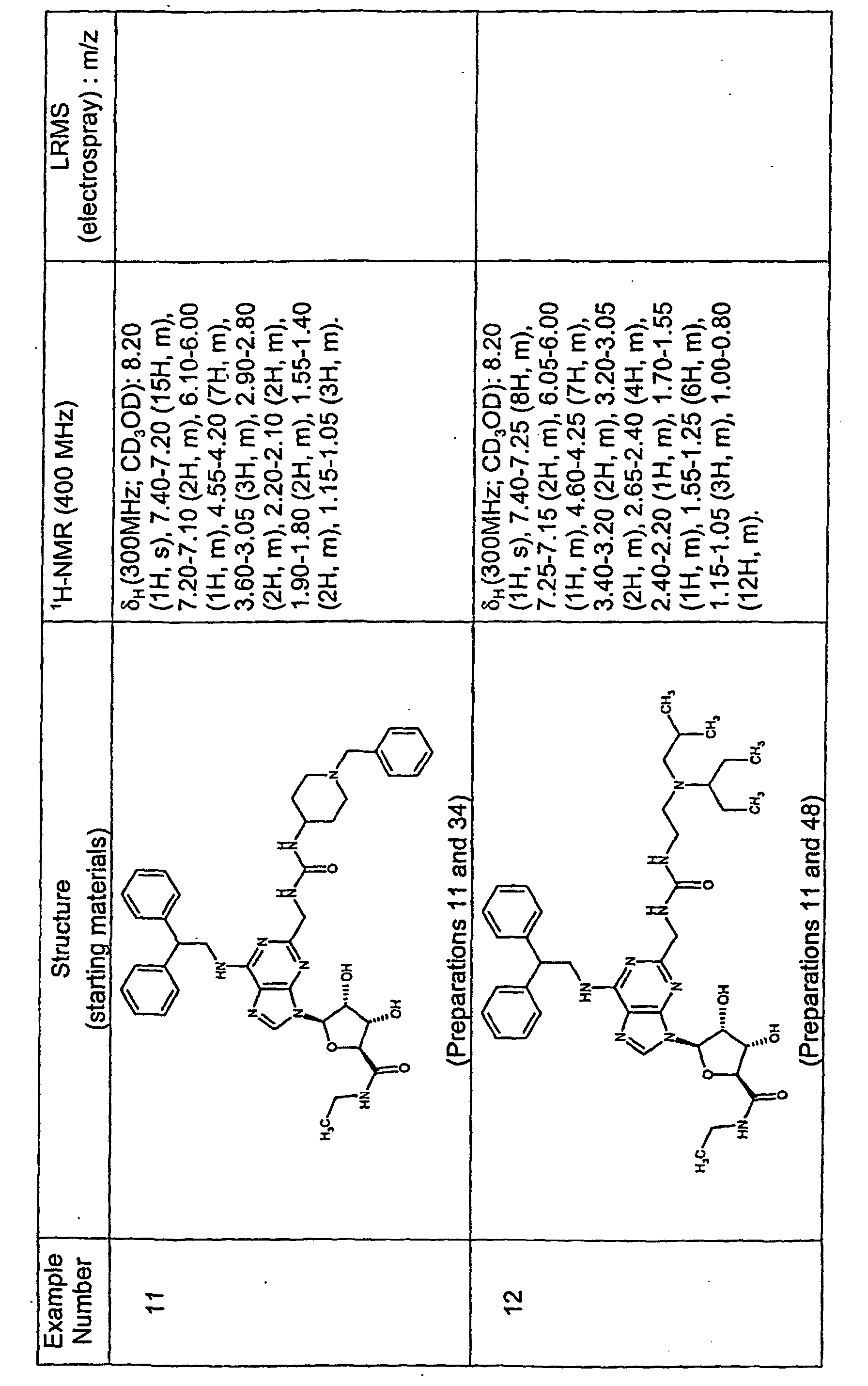

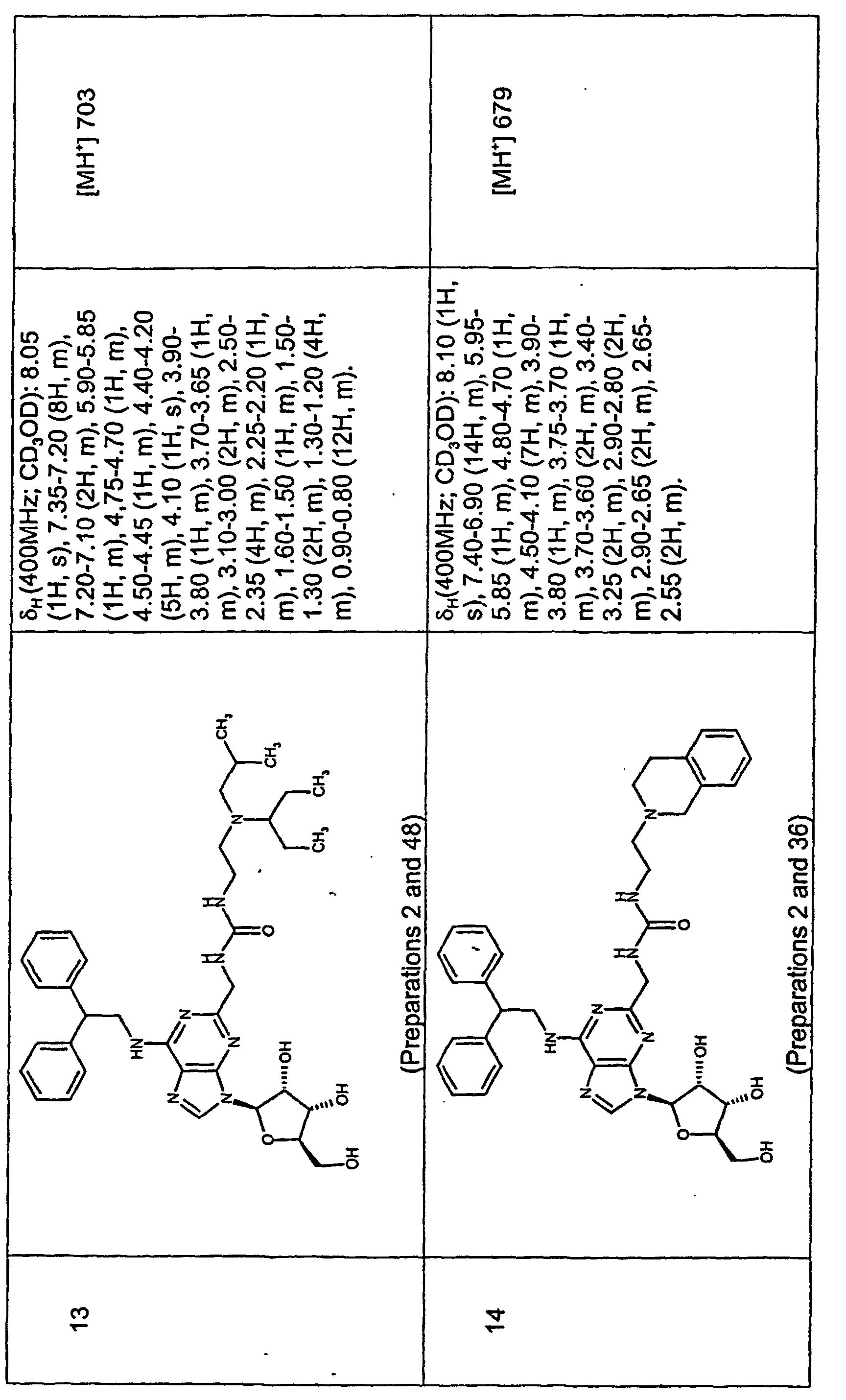

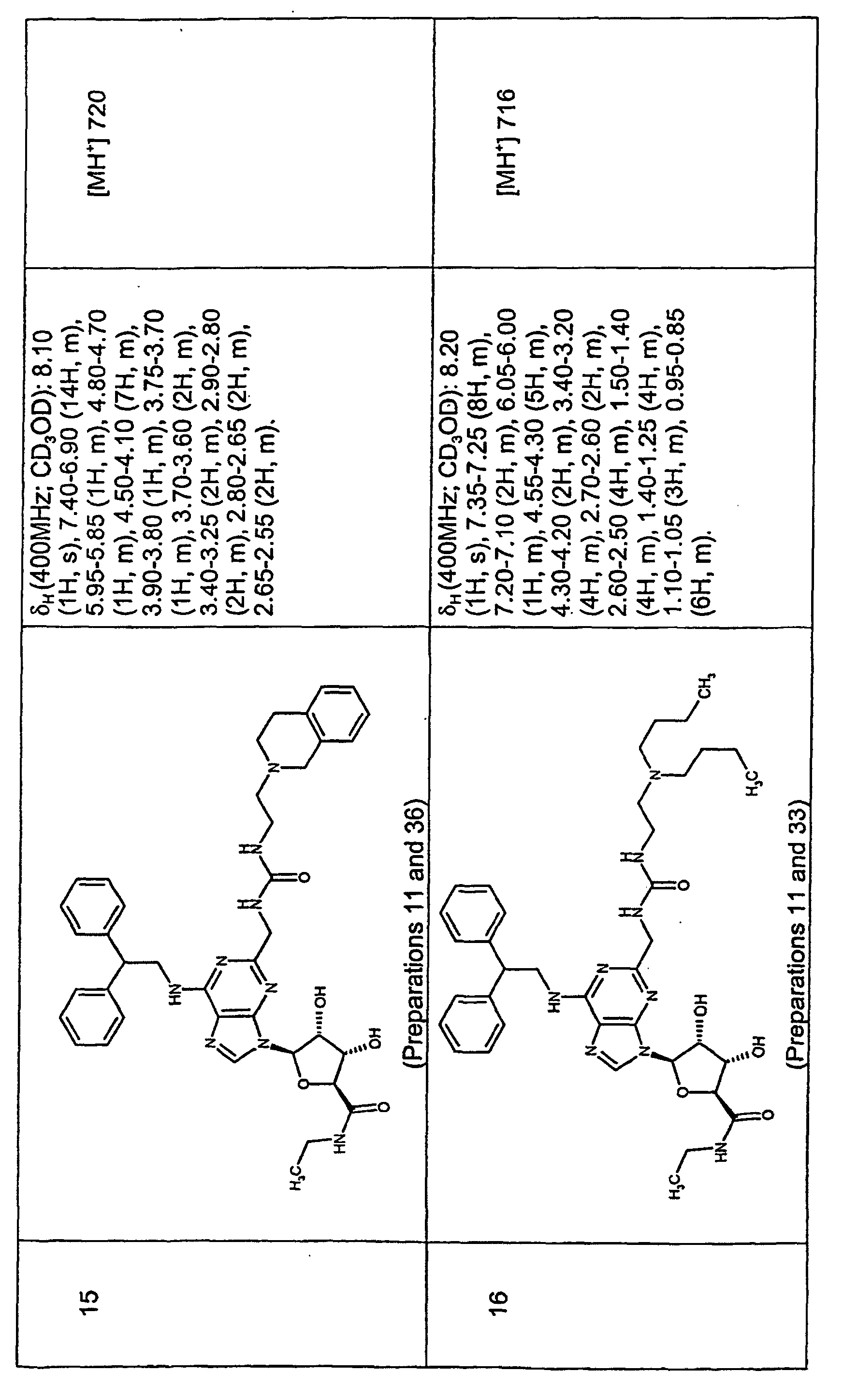

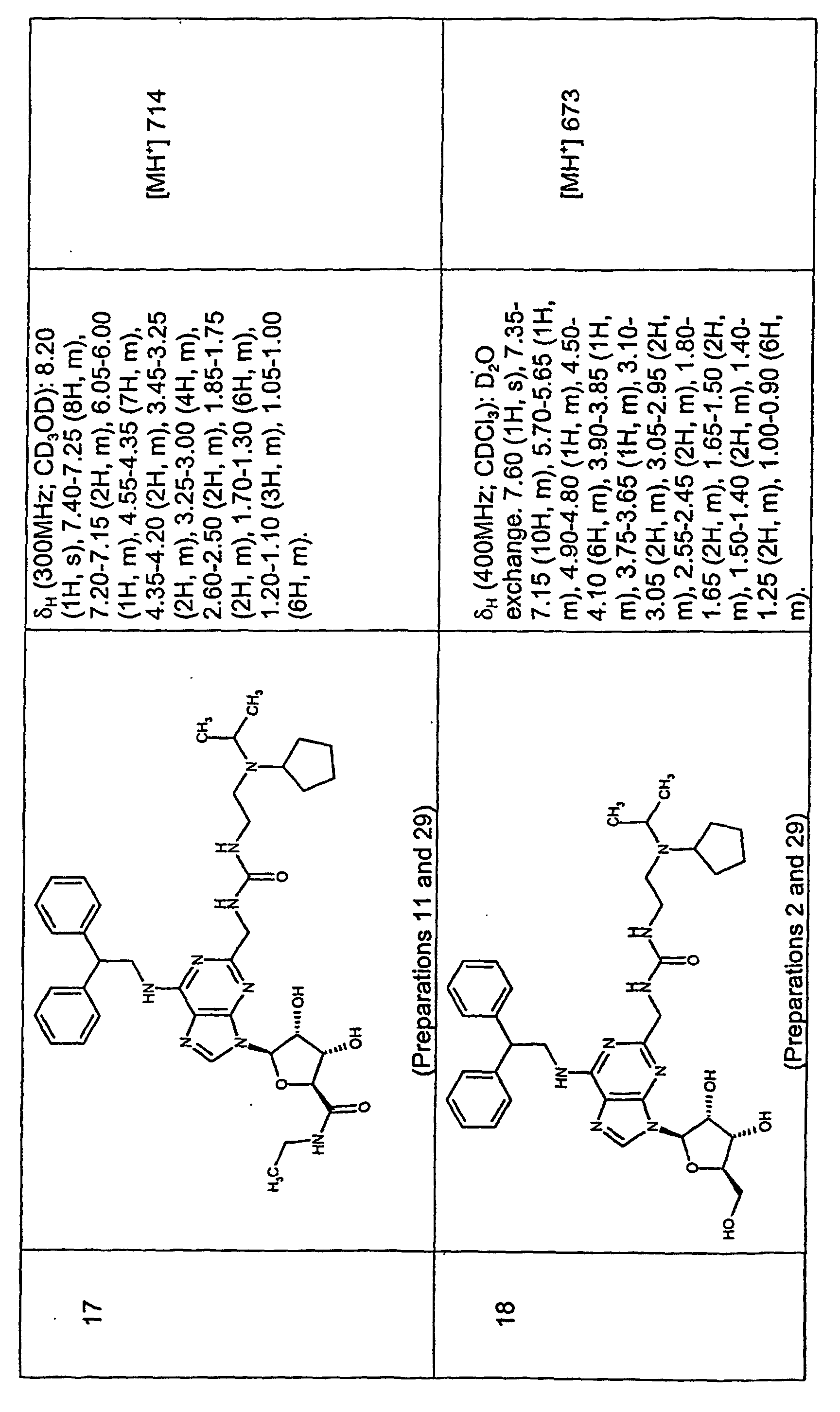

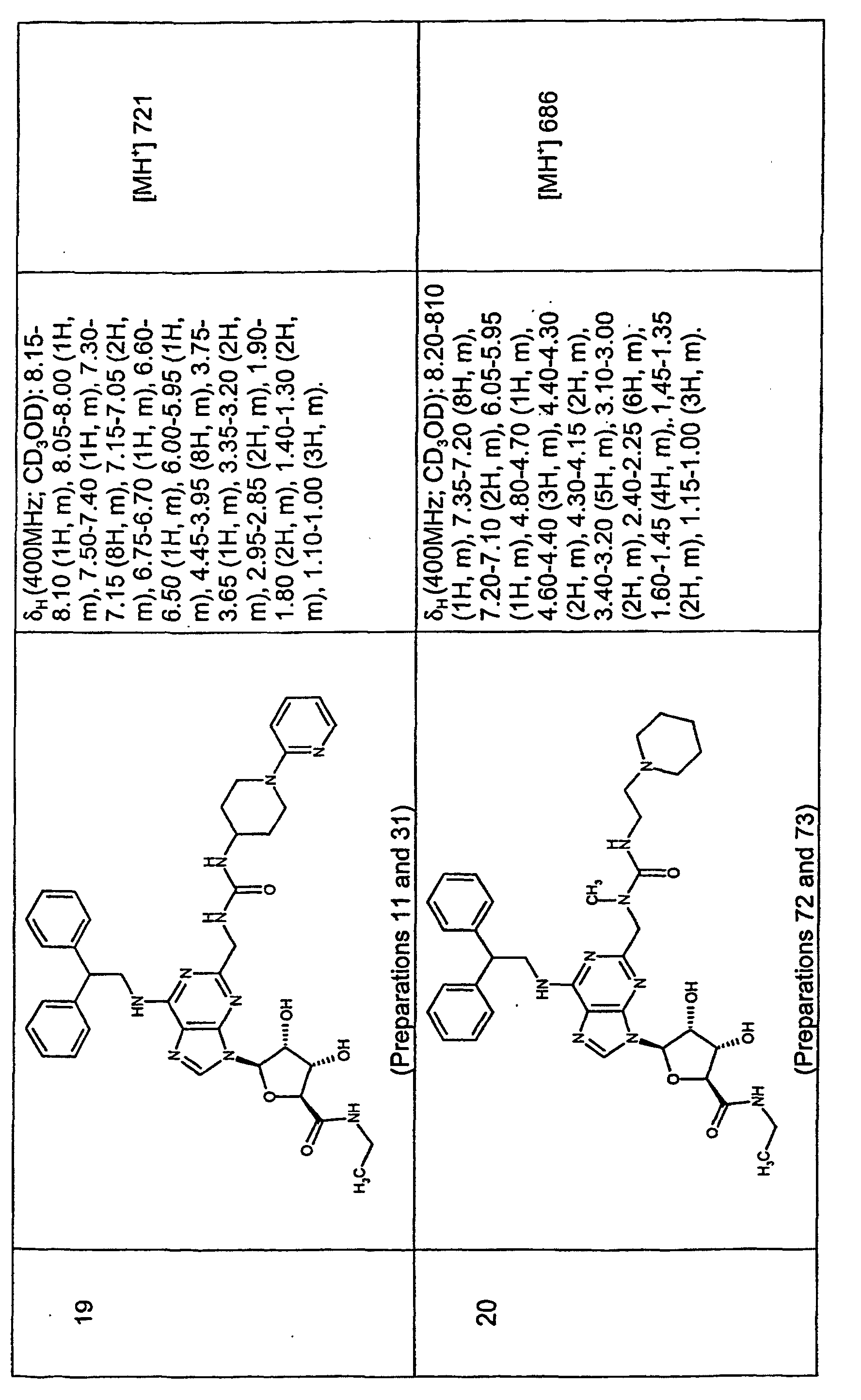

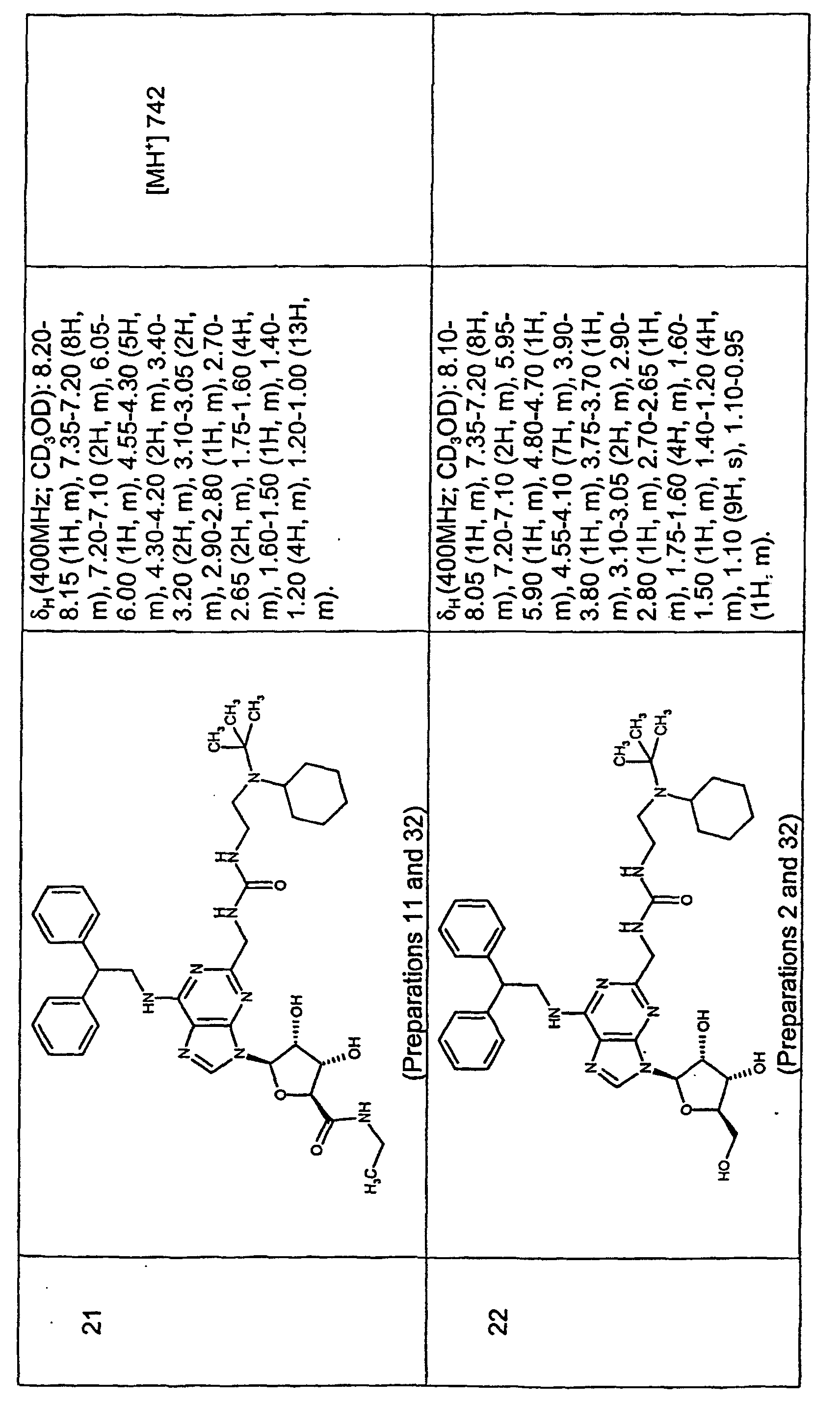

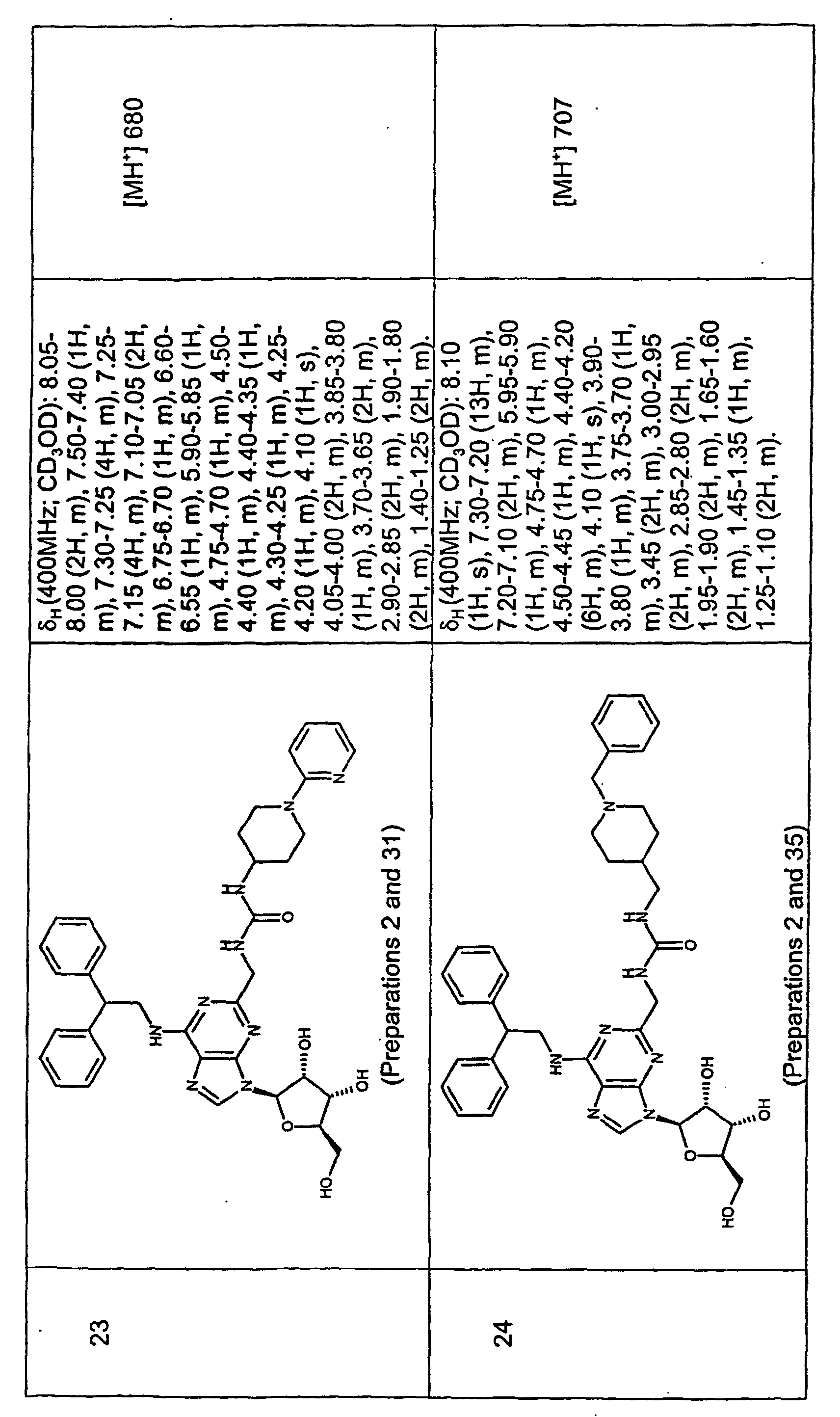

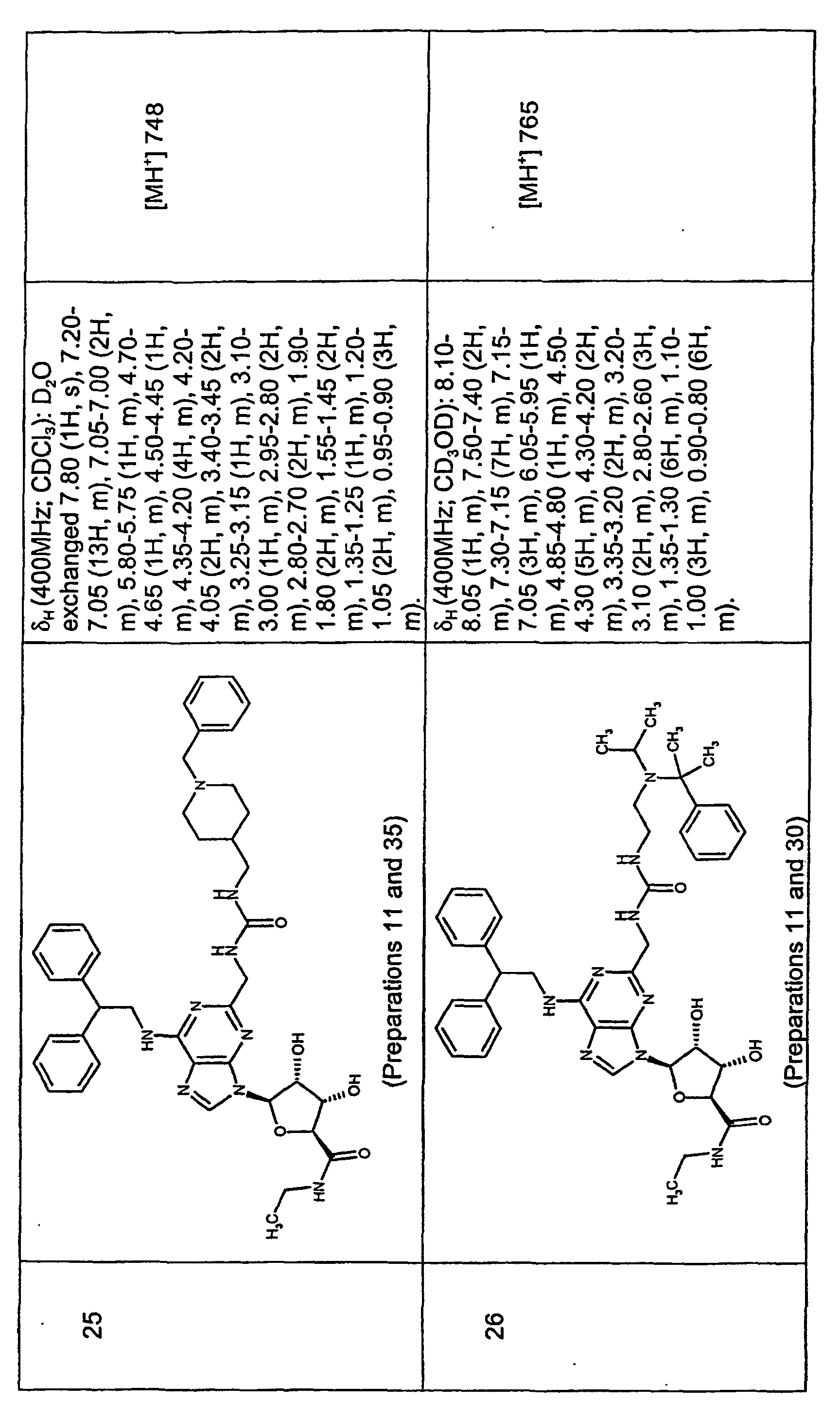

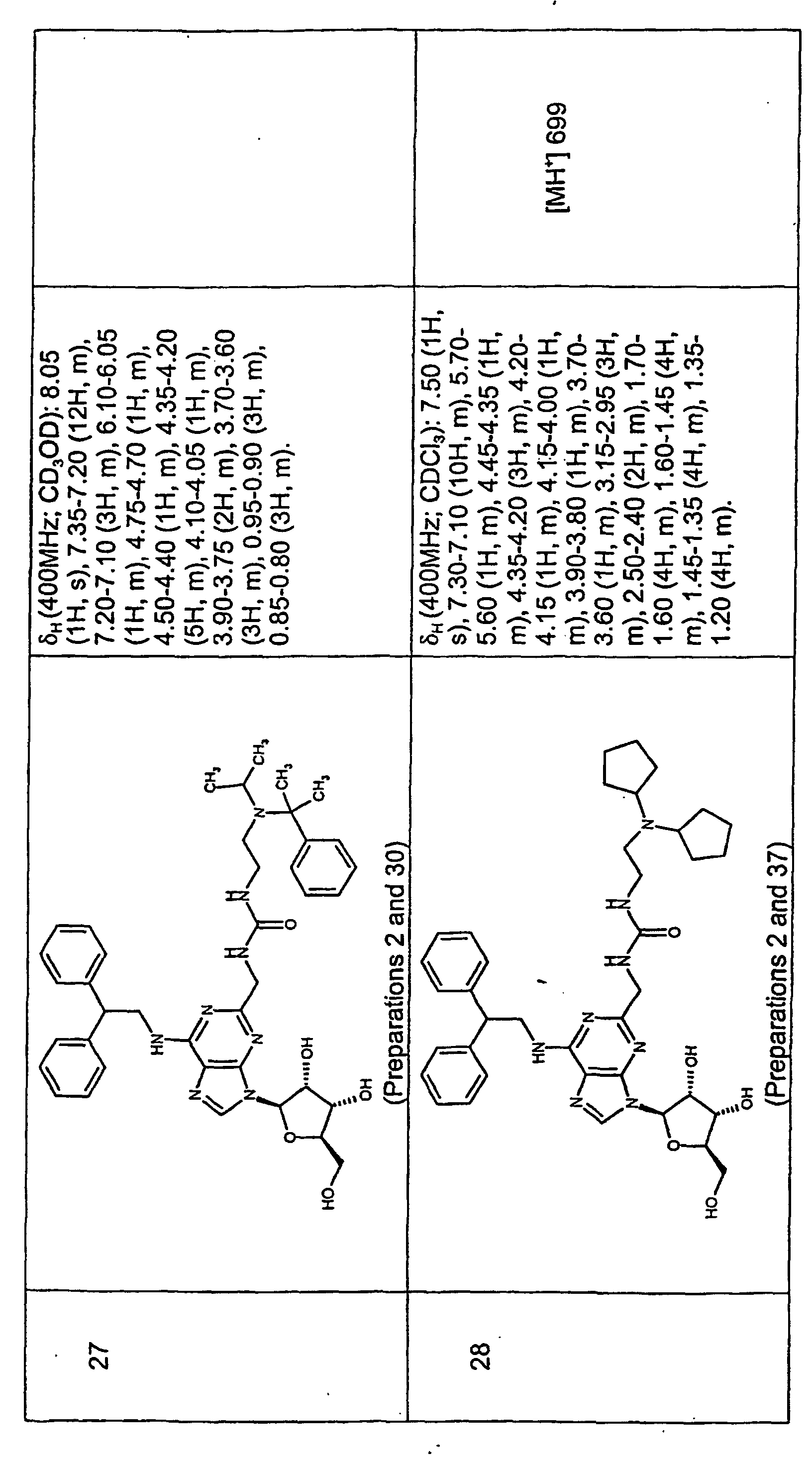

### EXAMPLE 29

### N-({9-[(2R,3R,4S,5R)-3,4-Dihydroxy-5-(hydroxymethyl)tetrahydro-2-furanyl]-6-[(9H-fluoren-9-ylmethyl)amino]-9H-purin-2-yl}methyl)-N'-[2-(diisopropylamino)ethyflurea

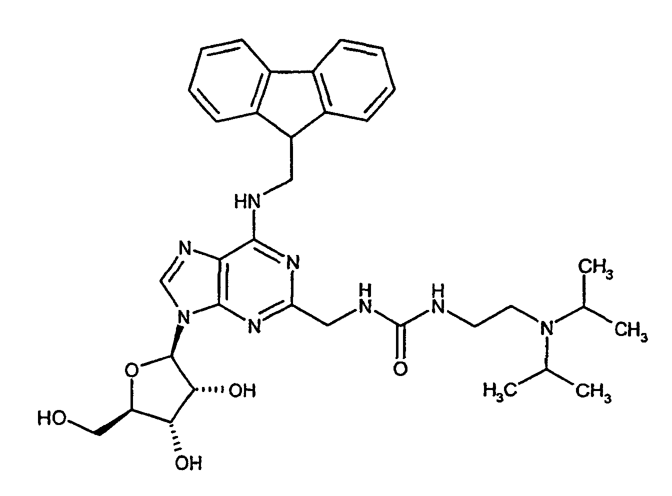

The title compound was prepared from (2*R*,3*R*,4*S*,5*R*)-2-{2-(aminomethyl)-6-[(9*H*-fluoren-9-ylmethyl)amino]-9*H*-purin-9-yl}-5-(hydroxymethyl)tetrahydro-3,4-furandiol (Preparation 13) and *N*-[2-(diisopropylamino)ethyl]-1*H*-imidazole-1-carboxamide (Preparation 27) in a similar procedure to Example 3.
m/z MH⁺ 645.
δ_{H} (400MHz; CD₃OD): 8.20 (1H, m), 7.85-7.75 (2H, m), 7.70-7.60 (2H, m), 7.40-7.20 (4H, m), 6.00-5.90 (1H, m), 4.40-4.35 (1H, m), 4.35-4.30 (1H, m), 4.20-4.00 (3H, m), 3.90-3.70 (4H, m).

### EXAMPLE 30

### N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(2,2,6,6-tetramethyl-1-piperidinyl)ethyl]urea

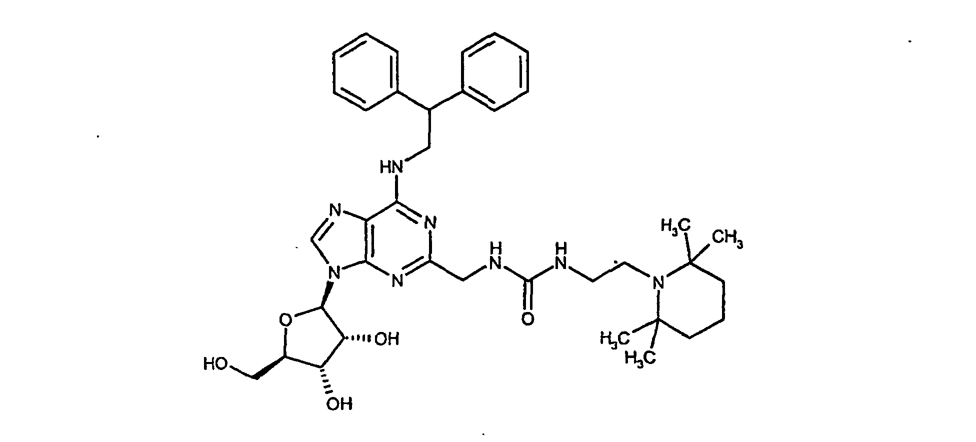

2-(2,2,6,6-Tetramethyl-1-piperidinyl)ethanamine (0.11g, 0.6mmol) (Preparation 43) was added to a stirred solution of N'N'-carbonyldiimidazole in tetrahydrofuran (5ml) at room temperature. The reaction mixture was stirred for 16 hours at room temperature. The solvent was evaporated and the residue was dissolved in ethyl acetate (30ml). The resulting solution was washed with water (20ml) and dried over anhydrous sodium sulphate. The solvent was evaporated to give impure *N*-[2-(2,2,6,6-tetramethyl-1-piperidinyl)ethyl]-1*H*-imidazole-1-carboxamide as a gum. This crude material (83mg, 0.3mmol) was added to a stirred solution of (2*R*,3*R*,4*S*,5*R*)-2-{2-(aminomethyl)-6-[(2,2-diphenylethyl)amino]-9*H*-purin-9-yl}-5-(hydroxymethyl)tetrahydro-3,4-furandiol (100mg, 0.21mmol) (Preparation 2) in toluene (5ml) and isopropanol (1ml) at room temperature. The reaction mixture was heated under reflux for 3 hours and then allowed to cool to room temperature. The solvent was evaporated under reduced pressure to give a residue that was purified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume). The solvent was evaporated under reduced pressure to give a residue that was found to still be impure. The residue was repurified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume). The solvent was removed under reduced pressure to give the title compound (22mg) as a gum.
m/z MH⁺ 686.
δ_{H} (400MHz; CD₃OD): 8.10 (1H, s), 7.35-7.20 (8H, m), 7.20-7.10 (2H, m), 5.95-5.90 (1H, m), 4.75-4.70 (1H, m), 4.55-4.45 (1H, m), 4.45-4.20 (5H, m), 4.20-4.10 (1H, m), 3.90-3.85 (1H, m), 3.75-3.70 (1H, m), 3.15-3.05 (2H, m), 2.60-2.55 (2H, m), 1.60-1.50 (2H, m), 1.45-1.40 (4H, m), 1.05 (12H, s).

### EXAMPLE 31

### (2S,3S,4R,5R)-5-(6-[(2,2-Diphenylethyl)amino]-2-{[({[2-(4-isopropyl-1-piperidinyl)ethyl]amino}carbonyl)amino]methyl}-9H-purin-9-yl)-N-ethyl-3,4-dihydroxytetrahydro-2-furancarboxamide

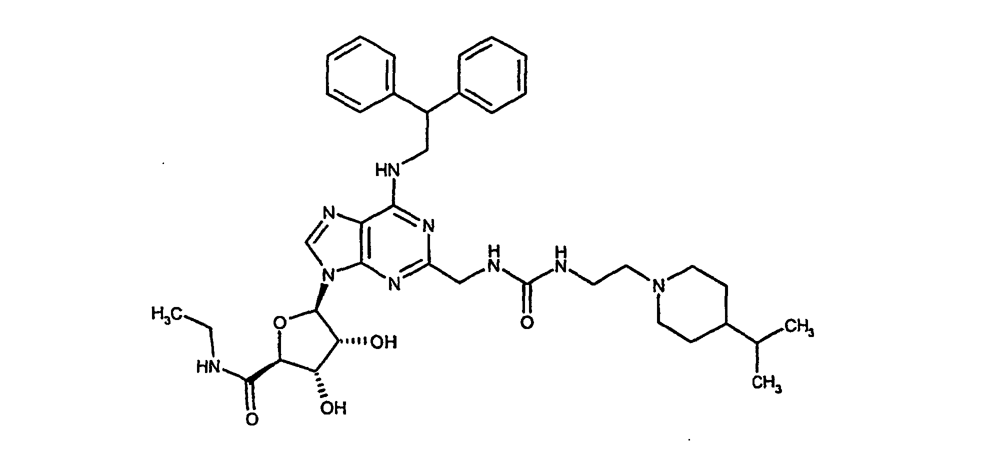

2-(4-Isopropyl-1-piperidinyl)ethylamine (0.3g, 1.76mmol) (Preparation 57) was added to a stirred solution of N'N'-carbonyldiimidazole (0.295g, 1.82mmol) in tetrahydrofuran (15ml). The reaction mixture was stirred for two hours. The solvent was removed under reduced pressure and the residue was partitioned between ethyl acetate (40ml) and water (20ml). The organic layer was dried over anhydrous sodium sulphate and the solvent was removed to give impure *N*-[2-(4-isopropyl-1-piperidinyl)ethyl]-1*H*-imidazole-1-carboxamide (Preparation 28). This crude material was added to a solution of (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (130mg, 0.25mmol) (Preparation 11) in toluene (3ml) and isopropyl alcohol (1ml) at room temperature. The reaction mixture was heated under reflux for 3 hours. The solvent was removed under reduced pressure and the residue was purified by column chromatography eluting with dichloromethane : methanol : 0.88 concentrated aqueous ammonia (95 : 5 : 0.5 by volume) increasing in polarity to dichloromethane : methanol : 0.88 concentrated aqueous ammonia (80 : 20 : 3 by volume). The solvent was removed under reduced pressure to give a residue that was impure. The material was triturated with diethylether (5ml) three times. The insoluble solid was repurified by column chromatography eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume). The solvent was removed under reduced pressure to give the title compound (37mg) as a foam.
m/z MH⁺ 714.
δ_{H} (400MHz; CD₃OD): 8.20 (1H, s), 7.40-7.20 (8H, m), 7.20-7.10 (2H, m), 6.05-6.00 (1H, m), 4.55-4.20 (7H, m), 3.40-3.20 (4H, m), 3.05-2.85 (2H, m), 2.50-2.35 (2H, m), 2.00-1.85 (2H, m), 1.70-1.60 (2H, m), 1.45-1.20 (3H, m), 1.10-0.95 (4H, m), 0.95-0.80 (6H, m).

### EXAMPLE 32

### (2S,3S,4R,5R)-5-(6-[(2,2-Diphenylethyl)amino]-2-{[({[2-(2,2,6,6-tetramethyl-1-piperidinyl)ethyl]amino}carbonyl)amino]methyl}-9H-purin-9-yl)-N-ethyl-3,4-dihydroxytetrahydro-2-furancarboxamide

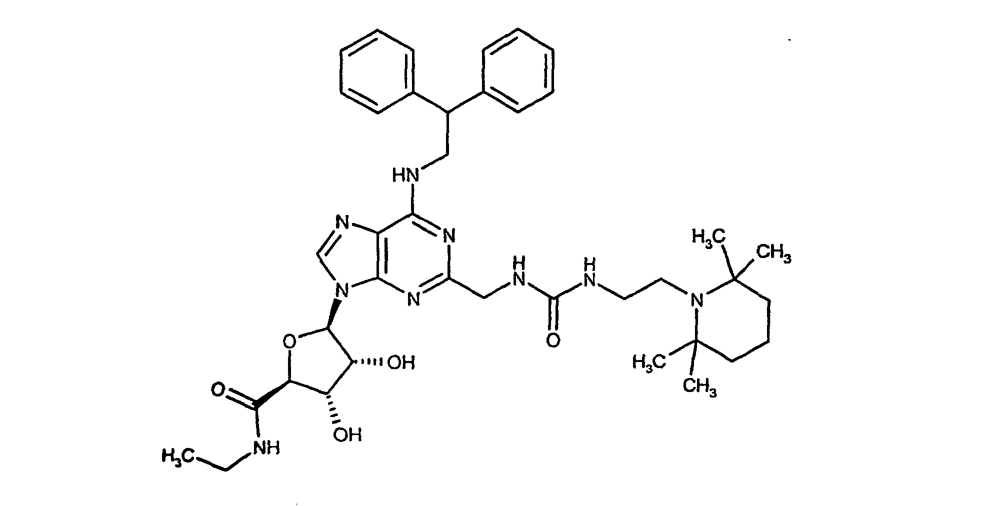

The title compound was prepared by a similar procedure to that used in Example 30 using (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (Preparation 11) and 2-(2,2,6,6-tetramethyl-1-piperidinyl)ethanamine (Preparation 43).
m/z MH⁺ 728.
δ_{H} (400MHz; CD₃OD): 8.20 (1H, s), 7.15-7.10 (4H, m), 7.10-7.05 (4H, m), 7.20-7.15 (2H, m), 6.05-6.00 (1H, m), 4.55-4.40 (5H, m), 4.35-4.20 (2H, m), 3.40-3.20 (2H, m), 3.15-3.05 (2H, m), 2.65-2.55 (2H, m), 1.60-1.50 (2H, m), 1.45-1.40 (4H, m), 1.15-1.05 (3H, m), 1.05-1.00 (12H, m).

### EXAMPLE 33

### N-[(3R)-1-Benzylpyrrolidinyl]-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)urea

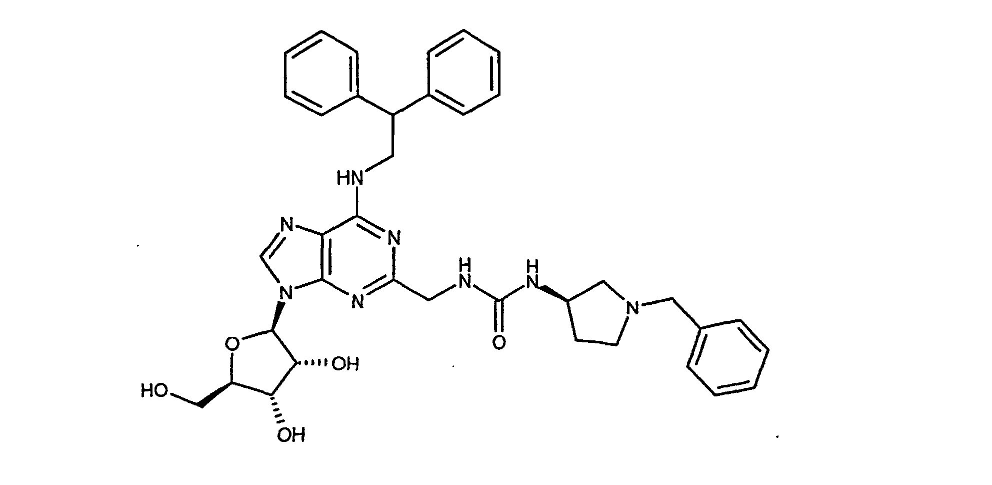

(3*R*)-1-Benzyl-3-pyrrolidinamine (0.64ml, 3.4mmol) was added to a stirred solution of N'N'-carbonyldiimidazole (0.6g, 3.7 mmol) in dichloromethane (150ml) at room temperature. The reaction mixture was stirred at room temperature for 1 hour. Dichloromethane (100ml) was added and the solution then washed three times with water (50ml). The organic phase was washed twice with saturated aqueous sodium chloride solution and then dried over anhydrous magnesium sulphate. The solvent was removed under reduced pressure to give *N*-[(3*R*)-1-benzylpyrrolidinyl]-1*H*-imidazole-1-carboxamide (1.23g) as an impure oil. This material (115mg, 0.43mmol) was added to a stirred solution of (2*R*,3*R*,4*S*,5*R*)-2-{2-(aminomethyl)-6-[(2,2-diphenylethyl)amino]-9*H*-purin-9-yl}-5-(hydroxymethyl)tetrahydro-3,4-furandiol (Preparation 2) (100mg, 0.21 mmol) in toluene (10ml) and isopropanol (2.5ml) at room temperature. The reaction mixture was heated under reflux for 40 minutes and then allowed to cool to room temperature. The solvent was removed under reduced pressure and the residue was purified by column chromatography eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume). The solvent was removed under reduced pressure to give the title compound (138mg).
δ_{H} (400MHz; CD₃OD): 8.10 (1H, s), 7.30-7.20 (13H, m), 7.20-7.10 (2H, m), 5.90-5.85 (1H, m), 4.80-4.75 (1H, m), 4.50-4.45 (1H, m), 4.45-4.10 (7H, m), 3.90-3.85 (1H, m), 3.75-3.70 (1H, m), 3.60-3.50 (2H, m), 2.75-2.65 (2H, m), 2.50-2.40 (2H, m), 2.20-2.10 (1H, m), 1.60-1.50 (1H, m).

### EXAMPLE 34

### (2S,3S,4R,5R)-5-{2-{[({[(3R)-Benzylpyrolidinyl]amino}carbonyl)amino]methyl}-6-[(2,2-diphenylethyl)amino]-9H-purin-9-yl}-N-ethyl-3,4-dihydroxytetrahydro-2-furancarboxamide

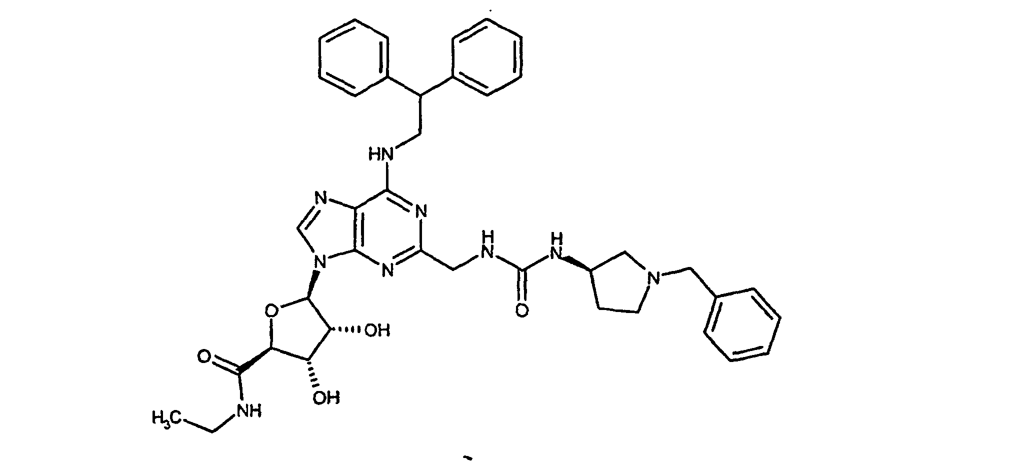

The title compound was prepared by a similar method to that of Example 33 using (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (Preparation 11) and (3*R*)-1-benzyl-3-pyrrolidinamine.
δ_{H} (400MHz; CD₃OD): 8.15 (1H, s), 7.35-7.20 (13H, m), 7.20-7.10 (2H, m), 6.05-6.00 (1H, m), 4.85-4.80 (1H, m), 4.55-4.10 (7H, m), 3.60-3.50 (2H, m), 3.40-3.20 (2H, m), 2.80-2.60 (2H, m), 2.50-2.35 (2H, m), 2.20-2.10 (1H, m), 1.65-1.45 (1H, m), 1.15-1.00 (3H, m).

### EXAMPLE 35

### (2S,3S,4R,5R)-5-(6-{[2,2-Bis(4-chlorophenyl)ethyl]amino}-2-{[({[2-(diisopropylamino)ethyl]amino}carbonyl)amino]methyl}-9H-purin-9-yl)-N-ethyl-3,4-dihydroxytetrahydro-2-furancarboxamide

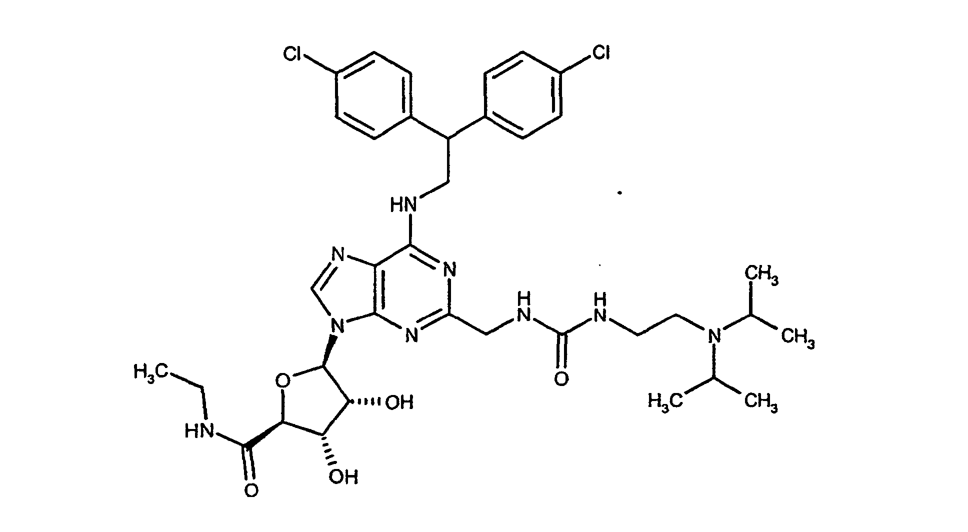

(2*R*,3*R*,4*S*,5*S*)-4-(Benzoyloxy)-2-(6-{[2,2-bis(4-chlorophenyl)ethyl]amino}-2-cyano-9*H*-purin-9-yl)-5-[(ethylamino)carbonyl]tetrahydro-3-furanyl benzoate (220mg, 0.28mmol) (Preparation 68) was added to a saturated solution of ammonia in ethanol (15ml). 10% Palladium on carbon (40mg) was added and the suspension was stirred under an atmosphere of hydrogen gas (413.7 kPa, 60psi) at room temperature for 16 hours. The reaction temperature was then stirred at 60°C under an atmosphere of hydrogen gas (413.7 kPa, 60psi) for a further 48 hours. The reaction mixture was filtered through Arbocel (Trade Mark) and the solvent was removed under reduced pressure. The crude residue was dissolved in toluene (8ml) and isopropyl alcohol (2ml) and *N*-[2-(diisopropylamino)ethyl]-1*H*-imidazole-1-carboxamide (50mg, 0.21mmol) (Preparation 27) were added. The solution was heated under reflux for 3 hours. The solvent was removed under reduced pressure and the residue was purified by column chromatography on silica gel eluting with dichloromethane : methanol : 0.88 concentrated aqueous ammonia (90 : 10 : 2 by volume). The solvent was removed under reduced pressure give a product that was contaminated with imidazole. The product was dissolved in dichloromethane (60ml) and the resulting solution was washed three times with water (10ml) and dried over anhydrous magnesium sulphate. The solvent was removed under reduced pressure to give the title compound (53mg) as a foam.
m/z MH⁺ 756.
δ_{H}(400MHz; CD₃OD): 8.20 (1H, s), 7.40-7.20 (8H, m), 6.05-6.00 (1H, m), 4.55-4.20 (7H, m), 3.40-3.25 (2H, m), 3.20-3.05 (4H, m), 1.15-1.00 (15H, m).

### EXAMPLE 36

### N-({6-{[2,2-Bis(4-chlorophenyl)ethyl]amino}-9-[(2R,3R,4S,5R)-3,4-dihydroxy-5-(hydroxymethyl)tetrahydro-2-furanyl]-9H-purin-2-yl}methyl)-N'-[2-(diisopropylamino)ethyl]urea

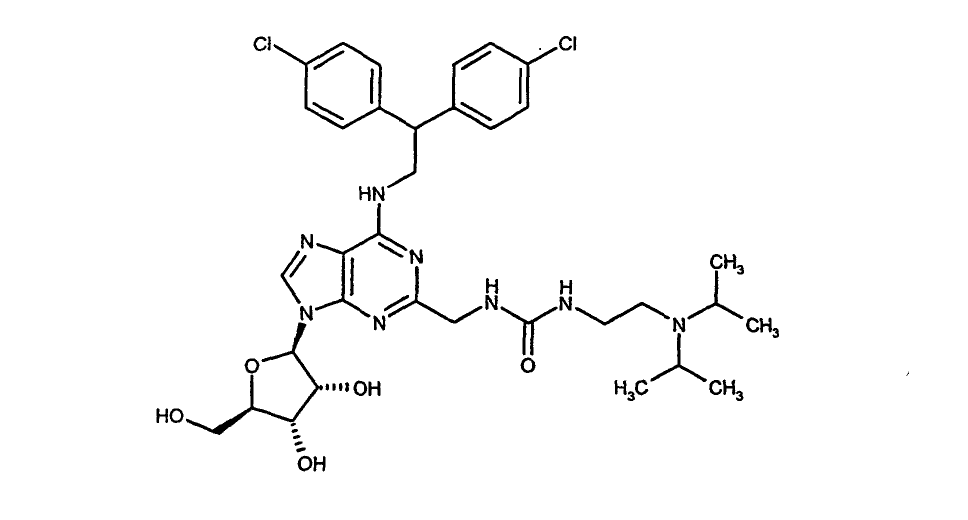

(2*R*,3*R*,4*S*,5*R*)-2-(2-(Aminomethyl)-6-{[2,2-bis(4-chlorophenyl)ethyl]amino}-9*H*-purin-9-yl)-5-(hydroxymethyl)tetrahydro-3,4-turandiol (0.1 g, 0.18mmol) (Preparation 63) was dissolved in a mixture of isopropanol (0.5ml) and Genklene (Trade Mark) (5ml). *N*-[2-(Diisopropylamino)ethyl]-1*H*-imidazole-1-carboxamide (48mg, 0.20mmol) (Preparation 27) was added and the reaction mixture was heated under reflux for 2 hours. More *N*-[2-(diisopropylamino)ethyl]-1*H*-imidazole-1-carboxamide (35mg, 0.15mmol) (Preparation 27) was then added and the reaction mixture was heated under reflux for a further 2 hours. The reaction mixture was then allowed to cool and the solvent was removed under reduced pressure. The residue was purified by flash chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 2 by volume) to give the title compound (0.09g) as a gum.
m/z MH⁺ 715.
δ_{H} (400MHz; CD₃OD): 8.10 (1H, s), 7.35-7.20 (8H, m), 7.20-7.15 (1H, m), 5.90-5.85 (1H, m), 4.75-4.70 (1H, m), 4.55-4.45 (1H, m), 4.40-4.35 (2H, m), 4.35-4.15 (3H, m), 4.15-4.10 (1H, m), 3.90-3.80 (1H, m), 3.70-3.65 (1H, m), 3.25-3.10 (2H, m), 1.20-1.00 (12H, m).

### EXAMPLE 37

### N-({6-{[2,2-Bis(4-methylphenyl)ethyl]amino}-9-[(2R,3R,4S,5R)-3,4-dihydroxy-5-(hydroxymethyl)tetrahydro-2-furanyl]-9H-purin-2-yl}methyl)-N'-[2-(diisopropylamino)ethyl]urea

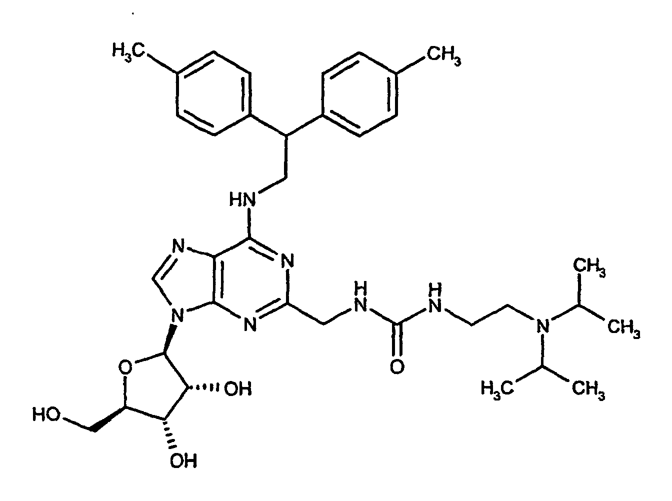

The title compound was prepared from (2*R*,3*R*,4*S*,5*R*)-2-(2-(aminomethyl)-6-{[2,2-bis(4-methylphenyl)ethyl]amino}-9*H*-purin-9-yl)-5-(hydroxymethyl)tetrahydro-3,4-furandiol (Preparation 60) and *N*-[2-(diisopropylamino)ethyl]-1*H*-imidazole-1-carboxamide (Preparation 27) using a similar procedure to that used in Example 36.
m/z MH⁺ 673.
δ_{H} (400MHz; CD₃OD): 8.05 (1H, s), 7.20-7.15 (4H, m), 7.05-7.00 (4H, m), 5.90-5.85 (1H, m), 4.75-4.70 (1H, m), 4.40-4.30 (3H, m), 4.30-4.25 (1H, m), 4.15-4.10 (1H, m), 3.90-3.80 (1H, m), 3.75-3.70 (1H, m), 3.10-3.05 (2H, m), 3.05-2.90 (2H, m), 2.50-2.45 (2H, m), 2.05 (6H, m), 1.00-0.95 (12H, m).

### EXAMPLE 38

### N-({6-{[2,2-Bis(3-chlorophenyl)ethyl]amino}-9-[(2R,3R,4S,5R)-3,4-dihydroxy-5-(hydroxymethyl)tetrahydro-2-furanyl]-9H-purin-2-yl}methyl)-N'-[2-(diisopropylamino)ethyl]urea

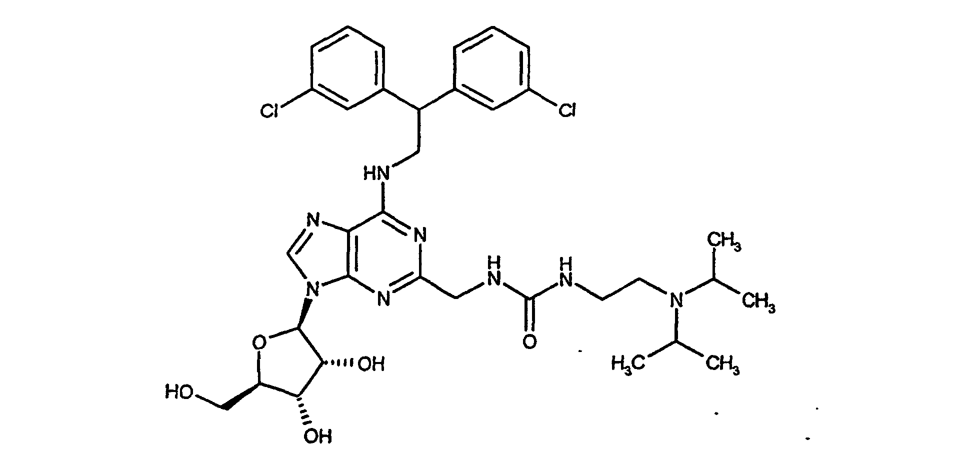

The title compound was prepared from (2*R*,3*R*,4*S*,5*R*)-2-(2-(aminomethyl)-6-{[2,2-bis(3-chlorophenyl)ethyl]amino}-9*H*-purin-9-yl)-5-(hydroxymethyl)tetrahydro-3,4-furandiol (Preparation 62) and *N*-[2-(diisopropylamino)ethyl]-1*H*-imidazole-1-carboxamide (Preparation 27) in a similar procedure to that used in Example 36.
m/z MH⁺ 715.
δ_{H} (400MHz; CD₃OD): 8.10-8.05 (1H, m), 7.35-7.10 (8H, m), 5.90-5.85 (1H, m), 4.75-4.70 (1H, m), 4.60-4.45 (1H, m), 4.40-4.15 (5H, m), 4.10-4.05 (1H, m), 3.85-3.80 (1H, m), 3.70-3.65 (1H, m), 3.15-3.00 (4H, m), 2.65-2.50 (2H, m), 1.05-0.90 (12H, m).

### EXAMPLE 39

### N-({6-{[2,2-Bis(3-methylphenyl)ethyl]amino}-9-[(2R,3R,4S,5R)-3,4-dihydroxy-5-(hydroxymethyl)tetrahydro-2-furanyl]-9H-purin-2-yl}methyl)-N'-[2-(diisopropylamino)ethyl]urea

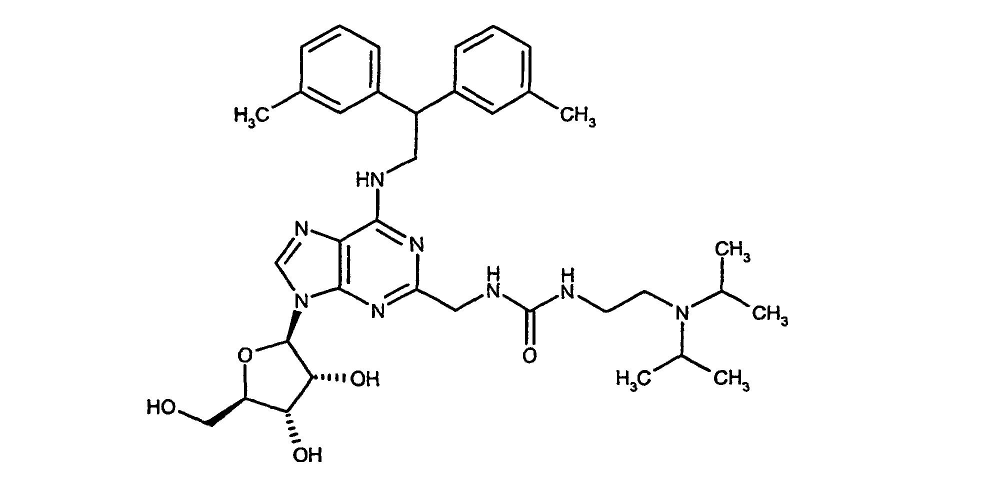

The title compound was prepared from (2*R*,3*R*,4*S*,5*R*)-2-(2-(aminomethyl)-6-{[2,2-bis(3-methylphenyl)ethyl]amino}-9*H*-purin-9-yl)-5-(hydroxymethyl)tetrahydro-3,4-furandiol (Preparation 65) and *N*-[2-(diisopropylamino)ethyl]-1*H*-imidazole-1-carboxamide (Preparation 27) using a similar procedure to that used in Example 36.
m/z MH⁺ 673.
δ_{H} (400MHz; CD₃OD): 8.10 (1H, s), 7.20-7.05 (6H, m), 5.90-5.85 (1H, m), 4.75-4.70 (1H, m), 4.40-4.25 (5H, m), 4.25-4.15 (2H, m), 4.15-4.10 (1H, m), 3.90-3.80 (1H, m), 3.70-3.65 (1H, m), 3.15-3.00 (4H, m), 2.60-2.45 (2H, m), 2.05 (6H, s), 1.10-0.95 (12H, m).

### EXAMPLE 40

### (2S,3S,4R,5R)-5-{2-{[({[2-(Diisopropylamino)ethyl]amino}carbonyl)amino]methyl}-6-[(1-naphthylmethyl)amino]-9H-purin-9-yl}-N-ethyl-3,4-dihydroxytetrahydro-2-furancarboxamide

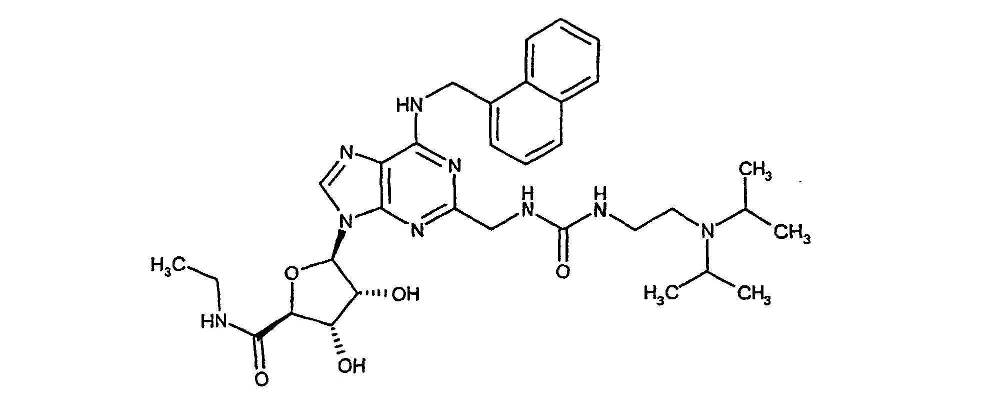

The title compound was prepared from (2*S*,3*S*,4*R*,5*R*)-5-{2-(aminomethyl)-6-[(1-naphthylmethyl)amino]-9*H*-purin-9-yl}-*N*-ethyl-3,4-dihydroxytetrahydro-2-furancarboxamide (Preparation 69) and *N*-[2-(diisopropylamino)ethyl]-1*H*-imidazole-1-carboxamide (Preparation 27) by similar procedure to that used in Example 3.
m/z MH⁺ 648.
δ_{H}(400MHz; CD₃OD): 8.20 (1H, s), 8.15-8.10 (1H, m), 7.90-7.85 (1H, m), 7.80-7.75 (1H, m), 7.55-7.50 (1H, m), 7.50-7.45 (1H, m), 7.45-7.40 (1H, m), 6.05-6.00 (1H, m), 5.35-5.25 (2H, m), 4.45-4.35 (4H, m), 3.35-3.20 (2H, m), 3.10-3.00 (2H, m), 3.00-2.90 (2H, m), 2.50-2.40 (2H, m), 1.10-1.05 (3H, m).

The following Preparations describe the preparation of certain intermediates used in the preceding Examples.

### PREPARATION 1

### (2R,3R,4R,5R)-4-(Acetyloxy)-2-[(acetyloxy)methyl]-5-{2-cyano-6-[(2,2-diphenylethyl)amino]-9H-purin-9-yl}tetrahydro-3-furanyl acetate

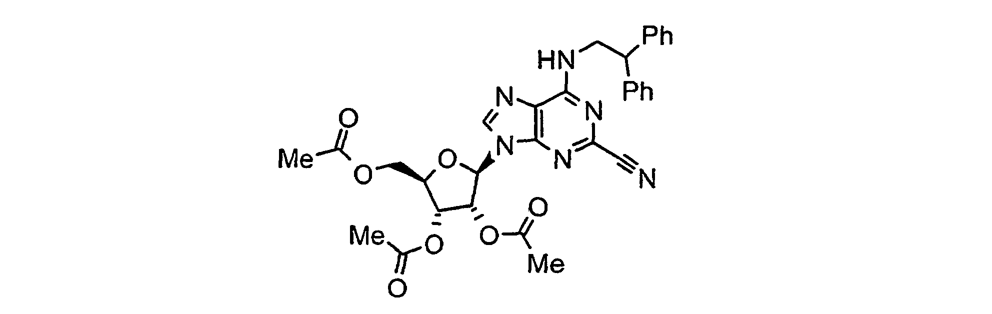

*N,O*-Bistrimethylsilylacetamide (44ml, 0.18mol) was added to a suspension of 6-[(2,2-diphenylethyl)amino]-9*H*-purine-2-carbonitrile (10.0g, 0.03mol) (Preparation 24) in 1,1,1-trichloroethane (250ml). The suspension was heated under reflux. When all suspended solid had dissolved the reaction mixture was allowed to cool to room temperature and the solvent was removed under reduced pressure. The residue was twice dissolved in toluene (50ml) and the solvent was removed under reduced pressure. The residue was then dissolved in toluene (100ml) and (2*R*,3*R*,4*R*,5*S*)-4,5-bis(acetyloxy)-2-[(acetyloxy)methyl]tetrahydro-3-furanyl acetate (10.3g, 0.032mol) was added. The solution was stirred at room temperature and trimethylsilyltrifluoromethanesulphonate (16ml, 0.088mol) was carefully added. The resulting solution was heated under reflux for 2 hours and then allowed to cool to room temperature. The reaction mixture was diluted by the addition of ethyl acetate (100ml) and then washed with saturated aqueous sodium hydrogen carbonate solution (ten portions of 100ml) and saturated aqueous sodium chloride solution (100ml). The aqueous extracts were combined and washed with ethyl acetate (three portions of 100ml). The combined organic layers were dried (anhydrous magnesium sulphate) and the solvent was removed under reduced pressure to give a solid that was purified by column chromatography on silica gel eluting with dichloromethane : methanol : 0.88 concentrated aqueous ammonia (97 : 3 : 0.5 by volume increasing to 80 : 20 : 3 by volume). This gave the title compound as a foam (8.5g).
δ_{H} (400MHz; CDCl₃): 7.95 (1H, s), 7.35-7.20 (10H, m), 6.15-6.10 (1H, m), 5.95-5.90 (1H, m), 5.80-5.75 (1H, m), 5.60-5.55 (1H, m), 4.45-4.35 (4H, m), 4.35-4.25 (2H, m), 2.15 (3H, s), 2.10 (3H, s), 2.05 (3H, s).

### PREPARATION 2

### (2R,3R,4S,5R)-2-{2-(Aminomethyl)-6-[(2,2-diphenylethyl)amino]-9H-purin-9-yl}-5-(hydroxymethyl)tetrahydro-3,4-furandiol

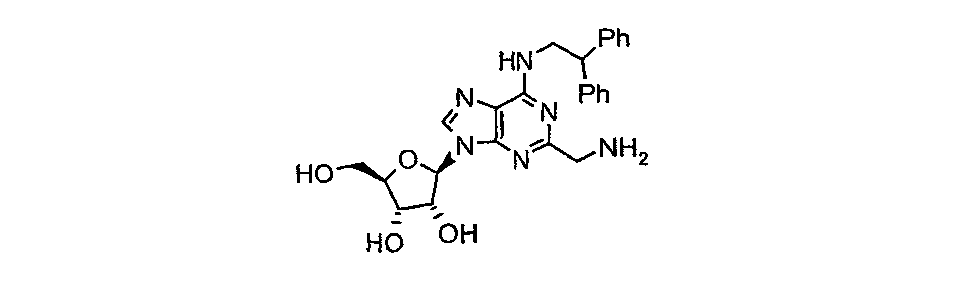

10% Palladium on carbon (200mg) was added to a solution of (2*R*,3*R*,4*R*,5*R*)-4-(acetyloxy)-2-[(acetyloxy)methyl]-5-{2-cyano-6-[(2,2-diphenylethyl)amino]-9*H*-purin-9-yl}tetrahydro-3-furanyl acetate (Preparation 1) (1.9g, 3.2mmol) in a saturated solution of ammonia in ethanol (100ml). The reaction mixture was stirred under an atmosphere of hydrogen (414kPa, 60psi) for 16 hours at room temperature. The solids were removed by filtration through Arbocel (Trade Mark) and the solvent was removed under reduced pressure. The residue was purified by column chromatography on silica gel eluting with dichloromethane : methanol : 0.88 concentrated aqueous ammonia (90 : 10 : 1 by volume increasing to 80 : 20 : 2 by volume). This gave the title compound as a solid (770mg).
m/z: MH⁺ 477.
δ_{H} (400MHz; CDCl₃): 8.10 (1H, s), 7.35-7.20 (8H, m), 5.90-5.85 (1H, m), 4.75-4.70 (1H, m), 4.50-4.40 (1H, m), 4.30-4.20 (2H, m), 4.10 (1H, m), 3.90-3.80 (2H, m), 3.70-3.65 (1H, m).

### PREPARATION 3

### 2-(Aminomethyl)-N-(2,2-diphenylethyl)-9-tetrahydro-2H-pyran-2-yl-9H-purin-6-amine

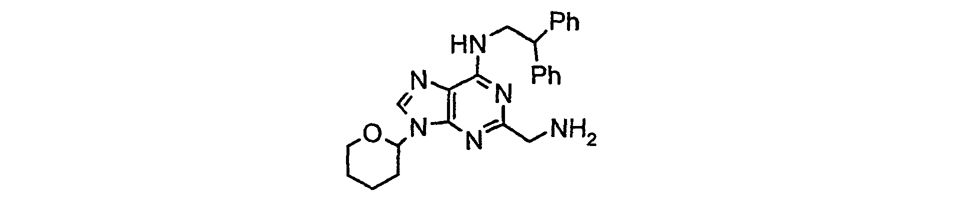

6-[(2,2-Diphenylethyl)amino]-9-(tetrahydro-2*H*-pyran-2-yl)-9*H*-purine-2-carbonitrile (19.7g, 0.046mol) (Preparation 23) was dissolved in a saturated solution of ammonia in ethanol (500ml). 10% Palladium on carbon (2g) was added and the suspension was stirred under an atmosphere of hydrogen (414kPa, 60psi) for 36 hours. The suspension was filtered through Arbocel (Trade Mark) and the solvent was removed under reduced pressure. This gave the title compound as a foam (17.7g).
δ_{H} (400MHz; CDCl₃): 7.84 (1H, s), 7.36-7.14 (10H, m), 5.70 (1H, d), 5.60 (1H, br s), 4.42-4.20 (3H, m), 4.14 (1H, d), 3.95 (2H, s), 3.78 (1H, t), 2.20-1.90 (5H, m), 1.88-1.50 (3H, m).

### PREPARATION 4

### N-({6-[(2,2-Diphenylethyl)amino]-9-tetrahydro-2H-pyran-2-yl-9H-purin-2-yl}methyl)-N'-[2-(1-piperidinyl)ethyl]urea

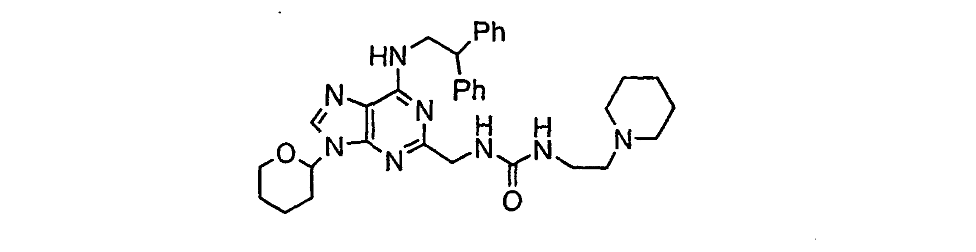

2-(1-Piperidinyl)ethanamine (0.35ml, 2.46mmol) was added to a solution of N,N'-carbonyldiimidazole (420mg, 2.6mmol) in dichloromethane (100ml). The reaction mixture was stirred for ten minutes at room temperature and then 2-(aminomethyl)-*N*-(2,2-diphenylethyl)-9-tetrahydro-2*H*-pyran-2-yl-9*H*-purin-6-amine (1.0g, 2.33mmol) (Preparation 3) was added. The reaction mixture was then stirred for 3 hours at room temperature. Dichloromethane (50ml) was then added and the resulting solution was washed with water (50ml) and saturated aqueous sodium chloride solution (50ml). The organic layer was dried with anhydrous magnesium sulphate and the solvent was removed under reduced pressure. The residue was purified by column chromatography on silica gel eluting with dichloromethane : methanol : 0.88 concentrated aqueous ammonia (93 : 7 : 1 by volume). This gave the title compound as an oil (300mg).
m/z: MH⁺ 583.
δ_{H} (400MHz; CDCl₃): 7.85 (1H, s), 7.55 (1H, s), 7.30-7.05 (10H, m), 5.70-5.60 (1H, m), 4.50-4.00 (6H, m), 3.75-3.60 (1H, m), 3.30-3.10 (2H, m), 2.45-2.20 (6H, m), 2.05-1.85 (2H, m), 1.85-1.25 (10H, m).

### PREPARATION 5

### N-({6-[(2,2-Diphenylethyl)amino]-9H-purin-2-yl}methyl)-N'-[2-(1-piperidinyl)ethyl]urea

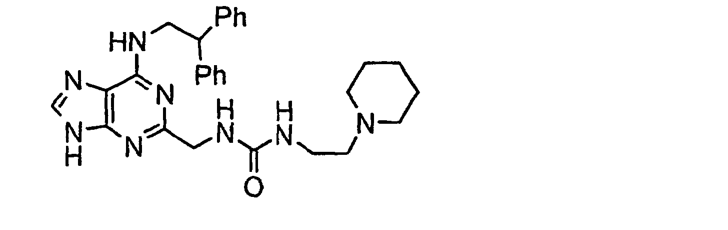

A solution of *N*-({6-[(2,2-diphenylethyl)amino]-9-tetrahydro-2*H*-pyran-2-yl-9*H*-purin-2-yl}methyl)-*N*'-[2-(1-piperidinyl)ethyl]urea (300mg, 0.51mmol) (Preparation 4) in methanol (150ml) was treated with aqueous hydrochloric acid (2M, 100ml). The reaction mixture was stirred at room temperature for 2 hours. The solvent volume was then reduced to 100ml by evaporation under reduced pressure. Saturated aqueous sodium hydrogen carbonate solution (50ml) and ethyl acetate (200ml) were added. The two phases were separated. The organic layer was washed with saturated aqueous sodium chloride solution (100ml), dried (anhydrous magnesium sulphate) and evaporated to give the title compound as a solid (255mg).
m/z: MH⁺ 499.
δ_{H} (400MHz; CDCl₃): 7.80 (1H, s), 7.35-7.10 (10H, m), 4.55-4.10 (5H, m), 3.40-3.20 (2H, m), 2.60-2.30 (6H, m), 1.60-1.25 (6H, m).

### PREPARATION 6

### (2R,3R,4R,5R)-4-(Acetyloxy)-2-[(acetyloxy)methyl]-5-(6-[(2,2-diphenylethyl)amino]-2-{[({[2-(1-piperidinyl)ethyl]amino}carbonyl) amino]methyl}-9H-purin-9-yl)tetrahydro-3-furanyl acetate

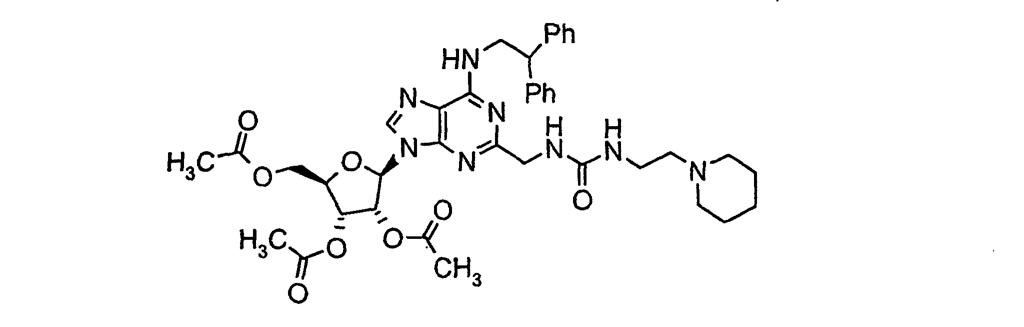

*N,O*-Bistrimethylsilylacetamide (0.34ml, 1.4mmol) was added to a stirred suspension of *N*-({6-[(2,2-diphenylethyl)amino]-9*H*-purin-2-yl)methyl)-*N*'-[2-(1-piperidinyl)ethyl]urea (100mg, 0.2mmol) (Preparation 5) in 1,1,1-trichloroethane (20ml) at 50°C. The reaction mixture was stirred at this temperature for 30 minutes, allowed to cool to room temperature and then evaporated under reduced pressure. Toluene (5ml) was added and the solvent was removed under reduced pressure. The residue was redissolved in toluene (20ml) and (2*R*,3*R*,4*R*,5*S*)-4,5-bis(acetyloxy)-2-[(acetyloxy)methyl]tetrahydro-3-furanyl acetate (0.064g, 0.2mmol) and then trimethylsilyltrifluoromethanesulphonate (0.1ml, 0.35mmol) were added. The reaction mixture was then heated under reflux for 2 hours. The reaction was allowed to cool to room temperature and diluted with ethyl acetate (100ml). The solution was washed with saturated aqueous sodium hydrogen carbonate solution (50ml) and saturated aqueous sodium chloride solution (50 ml) and then dried over anhydrous magnesium sulphate. The solvent was removed to give a residue that was purified by column chromatography on silica gel eluting with dichloromethane : methanol : 0.88 concentrated aqueous ammonia (90 : 10 : 1 by volume) to give the title compound as an oil (100mg).
m/z: MH⁺ 757.
δ_{H} (400MHz; CDCl₃): 7.65 (1H, s), 7.35-7.15 (10H, m), 6.05-6.00 (1H, m), 6.00-5.90 (2H, m), 5.85-5.75 (1H, m), 5.45-5.40 (1H, m), 4.60-4.15 (7H, m), 3.35-3.25 (2H, m), 2.50-2.35 (6H, m), 2.15 (3H, s), 2.10 (3H, s), 1.90 (3H, s), 1.60-1.35 (6H, m).

### PREPARATION 7

### (2S,3S,4R,5R)-4-(Benzoyloxy)-5-{2-cyano-6-[(2,2-diphenylethyl)amino]-9H-purin-9-yl}-2-[(ethylamino)carbonyl]tetrahydro-3-furanyl benzoate

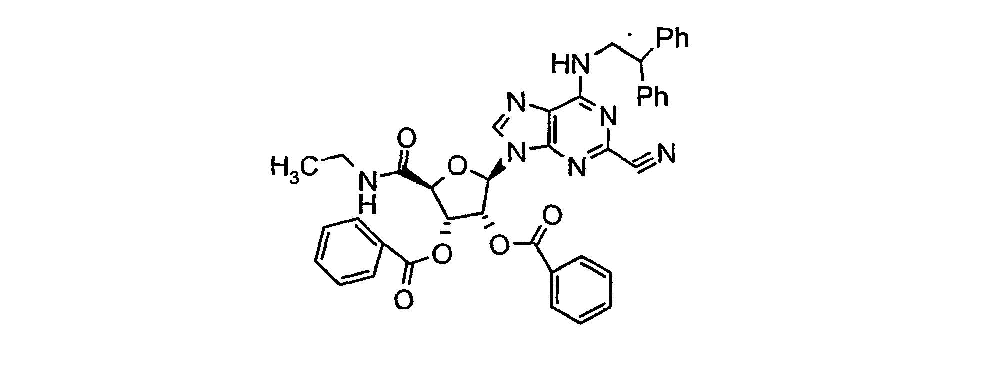

A mixture of 6-[(2,2-diphenylethyl)amino]-9*H*-purine-2-carbonitrile (Preparation 24) (5.00g, 14.7mmol), (2*S*,3*S*,4*R*,5*R*)-5-(acetyloxy)-4-(benzoyloxy)-2-[(ethylamino)carbonyl]tetrahydro-3-furanyl benzoate and (2*S*,3*S*,4*R*,5*S*)-5-(acetyloxy)-4-(benzoyloxy)-2-[(ethylamino)carbonyl]tetrahydro-3-furanyl benzoate (Preparation 18) (6.50g, 14.7mmol) and iodine (0.38g, 15.0mmol) were heated together at 150°C under reduced pressure (7kPa, 1 psi) for 2.5 hours. The reaction was then allowed to stand at room temperature for 18 hours. The residue was purified by column chromatography on silica gel eluting with a gradient system of ethyl acetate : pentane (40 : 60 by volume) increasing in polarity to neat ethyl acetate to afford the title compound as a foam (4.95g).
δ_{H} (400MHz; CDCl₃): 8.12 (3H, m), 7.79 (3H, m), 7.63 (1H, m), 7.50 (3H, m), 7.38-7.16 (11H, m), 6.35 (2H, m), 6.10 (1H, t), 6.03 (1H, d), 4.94 (1H, m), 4.35 (3H, m), 3.57 (2H, m), 1.30 (3H, t).

### PREPARATION 8

### (2S,3S,4R,5R)-5-{2-Cyano-6-[(2,2-diphenylethyl)amino]-9H-purin-9-yl}-N-ethyl-3,4-dihydroxytetrahydro-2-furancarboxamide

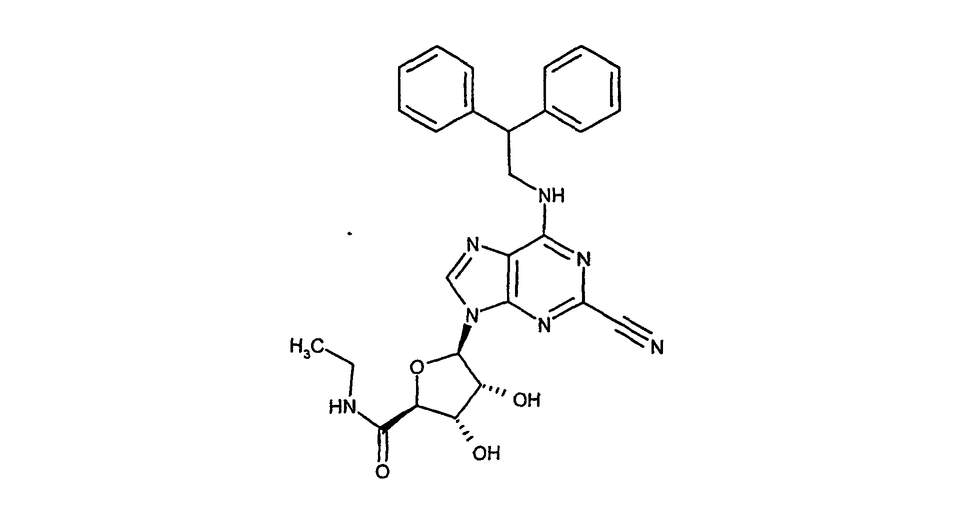

A solution of (2*S*,3*S*,4*R*,5*R*)-4-(benzoyloxy)-5-{2-cyano-6-[(2,2-diphenylethyl)amino]-9*H*-purin-9-yl}-2-[(ethylamino)carbonyl]tetrahydro-3-furanyl benzoate (Preparation 7) (4.75g, 6.59mmol) in ethanol (200ml) was saturated with ammonia gas and stirred at room temperature for 18 hours. The solvent was removed under reduced pressure and the residue was purified by column chromatography on silica gel eluting with a gradient system of dichloromethane : methanol (95 : 5 by volume) gradually changing to dichloromethane : methanol (90 : 10 by volume) to afford the title compound as a solid (2.80g).
δ_{H} (400MHz; d₆DMSO): 8.65 (1H, s), 8.54 (1H, br t), 8.18 (1H, br m), 7.13-7.42 (10H, m), 5.98 (1H, m), 5.65 (1H, m), 5.57 (1H, m), 4.59 (2H, m), 4.32 (1H, m), 4.08-4.28 (3H, m), 3.20 (2H, m), 1.05 (3H, t).

### PREPARATION 9

### 2-Chloro-N-(1-naphthylmethyl)-9-tetrahydro-2H-pyran-2-yl-9H-purin-6-amine

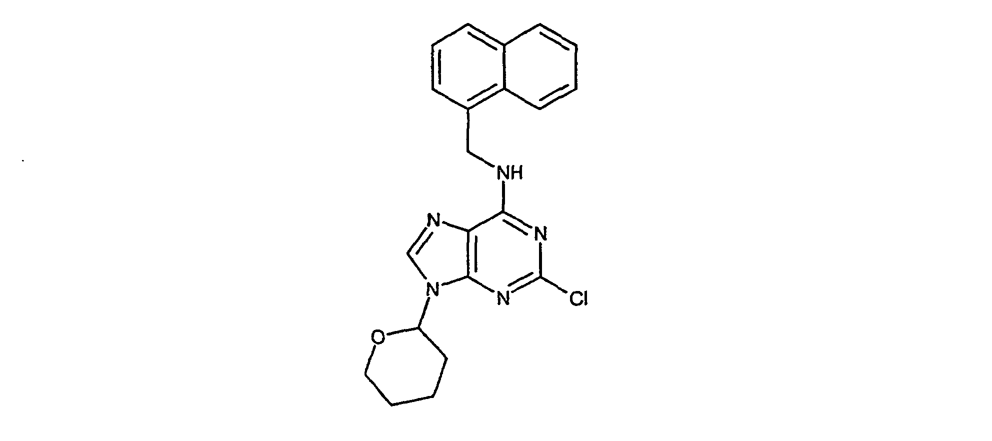

1-Naphthylmethanamine (5.4g, 19.7mmol) was added to a stirred solution of 2,6-dichloro-9-(tetrahydro-2*H*-pyran-2-yl)-9*H*-purine (3.5g, 22.3mmol) (Preparation 19) and triethylamine (4g, 39.6mmol) in acetonitrile (150ml) at room temperature. The reaction mixture was stirred at room temperature for 64 hours. The solvent was removed under reduced pressure, ethyl acetate (100ml) was added and then the solvent was removed under reduced pressure again. The residue was purified by column chromatography on silica gel eluting with pentane : ethyl acetate (1 : 1 by volume) increasing in polarity to neat ethyl acetate. The solvent was removed under reduced pressure to give the title compound (7.56g) as a solid.
δ_{H} (400MHz; CDCl₃): 8.10-8.00 (1H, m), 7.90-7.80 (3H, m), 7.55-7.45 (3H, m), 7.45-7.40 (1H, m), 6.15 (1H, s), 5.70-5.60 (1H, m), 5.25 (1H, bs), 4.20-4.10 (1H, m), 3.80-3.70 (1H, m), 2.15-2.00 (2H, m), 2.00-1.80 (1H, m), 1.80-1.60 (3H, m).

### PREPARATION 10

### 6-[(1-Naphthylmethyl)amino]-9H-purine-2-carbonitrile

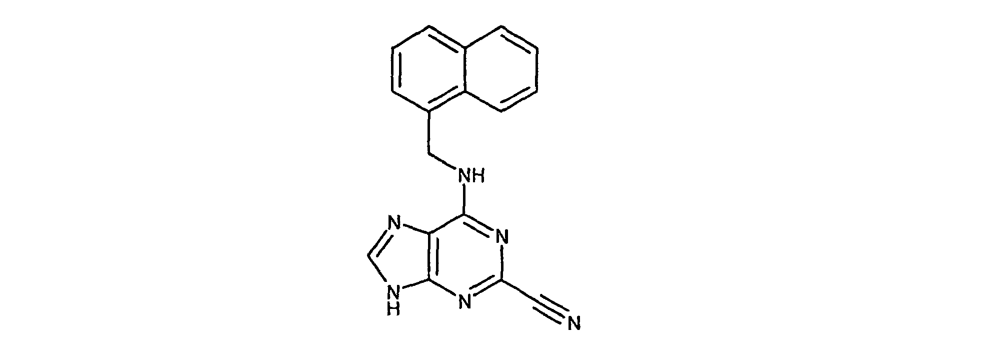

Tetrakistriphenylphosphine palladium (1.1g, 0.95mmol) was added to a solution of 2-chloro-*N*-(1-naphthylmethyl)-9-tetrahydro-2*H*-pyran-2-yl-9*H*-purin-6-amine (7.5g, 19mmol) (Preparation 9) , zinc cyanide (1.4g, 11.9mmol) and *N*-ethyl-*N*-isopropyl-2-propanamine (4ml, 23mmol) in N'N'-dimethylformamide (80ml). The reaction mixture was heated to 100°C for 16 hours. The reaction mixture was allowed to cool to room temperature and ethyl acetate (250ml) was added. The resulting mixture was extracted with aqueous sodium hydroxide solution (2M, 100ml). The sodium hydroxide layer was extracted with more ethyl acetate (50ml). The ethyl acetate layers were combined and washed with water (50ml) and then dried over anhydrous magnesium sulphate. The solvent was removed under reduced pressure, the residue was dissolved in dichloromethane (100ml) and the solvent was removed under reduced pressure again. The residue was purified by column chromatography on silica gel eluting with dichloromethane : methanol (99 : 2 by volume) increasing in polarity to dichloromethane : methanol (90 : 10 by volume). The solvent was removed under reduced pressure to give the title compound (1.8g) as a foam. The major product of this reaction was actually 6-[(1-naphthylmethyl)amino]-9-tetrahydro-2*H*-pyran-2-yl-9*H*-purine-2-carbonitrile (2.7g).
δ_{H} (400MHz; d₆DMSO): 8.35-8.25 (1H, m), 8.25-8.15 (1H, m), 7.95-7.90 (1H, m), 7.90-7.80 (1H, m), 7.60-7.40 (4H, m), 5.20-5.10 (2H, m).

### PREPARATION 11

### (2S,3S,4R,5R)-5-{2-(Aminomethyl)-6-[(2,2-diphenylethyl)amino]-9H-purin-9-yl}-N-ethyl-3,4-dihydroxytetrahydro-2-furancarboxamide

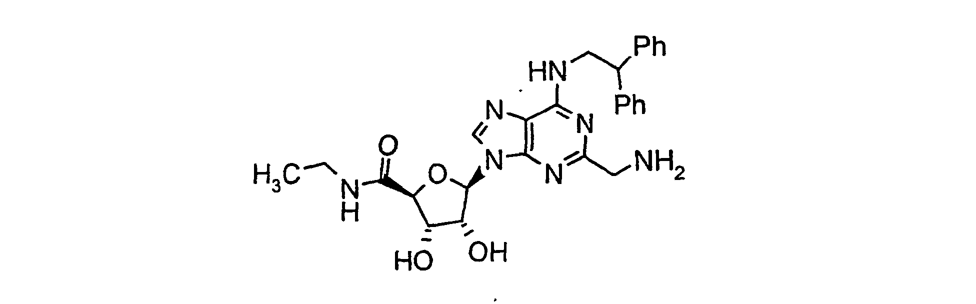

10% Palladium on carbon (400mg) was added to a solution of (2*S*,3*S*,4*R*,5*R*)-4-(benzoyloxy)-5-{2-cyano-6-[(2,2-diphenylethyl)amino]-9*H*-purin-9-yl}-2-[(ethylamino)carbonyl]tetrahydro-3-furanyl benzoate (Preparation 7) (2.0g, 2.70mmol) in a saturated solution of ammonia in ethanol (40ml). The reaction mixture was stirred under an atmosphere of hydrogen (414kPa, 60psi) for 16 hours at room temperature. The suspension was filtered through Arbocel (Trade Mark) and the solvent was removed under reduced pressure. The residue was purified by column chromatography on silica gel eluting with dichloromethane : methanol : 0.88 concentrated aqueous ammonia (95 : 5 : 0.5 by volume) increasing to (90 : 10 : 1 by volume). This gave the title compound as a solid (1.2g).
δ_{H} (400MHz; d₆DMSO): 8.55 (1H, s), 8.45-8.30 (1H, br s), 7.45-7.10 (10H, m), 6.10-6.00 (1H, m), 4.70-4.50 (2H, m), 4.35-4.10 (6H, m), 3.20-3.05 (2H, m), 1.10-0.95 (3H, m).

### PREPARATION 12

### (2S,3S,4R,5R)-5-(6-[(2,2-Diphenylethyl)amino]-2-{[({[2-(isopropylamino)ethyl] amino}carbonyl)amino]methyl}-9H-purin-9-yl)-N-ethyl-3,4-dihydroxytetrahydro-2-furancarboxamide

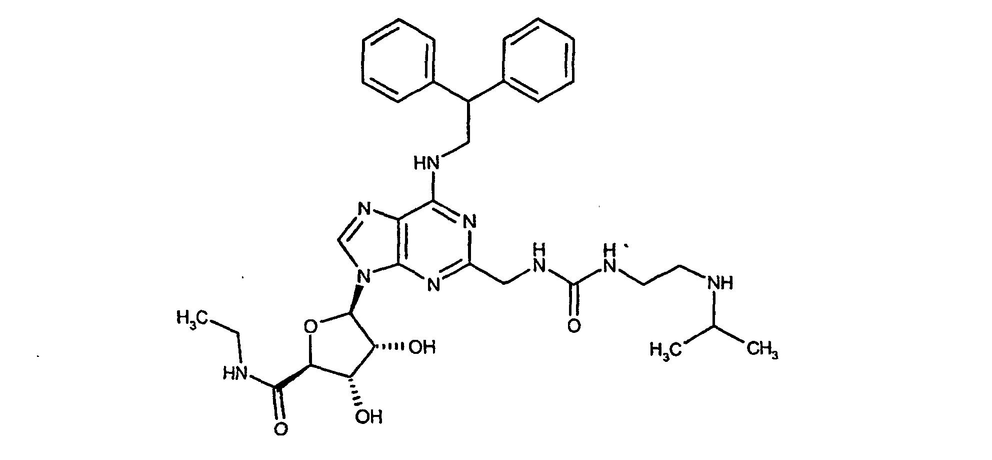

NN'-Carbonyldiimidazole (0.47g, 2.9mmol) was added to a stirred solution of (2*S*,3*S*,4*R*,5*R*)-5-{2-(aminomethyl)-6-[(2,2-diphenylethyl)amino]-9*H*-purin-9-yl}-*N*-ethyl-3,4-dihydroxytetrahydro-2-furancarboxamide (1.5g, 2.9mmol) (Preparation 11) in NN'-dimethylformamide (10ml) at room temperature. The reaction mixture was stirred at room temperature for 4 hours. N'-Isopropylethylene diamine was then added and the reaction mixture was stirred for 16 hours at room temperature. The solvent was then removed under reduced pressure. The residue was purified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (80 : 20 : 3 by volume). This gave the title compound as a foam (0.66g).
m/z MH⁺ 647.
δ_{H} (400MHz; CD₃OD): 8.20 (1H, m), 7.35-7.20 (8H, m), 7.20-7.10 (2H, m), 6.05-6.00 (1H, m), 4.55-4.40 (5H, m), 4.30-4.20 (2H, m), 3.35-3.20 (4H, m), 2.90-2.80 (1H, m), 2.70-2.65 (2H, m), 1.15-1.05 (6H, m).

### PREPARATION 13

### (2R,3R,4S,5R)-2-{2-(Aminomethyl)-6-[(9H-fluoren-9-ylmethyl)amino]-9H-purin-9-yl}-5-(hydroxymethyl)tetrahydro-3,4-furandiol

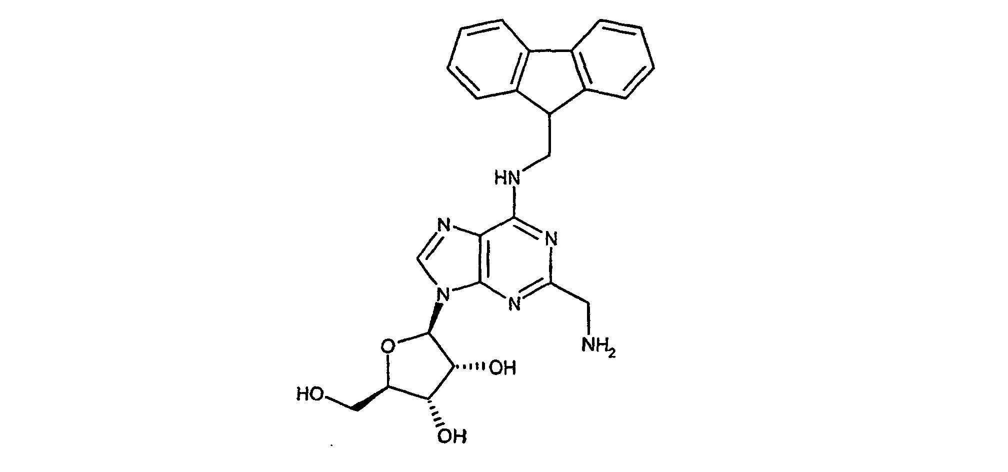

Ammonia gas was passed through an ice cold solution of (2*R*,3*R*,4*R*,5*R*)-4-(acetyloxy)-2-[(acetyloxy)methyl]-5-{2-cyano-6-[(9*H*-fluoren-9-ylmethyl)amino]-9*H*-purin-9-yl}tetrahydro-3-furanyl acetate (1.2g, 2mmol) (Preparation 71) in ethanol (40ml) until the solution was saturated. 10% Palladium on carbon (120mg) was added and the reaction mixture was stirred under an atmosphere of hydrogen gas (413.7 kPa, 60psi) at room temperature for 40 hours. The suspension was filtered through Arbocel (Trade Mark) and the solvent was removed from the filtrate under reduced pressure. The residue was purified by column chromatography on silica gel eluting with dichloromethane : methanol : 0.88 concentrated aqueous ammonia (96 : 4 : 0.4 by volume) increasing in polarity to dichloromethane : methanol : 0.88 concentrated aqueous ammonia (94 : 6 : 0.5 by volume). The solvent was removed under reduced pressure to give the title compound (358mg) as a foam.
m/z MH⁺ 475.
δ_{H} (400MHz; CD₃OD): 8.20 (1H, m), 7.85-7.75 (2H, m), 7.70-7.60 (2H, m), 7.40-7.20 (4H, m), 6.00-5.90 (1H, m), 4.40-4.35 (1H, m), 4.35-4.30 (1H, m), 4.20-4.00 (3H, m), 3.90-3.70 (4H, m).

### PREPARATION 14

### (2S,3S,4R,5R)-4-(Benzoyloxy)-5-(6-[(2,2-diphenylethyl)amino]-2-{[({[2-(1-piperidinyl)ethyl]amino}carbonyl)amino]methyl]-9H-purin-9-yl)-2-[(ethylamino)carbonyl]tetrahydro-3-furanyl benzoate

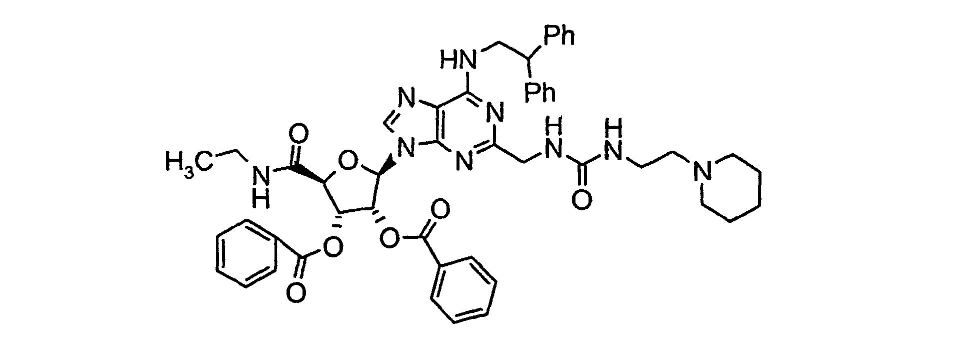

*N,O*-Bistrimethylsilylacetamide (0.34ml, 1.4mmol) was added to a stirred suspension of *N*-({6-[(2,2-diphenylethyl)amino]-9*H*-purin-2-yl}methyl)-*N*'-[2-(1-piperidinyl)ethyl]urea (100mg, 0.2mmol) (Preparation 5) in 1,1,1-trichloroethane (20ml) at 50°C. The reaction mixture was stirred at this temperature for 30 minutes, allowed to cool to room temperature and then evaporated under reduced pressure. Toluene (5ml) was added and the solvent was removed under reduced pressure. The residue was redissolved in toluene (20ml). (2*S*,3*S*,4*R*,5*R*)-5-(Acetyloxy)-4-(benzoyloxy)-2-[(ethy(amino)carbonyl]tetrahydro-3-furanyl benzoate and (2*S*,3*S*,4*R*,5*S*)-5-(acetyloxy)-4-(benzoyloxy)-2-[(ethylamino)carbonyl]tetrahydro-3-furanyl benzoate (Preparation 18) and then trimethylsilyltrifluoromethanesulphonate (0.1 ml, 0.35mmol) were added and the reaction mixture was heated under reflux for 2 hours. The reaction was then allowed to cool to room temperature and diluted with ethyl acetate (100ml). The solution was washed with saturated aqueous sodium hydrogen carbonate solution (2 x 50ml) and saturated aqueous sodium chloride solution (50 ml) and then dried (anhydrous magnesium sulphate). The solvent was removed to give a residue that was purified by column chromatography on silica gel eluting with dichloromethane : methanol : 0.88 concentrated aqueous ammonia (95 : 5 : 0.5 by volume). This gave an impure oil (100mg) which was used without further purification in subsequent experiments.

### PREPARATION 15

### (3aR,4S,6R,6aR)-N-Ethyl-6-methoxy-2,2-dimethyltetrahydrofuro[3,4-d][1,3]dioxole-4-carboxamide

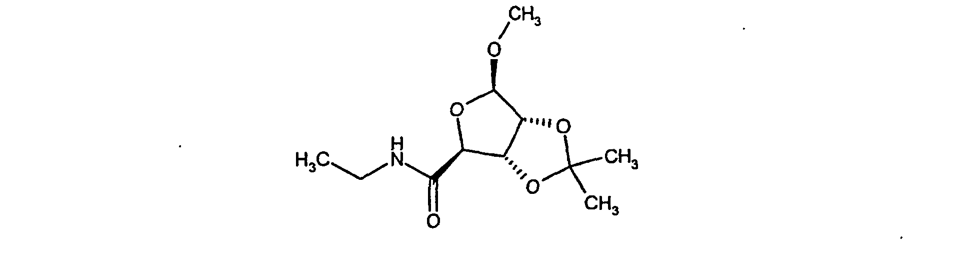

Oxalyl chloride (14.0 ml, 160 mmol) was added dropwise to a stirred solution of (3a*R*,4*S*,6*R*,6a*R*)-6-methoxy-2,2-dimethyltetrahydrofuro[3,4-*d*][1,3]dioxole-4-carboxylic acid (*J. Am. Chem*. *Soc*., **80,** 1958, 5168-5173) (23.30 g, 107 mmol) in anhydrous dichloromethane (120 ml) and N,N'-dimethylformamide (2 drops) and the mixture was stirred at room temperature for 3 hours until gas evolution had ceased. TLC analysis showed that some starting material still remained and so more N,N-dimethylformamide (2 drops) was added and stirring was continued for 1 Hour. The solvent was removed under reduced pressure and the residue was azeotroped twice with anhydrous dichloromethane. The residue was then dissolved in anhydrous dichloromethane (200 ml) and the solution was treated dropwise with a solution of ethylamine in tetrahydrofuran (2M, 140 ml, 280 mmol). This solution was left to stand at room temperature for 48 hours. Diethyl ether (250 ml) was added and the mixture was stirred for 15 minutes. The mixture was filtered and the solvent was removed from the filtrate under reduced pressure. The residue was purified by column chromatography on silica gel eluting with a gradient system of dichloromethane : ethyl acetate (100 : 0 by volume) gradually changing to dichloromethane : ethyl acetate (44 : 66 by volume) to afford the title compound as a yellow solid (24.70 g).
δ_{H} (400MHz; CDCl₃): 6.53 (1H, br m), 5.12 (1H, dd), 5.07 (1H, d), 4.60 (1H, d), 4.54 (1H, dd), 3.46 (3H, s), 3.32 (2H, m), 1.51 (3H, s), 1.34 (3H, s), 1.15 (3H, t).
m/z MH⁺ 246.

### PREPARATION 16

### (2S,3S,4R,5R)-N-Ethyl-3,4-dihydroxy-5-methoxytetrahydro-2-furancarboxamide and (2S,3S,4R,5S)-N-ethyl-3,4-dihydroxy-5-methoxytetrahydro-2-furancarboxamide

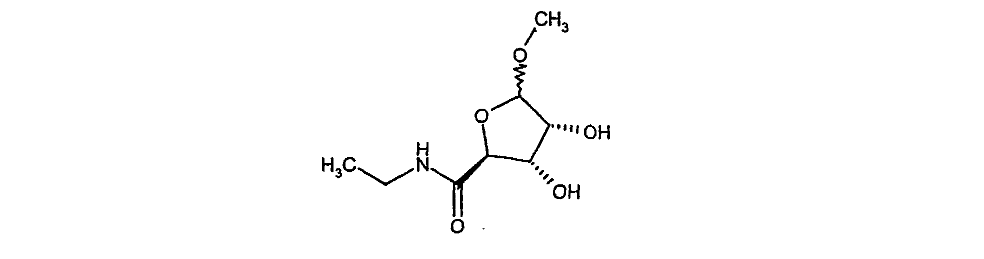

A solution of (3a*R*,4*S*,6*R*,6a*R*)-*N*-ethyl-6-methoxy-2,2-dimethyltetrahydrofuro[3,4-*d*][1,3]dioxole-4-carboxamide (Preparation 15) (24.60 g, 100 mmol) and pyridinium *p*-toluenesulphonate (2.50 g, 10 mmol) in methanol (500 ml) was heated under reflux for 18 hours. NMR analysis showed that some starting material still remained and therefore the solvent was removed under reduced pressure. The residue was dissolved in methanol (500 ml) and heated under reflux for 8 hours. NMR analysis showed that some starting material still remained therefore the solvent was removed under reduced pressure once more, the residue was dissolved in methanol (500 ml) and the resulting solution was heated under reflux for 24 hours. The solvent was then removed under reduced pressure and the residue was azeotroped three times with dichloromethane to afford the title compound as an oil and as a mixture of α and β anomers (20.50 g).
δ_{H} (400MHz; CDCl₃): 6.58 (1H, br m), 4.99 (0.25H, d), 4.94 (0.75H, d), 4.46 (0.25H, d), 4.37 (1.5H, m), 4.24 (0.25H, dd), 4.05 (1H, m), 3.52 (0.75H, s), 3.47 (2.25H, s), 3.30 (2H, m), 1.16 (3H, m).

### PREPARATION 17

### (2S,3S,4R,5R)-4-(Benzoyloxy)-2-[(ethylamino)carbonyl]-5-methoxytetrahydro-3-furanyl benzoate and (2S,3S,4R,5S)-4-(benzoyloxy)-2-[(ethylamino)carbonyl]-5-methoxytetrahydro-3-furanyl benzoate

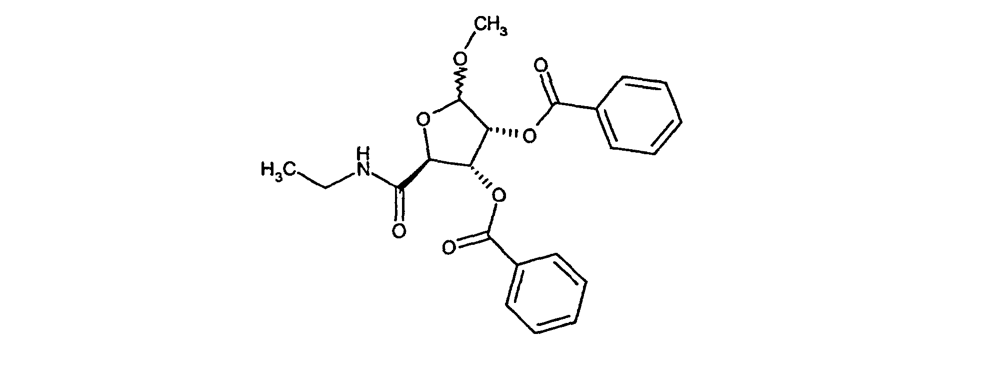

A solution of benzoyl chloride (30.0 ml, 259 mmol) in dichloromethane (100 ml) was added slowly to a solution of (2*S*,3*S*,4*R*,5*R*)-*N*-ethyl-3,4-dihydroxy-5-methoxytetrahydro-2-furancarboxamide and (2*S*,3*S*,4*R*,5*S*)-*N*-ethyl-3,4-dihydroxy-5-methoxytetrahydro-2-furancarboxamide (Preparation 16) (20.50 g, 100 mmol) and pyridine (33.0 ml, 409 mmol) in dichloromethane (400 ml) and the resulting mixture was stirred at room temperature for 18 hours. The solvent was removed under reduced pressure and the residue was partitioned between diethyl ether and aqueous hydrochloric acid (1M, 300 ml). The layers were separated and the aqueous layer was re-extracted with diethyl ether. The organic layers were combined, washed sequentially with water and brine, dried over anhydrous magnesium sulphate, filtered and evaporated under reduced pressure. The residue was purified by column chromatography on silica gel eluting with a gradient system of dichloromethane : diethyl ether (95 : 5 by volume) gradually changing to dichloromethane : diethyl ether (80 : 20 by volume) to afford the title compound as an oil and as a mixture of α and β anomers (37.0 g).
δ_{H} (400MHz; CDCl₃): 8.16 (0.5H, d), 7.95 (1.5H, d), 7.88 (1.5H, d), 7.81 (0.5H, d), 7.25-7.66 (6H, m), 6.65 (1H, br m), 5.88 (1H, m), 5.60 (0.75H, dd), 5.46 (0.25H, d), 5.23 (0.75H, d), 5.17 (0.25H, t), 4.80 (1H, m), 3.59 (2.25H, s), 3.49 (0.75H, s), 3.39 (2H, m), 1.23 (3H, t).

### PREPARATION 18

### (2S,3S,4R,5R)-5-(Acetyloxy)-4-(benzoyloxy)-2-[(ethylamino)carbonyl]tetrahydro-3-furanxl benzoate and (2S,3S,4R,5S)-5-(acetyloxy)-4-(benzoyloxy)-2-[(ethylamino)carbonyl]tetrahydro-3-furanyl benzoate

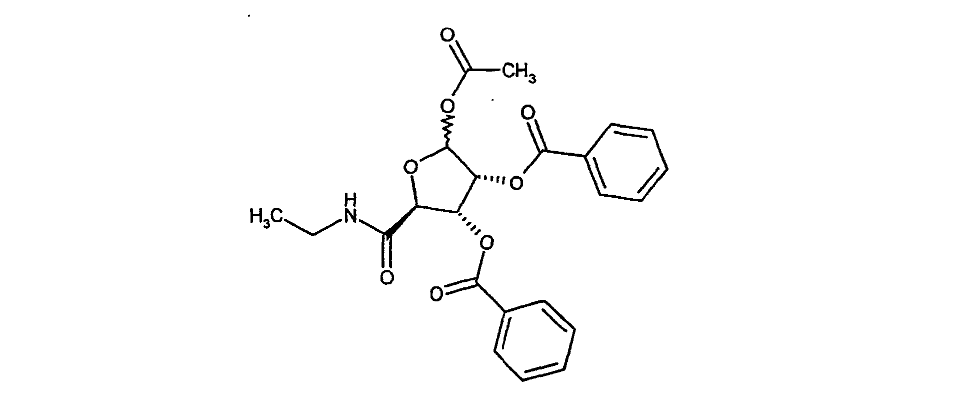

A solution of (2*S*,3*S*,4*R*,5*R*)-4-(benzoyloxy)-2-[(ethylamino)carbonyl]-5-methoxytetrahydro-3-furanyl benzoate and (2*S*,3*S*,4*R*,5*S*)-4-(benzoyloxy)-2-[(ethylamino)carbonyl]-5-methoxytetrahydro-3-furanyl benzoate (Preparation 17) (37.0 g, 89.6 mmol) in a mixture of acetic acid (330 ml, 5.77 mol) and acetic anhydride (67 ml, 709 mmol) was cooled to -10°C and treated dropwise with hydrochloric acid (12 N, 7.0 ml, 132 mmol). The mixture was stirred for 18 hours, during which time it was allowed to warm to room temperature. After cooling the mixture to 0°C, water (1000 ml) was added slowly. The mixture was then extracted three times with ethyl acetate (3 portions of 500 ml). The organic layers were combined, washed sequentially with water, a saturated aqueous solution of sodium hydrogen carbonate and brine, dried over anhydrous magnesium sulphate, filtered and evaporated under reduced pressure. The residue was purified by column chromatography on silica gel eluting with a gradient system of diethyl ether : pentane (66 : 44 by volume) gradually changing to diethyl ether:pentane (100:0 by volume). The residue was further purified by column chromatography on silica gel eluting with a gradient system of dichloromethane : diethyl ether (95 : 5 by volume) gradually changing to dichloromethane : diethyl ether (90 : 10 by volume) to afford the title compound as a mixture of α- and β-anomers (15.40 g).
δ_{H} (400MHz; CDCl₃): 8.12 (0.8H, d), 7.97 (1.2H, d), 7.92 (1.2H, d), 7.79 (0.8H, d), 7.24-7.65 (6H, m), 6.73 (0.4H, d), 6.62 (0.4H, br m), 6.46 (0.6H, br m), 6.42 (0.6H, d), 6.07 (0.4H, dd), 5.95 (0.6H, t), 5.72 (0.6H, d), 5.44 (0.4H, t), 4.94 (0.4H, d), 4.86 (0.6H, d), 3.36 (2H, m), 2.17 (1.8H, s), 2.10 (1.2H, s), 1.20 (3H, m).

### PREPARATION 19

### 2,6-Dichloro-9-(tetrahydro-2H-pyran-2-yl)-9H-purine

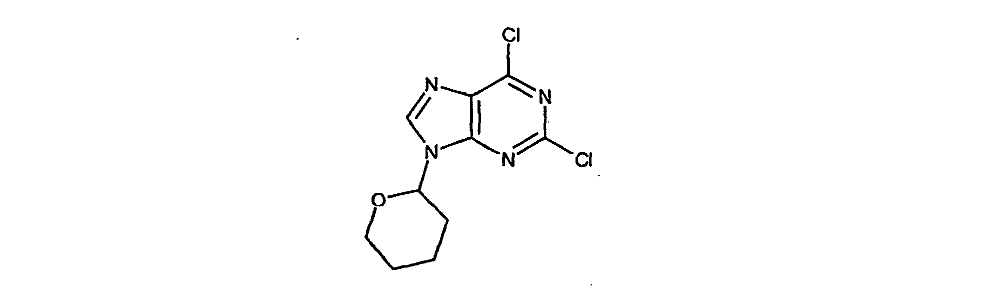

2,6-Dichloro-9*H*-purine (20 g, 0.11 mol) and 4-toluenesulphonic acid monohydrate (0.2 g) were dissolved in ethyl acetate (300 ml), the mixture was heated to 50°C and a solution of 3,4-dihydro-2H-pyran (12.6 ml, 0.14 mol) in ethyl acetate (50 ml) was added slowly over 30 minutes. The reaction mixture was cooled to room temperature, water (100 ml) was added and the pH of the solution was adjusted to 7 by addition of a saturated aqueous solution of sodium hydrogen carbonate. The organic layer was separated, washed sequentially with water and brine, dried over anhydrous magnesium sulphate, filtered and evaporated under reduced pressure. The residue was azeotroped with pentane (100ml) to afford the title compound as a slightly impure white solid (30.9 g).
δ_{H} (400MHz; CDCl₃): 8.30 (1H, s), 5.75 (1H, dd), 4.25-4.15 (1H, m), 3.85-3.70 (1H, m), 2.20-1.60 (6H, m).

### PREPARATION 20

### 2-Chloro-N-(2,2-diphenylethyl)-9-(tetrahydro-2H-pyran-2-yl)-9H-purin-6-amine

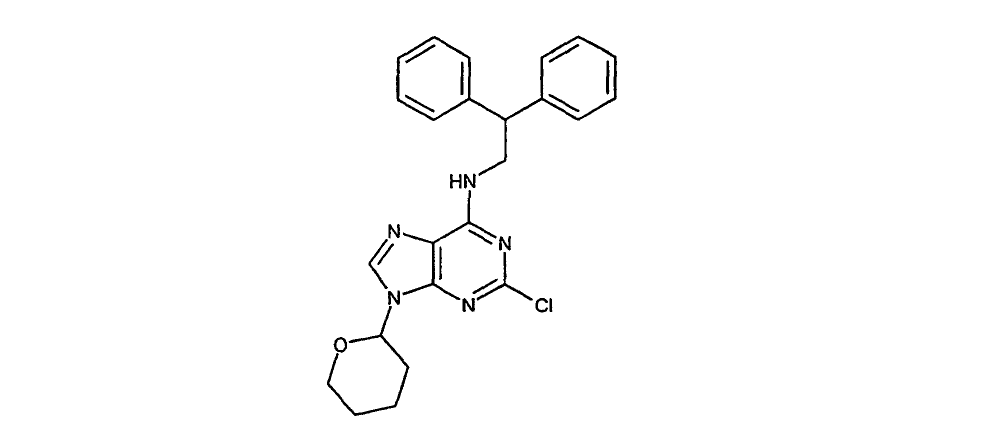

A solution of 2,6-dichloro-9-(tetrahydro-2*H*-pyran-2-yl)-9*H*-purine (Preparation 19) (30.9 g, 0.11 mol) in isopropyl alcohol (600 ml) was treated with *N*-ethyl-*N*-isopropyl-2-propanamine (47.5ml, 0.27mol) and 2,2-diphenylethylamine (24.8 g, 0.13 mol) and the resulting mixture was heated under reflux for 3 hours. The solvent was removed under reduced pressure and the residue was azeotroped with ethyl acetate. The residue was purified by column chromatography on silica gel eluting with a gradient system of ethyl acetate : hexane (40 : 60, by volume) gradually changing to ethyl acetate : hexane (60 : 40, by volume) to afford the title compound as a foam (49.7 g).
δ_{H} (400MHz; CDCl₃): 7.95-7.75 (1H, br s), 7.35-7.15 (10H, m), 5.80-5.70 (1H, br s), 5.65 (1H, d), 4.35 (1H, m), 4.30-4.18 (1H, br s), 4.10 (1H, d), 3.70 (1H, t), 2.05-1.95 (2H, m), 1.95-1.80 (1H, m), 1.80-1.55 (3H, m).

### PREPARATION 21

### N-(2,2-Diphenylethyl)-2-(methylsulfanyl)-9-(tetrahydro-2H-pyran-2-yl)-9H-purin-6-amine

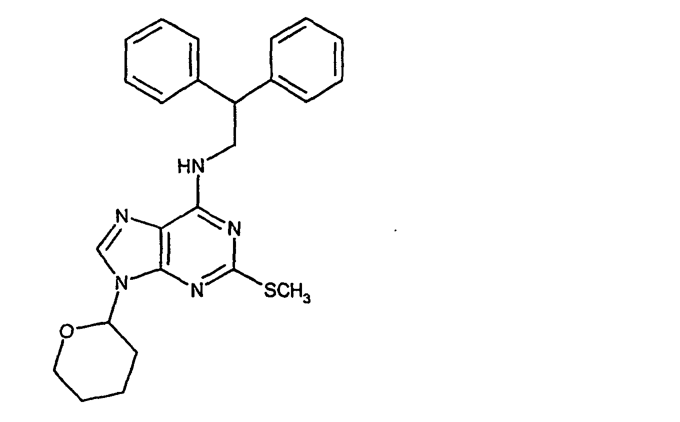

A solution of 2-chloro-*N*-(2,2-diphenylethyl)-9-(tetrahydro-2*H*-pyran-2-yl)-9*H*-purin-6-amine (Preparation 20) (49.7 g, 0.11 mol) in dry N,N-dimethylformamide (200 ml) was treated with sodium thiomethoxide (10 g, 0.14 mol) and the resulting mixture was heated under an atmosphere of nitrogen at 100°C for 90 minutes. The mixture was stirred at room temperature for 72 hours and then reheated at 100 °C for a further 2 hours. The reaction mixture was cooled and diluted with water (1000 ml). A suspension was formed which was extracted twice with diethyl ether (500ml). The combined organic layers were washed sequentially with water and brine, dried over anhydrous magnesium sulphate, filtered and evaporated under reduced pressure. The residue was azeotroped sequentially with diethyl ether and pentane to afford the title compound as a foam (48.9 g).
δ_{H} (400MHz; CDCl₃): 7.80 (1H, s), 7.20-7.10 (10H, m), 5.70-5.55 (2H, d), 4.40-4.20 (3H, m), 4.20-4.05 (1H, m), 3.80-3.65 (1H, m), 2.60 (3H, s), 2.15-1.90 (3H, m), 1.90-1.60 (3H, m).

### PREPARATION 22

### N-(2,2-Diphenylethyl)-2-(methylsulfonyl)-9-(tetrahydro-2H-pyran-2-yl)-9H-purin-6-amine

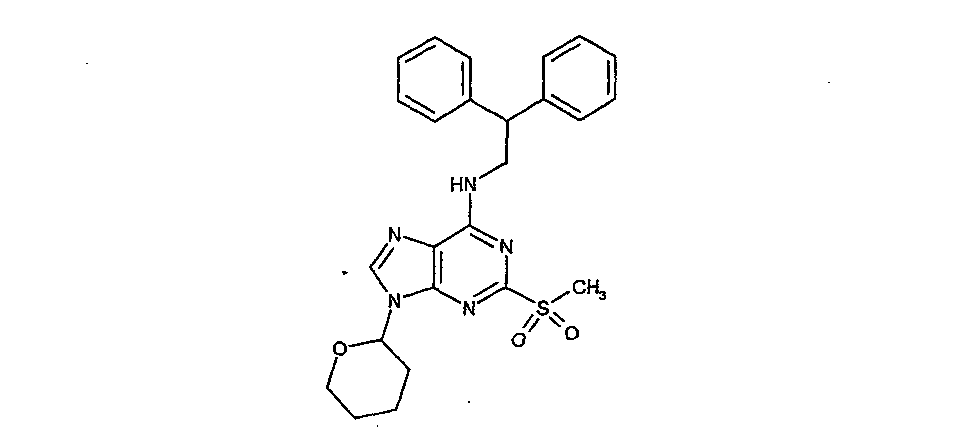

A solution of Oxone (Trade Mark) (potassium peroxymonosulphate) (44 g, 71.7 mmol) in water (200 ml) was added dropwise over 2 hours to a solution of *N*-(2,2-diphenylethyl)-2-(methylsulfanyl)-9-(tetrahydro-2*H*-pyran-2-yl)-9*H*-purin-6-amine (Preparation 21) (25 g, 56.2 mmol) and sodium hydrogencarbonate (20 g, 238 mmol) in a mixture of acetone (1000 ml) and water (250 ml). The resultant mixture was stirred at room temperature for 24 hours and filtered and the residue was washed with acetone. The acetone was removed from the filtrate under reduced pressure and the resulting aqueous residue was extracted with ethyl acetate and then dichloromethane. The combined organic layers were washed with brine, dried over anhydrous magnesium sulphate, filtered and evaporated under reduced pressure. The residue was triturated with diethyl ether, filtered, washed with diethyl ether and pentane and then dried to afford the title compound as a white solid (20.32 g).
δ_{H}(400MHz; CDCl₃): 8.00 (1H, s), 7.35-7.15 (10H, m), 6.05-5.95 (1H, br s), 5.75 (1H, d), 4.40-4.35 (1H, m), 4.35-4.20 (2H, br s), 4.15-4.05 (1H, m), 3.75 (1H, t), 3.30 (3H, s), 2.18-2.05 (1H, m), 2.05-1.98 (1H, m), 1.98-1.80 (1H, m), 1.80-1.60 (3H, m).

### PREPARATION 23

### 6-[(2,2-Diphenylethyl)amino]-9-(tetrahydro-2H-pyran-2-yl)-9H-purine-2-carbonitrile

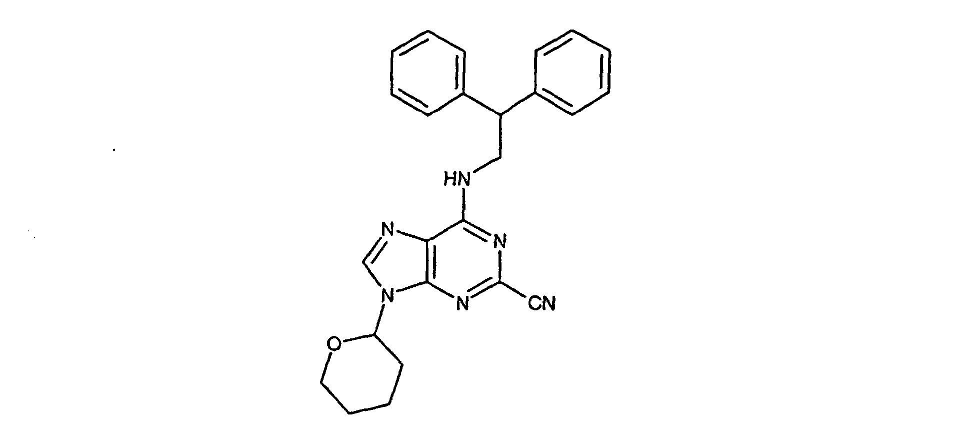

A solution of *N*-(2,2-diphenylethyl)-2-(methylsulfonyl)-9-(tetrahydro-2*H*-pyran-2-yl)-9*H*-purin-6-amine (Preparation 22) (20.1 g, 42.1 mmol) in dry N,N-dimethylformamide (100ml) was treated with potassium cyanide (5.5 g, 84.6 mmol) and the mixture was heated at 120°C for 24 hours under a nitrogen atmosphere. The mixture was cooled to room temperature and diluted with water (1000 ml) and stirring was continued for a further 1 Hour. The resultant solid was filtered off and washed several times with water. The solid was then dissolved in dichloromethane and the solution was washed with water, dried over anhydrous magnesium sulphate, filtered and evaporated under reduced pressure. The residue was azeotroped with diethyl ether (twice) to afford the title compound as an oil (17 g).
δ_{H}(400MHz; CDCl₃): 8.00 (1H, s), 7.40-7.20 (10H, m), 6.00-5.75 (1H, br s), 5.70 (1H, d), 4.40-4.20 (3H, m), 4.20-4.10 (1H, m), 3.80-3.70 (1H, m), 2.20-1.90 (3H, m), 1.90-1.60 (3H, m).

### PREPARATION 24

### 6-[(2,2-Diphenylethyl)amino]-9H-purine-2-carbonitrile

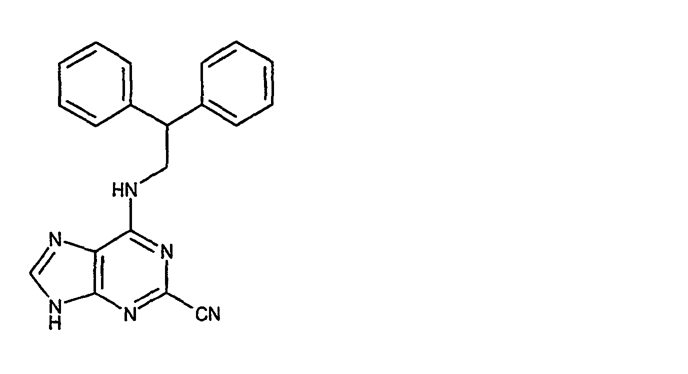

A solution of 6-[(2,2-diphenylethyl)amino]-9-(tetrahydro-2H-pyran-2-yl)-9H-purine-2-carbonitrile (Preparation 23) (17 g, 40.1 mmol) in ethanol (850 ml) was treated with 2 N aqueous hydrochloric acid (50 ml) and the mixture was stirred at room temperature for 24 hours. The solvent was removed under reduced pressure, the residue was dissolved in ethanol and the solvent was again removed under reduced pressure. The residue was triturated with diethyl ether, filtered, washed with diethyl ether and pentane and dried to afford the title compound as a solid (13.6 g).
δ_{H} (400MHz; d₆DMSO): 8.30 (1H, s), 8.20-8.05 (1H, br s), 7.40-7.10 (10H, m), 4.60-4.40 (1.4H, m), 4.20-4.00 (1.6H, m).
m/z [MH⁺] 341.

### PREPARATION 25

### N'-({6-[(2,2-diphenylethyl)amino]-9-tetrahydro-2H-pyran-2-yl-9H-purin-2-yl}methyl)-N-methyl-N-[2-(2-pyridinyl)ethyl]urea

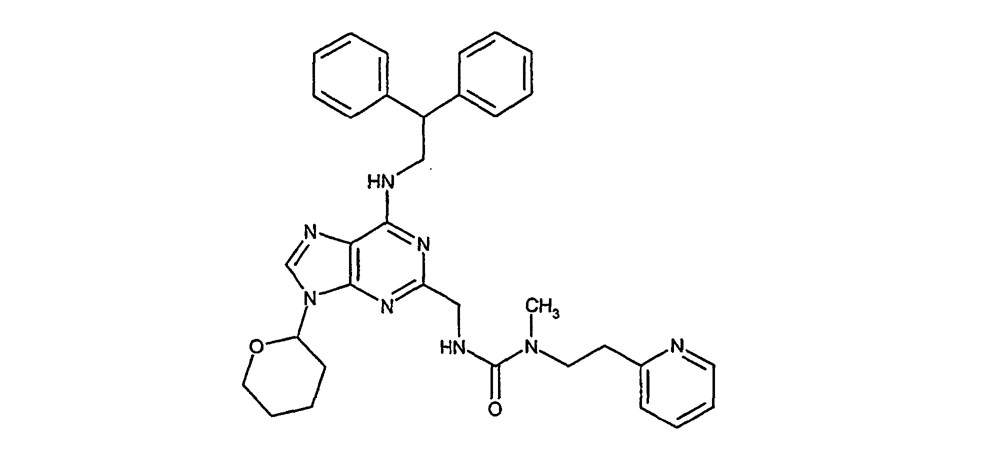

N'N'-Carbonyldiimidazole (0.42g, 2.6mmol) was added to a solution of 2-(aminomethyl)-*N*-(2,2-diphenylethyl)-9-tetrahydro-2*H*-pyran-2-yl-9*H*-purin-6-amine (1.0g, 2.3mmol) (Preparation 3) in dichloromethane (100ml). The reaction mixture was stirred for 16 hours at room temperature. Dichloromethane (100ml) was added and the solution was washed with water (50ml) and saturated aqueous sodium chloride solution (50ml). The organic layer was dried over anhydrous magnesium sulphate and the solvent was removed under reduced pressure. A portion of this residue (200mg) was dissolved in tetrahydrofuran (50ml) and *N*-methyl-2-(2-pyridinyl)ethanamine (200mg, 1.5mmol) and triethylamine (0.14ml, 1.0mmol) were added. The reaction mixture was heated under reflux for 2.5 hours. The reaction mixture was allowed to cool to room temperature and the solvent was removed under reduced pressure. The residue was redissolved in ethylacetate (100ml) and washed with water (100ml) and saturated aqueous sodium chloride solution (100ml). The organic phase was dried over anhydrous magnesium sulphate and the solvent was removed under reduced pressure. The residue was purified by column chromatography on silica gel eluting with dichloromethane : methanol : 0.88 concentrated aqueous ammonia (97 : 3 : 0.5 by volume). The solvent was removed under reduced pressure to give the title compound as a foam (100mg).
δ_{H}(400MHz; CDCl₃): 8.50-8.40 (1H, m), 7.85-7.75 (1H, m), 7.60-7.50 (1H, m), 7.30-7.10 (11H, m), 7.10-7.00 (1H, m), 6.20-6.10 (1H, m), 6.10-6.00 (1H, m), 5.70-5.60 (1H, m), 4.55-4.45 (2H, m), 4.35-4.15 (3H, m), 4.15-4.05 (1H, m), 3.75-3.60 (3H, m), 3.05-2.95 (2H, m), 2.75 (3H, s), 2.10-1.90 (3H, m), 1.80-1.50 (3H, m).

### PREPARATION 26

### N'-({6-[(2,2-Diphenylethyl)amino]-9H-purin-2-yl}methyl)-N-methyl-N-[2-(2-pyridinyl)ethyl]urea

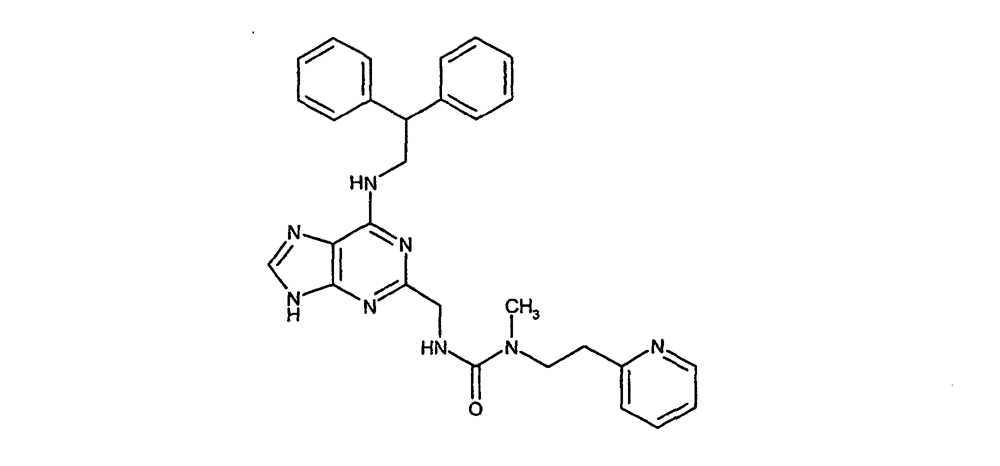

Aqueous hydrochloric acid (2M, 5ml) was added to a solution of *N*'-({6-[(2,2-diphenylethyl)amino]-9-tetrahydro-2*H*-pyran-2-yl-9*H*-purin-2-yl}methyl)-*N*-methyl-*N*-[2-(2-pyridinyl)ethyl]urea (Preparation 25) in methanol (50ml). The solution was stirred for 16 hours at room temperature. The solvent was removed under reduced pressure. The residue was dissolved in ethyl acetate (100ml) and the solution was washed with saturated aqueous sodium hydrogen carbonate solution (50ml) and then dried over anhydrous magnesium sulphate. The solvent was removed under reduced pressure to give the title compound (0.161g) as a gum.
δ_{H} (400MHz; CD₃OD): 8.45-8.40 (1H, m), 7.90 (1H, m), 7.70-7.65 (1H, m), 7.30-7.20 (10H, m), 7.15-7.10 (2H, m), 4.45-4.35 (3H, m), 4.30-4.20 (2H, m), 3.55-3.45 (2H, m), 2.95-2.90 (2H, m), 2.75 (3H, m).

### PREPARATION 27

### N-[2-(Diisopropylamino)ethyl]-1H-imidazole-1-carboxamide

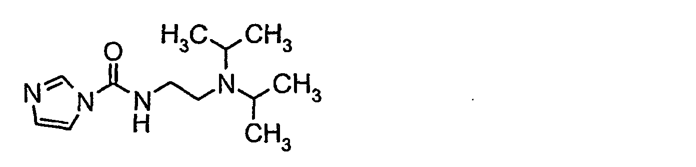

*N*¹,*N*¹-Diisopropyl-1,2-ethanediamine (1g, 6.94 mmol) was added to a stirred solution of N,N'-carbonyldiimidazole (1.12g, 6.94mmol) in dichloromethane (50ml) at room temperature. The reaction mixture was stirred for 1 hour and then diluted with more dichloromethane (50ml), washed with water (60ml), dried with anhydrous magnesium sulphate and evaporated under reduced pressure. This gave the title compound as a solid (600mg).
δ_{H} (400MHz; CDCl₃): 8.05 (1H, s), 7.25 (1H, s), 7.05 (1H, s), 6.65 (1H, br s), 3.40-3.35 (2H, m), 3.10-3.00 (2H, m), 2.75-2.70 (2H, m), 1.05-1.00 (6H, m).

### PREPARATIONS 28-37

The following compounds were prepared by the method of Preparation 27 using the appropriate amine.

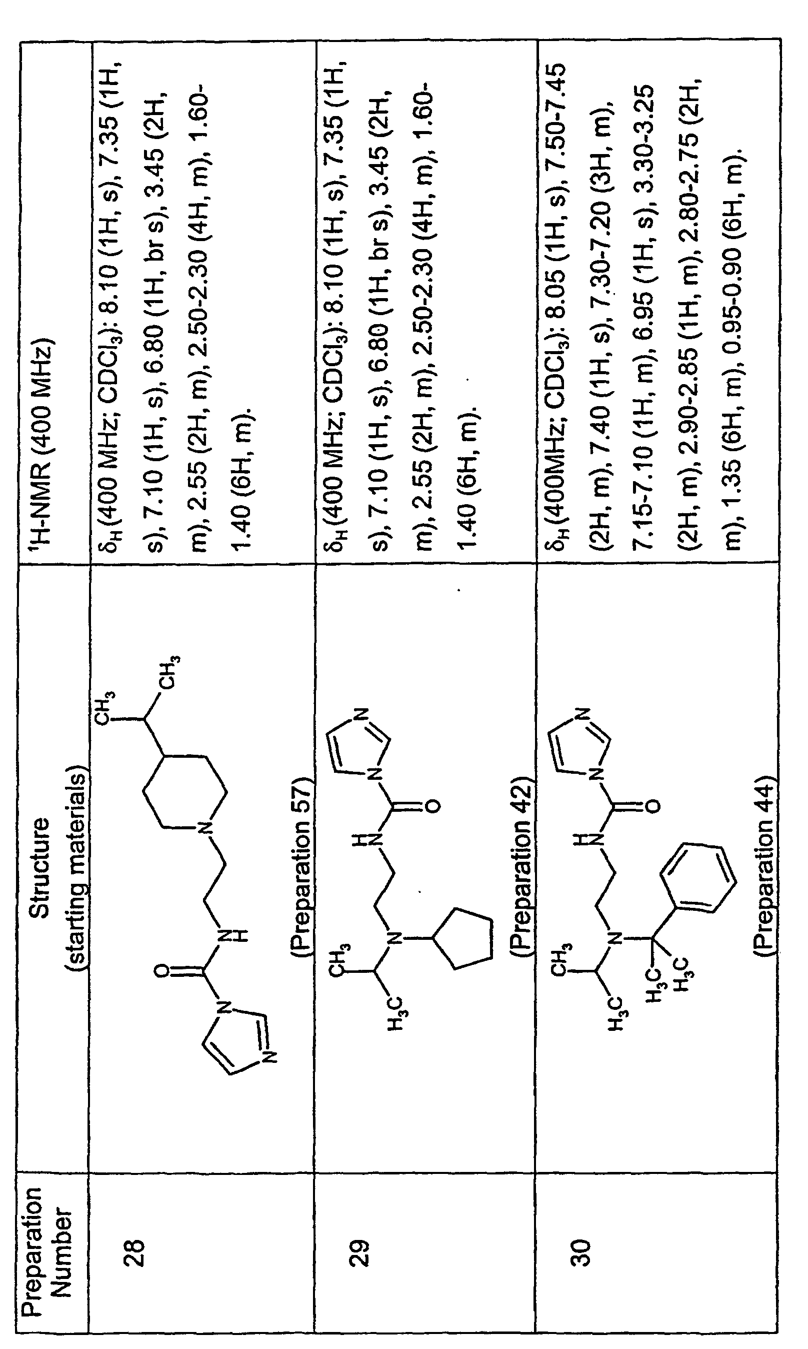

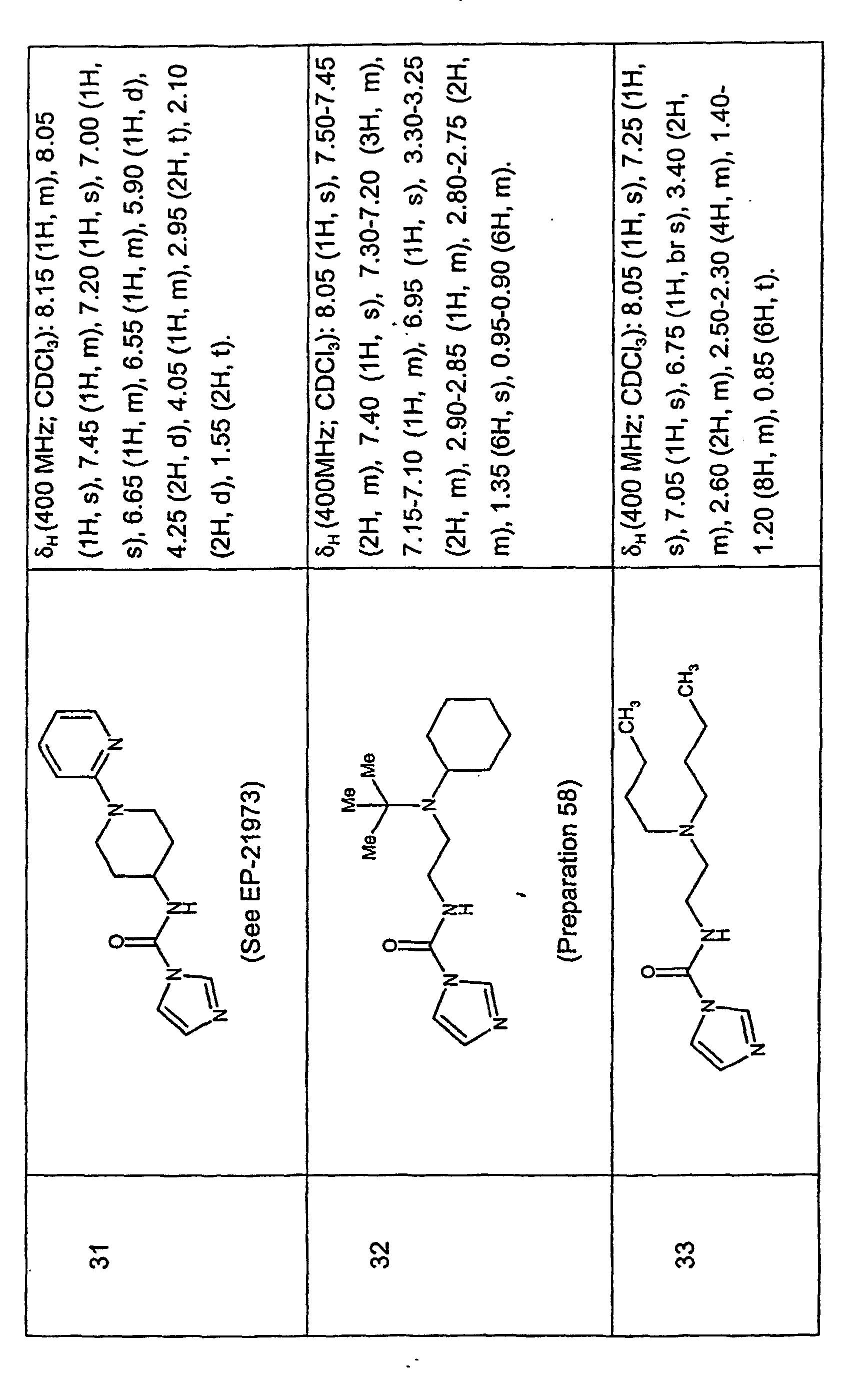

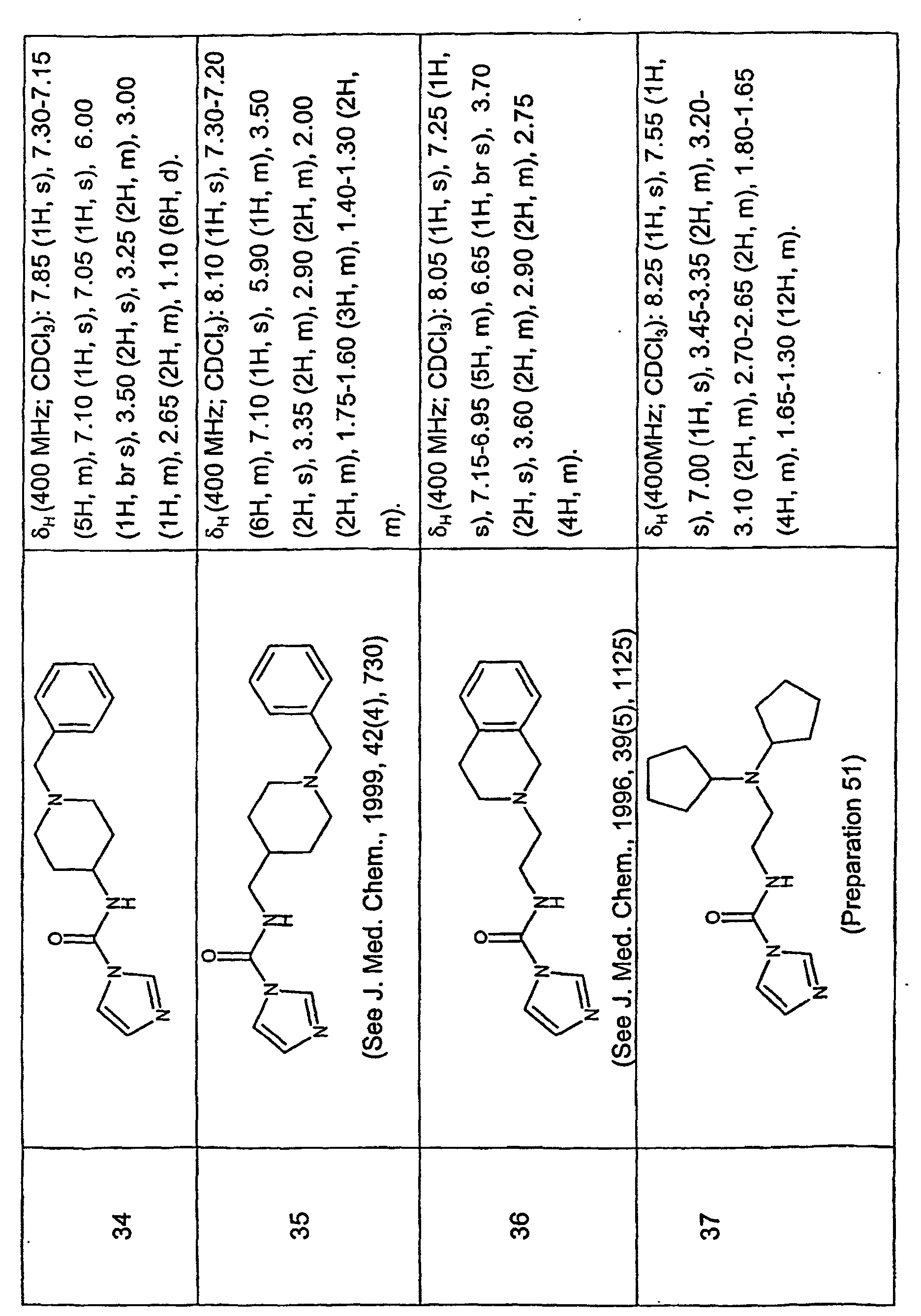

### PREPARATION 38

### N-Isopropylcyclopentanamine

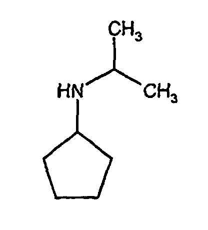

Pearlman's catalyst (20% w/w palladium hydroxide-on-carbon) (1.5g) was added to a solution of cyclopentylamine (15 ml, 0.21 mol) in acetone (200 ml). The reaction mixture was stirred under an atmosphere of hydrogen gas at 414kPa (60psi). After stirring for 16 hours the reaction mixture was filtered through Arbocel (Trade Mark) and the solvent was removed under reduced pressure to give the title compound (15 ml) as a thin oil.
δ_{H} (400MHz; CDCl₃): 3.20-3.10 (1H, m), 2.90-2.80 (1H, m), 1.95-1.85 (2H, m), 1.75-1.45 (4H, m), 1.35-1.20 (2H, m), 1.10-1.00 (6H, m).

### PREPARATION 39

### [Cyclopentyl(isopropyl)amino]acetonitrile

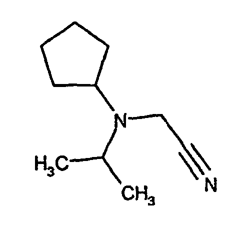

Hydroxyacetonitrile (8.2ml of a 70% w/w solution in water, 0.1mol) was added to a solution of *N*-isopropylcyclopentanamine (11.43g, 0.09mol) (Preparation 38) in ethanol (60ml). The reaction mixture was heated under reflux for 3 hours, allowed to cool and evaporated under reduced pressure. The residue was purified by chromatography on silica gel eluting with dichloromethane : methanol (98 : 2 by volume) to give the title compound (14.1g) as a clear oil.
δ_{H} (400MHz; CDCl₃): 3.60-3.50 (2H, s), 3.30-3.20 (2H, m), 2.00-1.85 (2H, m), 1.80-1.55 (4H, m), 1.45-1.30 (2H, m), 1.15-1.05 (6H, m).

### PREPARATION 40

### [Isopropyl(1-methyl-1-phenylethyl)amino]acetonitrile

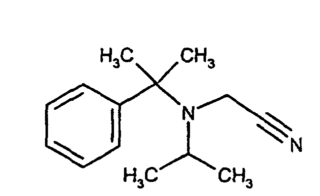

The title compound was prepared from *N*-isopropyl-2-phenyl-2-propanamine (Preparation 55) using a similar method to Preparation 39.
δ_{H} (400MHz; CDCl₃): 7.55-7.45 (2H, m), 7.35-7.30 (2H, m), 7.25-7.20 (1H, m), 3.60 (2H, m), 3.10-3.00 (1H, m), 1.60 (6H, m), 1.10-1.05 (6H, m).
m/z MH⁺ 217.

### PREPARATION 41

### (2,2,6,6-Tetramethyl-1-piperidinyl)acetonitrile

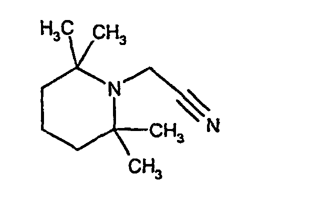

The title compound was prepared from 2,2,6,6-tetramethylpiperidine using a similar method to that of Preparation 39.
δ_{H} (400MHz; CDCl₃): 3.45 (2H, s), 1.55-1.50 (4H, m), 1.10 (12H, m).

### PREPARATION 42

### N¹-Cyclopentyl-N¹-isopropyl-1,2-ethanediamine

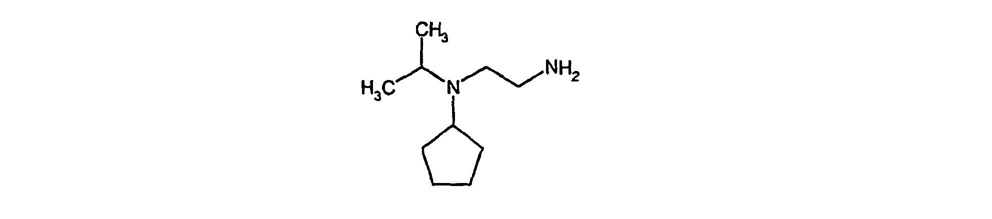

Lithium aluminium hydride (66ml of a 1 molar solution in tetrahydrofuran, 0.066mol) was added to a stirred solution of [cyclopentyl(isopropyl)amino]acetonitrile (10g, 0.66mol) (Preparation 39) in tetrahydrofuran (100ml) at 0°C. The reaction mixture was stirred at 0°C for 20 minutes and then heated under reflux for 2 hours. The reaction mixture was allowed to cool to room temperature and left to stand overnight. The reaction mixture was cooled in an icebath and treated dropwise with 4.8ml of a 7.5% w/w aqueous sodium hydroxide solution followed by 7.4 ml of water. The solvent was removed under reduced pressure and the residue was slurried with diethyl ether (200ml) for 30 minutes and then filtered. The filtrate was evaporated under reduced pressure to give the title compound as a colourless oil (10.30g).
δ_{H} (400MHz; CDCl₃): 3.10-2.95 (2H, m), 2.70-2.60 (2H, m), 2.50-2.40 (2H, m), 1.80-1.45 (10H, m), 1.05-0.95 (6H, m).
m/z MH⁺ 171.

### PREPARATION 43

### 2-(2,2,6,6-Tetramethyl-1-piperidinyl)ethanamine

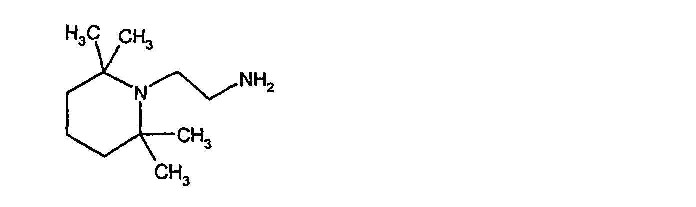

The title compound was prepared by a similar method to that of Preparation 42 using (2,2,6,6-tetramethyl-1-piperidinyl)acetonitrile (Preparation 41).
δ_{H} (400MHz; CDCl₃): 2.70-2.60 (2H, m), 2.50-2.40 (2H, m), 1.60-1.40 (4H, m), 1.40-1.30 (4H, m), 1.00 (12H, s).

### PREPARATION 44

### N¹-Isopropyl-N¹-(1-methyl-1-phenylethyl)-1,2-ethanediamine

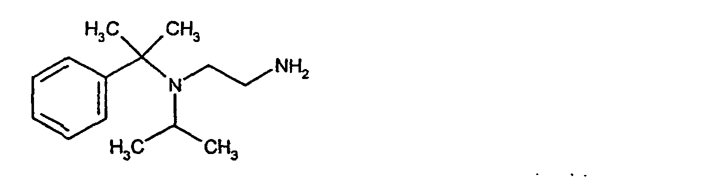

The title compound was prepared by a similar method to that of Preparation 42 using [isopropyl(1-methyl-1-phenylethyl)amino]acetonitrile (Preparation 40).
δ_{H} (400MHz; CDCl₃): 7.55-7.50 (2H, m), 7.30-7.25 (2H, m), 7.20-7.15 (1H, m), 2.90-2.80 (1H, m), 2.70-2.60 (4H, m), 1.35 (6H, m), 0.95-0.90 (6H, m).

### PREPARATION 45

### (2R,3R,4R,5R)-4-(Acetyloxyl-2-[(acetyloxy)methyl]-5-(6-chloro-2-cyano-9H-purin-9-yl)tetrahydro-3-furanyl acetate

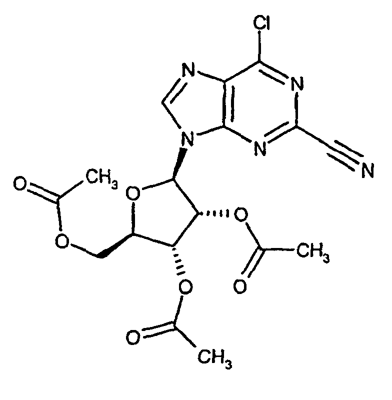

Copper (I) cyanide was added to a solution of (2*R*,3*R*,4*R*,5*R*)-4-(acetyloxy)-2-[(acetyloxy)methyl]-5-(2,6-dichloro-9*H*-purin-9-yl)tetrahydro-3-furanyl acetate (*J*. *Med. Chem*., 248, **35**, 1992) (22g, 40mmol) in N'N'-dimethylformamide (150ml). The suspension was heated to 100°C for 2 hours and was then allowed to cool to room temperature. The reaction mixture was poured into water (700ml) with stirring. The solid was filtered off and stirred in dichloromethane (700ml) for 30 minutes. The solid was filtered off again and then stirred in dichloromethane (700ml) for another 30 minutes. The dichloromethane portions were combined and evaporated under reduced pressure to a volume of 500ml. The dichloromethane was then dried over anhydrous sodium sulphate. The solvent was removed under reduced presure. The residue was redissolved in dichloromethane (300ml). The solvent was removed under reduced pressure. The residue was then stirred in diethylether (300ml) for 20 minutes. The solid was filtered off and dried to give the title compound (14.8g).
δ_{H} (300MHz; CDCl₃): 8.50 (1H, s), 6.10-6.05 (1H, m), 5.80-5.75 (1H, m), 5.55-5.50 (1H, m), 4.55-4.50 (1H, m), 4.50-4.40 (2H, m), 2.20-2.15 (6H, m), 2.10 (3H, s).

### PREPARATION 46

### (2R,3R,4S,5S)-2-(2-Amino-6-chloro-9H-purin-9-yl)-4-(benzoyloxy)-5-[(ethylamino)carbonyl]-tetrahydro-3-furanyl benzoate

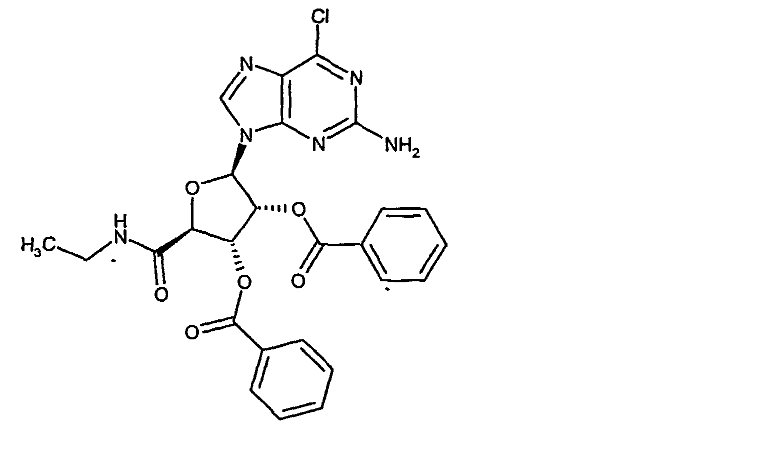

A suspension of 2-amino-6-chloropurine (4.60 g, 27.13 mmol) in 1,1,1-trichloroethane (230 ml) was treated with N,O-bis(trimethylsilyl)acetamide (20 ml, 81.4 mmol). The mixture was heated under reflux for 6 hours. The solution was allowed to cool to room temperature and the solvent was removed under reduced pressure. The residue was treated with a solution of (2*S*,3*S*,4*R*,5*R*)-5-(acetyloxy)-4-(benzoyloxy)-2-[(ethylamino)carbonyl]tetrahydro-3-furanyl benzoate and (2*S*,3*S*,4*R*,5*S*)-5-(acetyloxy)-4-(benzoyloxy)-2-[(ethylamino)carbonyl]tetrahydro-3-furanyl benzoate (Preparation 18) (14.39 g, 32.6 mmol) in anhydrous toluene (230 ml) and trimethylsilyl trifluoromethanesulfonate (20 ml, 108.5 mmol). The resulting solution was then heated at 90°C under a nitrogen atmosphere for 90 minutes. The mixture was cooled to room temperature, diluted with ethyl acetate (250 ml) and washed with a saturated aqueous solution of sodium hydrogen carbonate (350 ml) and then brine (350 ml). The organic layer was separated, dried over anhydrous magnesium sulphate, filtered and evaporated under reduced pressure. The residue was purified by column chromatography on silica gel eluting with dichloromethane : methanol (98 : 2 by volume) to afford the title compound as a foam (8.1 g).
m/z MH⁺ 552.
δ_{H}(400 MHz; CDCl₃): 8.10-7.95 (3H, m), 7.80 (2H, m), 7.50-7.30 (6H, m), 6.90 (1H, m), 6.40-6.20 (3H, m), 5.20 (2H, br s), 4.90 (1H, m), 3.45 (1H, m), 3.30 (1H, m), 1.15 (3H, t).

### PREPARATION 47

### (2R,3R,4S,5S)-4-(Benzoyloxy)-2-(6-chloro-2-iodo-9H-purin-9-yl)-5-[(ethylamino)carbonyl]-tetrahydro-3-furanyl benzoate

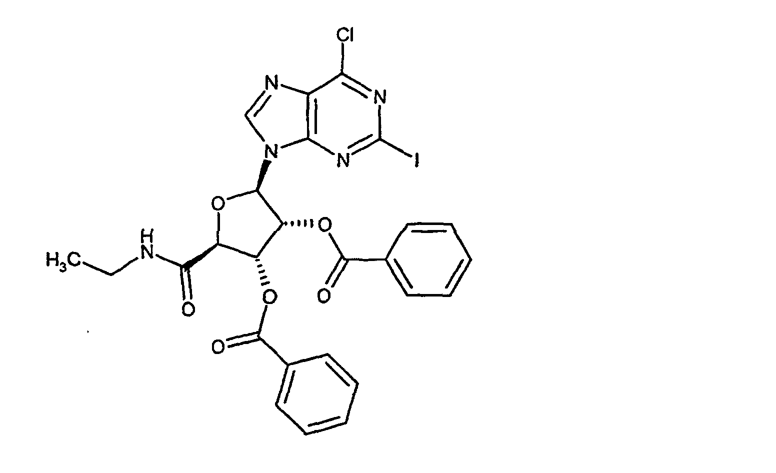

*n*-Butyl nitrite (4.65 ml, 39.7 mmol) was added to a suspension of (2*R*,3*R*,4*S*,5*S*)-2-(2-amino-6-chloro-9*H*-purin-9-yl)-4-(benzoyloxy)-5-[(ethylamino)carbonyl]-tetrahydro-3-furanyl benzoate (Preparation 46) (8.10 g, 14.7 mmol), iodine (3.73 g, 14.7 mmol), copper(I) iodide (6.16 g, 32.3 mmol) and diiodomethane (12.55 ml, 155.8 mmol) in THF (100 ml) and the mixture was heated under reflux for 2.5 hours. The solution was allowed to cool to room temperature and the solvent was removed under reduced pressure. The residue was partitioned between 5% w/w aqueous sodium metabisulfite solution (100 ml) and dichloromethane (100 ml). The organic layer was separated, filtered through Arbocel (Trade Mark), dried over anhydrous magnesium sulphate and evaporated under reduced pressure. The residue was purified by column chromatography on silica gel eluting with dichloromethane : methanol (99 : 1 by volume) to afford the title compound as a yellow foam (7.55 g).
m/z MNa⁺ 684.
δ_{H} (400 MHz; CDCl₃): 8.55 (1H, s), 8.05 (2H, m), 7.80 (2H, m), 7.65-7.30 (6H, m), 6.75 (1H, m), 6.50 (1H, m), 6.10-6.00 (2H, m), 4.90 (1H, m), 3.60-3.40 (2H, m), 1.25 (3H, t).

### PREPARATION 48

### N-{2-[(1-Ethylpropyl)(isobutyl)amino]ethyl}-1H-imidazole-1-carboxamide

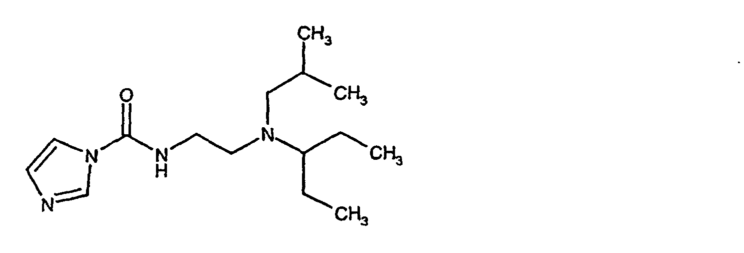

A suspension of Pearlman's catalyst (200mg) and benzyl 2-[(1-ethylpropyl)(isobutyl)amino]ethylcarbamate (1.3g, 4.06mmol) (Preparation 49) in ethyl acetate (20ml) was stirred under an atmosphere of hydrogen gas (414 kPa, 60psi) at room temperature for 12 hours. The reaction mixture was filtered through Arbocel (Trade Mark) and then the solvent was removed under reduced pressure. The crude material was dissolved in dichloromethane (10ml) and the resulting solution was added to a solution of N'N'-carbonyldiimidazole (0.66g, 4.10mmol) in dichloromethane (10ml). The reaction mixture was stirred at room temperature for 30 minutes and then diluted with dichloromethane (50ml). The solution was washed with water (30ml) and dried over anhydrous magnesium sulphate. The solvent was removed under reduced pressure and the residue was purified by column chromatography on silica gel eluting with ethyl acetate increasing in polarity to ethyl acetate : methanol (97 : 3 by volume). This gave the title compound as a gum (0.5g).
δ_{H} (400MHz; CDCl₃): 8.05 (1H, s). 7.25 (1H, s), 7.10 (1H, s), 6.45 (1H, bs), 3.45-3.40 (2H; m), 2.70-2.65 (2H, m), 2.45-2.40 (2H, m), 2.30-2.20 (1H, m), 1.60-1.50 (1H, m), 1.50-1.20 (4H, m), 0.95-0.85 (12H, m).

### PREPARATION 49

### Benzyl 2-[(1-ethylpropyl)(isobutyl)amino]ethylcarbamate.

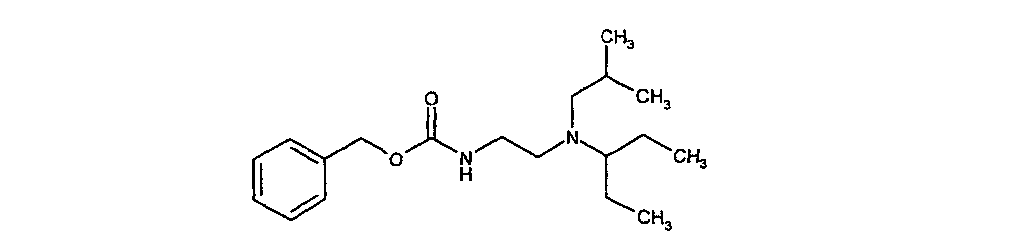

Sodium triacetoxyborohydride (1.87g, 8.84mmol) was added to a solution of benzyl 2-[(1-ethylpropyl)amino]ethylcarbamate (1.8g, 6.8mmol) (Preparation 50), 3-methylbutanal (0.8ml, 7.5mmol) and acetic acid (1ml, 8.85mmol) in dichloromethane (50ml). The reaction mixture was stirred for 16 hours at room temperature. The reaction mixture was diluted with dichloromethane (50ml) and then washed with saturated aqueous sodium hydrogen carbonate solution (100ml). The organic phase was dried over anhydrous magnesium sulphate and then the solvent was removed under reduced pressure. The residue was purified by column chromatography on silica gel eluting with dichloromethane : methanol (95 : 5 by volume) increasing in polarity to dichloromethane : methanol : 0.88 concentrated aqueous ammonia (95 : 5 : 0.5 by volume). This gave the title compound as an oil (1.3g).
δ_{H} (400MHz; CDCl₃): 7.40-7.25 (5H, m), 5.30-5.20 (1H, m), 5.10-5.05 (2H, m), 3.25-3.15 (2H, m), 2.60-2.50 (2H, m), 2.45-2.35 (2H, m), 2.25-2.15 (1H, m), 1.65-1.50 (1H, m), 1.50-1.20 (5H, m), 1.00-0.80 (12H, m).

### PREPARATION 50

### Benzyl 2-[(1-ethylpropyl)amino]ethylcarbamate

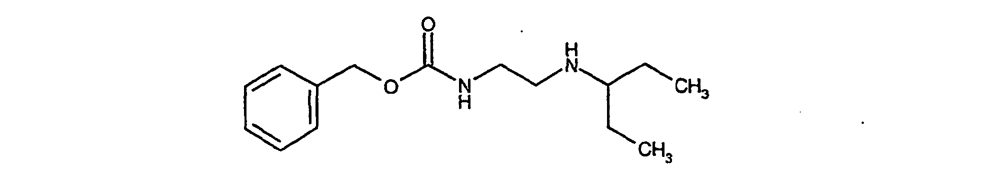

Benzyl 2-aminoethylcarbamate hydrochloride (2g, 8.65mmol) was dissolved in dichloromethane (50ml) and 3-pentanone (3.7ml, 35mmol) was added. The reaction mixture was stirred for 1 Hour at room temperature. Sodium triacetoxy borohydride (5.5g, 26mmol) was added and the reaction mixture was then stirred for 16 hours at room temperature. The solution was washed with saturated aqueous sodium hydrogen carbonate solution (40ml). The dichloromethane phase was dried over anhydrous magnesium sulphate and the solvent was removed. The residue was purified by column chromatography on silica gel eluting with dichloromethane : methanol : 0.88 concentrated aqueous ammonia (95 : 5 : 0.5 by volume). This gave the title compound as an oil (1.8g).
δ_{H} (300MHz; CDCl₃): 7.20-7.10 (5H, m), 5.30 (1H, bs), 5.10 (2H, s), 3.35-3.20 (2H, m), 2.85-2.70 (2H, m), 2.40-2.30 (1H, m), 1.50-1.30 (4H, m), 0.90-0.80 (6H, m).

### PREPARATION 51

### N¹,N¹-Dicyclopentyl-1,2-ethanediamine hydrochloride

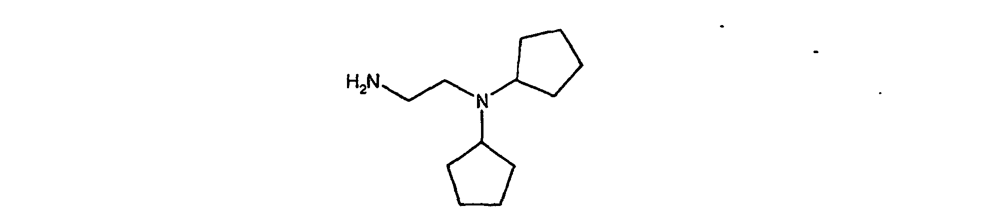

Hydrogen chloride gas was passed through a stirred ice cold solution of *tert*-butyl 2-(dicyclopentylamino)ethylcarbamate (0.22g, 0.74mmol) (Preparation 52) in dichloromethane (15ml) until the solution was saturated. The reaction mixture was allowed to warm to room temperature and then stirred for 1 hour. The solvent was removed under reduced pressure to give the title compound (0.15g) as a brown foam.
m/z MH⁺ 197.
δ_{H} (400MHz; CDCl₃): 10.80 (1H, s), 8.75 (3H, s), 3.80-3.60 (6H, m), 2.25-1.50 (16H, m).

### PREPARATION 52

### tert-Butyl 2-(dicyclopentylamino)ethylcarbamate

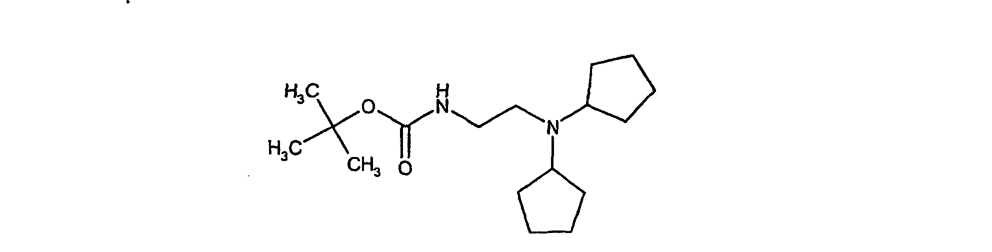

lodocyclopentane (1.68g, 8.6mmol) was added to a suspension of potassium carbonate (1.8g, 13.2mmol) and *tert*-butyl 2-[cyclopentylamino]ethylcarbamate (1.5g, 6.6mmol) (Preparation 53) in N',N'-dimethylformamide (10ml). The reaction mixture was stirred at 60°C for 72 hours. The reaction mixture was allowed to cool and was then partitioned between ethyl acetate (50ml) and water (50ml). The ethyl acetate layer was washed with brine (30ml) and dried over anhydrous magnesium sulphate. The solvent was removed to give a residue that was purified by chromatography on silica gel eluting with dichloromethane : methanol (98 : 2 by volume) increasing in polarity to dichloromethane : methanol : 0.88 concentrated aqueous ammonia (90 : 10 : 1 by volume) to give the title compound (64mg) as a brown oil.
m/z MH⁺ 297
δ_{H}(400MHz; CDCl₃): 5.10-4.90 (1H, m), 3.30-3.05 (4H, m), 2.65-2.50 (2H, m), 1.85-1.30 (25H, m).

### PREPARATION 53

### tert-Butyl 2-[cyclopentylamino]ethylcarbamate

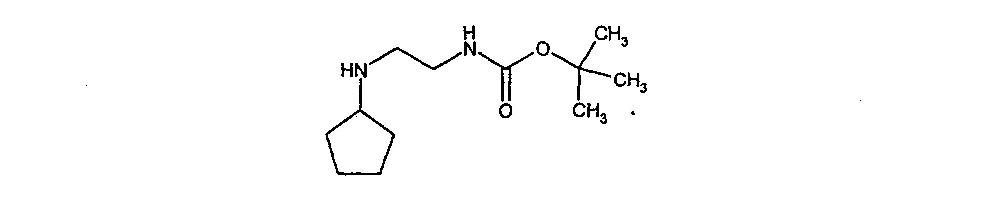

*tert*-Butyl 2-aminoethylcarbamate (3.0g, 18.8mmol) (Preparation 54) was dissolved in cyclopentanone (30ml). Pearlman's Catalyst (0.1g) was added and the reaction mixture was stirred under an atmosphere of hydrogen gas (414 kPa, 100psi) for 48 hours. The catalyst was filtered off through Arbocel (Trade Mark) and the solvent was removed under reduced pressure. The residue was purified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume). The solvent was removed under reduced pressure to give the title compound (1.5g) as an oil.
m/z MH⁺ 229
δ_{H}(400MHz; CDCl₃): 4.90 (1H, s), 3.25-3.10 (2H, m), 3.05-2.95 (1H, m), 2.70-2.60 (2H, m), 1.85-1.75 (2H, m), 1.70-1.20 (17H, m).

### PREPARATION 54

### tert-Butyl 2-aminoethylcarbamate

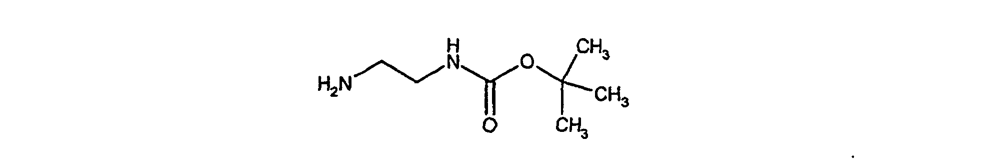

A solution of di(*tert*-butyl) dicarbonate (27.3g, 0.125mol) in dichloromethane (100ml) was added dropwise to a solution of ethylenediamine (30g, 0.5mol) over an hour. The reaction mixture was stirred for a further hour at room temperature. The solvent was then removed under reduced pressure and the residue was partitioned between ethyl acetate (600ml) and 5% w/w aqueous sodium hydroxide solution (200ml). The ethyl acetate layer was washed with water (100ml) and brine (100ml) and dried over anhydrous sodium sulphate. The solvent was removed under reduced pressure to give the title compound as a white solid (19.1g).
δ_{H} (60MHz; CDCl₃): 3.30-2.60 (4H, m), 1.45-1.30 (9H, m), 1.20-1.05 (2H, m).

### PREPARATION 55

### N-Isopropyl-2-phenyl-2-propanamine

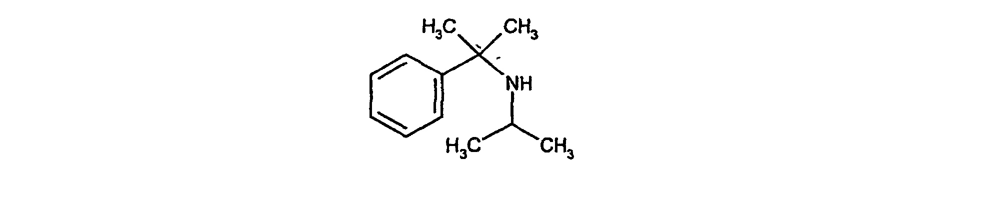

Sodium triacetoxyborohydride (4.5g, 21.2mmol) was added portionwise to a solution of 1-methyl-1-phenylethylamine (0.96g, 7.1mmol) in a mixture of acetone (5ml) and dichloromethane (120ml). The reaction mixture was stirred at room temperature for 16 hours. The solvent was removed under reduced pressure and the residue was partitioned between aqueous sodium hydroxide solution (2M, 100ml) and ethyl acetate (200ml). The ethyl acetate layer was washed with water (100ml) and brine (100ml) and then dried over anhydrous magnesium sulphate.
The solvent was removed under reduced pressure to give the title compound (1g) as a colourless oil.
δ_{H} (400MHz; CDCl₃): 7.40-7.35 (2H, m), 7.30-7.20 (2H, m), 7.15-7.10 (1H, m), 3.40 (1H, bs), 2.60-2.50 (1H, m), 1.40 (6H, s), 0.90-0.85 (6H, m).

### PREPARATION 56

### 2-[2-(4-Isopropyl-1-piperidinyl)ethyl]-1H-isoindole-1,3(2H)-dione

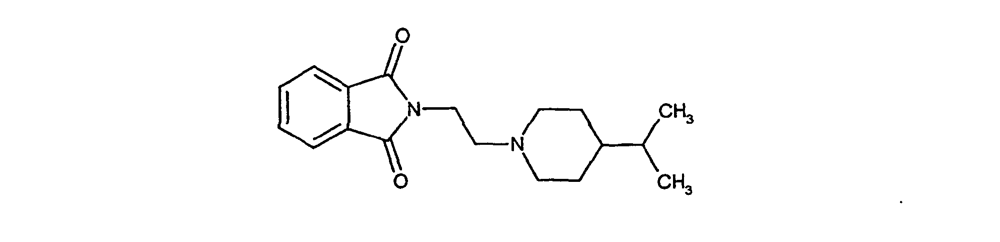

A solution of 4-isopropylpiperidine (3.3 g, 20.2 mmol), N-(2-bromoethyl)phthalimide (5.4 g, 21.3 mmol), potassium carbonate (5.9 g, 45.4 mmol) and acetonitrile (100 ml) and was heated under reflux for 2.5 hours and then stirred at room temperature overnight. The solvent was removed under reduced pressure and the residue was partitioned between ethyl acetate (100 ml) and water (100 ml). The organic layer was separated and the aqueous layer was extracted with further ethyl acetate (100 ml). The combined organic extracts were dried over anhydrous sodium sulphate and the solvent was removed by evaporation under reduced pressure. The resulting oil was purified by column chromatography on silica gel eluting with a gradient system of dichloromethane changing to dichloromethane : diethyl ether (50 : 50, by volume) changing to diethyl ether to afford the title compound (3.3 g).
m/z MH⁺ 301
δ_{H} (400 MHz, CDCl₃): 7.80 (2H, m), 7.70 (2H, m), 3.80 (2H, t), 3.00 (2H, m), 2.60 (2H, t), 1.95 (2H, m), 1.60 (2H, m), 1.40 (1H, m), 1.20 (2H, qd), 0.95 (1H, m), 0.80 (6H, d).

### PREPARATION 57

### 2-(4-Isopropyl-1-piperidinyl)ethylamine

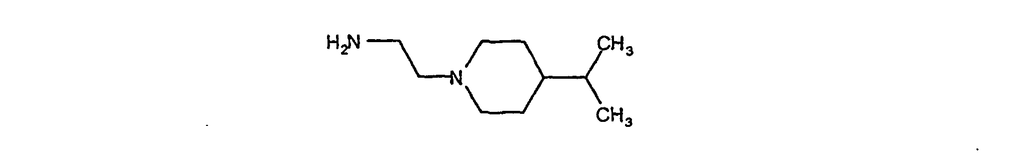

A solution of 2-[2-(4-isopropyl-1-piperidinyl)ethyl]-1*H*-isoindole-1,3(2*H*)-dione (Preparation 56) (3.2 g, 10.6 mmol) in a 33 % w/w solution of methylamine in ethanol (60 ml) was heated under reflux for three hours. The solvent was removed under reduced pressure, more ethanol was added (60 ml) and the solvent was again removed under reduced pressure. The residue was suspended in dichloromethane (100 ml) and the solid was filtered off. This was washed with dichloromethane (100 ml). The filtrate was evaporated under reduced pressure and the resulting oil was purified by column chromatography on silica gel eluting with dichloromethane : methanol : 0.88 aqueous ammonia solution (90 : 10 : 1, by volume) to give a colourless oil. Bulb-to-bulb distillation (150-160 °C, 4kPa) yielded the title compound (1.0 g, 55 %).
m/z MH⁺ 171.
δ_{H}(400 MHz; CDCl₃): 2.90 (2H, m), 2.80 (2H, t), 2.40 (2H, t), 1.95 (2H, m), 1.65 (2H, m), 1.40 (1H, m), 1.30-1.20 (4H, m), 1.00 (1H, m), 0.85 (6H, d).

### PREPARATION 58

### N¹-(tert-Butyl)-N¹-cyclohexyl-1,2-ethanediamine

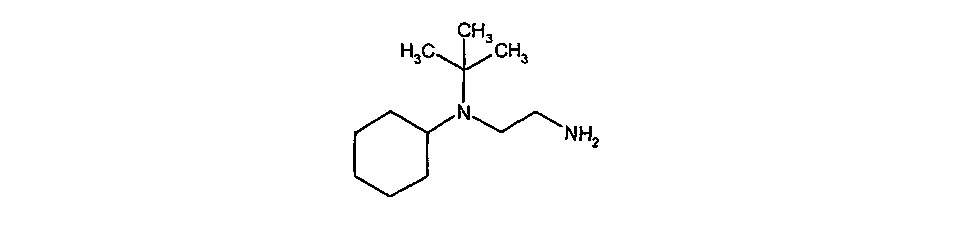

Hydroxyacetonitrile (5.3ml of of a 70% w/w solution in water, 32mmol) was added to a stirred solution of *N*-(*tert*-butyl)cyclohexanamine (5.3ml, 32mmol) in ethanol (50ml) at room temperature. The reaction mixture was heated under reflux for 16 hours. The solution was allowed to cool to room temperature and the solvent was removed under reduced pressure. The residue was partitioned between dichloromethane (50ml) and water (50ml). The organic layer was dried over anhydrous magnesium sulphate and the solvent was removed under reduced pressure. The crude residue was dissolved in tetrahydrofuran (30ml) and a solution of lithium aluminium hydride in tetrahydrofuran (1M, 55ml, 55mmol) was slowly added. The reaction mixture was heated under reflux for 2 hours. An aqueous solution of sodium hydroxide (2M, 6ml) was then carefully added. The suspension was filtered and the liquid was concentrated under reduced pressure to give the title compound (3g) as an oil.
δ_{H} (300MHz; CDCl₃): 2.90-2.70 (1H, m), 2.70-2.55 (2H, m), 2.20-1.95 (2H, m), 1.85-1.55 (4H, m), 1.40-1.15 (4H, m), 1.15-0.95 (11H, m).

### PREPARATION 59

### (2R,3R,4R,5R)-4-(Acetyloxy)-2-[(acetyloxy)methyl]-5-(6-{[2,2-bis(4-methylphenyl)ethyl]amino}-2-cyano-9H-purin-9-yl)tetrahydro-3-furanyl acetate

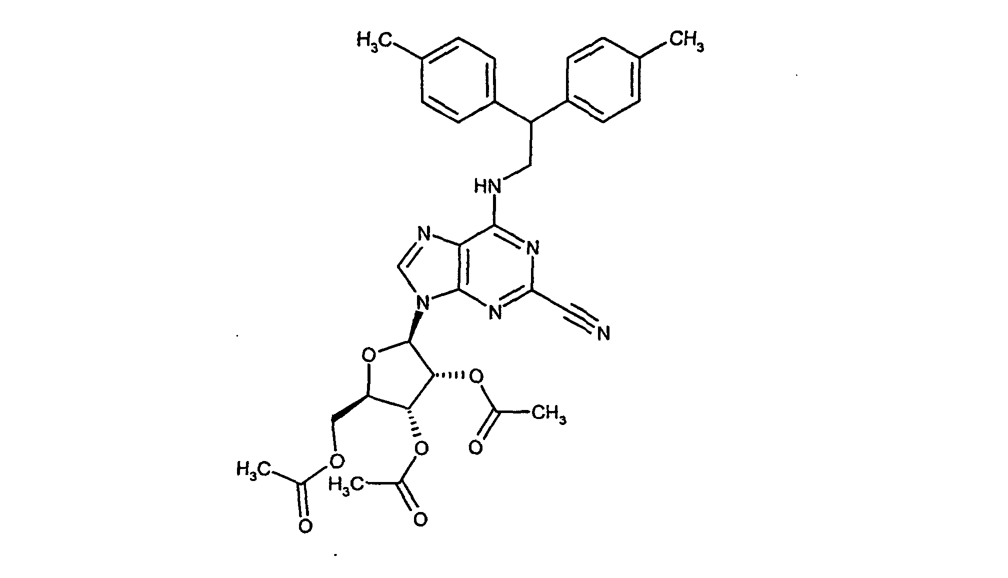

The compound was prepared from (2*R*,3*R*,4*R*,5*R*)-4-(acetyloxy)-2-[(acetyloxy)methyl]-5-(6-chloro-2-cyano-9*H*-purin-9-yl)tetrahydro-3-furanyl acetate (Preparation 45) and 2,2-bis(4-methylphenyl)ethanamine (J. Med. Chem., 1969, 12(1), 9) using a similar method to that of Preparation 64.
δ_{H} (400MHz; CDCl₃): 7.95 (1H, s), 7.20-7.05 (8H, m), 6.15-6.10 (1H, m), 5.95-5.90 (1H, m), 5.75-5.70 (1H, m), 5.55-5.50 (1H, m), 4.50-4.35 (3H, m), 4.30-4.20 (2H, m), 2.30 (6H, m), 2.20-2.00 (9H, m).

### PREPARATION 60

### (2R,3R,4S,5R)-2-(2-(Aminomethyl)-6-{[2,2-bis(4-methylphenyl)ethyl]amino}-9H-purin-9-yl)-5-(hydroxymethyl)tetrahydro-3,4-furandiol

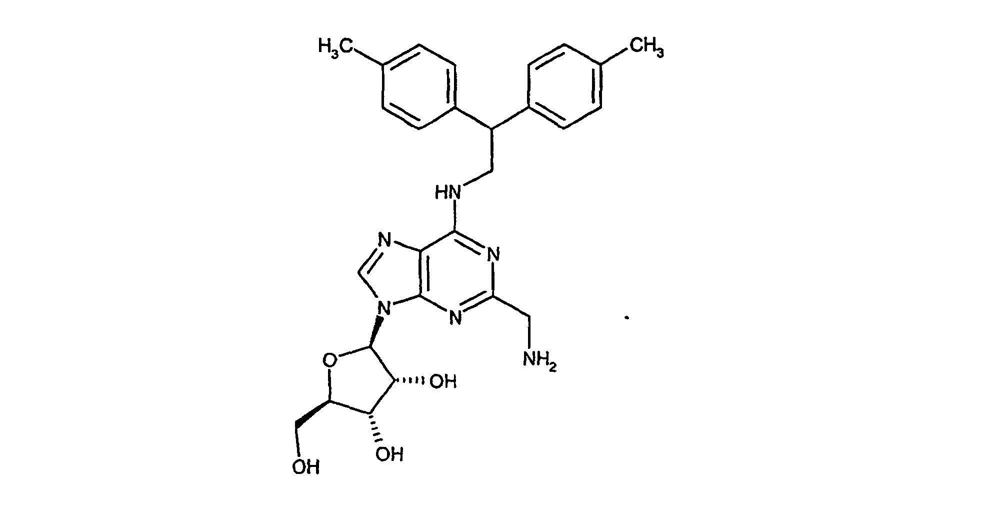

(2*R*,3*R*,4*R*,5*R*)-4-(Acetyloxy)-2-[(acetyloxy)methyl]-5-(6-{[2,2-bis(4-methylphenyl)ethyl]amino}-2-cyano-9*H*-purin-9-yl)tetrahydro-3-furanyl acetate (Preparation 59) (837mg, 1.33mmol) was dissolved in a saturated solution of ammonia in ethanol (25ml). 10% Palladium on carbon (168mg) was added and the suspension was stirred under an atmosphere of hydrogen (414 kPa, 60psi) for 16 hours. The reaction mixture was filtered through Arbocel (Trade Mark) and the filtrate was evaporated under reduced pressure. The residue was dissolved in methanol (100ml) and sodium carbonate (100mg) was added. The suspension was stirred at room temperature for 2 hours. The solvent was removed under reduced pressure and the residue was dissolved in a mixture of dichloromethane (15ml) and water (15ml). The dichloromethane layer was washed with water (15ml) and saturated aqueous sodium chloride solution. The solvent was removed under reduced pressure. The residue was triturated with diethyl ether to give the title compound as a solid (340mg).
m/z MH⁺ 503.
δ_{H} (400MHz; CDCl₃): 8.10 (1H, s), 7.20-7.10 (4H, m), 7.05-7.00 (4H, m), 5.90-5.85 (1H, m), 4.70-4.65 (1H, m), 4.40-4.35 (1H, m), 4.35-4.05 (3H, m), 3.85-3.80 (3H, m), 3.70-3.65 (1H, m), 2.05 (6H, m).

### PREPARATION 61

### (2R,3R,4R,5R)-4-(Acetyloxy)-2-[(acetyloxy)methyl]-5-(6-{[2,2-bis(3-chlorophenyl)ethyl]amino}-2-cyano-9H-purin-9-yl)tetrahydro-3-furanyl acetate

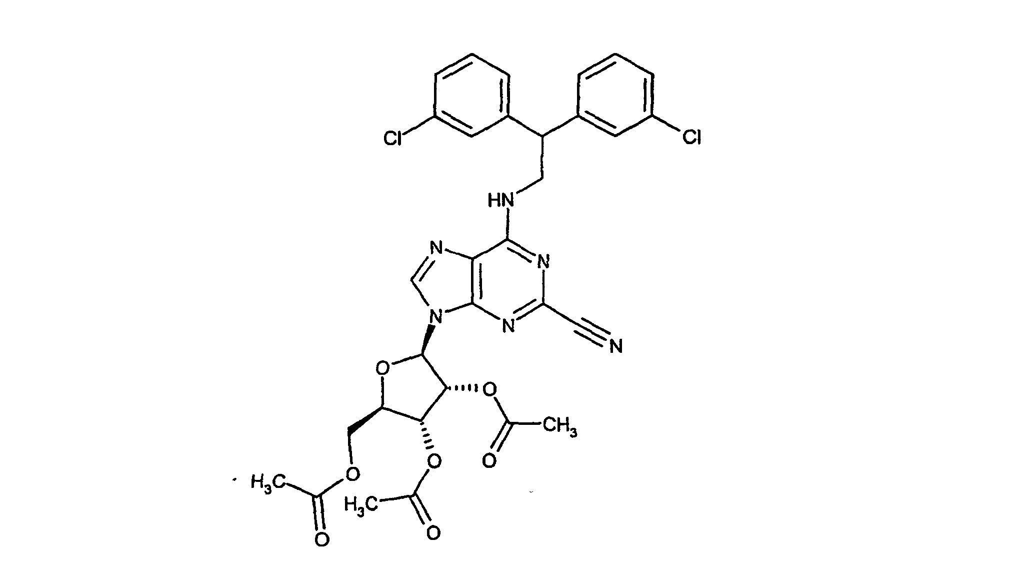

The compound was prepared from (2*R*,3*R*,4*R*,5*R*)-4-(acetyloxy)-2-[(acetyloxy)methyl]-5-(6-chloro-2-cyano-9*H*-purin-9-yl)tetrahydro-3-furanyl acetate (Preparation 45) and 2,2-bis(3-chlorophenyl)ethanamine (J. Med. Chem., 1988, 31(7), 1282) using a similar method to that of Preparation 64.
δ_{H} (400MHz; CDCl₃): 8.00 (1H, s), 7.30-7.10 (8H, m), 6.20-6.10 (1H, m), 5.80-5.70 (1H, m), 5.60-5.50 (1H, m), 4.50-4.30 (4H, m), 4.30-4.15 (2H, m), 2.20-2.00 (9H, m).

### PREPARATION 62

### (2R,3R,4S,5R)-2-(2-(Aminomethyl)-6-{[2,2-bis(3-chlorophenyl)ethyl]amino}-9H-purin-9-yl)-5-(hydroxymethyl)tetrahydro-3,4-furandiol

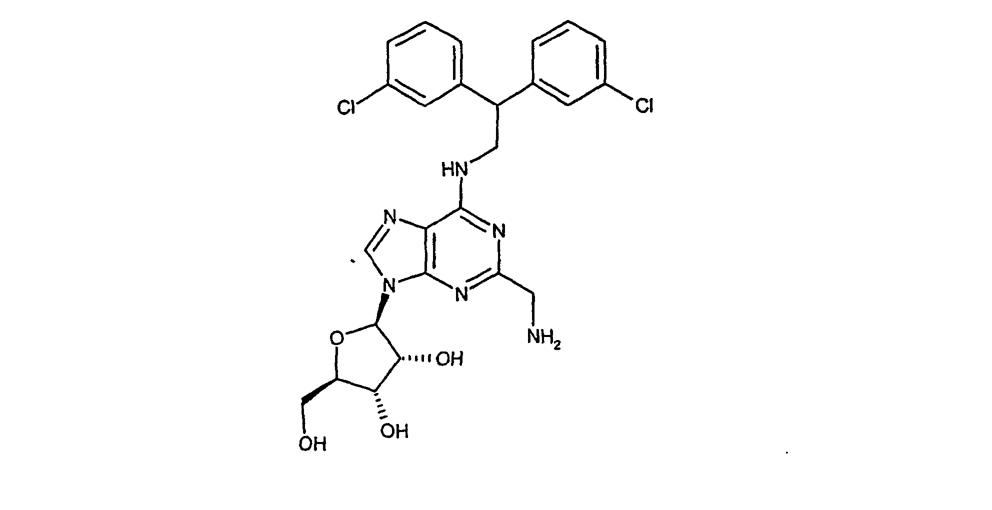

The title compound was prepared from (2*R*,3*R*,4*R*,5*R*)-4-(acetyloxy)-2-[(acetyloxy)methyl]-5-(6-{[2,2-bis(3-chlorophenyl)ethyl]amino}-2-cyano-9*H*-purin-9-yl)tetrahydro-3-furanyl acetate (Preparation 61) by the method used in Preparation 60.
δ_{H} (400MHz; CD₃OD): 8.15-8.10 (1H, m), 7.35-7.10 (8H, m), 5.90-5.85 (1H, m), 4.70-4.65 (1H, m), 4.40-4.20 (3H, m), 4.10-4.05 (1H, m), 3.90-3.80 (3H, m), 3.70-3.65 (3H, m).

### PREPARATION 63

### (2R,3R,4S,5R)-2-(2-(Aminomethyl)-6-{[2,2-bis(4-chlorophenyl)ethyl]amino}-9H-purin-9-yl)-5-(hydroxymethyl)tetrahydro-3,4-furandiol

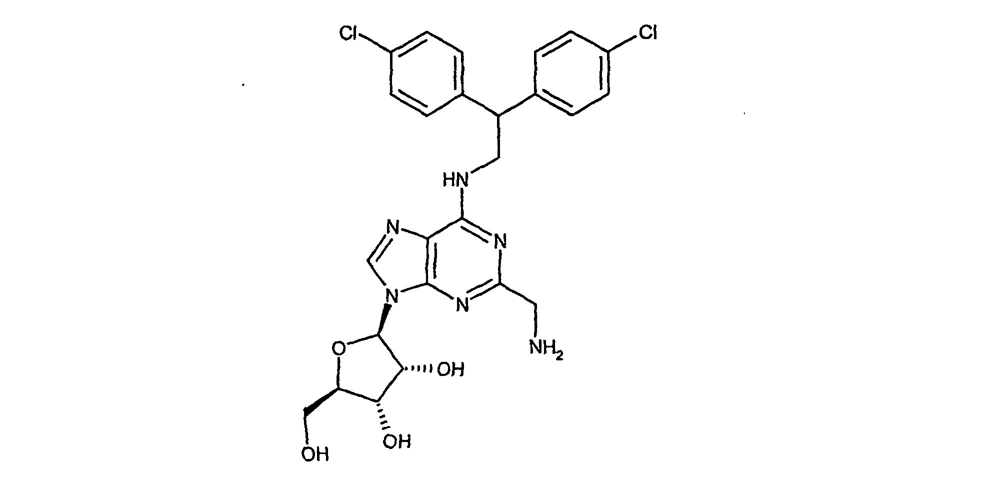

A solution of (2*R*,3*R*,4*R*,5*R*)-4-(acetyloxy)-2-[(acetyloxy)methyl]-5-(6-{[2,2-bis(4-chlorophenyl)ethyl]amino}-2-cyano-9*H*-purin-9-yl)tetrahydro-3-furanyl acetate (700mg, 1 mmol) (Preparation 64) in ethanol (20ml) was saturated with ammonia gas. 10% Palladium on carbon (140mg) was added and the reaction mixture stirred under an atmosphere of hydrogen gas (414 kPa, 60psi) at room temperature for 16 hours. The Palladium on carbon was filtered off through Arbocel (Trade Mark) and the filtrate was evaporated under reduced pressure. The residue was dissolved in methanol (50ml) and sodium carbonate (60mg, 0.57mmol) was added. The reaction mixture was stirred for 1.5 hours and then the solvent was removed under reduced pressure. The residue was purified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 2 by volume). The solvent was removed under reduced pressure to give the title compound (275mg) as a foam.
m/z MH⁺ 545.
δ_{H} (400MHz; CD₃OD): 8.10 (1H, s), 7.30-7.20 (8H, m), 7.20-7.10 (1H, m), 5.90-5.85 (1H, m), 4.70-4.65 (1H, m), 4.50-4.40 (1H, m), 4.30-4.15 (3H, m), 4.15-4.10 (1H, m), 3.90-3.80 (3H, m), 3.70-3.65 (1H, m).

### PREPARATION 64

### (2R,3R,4R,5R)-4-(Acetyloxy)-2-[(acetyloxy)methyl]-5-(6-{[2,2-bis(4-chlorophenyl)ethyl]amino}-2-cyano-9H-purin-9-yl)tetrahydro-3-furanyl acetate

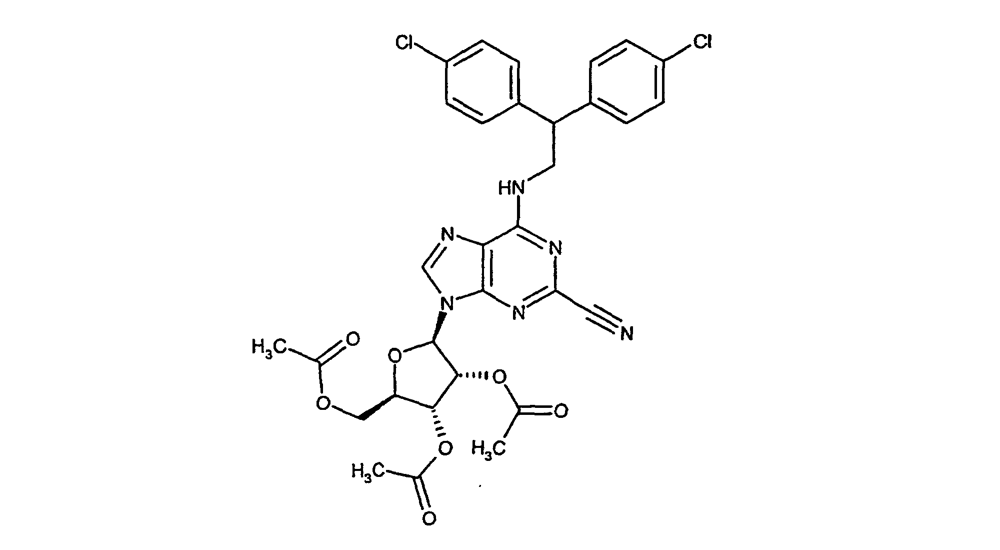

Triethylamine (0.2ml, 1.14mmol) was added to a stirred solution of (2*R*,3*R*,4*R*,5*R*)-4-(acetyloxy)-2-[(acetyloxy)methyl]-5-(6-chloro-2-cyano-9*H*-purin-9-yl)tetrahydro-3-furanyl acetate (500mg, 1.14mmol) (Preparation 45) and 2,2-bis(4-chlorophenyl)ethanamine (919mg, 1.2mmol) (J. Am. Chem. Soc., 1983, 105(10), 3138) in acetonitrile (5ml) at room temperature. The reaction mixture was stirred for 16 hours. The solvent was then removed under reduced pressure and the residue was dissolved in dichloromethane (20ml). The solution was washed with water (10ml) and saturated sodium chloride solution (10ml), dried over anhydrous magnesium sulphate and evaporated under reduced pressure. The residue was purified by column chromatography on silica gel eluting with dichloromethane : methanol : (99 :1 by volume) increasing in polarity to dichloromethane : methanol (98.5 : 1.5 by volume). The solvent was removed under reduced pressure to give the title compound (698mg) as a foam.
m/z MH⁺ 667.
δ_{H} (400MHz; CD₃OD): 7.95 (1H, s), 7.35-7.10 (8H, m), 6.15-6.10 (1H, m), 5.95-5.85 (1H, m), 5.75-5.70 (1H, m), 5.55-5.50 (1H, m), 4.45-4.30 (4H, m), 4.30-4.10 (2H, m), 2.20-2.05 (9H, m).

### PREPARATION 65

### (2R,3R,4S,5R)-2-(2-(Aminomethyl)-6-{[2,2-bis(3-methylphenyl)ethyl]amino}-9H-purin-9-yl)-5-(hydroxymethyl)tetrahydro-3,4-furandiol

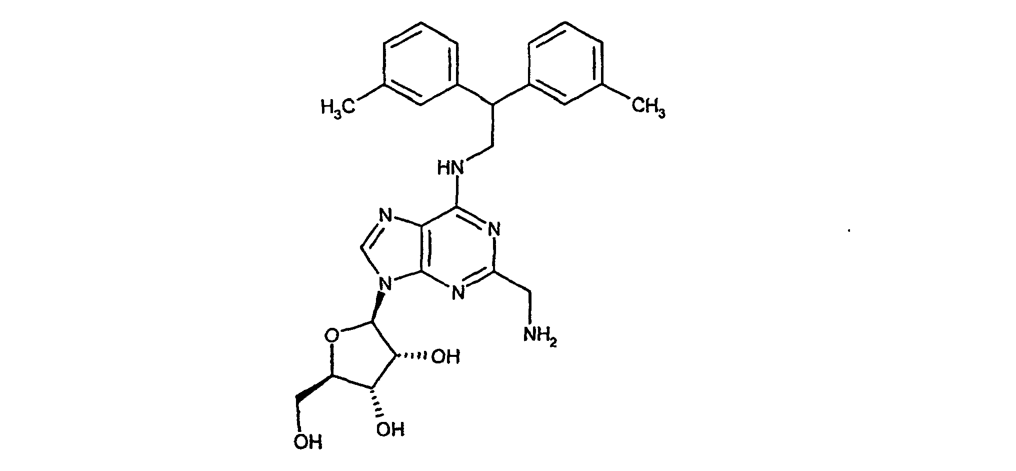

(2*R*,3*R*,4*R*,5*R*)-4-(Acetyloxy)-2-[(acetyloxy)methyl]-5-(6-{[2,2-bis(3-methylphenyl)ethyl]amino}-2-cyano-9*H*-purin-9-yl)tetrahydro-3-furanyl acetate (620mg, 1mmol) (Preparation 66) was dissolved in a saturated solution of 0.88 concentrated aqueous ammonia in ethanol (20ml). The solution was then stirred under an atmosphere of hydrogen gas (414 kPa, 60psi) at room temperature for 64 hours in the presence of 10% Palladium on carbon (120mg). The reaction mixture was then filtered through Arbocel (Trade Mark) and the filtrate was evaporated to dryness. The residue was purified by column chromatography on silica gel eluting with dichloromethane : methanol : (90 :10 by volume) increasing in polarity to dichloromethane : methanol : 0.88 concentrated aqueous ammonia (80 : 20 : 2 by volume). The solvent was removed under reduced pressure to give the title compound (253mg) as a foam.
m/z MH⁺ 503.
δ_{H} (400MHz; CD₃OD): 8.10 (1H, s), 7.20-7.05 (6H, m), 7.05-6.90 (2H, m), 5.95-5.90 (1H, m), 4.70-4.65 (1H, m), 4.40-4.35 (1H, m), 4.35-4.20 (2H, m),4.20-4.10 (1H, m), 3.90-3.80 (2H, m), 3.70-3.65 (1H, m), 2.05 (6H, m).

### PREPARATION 66

### (2R,3R,4R,5R)-4-(Acetyloxy)-2-[(acetyloxy)methyl]-5-(6-{[2,2-bis(3-methylphenyl)ethyl]amino}-2-cyano-9H-purin-9-yl)tetrahydro-3-furanyl acetate

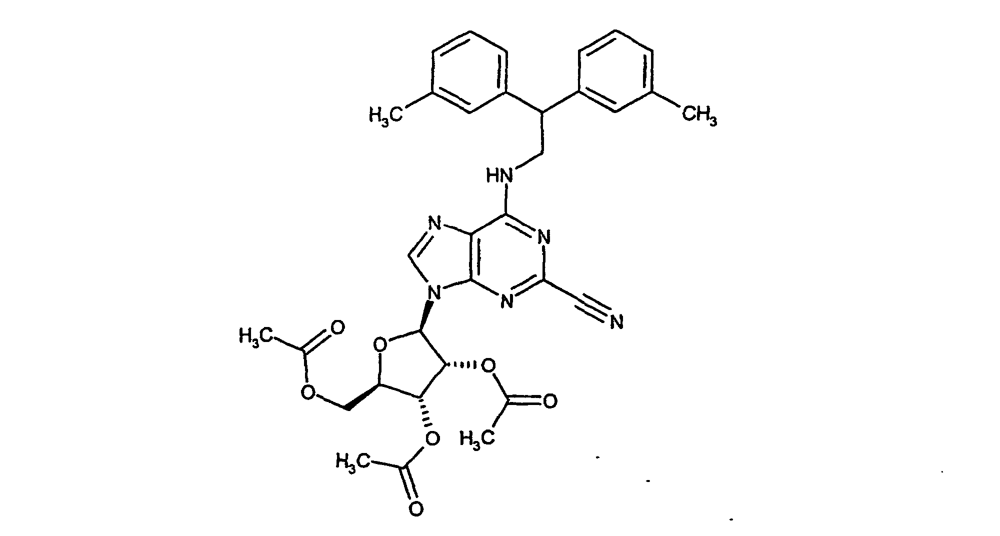

The title compound was prepared from (2*R*,3*R*,4*R*,5*R*)-4-(acetyloxy)-2-[(acetyloxy)methyl]-5-(6-chloro-2-cyano-9*H*-purin-9-yl)tetrahydro-3-furanyl acetate (Preparation 45) and 2,2-bis(3-methylphenyl)ethanamine (J. Med. Chem., 1988, 31 (7), 1282) using a similar procedure to that of Preparation 64.
m/z MH⁺ 627.
δ_{H} (400MHz; CD₃OD): 7.95 (1H, s), 7.25-7.15 (2H, m), 7.10-7.00 (6H, m), 6.15-6.10 (1H, m), 5.95-5.90 (1H, m), 5.80-5.70 (1H, m), 5.60-5.55 (1H, m), 4.50-4.35 (3H, m), 4.30-4.20 (3H, m), 3.30 (6H, s), 2.20-2.05 (9H, m).

### PREPARATION 67

### (2R,3R,4S,5S)-4-(Benzoyloxy)-2-(6-{[2,2-bis(4-chlorophenyl)ethyl]amino}-2-iodo-9H-purin-9-yl)-5-[(ethylamino)carbonyl]tetrahydro-3-furanyl benzoate

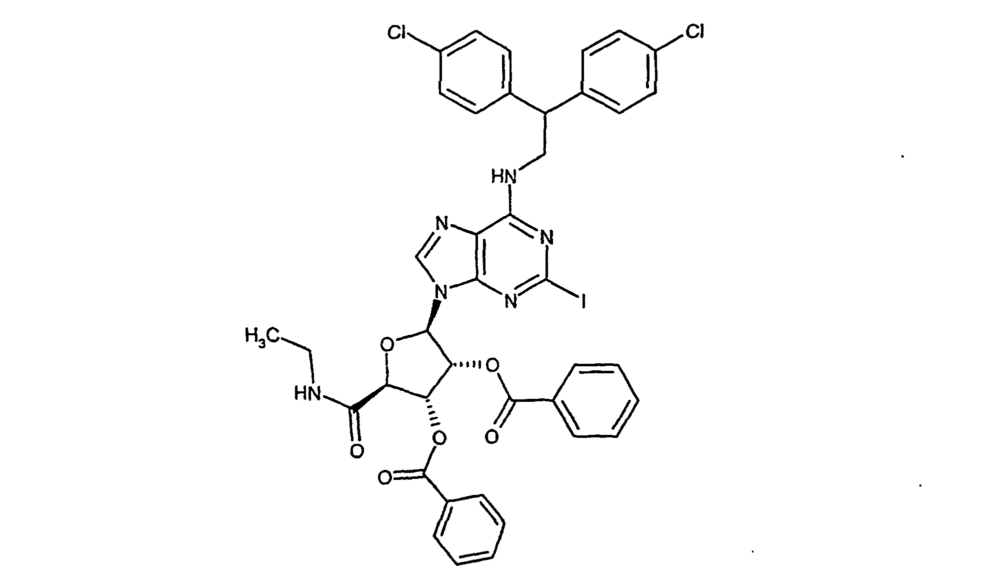

2,2-Bis(4-chlorophenyl)ethanamine (426mg, 1.51 mmol) (J. Am. Chem. Soc., 1983, 105(10), 3138) was added to a stirred solution of (2*R*,3*R*,4*S*,5*S*)-4-(benzoyloxy)-2-(6-chloro-2-iodo-9*H*-purin-9-yl)-5-[(ethylamino)carbonyl]tetrahydro-3-furanyl benzoate (500mg, 0.755mmol) (Preparation 47) in isopropyl alcohol (20ml) at room temperature. The reaction mixture was stirred at room temperature for 48 hours. The solvent was removed under reduced pressure to give a residue that was purified by column chromatography on silica gel eluting with dichloromethane : methanol : (99 :1 by volume). The solvent was removed under reduced pressure to give the title compound (329mg) as a foam.
m/z MH⁺ 891.
δ_{H} (400MHz; CD₃OD): 8.10-8.00 (2H, m), 7.85-7.75 (3H, m), 7.65-7.55 (1H, m), 7.55-7.40 (4H, m), 7.30-7.15 (8H, m), 6.30-6.20 (1H, m), 6.15-6.10 (1H, m), 6.10-6.05 (1H, m), 5.85-5.75 (1H, m), 4.90 (1H, s), 4.40-4.30 (2H, m), 4.25-4.10 (2H, m), 3.75-3.60 (1H, m), 3.60-3.45 (1H, m), 1.30-1.20 (3H, m).

### PREPARATION 68

### (2R,3R,4S,5S)-4-(Benzoyloxy)-2-(6-{[2,2-bis(4-chlorophenyl)ethyl]amino}-2-cyano-9H-purin-9-yl)-5-[(ethylamino)carbonyl]tetrahydro-3-furanyl benzoate

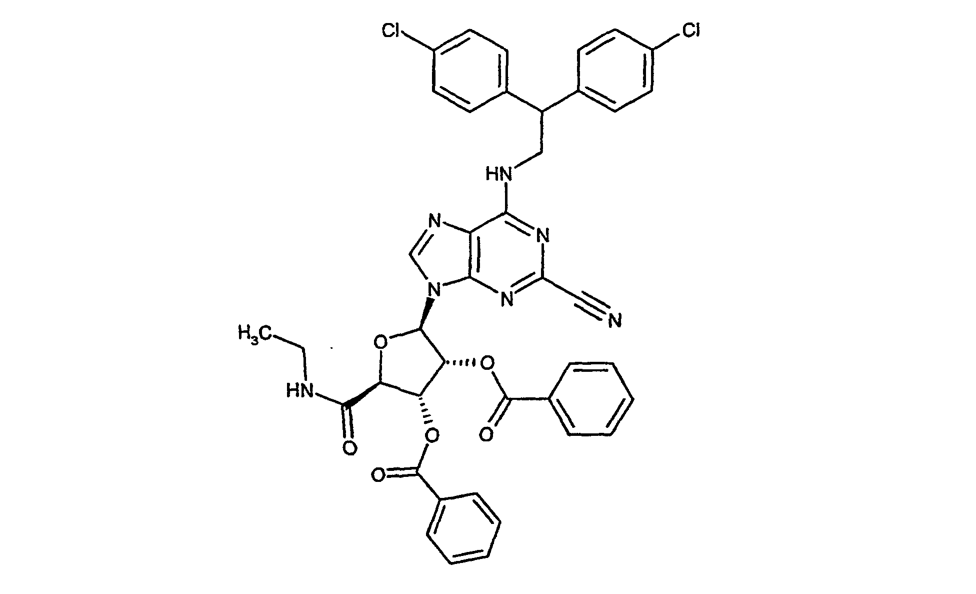

Copper (I) cyanide was added to a solution of (2*R*,3*R*,4*S*,5*S*)-4-(benzoyloxy)-2-(6-{[2,2-bis(4-chlorophenyl)ethyl]amino}-2-iodo-9*H*-purin-9-yl)-5-[(ethylamino)carbonyl]tetrahydro-3-furanyl benzoate (440mg, 0.494mmol) (Preparation 67) in N'N'-dimethylformamide (10ml). The reaction mixture was stirred at 90°C overnight. The reaction mixture was poured into water (15ml). The precipitate was filtered off and stirred in dichloromethane (50ml) for 10 minutes. The dichloromethane was filtered, dried over anhydrous magnesium sulphate and evaporated under reduced pressure. The residue was purified by column chromatography on silica gel eluting with dichloromethane : methanol (98 : 2 by volume). The solvent was removed under reduced pressure to give the title compound (227mg) as a foam.
δ_{H} (400MHz; CD₃OD): 8.20 (1H, m), 8.10-8.00 (3H, m), 7,85-7.75 (2H, m), 7.65-7.60 (1H, m), 7.60-7.40 (4H, m), 7.35-7.15 (8H, m), 6.40-6.35 (1H, m), 6.30-6.20 (1H, m), 6.10-6.00 (2H, m), 4.90 (1H, m), 4.45-4.35 (1H, m), 4.30-4.15 (2H, m), 3.65-3.45 (2H, m), 1.30-1.20 (3H, m).

### PREPARATION 69

### (2S,3S,4R,5R)-5-{2-(Aminomethyl)-6-[(1-naphthylmethyl)amino]-9H-purin-9-yl}-N-ethyl-3,4-dihydroxytetrahydro-2-furancarboxamide

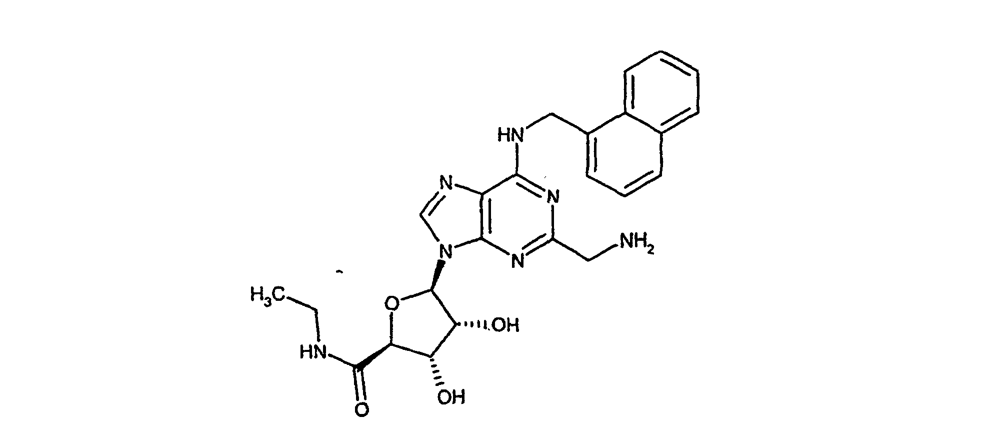

10% Palladium on carbon was added to a solution of (2*R*,3*R*,4*S*,5*S*)-4-(benzoyloxy)-2-{2-cyano-6-[(1-naphthylmethyl)amino]-9*H*-purin-9-yl}-5-[(ethylamino)carbonyl]tetrahydro-3-furanyl benzoate (487mg, 0.71 mmol) (Preparation 70) dissolved in a saturated solution of ammonia in ethanol. The reaction mixture was stirred for 16 hours under an atmosphere of hydrogen gas (414 kPa, 60psi). The reaction mixture was then filtered through Arbocel (Trade Mark) and the solvent was removed under reduced pressure. The residue was purified by column chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (80 : 20 : 2 by volume). The solvent was removed under reduced pressure to give the title compound (240mg) as a foam.
δ_{H} (400MHz; D₆DMSO): 8.45-8.20 (4H, m), 7.90-7.85 (1H, m), 7.80-7.75 (1H, m), 7.55-7.45 (3H, m), 7.45-7.35 (1H, m), 5.95-5.90 (1H, m), 5.65-5.55 (1H, m), 5.55-5.45 (1H, m), 5.20-5.10 (2H, m), 4.65-4.55 (1H, m), 4.25 (1H, s), 4.15-4.10 (1H, m), 4.10-3.90 (1H, m), 3.65 (2H, m), 3.20-3.00 (2H, m), 1.05-0.90 (3H, m).

### PREPARATION 70

### (2R,3R,4S,5S)-4-(Benzoyloxy)-2-{2-cyano-6-[(1-naphthylmethyl)amino]-9H-purin-9-yl}-5-[(ethylamino)carbonyl]tetrahydro-3-furanyl benzoate

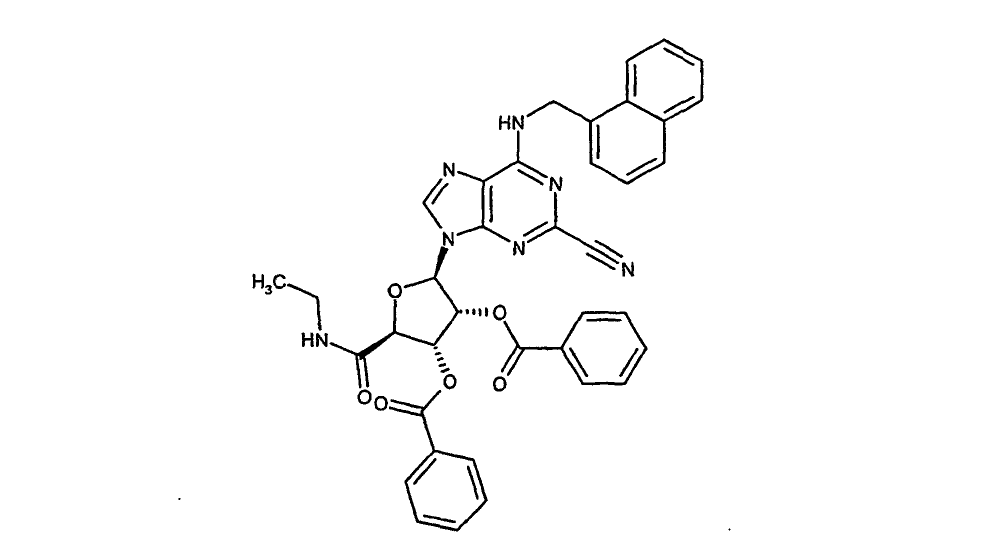

Diazabicycloundecane (0.18ml, 1.2mmol) was added to a solution of (6-[(1-naphthylmethyl)amino]-9*H*-purine-2-carbonitrile (200mg, 0.67mmol) (Preparation 10) and (2*S*,3*S*,4*R*,5*R*)-5-(acetyloxy)-4-(benzoyloxy)-2-[(ethylamino)carbonyl]tetrahydro-3-furanyl benzoate and (2*S*,3*S*,4*R*,5*S*)-5-(acetyloxy)-4-(benzoyloxy)-2-[(ethylamino)carbonyl]tetrahydro-3-furanyl benzoate (309mg, 0.70mmol) (Preparation 18) in acetonitrile (5ml). Trimethylsilyl trifluoromethanesulfonate (0.24ml, 1.33mmol) was added and the reaction mixture heated under reflux for 30 minutes. The reaction mixture was then allowed to cool to room temperature, diluted with ethyl acetate (20ml) and washed twice with water (20ml). The ethyl acetate layer was then washed with 10% w/w aqueous citric acid and saturated aqueous sodium chloride solution (20ml). The organic phase was dried over anhydrous magnesium sulphate and evaporated under reduced pressure. The residue was purified by column chromatography on silica gel eluting with dichloromethane increasing in polarity to dichloromethane : methanol (98 :2 by volume). The solvent was removed under reduced pressure to give the title compound (235mg) as a foam.
δ_{H} (400MHz; d₆DMSO): 9.20-9.10 (1H, m), 8.75 (1H, m), 8.30-8.20 (2H, m), 8.00-7.90 (3H, m), 7.80-7.70 (3H, m), 7.70-7.60 (1H, m), 7.60-7.30 (8H, m), 6.70-6.60 (1H, m), 6.30-6.20 (1H, m), 6.10-6.05 (1H, m), 5.15-5.10 (2H, m), 4.90-4.85 (1H, m), 3.20-3.05 (2H, m), 1.00-0.90 (3H, m).

### PREPARATION 71

### (2R,3R,4R,5R)-4-(Acetyloxy)-2-[(acetyloxy)methyl]-5-{2-cyano-6-[(9H-fluoren-9-ylmethyl)amino]-9H-purin-9-yl}tetrahydro-3-furanyl acetate

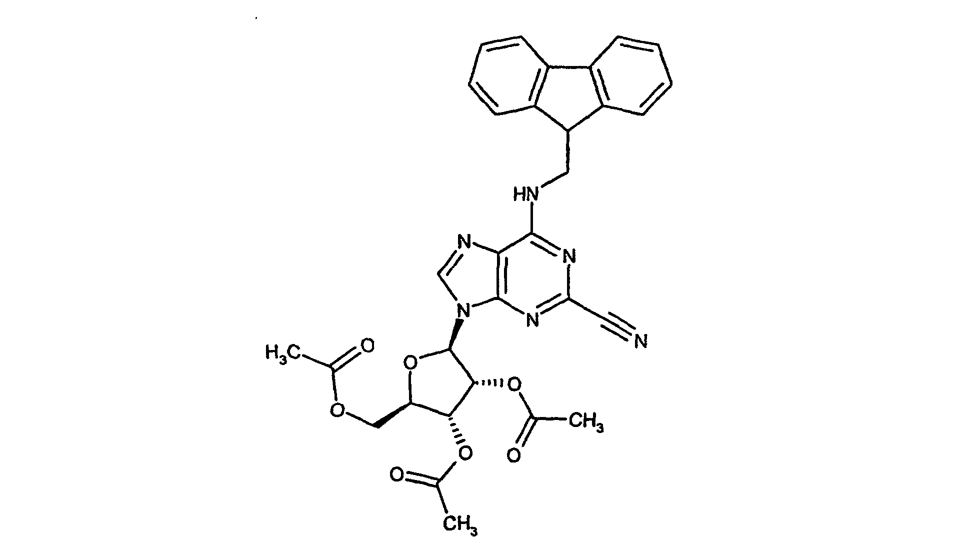

Triethylamine (0.38ml, 2.7mmol) was added to a stirred solution of (2*R*,3*R*,4*R*,5*R*)-4-(acetyloxy)-2-[(acetyloxy)methyl]-5-(6-chloro-2-cyano-9*H*-purin-9-yl)tetrahydro-3-furanyl acetate (1.0g, 2.3mmol) (Preparation 45) and 9*H*-fluoren-9-ylmethanamine (0.49g, 2.5mmol) (J. Org. Chem., 1971, 36(23), 3539) in acetonitrile (30ml) at room temperature. The reaction mixture was stirred at room temperature for 16 hours. The solvent was removed under reduced pressure. The residue was partially redissoved in ethyl acetate (30ml). The ethyl acetate was washed with water (1ml) and a saturated aqueous sodium chloride solution (10ml). The organic phase was evaporated under reduced pressure and the residue was purified by column chromatography on silica gel eluting with dichloromethane : ethyl acetate (96 : 4 by volume) increasing in polarity to dichloromethane : ethyl acetate (94 :6 by volume). The solvent was removed under reduced pressure to give the title compound (1.23g) as a foam.
m/z MH⁺ 595.
δ_{H} (300MHz; CDCl₃): 8.00 (1H, s), 7.80-7.75 (2H, m), 7.65-7.60 (2H, m), 7.45-7.30 (4H, m), 6.20-6.10 (1H, m), 6.05-5.95 (1H, m), 5.80-5.75 (1H, m), 5.60-5.55 (1H, m), 4.50-4.30 (4H, m), 4.30-4.20 (2H, m), 2.20-2.05 (9H, m).

### PREPARATION 72

### (2S,3S,4R,5R)-5-{6-[(2,2-Diphenylethyl)amino]-2-[(methylamino)methyl]-9H-purin-9-yl}-N-ethyl-3,4-dihydroxytetrahydro-2-furancarboxamide

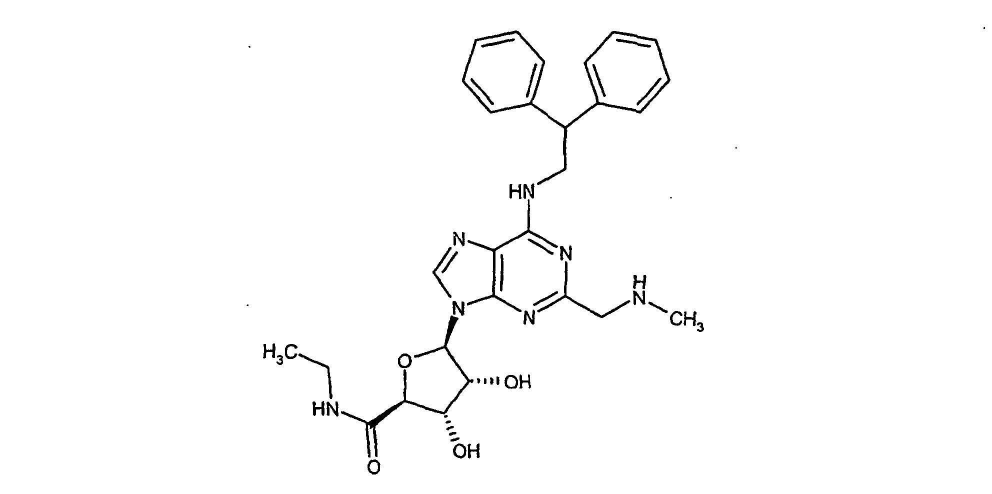

10% Palladium on carbon (0.2g) was added to a solution of (2*S*,3*R*,4*R*,5*R*)-4-(benzoyloxy)-5-{2-cyano-6-[(2,2-diphenylethyl)amino]-9*H*-purin-9-yl}-2-[(ethylamino)carbonyl]tetrahydro-3-furanyl benzoate (1.0g, 1.4mmol) (Preparation 7) in 33% w/w methylamine in ethanol (75ml). The reaction mixture was stirred under an atmosphere of hydrogen gas (4414 kPa, 60psi) for 16 hours at room temperature. The solid was filtered off and the solvent was removed from the filtrate under reduced pressure. The residue was purified by flash chromatography on silica gel eluting with dichloromethane : methanol : 0.88 concentrated aqueous ammonia (95 : 5 : 0.5 by volume) increasing in polarity to dichloromethane : methanol : 0.88 concentrated aqueous ammonia (90 : 10 : 1 by volume) to give the title compound (0.52g) as a solid.
m/z MH⁺ 532.
δ_{H}(400MHz; CDCl₃): 8.25 (1H, s), 7.35-7.20 (8H, m), 7.20-7.10 (2H, m), 6.05-6.00 (1H, m), 4.55-4.45 (1H, m), 4.45 (2H, m), 4.40-4.20 (2H, m), 3.80 (2H, m), 3.40-3.20 (2H, m), 2.45 (3H, m), 1.15-1.05 (3H, m).

### PREPARATION 73

### N-[2-(1-Piperidinyl)ethyl]-1H-imidazole-1-carboxamide

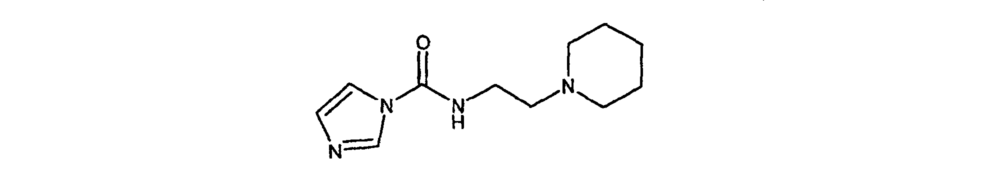

2-(1-Piperidinyl)ethylamine (1.28 g, 10 mmol) was added to a stirred solution of N,N'-carbonyldiimidazole (1.62 g, 10 mmol) in THF (25 ml) at room temperature. The reaction mixture was stirred overnight and the solvent was then removed by evaporation under reduced pressure. The residue was partitioned between ethyl acetate (100 ml) and water (50 ml) and the ethyl acetate layer was separated, washed with brine (30 ml) and dried with anhydrous sodium sulphate. Evaporation of the solvent under reduced pressure yielded the title compound as a white solid (1.8 g).
δ_{H}(400 MHz; CDCl₃): 8.10 (1H, s), 7.35 (1H, s), 7.10 (1H, s), 6.80 (1H, br s), 3.45 (2H, m), 2.55 (2H, m), 2.50-2.30 (4H, m), 1.60-1.40 (6H, m).

### PHARMACOLOGICAL ACTIVITY

All the compounds of the Examples were tested for anti-inflammatory activity by their ability to inhibit neutrophil function (which indicates A2a receptor agonist activity) by the method described on page 42 and all had an IC₅₀ of less than 1 micromolar.

## Claims

1. A compound of the formula 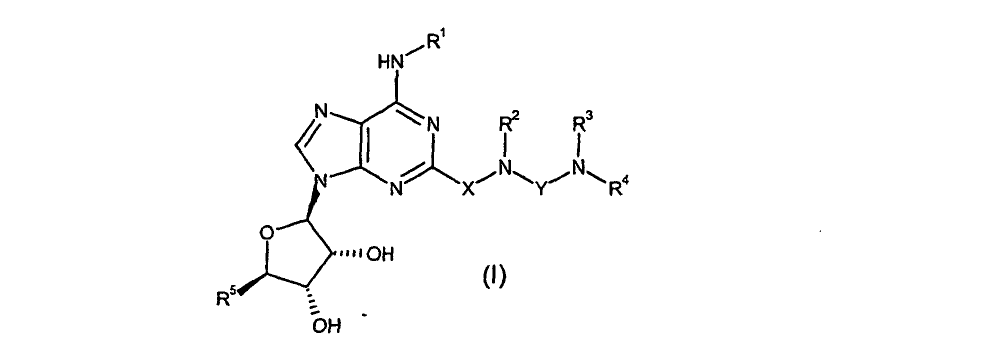 or a pharmaceutically acceptable salt or solvate thereof, wherein
R¹ is (i) H, (ii) C₁-C₆ alkyl optionally substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano, or (iii) fluorenyl;
R² is H or C₁-C₆ alkyl;
either, R³ and R⁴, taken together with the nitrogen atom to which they are attached, represent azetidinyl, pyrrolidinyl, piperidinyl, piperazinyl, homopiperidinyl or homopiperazinyl, each being optionally substituted on a ring nitrogen or carbon atom by C₁-C₆ alkyl or C₃-C₈ cycloalkyl and optionally substituted on a ring carbon atom not adjacent to a ring nitrogen atom by -NR⁶R⁷ or -OR⁹,
or, R³ is H, C₁-C₆ alkyl, C₃-C₈ cycloalkyl or benzyl, said C₁-C₆ alkyl being optionally substituted by C₃-C₈ cycloalkyl, and R⁴ is
(a) C₁-C₆ alkyl, C₃-C₈ cycloalkyl or R¹⁵, said C₁-C₆ alkyl being optionally substituted by R¹⁵, or
(b) -(C₂-C₆ alkylene)-R⁸, or
(c) -(C₁-C₆ alkylene)-R¹³;
R⁵ is -CH₂OH or -CONR¹⁴R¹⁴;
R⁶ and R⁷ are either each independently H or C₁-C₆ alkyl or, taken together with the nitrogen atom to which they are attached, represent azetidinyl, pyrrolidinyl or piperidinyl, said azetidinyl, pyrrolidinyl and piperidinyl being optionally substituted by C₁-C₆ alkyl;
R⁸ is
(i) azetidin-1-yl, pyrrolidin-1-yl, piperidin-1-yl, morpholin-4-yl, piperazin-1-yl, homopiperidin-1-yl, homopiperazin-1-yl or tetrahydroisoquinolin-1-yl, each being optionally substituted on a ring carbon atom by C₁-C₆ alkyl, C₃-C₈ cycloalkyl, phenyl, C₁-C₆ alkoxy-(C₁-C₆)-alkyl, R⁹R⁹N-(C₁-C₆)-alkyl, fluoro-(C₁-C₆)-alkyl, -CONR⁹R⁹, -COOR⁹ or C₂-C₅ alkanoyl and optionally substituted on a ring carbon atom not adjacent to a ring nitrogen atom by fluoro-(C₁-C₆)-alkoxy, halo, -OR⁹, cyano, -S(O)ₘR¹⁰, -NR⁹R⁹, -SO₂NR⁹R⁹, -NR⁹COR¹⁰ or -NR⁹SO₂R¹⁰, and said piperazin-1-yl and homopiperazin-1-yl being optionally substituted on the ring nitrogen atom not attached to the C₂-C₆ alkylene group by C₁-C₆ alkyl, phenyl, C₁-C₆ alkoxy-(C₂-C₆)-alkyl, R⁹R⁹N-(C₂-C₆)-alkyl, fluoro-(C₁-C₆)-alkyl, C₂-C₅ alkanoyl, -COOR¹⁰, C₃-C₈ cycloalkyl, -SO₂R¹⁰, -SO₂NR⁹R⁹ or -CONR⁹R⁹, or
(ii) -NR¹¹R¹²;
R⁹ is H, C₁-C₆ alkyl, C₃-C₈ cycloalkyl or phenyl;
R¹⁰ is C₁-C₆ alkyl, C₃-C₈ cycloalkyl or phenyl;
R¹¹ is C₁-C₆ alkyl, C₃-C₈ cycloalkyl or benzyl;
R¹² is C₁-C₆ alkyl, C₃-C₈ cycloalkyl, phenyl, benzyl, fluoro-(C₁-C₆)-alkyl, -CONR⁹R⁹, -COOR¹⁰, -COR¹⁰, -SO₂R¹⁰ or -SO₂NR⁹R⁹, said C₁-C₆ alkyl being optionally substituted by phenyl;
R¹³ is phenyl, pyridin-2-yl, pyridin-3-yl or pyridin-4-yl, each being optionally substituted by C₁-C₆ alkyl, C₁-C₆ alkoxy, halo or cyano;
R¹⁴ is H or C₁-C₆ alkyl optionally substituted by cyclopropyl;
R¹⁵ is azetidin-3-yl, pyrrolidin-3-yl, piperidin-3-yl, piperidin-4-yl, homopiperidin-3-yl or homopiperidin-4-yl, each being optionally substituted by R¹³, C₁-C₆ alkyl, C₃-C₈ cycloalkyl or benzyl;
m is 0, 1 or 2;
X is -CH₂- or -CH₂CH₂-; and
Y is CO, CS, SO₂ or C=N(CN).

2. A compound of the formula (I), as defined in claim 1, wherein
R¹ is H, C₁-C₆ alkyl or fluorenyl, said C₁-C₆ alkyl being optionally substituted by 1 or 2 substituents each independently selected from phenyl and naphthyl, said phenyl and naphthyl being optionally substituted by C₁-C₆ alkyl, C₁-C₆ alkoxy, halo or cyano;
R² is H or C₁-C₆ alkyl;
either, R³ and R⁴, taken together with the nitrogen atom to which they are attached, represent azetidinyl, pyrrolidinyl, piperidinyl, piperazinyl, homopiperidinyl or homopiperazinyl, each being optionally substituted on a ring nitrogen or carbon atom by C₁-C₆ alkyl or C₃-C₈ cycloalkyl and optionally substituted on a ring carbon atom not adjacent to a ring nitrogen atom by -NR⁶R⁷,
or, R³ is H, C₁-C₆ alkyl, C₃-C₈ cycloalkyl or benzyl and R⁴ is
(a) azetidin-3-yl, pyrrolidin-3-yl, piperidin-3-yl, piperidin-4-yl, homopiperidin-3-yl or homopiperidin-4-yl, each being optionally substituted by C₁-C₆ alkyl, C₃-C₈ cycloalkyl or benzyl, or
(b) -(C₂-C₆ alkylene)-R⁸, or
(c) -(C₁-C₆ alkylene)-R¹³;
R⁵ is -CH₂OH or -CONR¹⁴R¹⁴;
R⁶ and R⁷ are either each independently H or C₁-C₆ alkyl or, taken together with the nitrogen atom to which they are attached, represent azetidinyl, pyrrolidinyl or piperidinyl, said azetidinyl, pyrrolidinyl and piperidinyl being optionally substituted by C₁-C₆ alkyl;
R⁸ is
(i) azetidin-1-yl, pyrrolidin-1-yl, piperidin-1-yl, morpholin-4-yl, piperazin-1-yl, homopiperidin-1-yl, homopiperazin-1-yl or tetrahydroisoquinolin-1-yl, each being optionally substituted on a ring carbon atom by C₁-C₆ alkyl, C₃-C₈ cycloalkyl, phenyl, C₁-C₆ alkoxy-(C₁-C₆)-alkyl, R⁹R⁹N-(C₁-C₆)-alkyl, fluoro-(C₁-C₆)-alkyl, -CONR⁹R⁹, -COOR⁹ or C₂-C₅ alkanoyl, and optionally substituted on a ring carbon atom not adjacent to a ring nitrogen atom by fluoro-(C₁-C₆)-alkoxy, halo, -OR⁹, cyano, -S(O)ₘR¹⁰, -NR⁹R⁹, -SO₂NR⁹R⁹, -NR⁹COR¹⁰ or -NR⁹SO₂R¹⁰, and said piperazin-1-yl and homopiperazin-1-yl being optionally substituted on the ring nitrogen atom not attached to the C₂-C₆ alkylene group by C₁-C₆ alkyl, phenyl, C₁-C₆ alkoxy-(C₂-C₆)-alkyl, R⁹R⁹N-(C₂-C₆)-alkyl, fluoro-(C₁-C₆)-alkyl, C₂-C₅ alkanoyl, -COOR¹⁰, C₃-C₈ cycloalkyl, -SO₂R¹⁰, -SO₂NR⁹R⁹ or -CONR⁹R⁹, or
(ii) -NR¹¹R¹²;
R⁹ is H, C₁-C₆ alkyl, C₃-C₈ cycloalkyl or phenyl;
R¹⁰ is C₁-C₆ alkyl, C₃-C₈ cycloalkyl or phenyl;
R¹¹ is H, C₁-C₆ alkyl, C₃-C₈ cycloalkyl or benzyl;
R¹² is H, C₁-C₆ alkyl, C₃-C₈ cycloalkyl, phenyl, benzyl, fluoro-(C₁-C₆)-alkyl, -CONR⁹R⁹, -COOR¹⁰, C₂-C₅ alkanoyl or -SO₂NR⁹R⁹;
R¹³ is phenyl, pyridin-2-yl, pyridin-3-yl or pyridin-4-yl, each being optionally substituted by C₁-C₆ alkyl, C₁-C₆ alkoxy, halo or cyano;
R¹⁴ is H or C₁-C₆ alkyl optionally substituted by cyclopropyl;
m is 0, 1 or 2;
X is -CH₂- or -CH₂CH₂-; and
Y is CO, CS, SO₂ or C=N(CN).

3. A compound as claimed in claim 1 wherein R¹ is C₁-C₆ alkyl optionally substituted by 1 or 2 substituents each independently selected from phenyl, naphthyl and fluorenyl, said phenyl, naphthyl and fluorenyl being optionally substituted by C₁-C₆ alkyl, C₁-C₆ alkoxy, halo or cyano.

4. A compound as claimed in claim 3 wherein R¹ is 2,2-diphenyleth-1-yl, 2,2-di(4-chlorophenyl)eth-1-yl, 2,2-di(3-chlorophenyl)eth-1-yl, 2,2-di(4-methylphenyl)eth-1-yl, 2,2-di(3-methylphenyl)eth-1-yl, naphth-1-ylmethyl or fluoren-9-ylmethyl.

5. A compound as claimed in any one of the preceding claims wherein R² is H or C₁-C₄ alkyl.

6. A compound as claimed in claim 5 wherein R² is H or methyl.

7. A compound as claimed in any one of the preceding claims wherein R³ is H or C₁-C₆ alkyl.

8. A compound as claimed in claim 7 wherein R³ is H or methyl.

9. A compound as claimed in claim 1 or any one of claims 3 to 8 wherein R⁴ is (a) C₁-C₄ alkyl substituted by -R¹⁵, C₃-C₆ cycloalkyl or -R¹⁵; or (b) -(C₂-C₄ alkylene)-R⁸, or (c) -(C₁-C₄ alkylene)-R¹³.

10. A compound as claimed in claim 9 wherein R⁴ is -CH₂R¹⁵, cyclohexyl, -R¹⁵, -CH₂CH₂R⁸, -CH₂R¹³ or -CH₂CH₂R¹³.

11. A compound as claimed in any one of the preceding claims wherein R⁵ is -CH₂OH or -CONH(C₁-C₆ alkyl).

12. A compound as claimed in claim 11 wherein R⁵ is -CH₂OH or -CONHCH₂CH₃.

13. A compound as claimed in claim 1 or any one of claims 3 to 12 wherein R⁸ is (i) piperidin-1-yl or 1,2,3,4-tetrahydroisoquinolin-2-yl, each being optionally substituted on a ring carbon atom by C₁-C₆ alkyl, C₃-C₈ cycloalkyl, phenyl, C₁-C₆ alkoxy-(C₁-C₆)-alkyl, R⁹R⁹N-(C₁-C₆)-alkyl, fluoro-(C₁-C₆)-alkyl, -CONR⁹R⁹, -COOR⁹ or C₂-C₅ alkanoyl and optionally substituted on a ring carbon atom not adjacent to a ring nitrogen atom by fluoro-(C₁-C₆)-alkoxy, halo, -OR⁹, cyano, -S(O)ₘR¹⁰, -NR⁹R⁹, -SO₂NR⁹R⁹, -NR⁹COR¹⁰ or -NR⁹SO₂R¹⁰, or (ii) -NR¹¹R¹².

14. A compound as claimed in claim 13 wherein R⁸ is piperidin-1-yl, 4-(2-propyl)piperidin-1-yl, 2,2,6,6-tetramethylpiperidin-1-yl, 1,2,3,4-tetrahydroisoquinolin-2-yl or -NR¹¹R¹².

15. A compound as claimed in any one of the preceding claims wherein R¹¹ is C₁-C₆ alkyl or C₃-C₈ cycloalkyl.

16. A compound as claimed in claim 15 wherein R¹¹ is -CH(CH₃)₂, -CH₂CH₂CH₂CH₃, -CH₂CH(CH₃)₂, -C(CH₃)₃, -CH(CH₂CH₃)₂, cyclohexyl or cyclopentyl.

17. A compound as claimed in claim 1 or any one of claims 3 to 16 wherein R¹² is C₁-C₆ alkyl, C₃-C₈ cycloalkyl, -COR¹⁰ or -SO₂R¹⁰ said C₁-C₆ alkyl being optionally substituted by phenyl.

18. A compound as claimed in claim 17 wherein R¹² is -CH(CH₃)₂, -CH₂CH₂CH₂CH₃, -CH₂CH(CH₃)₂, -C(CH₃)₃, -CH(CH₂CH₃)₂, -C(CH₃)₂Ph, -SO₂Ph, -COPh, cyclohexyl or cyclopentyl.

19. A compound as claimed in any one of the preceding claims wherein R¹³ is phenyl or pyridin-2-yl, each being optionally substituted by C₁-C₆ alkyl, C₁-C₆ alkoxy, halo or cyano.

20. A compound as claimed in claim 18 wherein R¹³ is phenyl or pyridin-2-yl.

21. A compound as claimed in claim 1 or any one of claims 3 to 20 wherein R¹⁵ is pyrrolidin-3-yl or piperidin-4-yl, each being optionally substituted by R¹³, C₁-C₆ alkyl, C₃-C₈ cycloalkyl or benzyl.

22. A compound as claimed in claim 21 wherein R¹⁵ is 1-benzyl-piperidin-4-yl, 1-(2-pyridinyl)piperidin-4-yl, or 1-benzyl-pyrrolidin-3-yl.

23. A compound as claimed in any one of the preceding claims wherein X is -CH₂-.

24. A compound as claimed in any one of the preceding claims wherein Y is CO or C=N(CN).

25. A compound as claimed in claim 1 which is selected from the group consisting of:
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-[2-(diisopropylamino)ethyl] urea;
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-[2-(1-piperidinyl)ethyl]urea;
(2*S*,3*S*,4*R*,5*R*)-5-{2-{[({[2-(diisopropylamino)ethyl]amino}carbonyl)amino] methyl}-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-(6-[(2,2-diphenylethyl)amino]-2-{[({[2-(1-piperidinyl)ethyl] amino}carbonyl)amino]methyl}-9*H*-purin-9-yl)-*N*-ethyl-3,4-dihydroxytetrahydro-2-furancarboxamide;
(2*S*,3*S*,4*R*,5*R*)-5-{2-{[((*E*)-(cyanoimino){[2-(1-piperidinyl)ethyl]amino} methyl)amino]methyl}-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-{2-({[(benzylamino)carbonyl]amino}methyl)-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-{2-({[(cyclohexylamino)carbonyl]amino}methyl)-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-{2-({[({2-[benzoyl(isopropyl)amino]ethyl}amino)carbonyl] amino}methyl)-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-[6-[(2,2-diphenylethyl)amino]-2-({[({2-[isopropyl(phenylsulfonyl)amino]ethyl}amino)carbonyl]amino}methyl)-9*H*-purin-9-yl]-*N*-ethyl-3,4-dihydroxytetrahydro-2-furancarboxamide;
*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*-methyl-*N*-[2-(2-pyridinyl) ethyl]urea;
(2*S*,3*S*,4*R*,5*R*)-5-{2-[({[(1-benzyl-4-piperidinyl)amino]carbonyl}amino)methyl]-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-[6-[(2,2-diphenylethyl)amino]-2-({[({2-[(1-ethylpropyl)(isobutyl)amino]ethyl}amino)carbonyl]amino}methyl)-9*H*-purin-9-yl]-*N*-ethyl-3,4-dihydroxytetrahydro-2-furancarboxamide;
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-{2-[(1-ethylpropyl)(isobutyl)amino]ethyl}urea;
*N*-[2-(3,4-dihydro-2(1*H*)-isoquinolinyl)ethyl]-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)urea;
(2*S*,3*S*,4*R*,5*R*)-5-{2-{[({[2-(3,4-dihydro-2(1*H*)-isoquinolinyl)ethyl]amino} carbonyl)amino]methyl}-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-{2-{[({[2-(dibutylamino)ethyl]amino}carbonyl)amino]methyl}-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-{2-({[({2-[cyclopentyl(isopropyl)amino]ethyl}amino)carbonyl] amino}methyl)-6-[(2,2-diphenylethyl)amino]-9*H*-purin-9-yl}-*N*-ethyl-3,4-dihydroxytetrahydro-2-furancarboxamide;
*N*-{2-[cyclopentyl(isopropyl)amino]ethyl}-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)urea;
(2*S*,3*S*,4*R*,5*R*)-5-(6-[(2,2-dipheny)ethyl)amino]-2-{[({[1-(2-pyridinyl)-4-piperidinyl]amino}carbonyl)amino]methyl}-9*H*-purin-9-yl)-*N*-ethyl-3,4-dihydroxytetrahydro-2-furancarboxamide;
(2*S*,3*S*,4*R*,5*R*)-5-(6-[(2,2-diphenylethyl)amino]-2-{[methyl({[2-(1-piperidinyl)ethyl]amino}carbonyl)amino]methyl}-9*H*-purin-9-yl)-*N*-ethyl-3,4-dihydroxytetrahydro-2-furancarboxamide;
(2*S*,3*S*,4*R*,5*R*)-5-{2-({[({2-[*tert*-butyl(cyclohexyl)amino]ethyl}amino)carbonyl] amino}methyl)-6-[(2,2-diphenylethyl)amino]-9*H*-purin-9-yl}-*N*-ethyl-3,4-dihydroxytetrahydro-2-furancarboxamide;
*N*-{2-[*tert*-butyl(cyclohexyl)amino]ethyl}-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)urea;
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-[1-(2-pyridinyl)-4-piperidinyl]urea;
*N*-[(1-benzyl-4-piperidinyl)methyl]-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H-*purin-2-yl}methyl)urea;
(2*S*,3*S*,4*R*,5*R*)-5-(6-[(2,2-diphenylethyl)amino]-2-{[({[(1-benzyl-4-(hydroxy piperidinyl)methyl]amino}carbonyl)amino]methyl}-9*H*-purin-9-yl)-*N*-ethyl-3,4-dihydroxytetrahydro-2-furancarboxamide;
(2*S*,3*S*,4*R*,5*R*)-5-[6-[(2,2-diphenylethyl)amino]-2-({[({2-[isopropyl(1-methyl-1-phenylethyl)amino]ethyl}amino)carbonyl]amino}methyl)-9*H*-purin-9-yl]-*N*-ethyl-3,4-dihydroxytetrahydro-2-furancarboxamide;
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-{2-[isopropyl(1-methyl-1-phenylethyl)amino]ethyl}urea;
*N*-[2-(dicyclopentylamino)ethyl]-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)urea;
*N*-({9-[(2*R*,3*R*,4*S*,5*R*)-3,4-dihydroxy-5-(hydroxymethyl)tetrahydro-2-furanyl]-6-[(9*H*-fluoren-9-ylmethyl)amino]-9*H*-purin-2-yl}methyl)-*N*'-[2-(diisopropylamino) ethyl]urea;
*N*-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)-*N*'-[2-(2,2,6,6-tetramethyl-1-piperidinyl)ethyl]urea;
(2*S*,3*S*,4*R*,5*R*)-5-(6-[(2,2-diphenylethyl)amino]-2-{[({[2-(4-isopropyl-1-piperidinyl)ethyl]amino}carbonyl)amino]methyl}-9*H*-purin-9-yl)-*N*-ethyl-3,4-dihydroxytetrahydro-2-furancarboxamide;
(2*S*,3*S*,4*R*,5*R*)-5-(6-[(2,2-diphenylethyl)amino]-2-{[({[2-(2,2,6,6-tetramethyl-1-piperidinyl)ethyl]amino}carbonyl)amino]methyl}-9*H*-purin-9-yl)-*N*-ethyl-3,4-dihydroxytetrahydro-2-furancarboxamide;
*N*-[(3*R*)-1-benzylpyrrolidinyl]-*N*'-({9-[(2*R*,3*R*,4*S*,5*R*)-3,4-dihydroxy-5-(hydroxymethyl)tetrahydro-2-furanyl]-6-[(2,2-diphenylethyl)amino]-9*H*-purin-2-yl}methyl)urea;
(2*S*,3*S*,4*R*,5*R*)-5-{2-{[({[(3*R*)-1-benzylpyrrolidinyl]amino}carbonyl)amino]methyl}-6-[(2,2-diphenylethyl)amino]-9*H*-purin-9-yl}-*N*-ethyl-3,4-dihydroxytetrahydro-2-furancarboxamide;
(2*S*,3*S*,4*R*,5*R*)-5-(6-{[2,2-bis(4-chlorophenyl)ethyl]amino}-2-{[({[2-(diisopropylamino)ethyl]amino}carbonyl)amino]methyl}-9*H*-purin-9-yl)-*N*-ethyl-3,4-dihydroxytetrahydro-2-furancarboxamide;
*N*-({6-{[2,2-bis(4-chlorophenyl)ethyl]amino}-9-[(2*R*,3*R*,4*S*,5*R*)-3,4-dihydroxy-5-(hydroxymethyl)tetrahydro-2-furanyl]-9*H*-purin-2-yl}methyl)-*N*'-[2-(diisopropylamino)ethyl]urea;
*N*-({6-{[2,2-bis(4-methylphenyl)ethyl]amino}-9-[(2*R*,3*R*,4*S*,5*R*)-3,4-dihydroxy-5-(hydroxymethyl)tetrahydro-2-furanyl]-9*H*-purin-2-yl}methyl)-*N*'-[2-(diisopropylamino)ethyl]urea;
*N*-({6-{[2,2-bis(3-chlorophenyl)ethyl]amino}-9-[(2*R*,3*R*,4*S*,5*R*)-3,4-dihydroxy-5-(hydroxymethyl)tetrahydro-2-furanyl]-9*H*-purin-2-yl}methyl)-*N*'-[2-(diisopropylamino)ethyl]urea;
*N*-({6-{[2,2-bis(3-methylphenyl)ethyl]amino}-9-[(2*R*,3*R*,4*S*,5*R*)-3,4-dihydroxy-5-(hydroxymethyl)tetrahydro-2-furanyl]-9*H*-purin-2-yl}methyl)-*N*'-[2-(diisopropylamino)ethyl]urea; and
(2*S*,3*S*,4*R*,5*R*)-5-{2-{[({[2-(Diisopropylamino)ethyl]amino}carbonyl)amino]methyl}-6-[(1-naphthylmethyl)amino]-9*H*-purin-9-yl}-*N*-ethyl-3,4-dihydroxytetrahydro-2-furancarboxamide;
and the pharmaceutically acceptable salts and solvates thereof.

26. A pharmaceutical composition including a compound of the formula (I) or a pharmaceutically acceptable salt or solvate thereof, as defined in any one of the preceding claims, together with a pharmaceutically acceptable excipient, diluent or carrier.

27. A compound of the formula (I) or a pharmaceutically acceptable salt, solvate or composition thereof, as defined in any one of claims 1 to 25 and 26, respectively, for use as a medicament.

28. The use of a compound of the formula (I) or a pharmaceutically acceptable salt, solvate or composition thereof, as defined in any one of claims 1 to 25 and 26, respectively, for the manufacture of a medicament to treat a disease for which an A2a receptor agonist is indicated.

29. The use of a compound of the formula (I) or a pharmaceutically acceptable salt, solvate or composition thereof, as defined in any one of claims 1 to 25 and 26, respectively, for the manufacture of an anti-inflammatory agent.

30. The use of a compound of the formula (I) or a pharmaceutically acceptable salt, solvate or composition thereof, as defined in any one of claims 1 to 25 and 26, respectively, for the manufacture of a medicament for the treatment of a respiratory disease.

31. Use as claimed in claim 30 where the disease is selected from the group consisting of adult respiratory distress syndrome (ARDS), bronchitis, chronic bronchitis, chronic obstructive pulmonary disease, cystic fibrosis, asthma, emphysema, bronchiectasis, chronic sinusitis and rhinitis.

32. The use of a compound of the formula (I) or a pharmaceutically acceptable salt, solvate or composition thereof, as defined in any one of claims 1 to 25 and 26, respectively, for the manufacture of a medicament for the treatment of septic shock, male erectile dysfunction, male factor infertility, female factor infertility, hypertension, stroke, epilepsy, cerebral ischaemia, peripheral vascular disease, post-ischaemic reperfusion injury, diabetes, rheumatoid arthritis, multiple sclerosis, psoriasis, allergic dermatitis, eczema, ulcerative colitis, Crohns disease, inflammatory bowel disease, *Heliobacter pylori*-gastritis, non*-Heliobacter pylori* gastritis, non-steroidal anti-inflammatory drug-induced damage to the gastro-intestinal tract or a psychotic disorder, or for wound healing.

33. A compound of the formula (I) or a pharmaceutically acceptable salt, solvate or composition thereof, as defined in any one of claims 1 to 25 and 26, respectively, for use as an A2a receptor agonist.

34. A compound of the formula (I) or a pharmaceutically acceptable salt, solvate or composition thereof, as defined in any one of claims 1 to 25 and 26, respectively, for use as an anti-inflammatory agent.

35. A compound of the formula (I) or a pharmaceutically acceptable salt, solvate or composition thereof, as defined in any one of claims 1 to 25 and 26, respectively, for use in the treatment of a respiratory disease.

36. A compound as claimed in claim 35 where the disease is selected from the group consisting of adult respiratory distress syndrome (ARDS), bronchitis, chronic bronchitis, chronic obstructive pulmonary disease, cystic fibrosis, asthma, emphysema, bronchiectasis, chronic sinusitis and rhinitis.

37. A compound of the formula (I) or a pharmaceutically acceptable salt, solvate or composition thereof, as defined in any one of claims 1 to 25 and 26, respectively, for use in the treatment of septic shock, male erectile dysfunction, male factor infertility, female factor infertility, hypertension, stroke, epilepsy, cerebral ischaemia, peripheral vascular disease, post-ischaemic reperfusion injury, diabetes, rheumatoid arthritis, multiple sclerosis, psoriasis, allergic dermatitis, eczema, ulcerative colitis, Crohns disease, inflammatory bowel disease, *Heliobacter pylori*-gastritis, non-*Heliobacter pylori* gastritis, non-steroidal anti-inflammatory drug-induced damage to the gastro-intestinal tract or a psychotic disorder, or for wound healing.

38. A process for the preparation of a compound of the formula (I), as defined in claim 1, or a pharmaceutically acceptable salt or solvate thereof, which includes
(a) deprotection of a compound of the formula 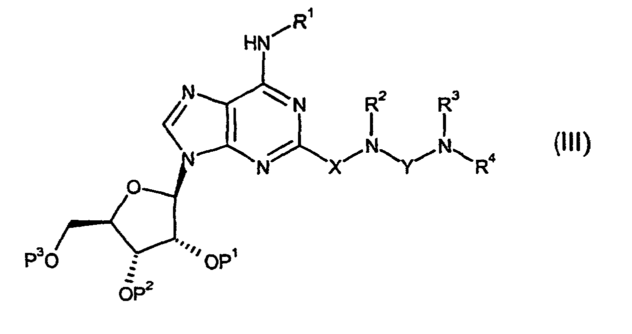 wherein R¹, R², R³, R⁴, X and Y are as defined in claim 1 and either P¹, P² and P³, when taken separately, are protecting groups or, P¹ and P², when taken together are a protecting group and P³ is a protecting group, the protecting groups being removed together or sequentially; or
(b) deprotection of a compound of the formula 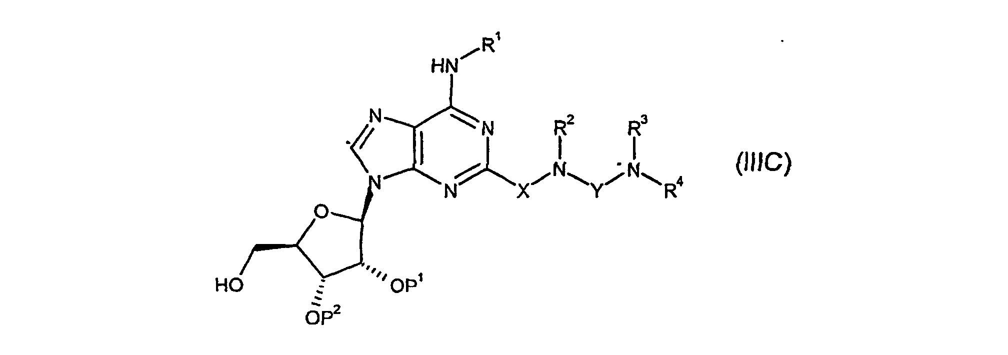 wherein R¹, R², R³, R⁴, X and Y are as defined in claim 1 and either P¹ and P², when taken separately, are protecting groups or, P¹ and P², when taken together are a protecting group, the protecting groups P¹ and P², when taken separately, being removed either together or sequentially; or
(c) deprotection of a compound of the formula 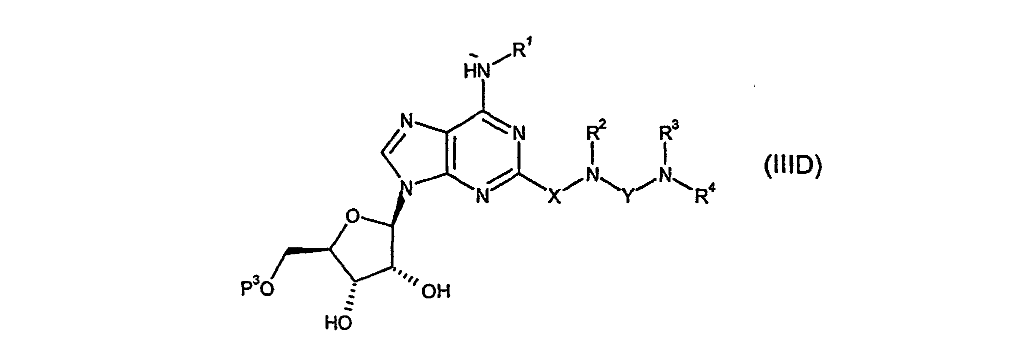 wherein P³ is a protecting group and R¹, R², R³, R⁴, X and Y are as defined in claim 1; or
(d) deprotection of a compound of the formula 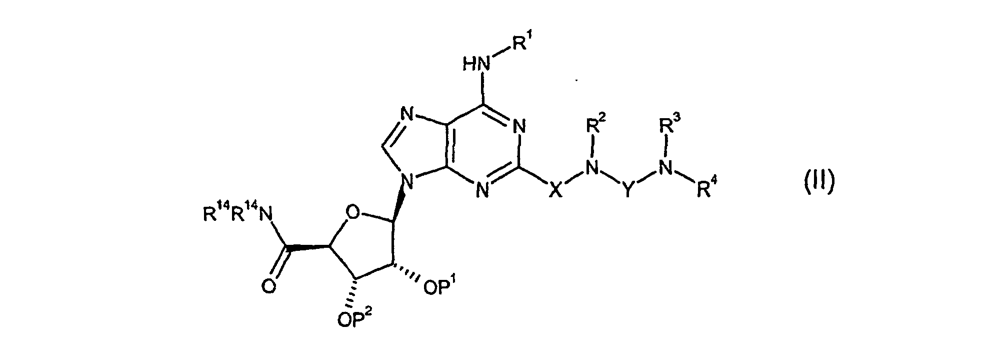
wherein R¹, R², R³, R⁴, R¹⁴, X and Y are as defined in claim 1 and either P¹ and P², when taken separately, are protecting groups or, P¹ and P², when taken together are a protecting group, the protecting groups P¹ and P², when taken separately, being removed either together or sequentially;
any one of said processes (a) to (d) being optionally followed by the conversion of the compound of the formula (I) to a pharmaceutically acceptable salt thereof.

39. A process for the preparation of a compound of the formula (I), as defined in claim 1, or a pharmaceutically acceptable salt or solvate thereof, which includes the reaction of a compound of the formula 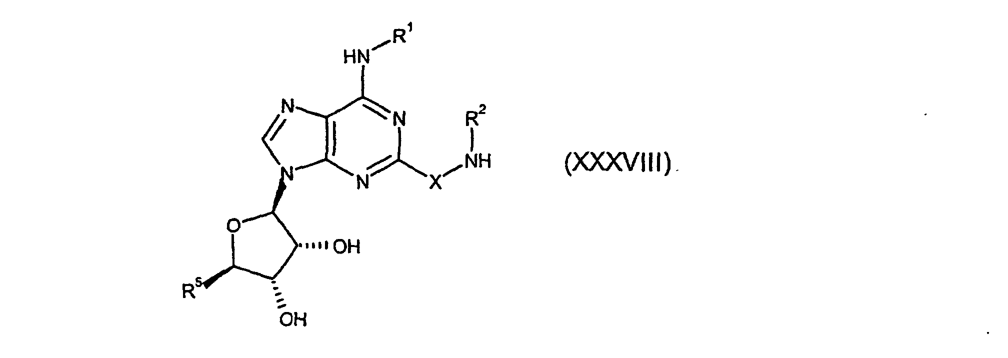 wherein R¹, R², R⁵ and X are as defined in claim 1 with
(a) a compound of the formula 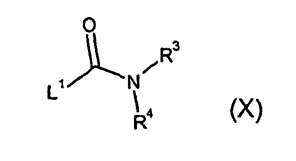 wherein R³ and R⁴ are as defined in claim 1 and L¹ is a suitable leaving group, preferably imidazol-1-yl; or
(b) a compound of the formula
R³R⁴NSO₂L⁹ (XVII)
wherein R³ and R⁴ are as defined in claim 1 and L⁹ is a suitable leaving group, preferably chloro; or
(c) a compound of the formula 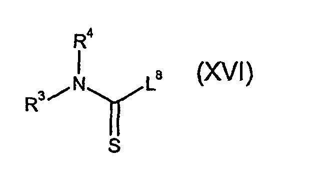 wherein R³ and R⁴ are as defined in claim 1 and L⁸ is a suitable leaving group, preferably methylthio or imidazol-1-yl; or
(d) a compound of the formula 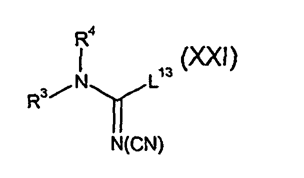
wherein R³ and R⁴ are as defined in claim 1 and L¹³ is a suitable leaving group, preferably methylthio; said process being optionally followed by the conversion of the compound of the formula (I) to a pharmaceutically acceptable salt thereof.

40. A process for the preparation of a compound of the formula (I), as defined in claim 1, or a pharmaceutically acceptable salt or solvate thereof, which includes
(a) the reaction of a compound of the formula 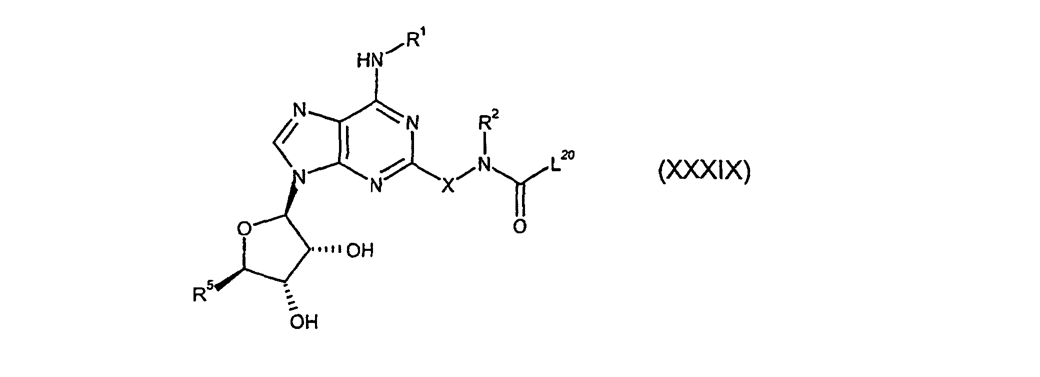 wherein R¹, R², R⁵ and X are as defined in claim 1 and L²⁰ is a suitable leaving group, preferably imidazol-1-yl, with a compound of the formula
R³R⁴NH (XI)
wherein R³ and R⁴ are as defined in claim 1; or
(b) the reaction of a compound of the formula 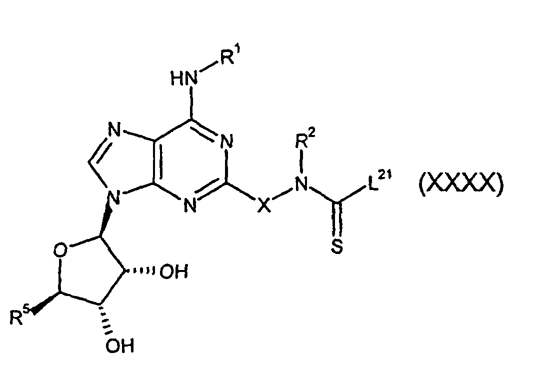 wherein R¹, R², R⁵ and X are as defined in claim 1 and L²¹ is a suitable leaving group, preferably methylthio or imidazol-1-yl, with a compound of the formula
R³R⁴NH (XI)
wherein R³ and R⁴ are as defined in claim 1; or
(c) the reaction of a compound of the formula 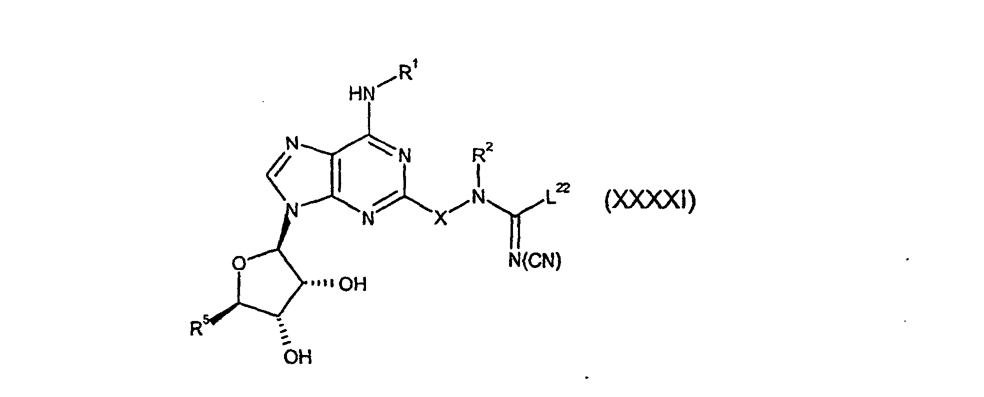
wherein R¹, R², R⁵ and X are as defined in claim 1 and L²² is a suitable leaving group, preferably methylthio, with a compound of the formula
R³R⁴NH (XI)
wherein R³ and R⁴ are as defined in claim 1; any one of said processes (a) to (c) being optionally followed by the conversion of the compound of the formula (I) to a pharmaceutically acceptable salt thereof.

41. A process for the preparation of a compound of the formula (I), as defined in claim 1, or a pharmaceutically acceptable salt or solvate thereof, which includes the acylation or suphonylation of a compound of the formula 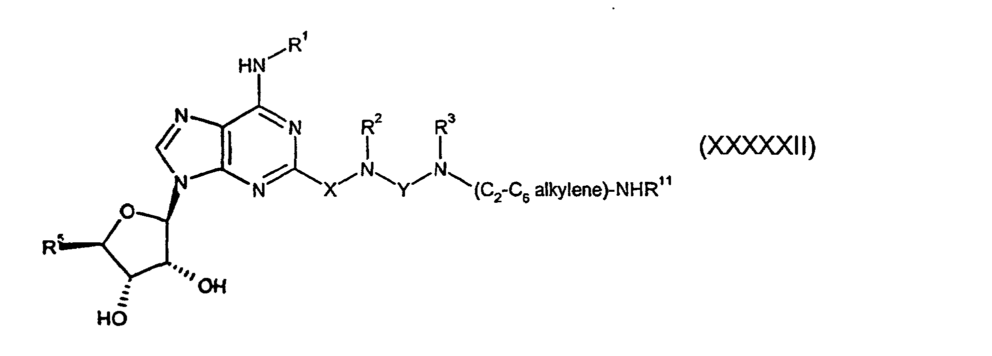 wherein R¹, R², R³, R⁵, R¹¹, X and Y are as defined in claim 1;
said process being optionally followed by the conversion of the compound of the formula (I) to a pharmaceutically acceptable salt thereof.

42. A compound of the formula 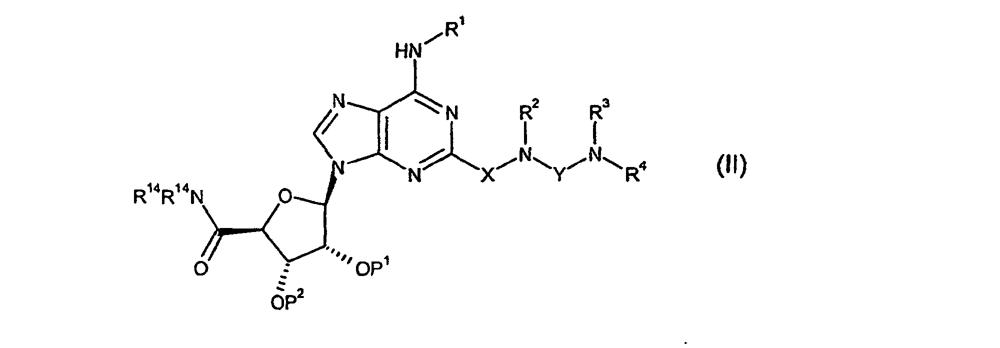 wherein P¹ and P² when taken separately, are protecting groups or, P¹ and P², when taken together are a protecting group; or 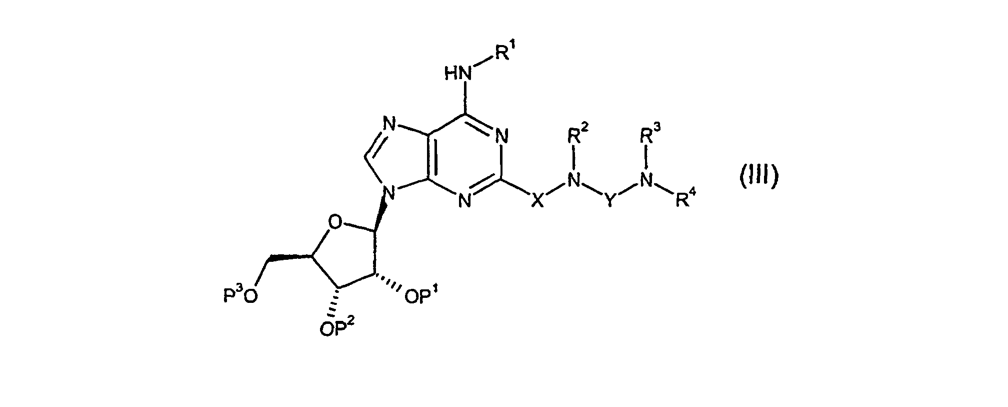 wherein either P¹, P² and P³, when taken separately, are protecting groups or, P¹ and P², when taken together are a protecting group and P³ is a protecting group; or 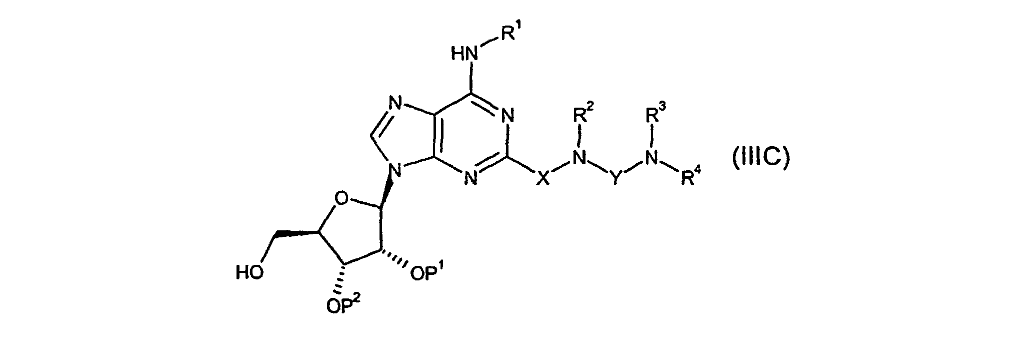 wherein either P¹ and P², when taken separately, are protecting groups or, P¹ and P², when taken together are a protecting group; or 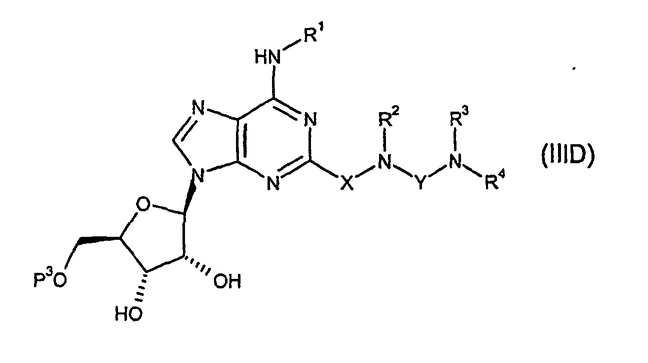 wherein P³ is a protecting group; or 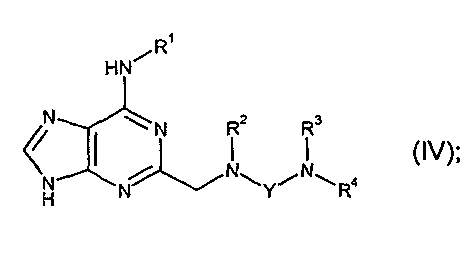 or 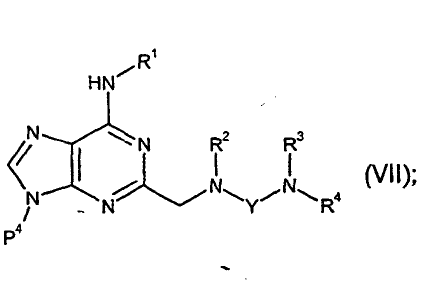 or
wherein P⁴ is a protecting group; or 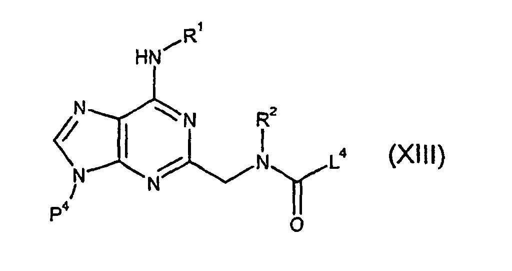 wherein L⁴ is a suitable leaving group, preferably imidazol-1-yl, and P⁴ is a protecting group; or 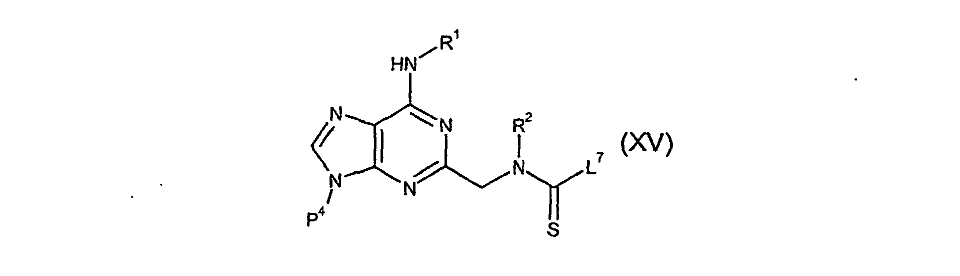 wherein L⁷ is a suitable leaving group, preferably methylthio or imidazol-1-yl, and P⁴ is a protecting group; or 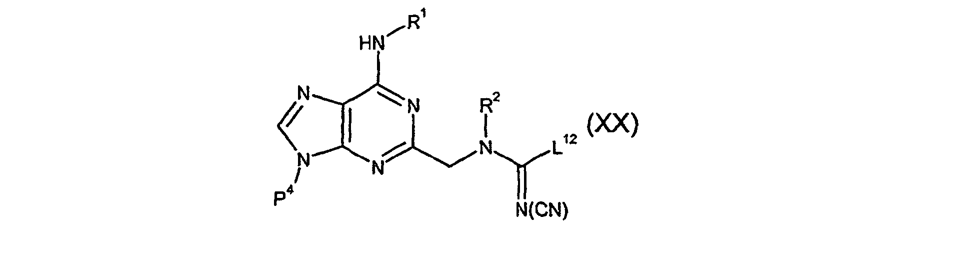 wherein L¹² is a suitable leaving group, preferably methylthio, and P⁴ is a protecting group; or 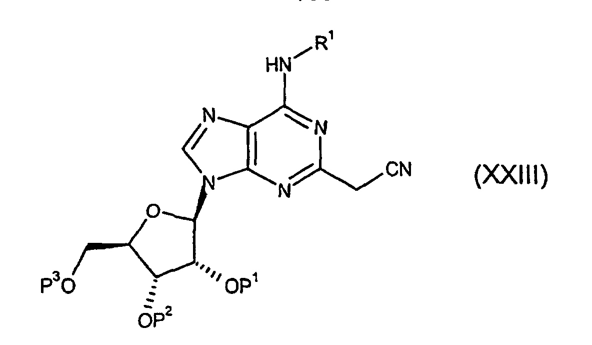 wherein either P¹, P² and P³, when taken separately, are protecting groups or, P¹ and P², when taken together are a protecting group and P³ is a protecting group; or 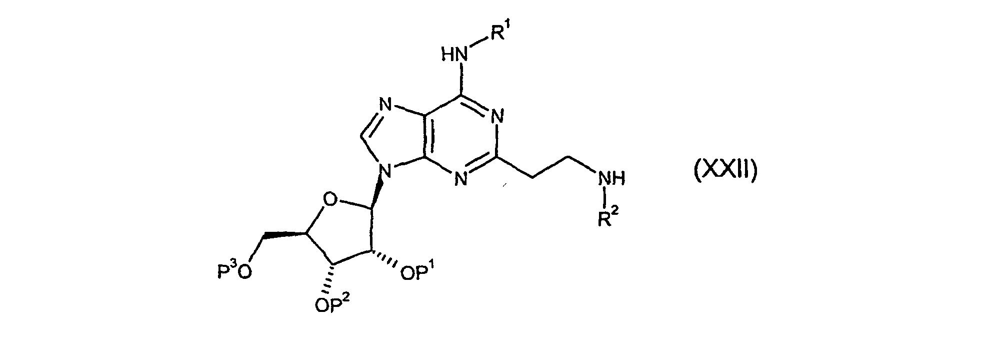 wherein either P¹, P² and P³, when taken separately, are protecting groups or, P¹ and P², when taken together are a protecting group and P³ is a protecting group; or 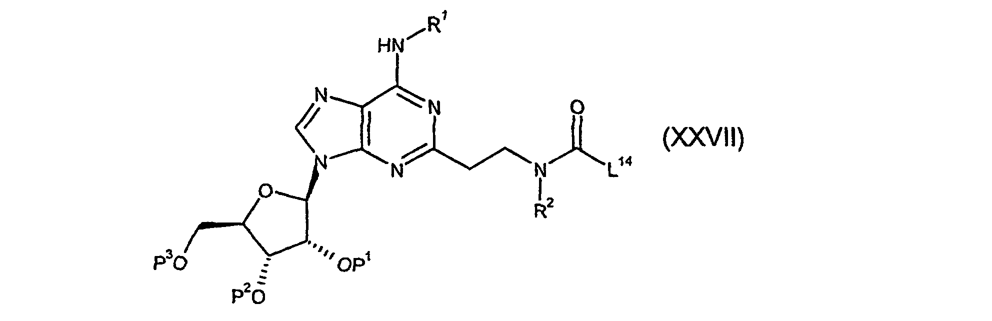 wherein L¹⁴ is a suitable leaving group, preferably imidazol-1-yl, and either P¹, P² and P³, when taken separately, are protecting groups or, P¹ and P², when taken together are a protecting group and P³ is a protecting group; or 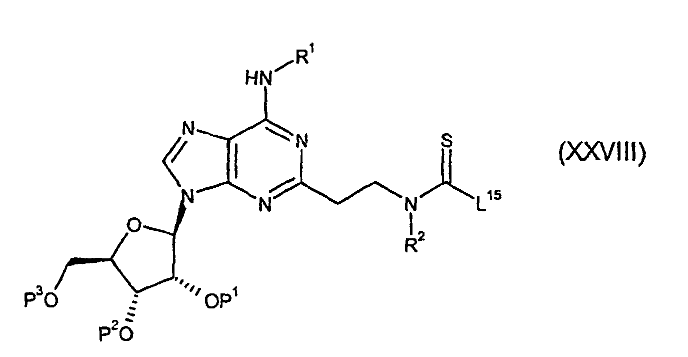 wherein L¹⁵ is a suitable leaving group, preferably methylthio or imidazol-1-yl, and either P¹, P² and P³, when taken separately, are protecting groups or, P¹ and P², when taken together are a protecting group and P³ is a protecting group; or 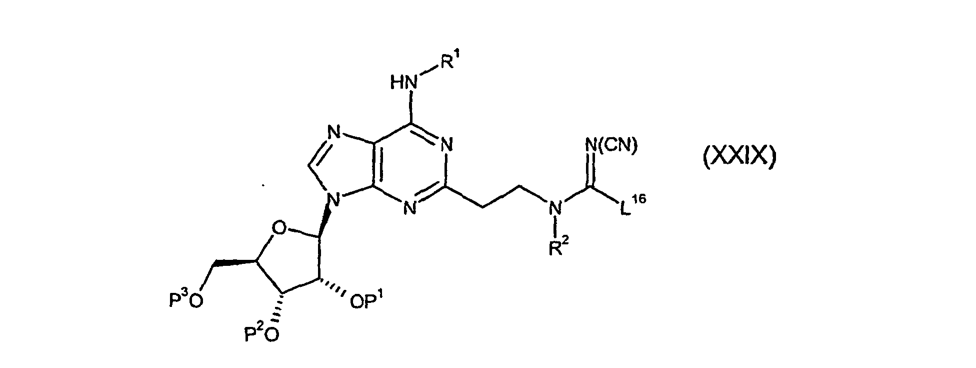 wherein L¹⁶ is a suitable leaving group, preferably methylthio, and either P¹, P² and P³, when taken separately, are protecting groups or, P¹ and P², when taken together are a protecting group and P³ is a protecting group; or 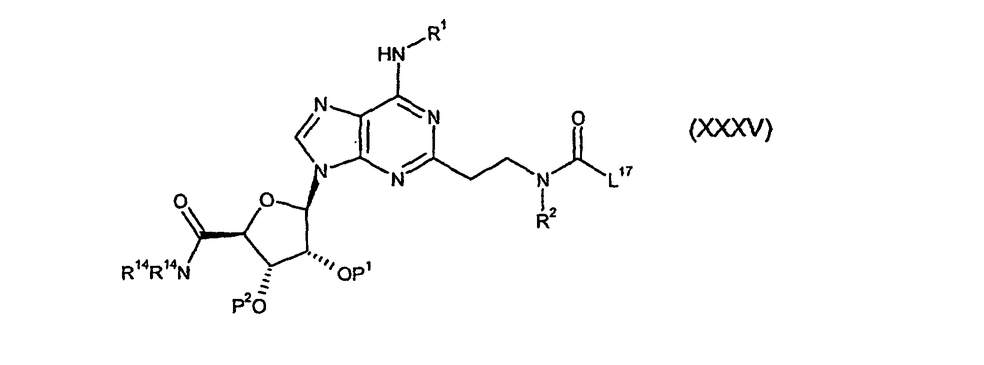 wherein L¹⁷ is a suitable leaving group, preferably imidazol-1-yl, and either P¹ and P², when taken separately, are protecting groups or, P¹ and P², when taken together are a protecting group; or 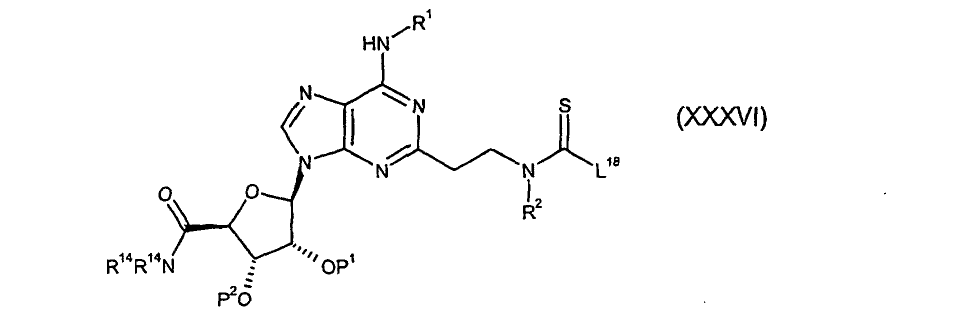 wherein L¹⁸ is a suitable leaving group, preferably imidazol-1-yl or methylthio, and either P¹ and P², when taken separately, are protecting groups or, P¹ and P², when taken together are a protecting group; or 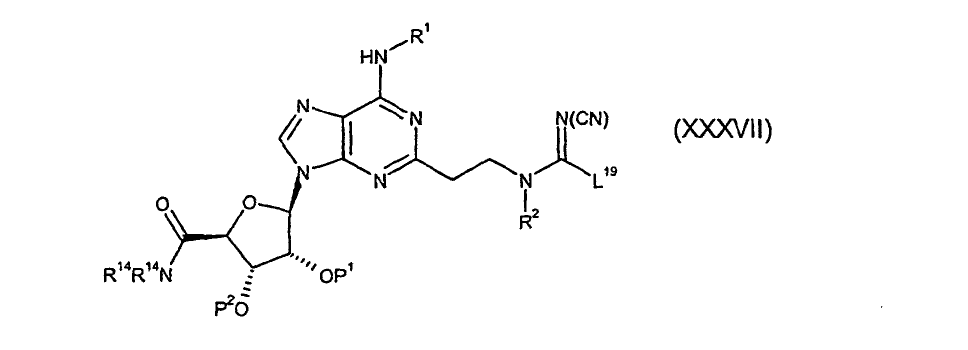 wherein L¹⁹ is a suitable leaving group, preferably methylthio, and either P¹ and P², when taken separately, are protecting groups or, P¹ and P², when taken together are a protecting group; or 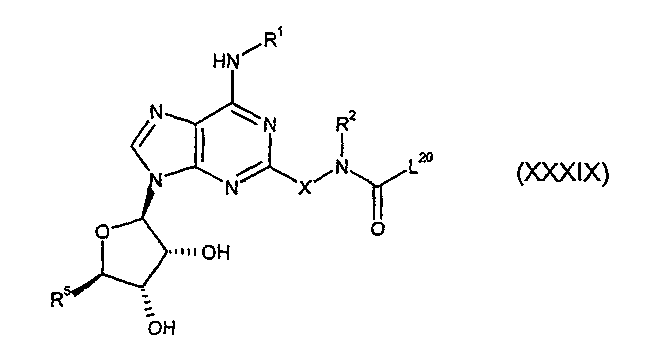 wherein L²⁰ is a suitable leaving group, preferably imidazol-1-yl; or 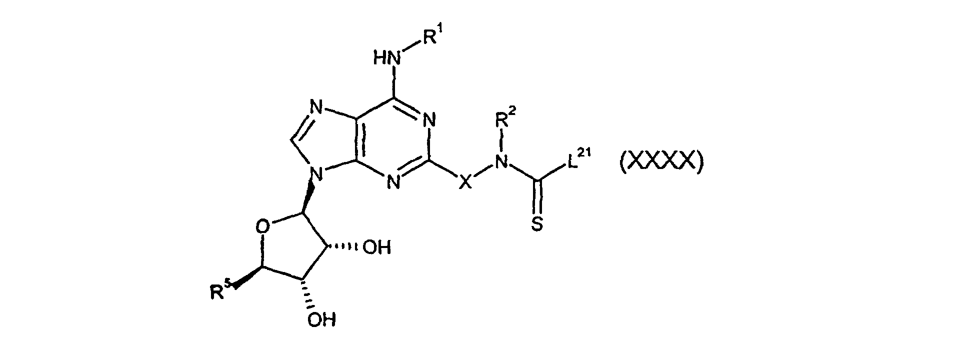 wherein L²¹ is a suitable leaving group, preferably methylthio or imidazol-1-yl; or 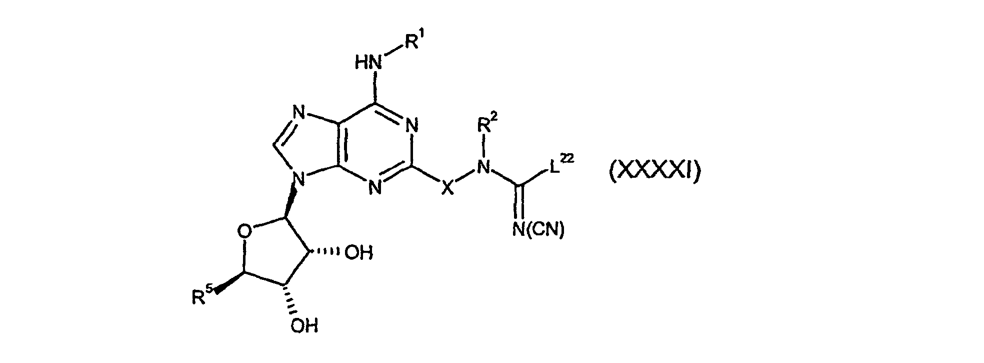 wherein L²² is a suitable leaving group, preferably methylthio; or 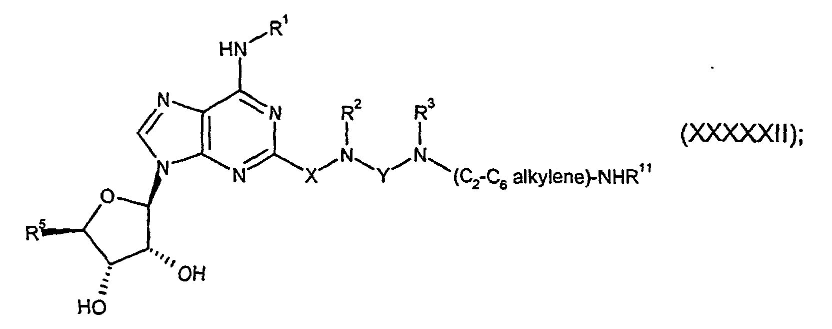 the groups R¹, R², R³, R⁴, R⁵, R¹¹, R¹⁴, X and Y being as defined in claim 1.

## Patentansprüche

1. Verbindung der Formel 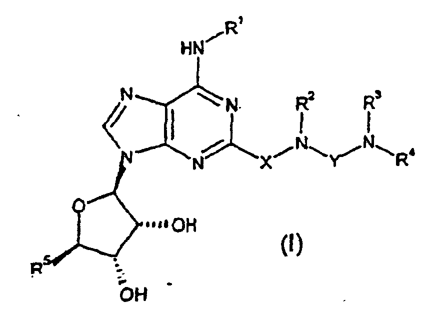 oder ein pharmazeutisch annehmbares Salz oder Solvat davon, worin
R¹ (i) H, (ii) C₁-C₆-Alkyl gegebenenfalls subsituiert durch ein oder zwei Substituenten jeweils unabhängig ausgewählt aus Phenyl, Naphthyl und Fluorenyl, wobei Phenyl, Naphthyl und Fluorenyl gegebenenfalls durch C₁-C₆-Alkyl, C₁-C₆-Alkoxy, Halogen oder Cyano substituiert sind oder (iii) Fluorenyl ist;
R² für H oder C₁-C₆-Alkyl steht;
entweder R³ und R⁴ zusammen mit dem Stickstoffatom, an das sie gebunden sind, für Azetidinyl, Pyrrolidinyl, Piperidinyl, Piperazinyl, Homopiperidinyl oder Homopiperazinyl stehen, wobei jeder Rest gegebenenfalls an einem Ringstickstoffatom oder -kohlenstoffatom durch C₁-C₆-Alkyl oder C₃-C₆-Cycloalkyl substituiert ist und gegebenenfalls an einem Ringkohlenstoffatom, das nicht einem Ringstickstoffatom benachbart ist, durch -NR⁶R⁷ oder -OR⁹ substituiert ist,
oder R³ H, C₁-C₆-Alkyl, C₃-C₈-Cycloalkyl oder Benzyl ist, wobei C₁-C₆-Alkyl gegebenenfalls durch C₃-C₈-Cycloalkyl substituiert ist und R⁴ bedeutet
(a) C₁-C₆-Alkyl, C₃-C₈-Cycloalkyl oder R¹⁵, wobei C₁-C₆-Alkyl gegebenenfalls durch R¹⁵ substituiert ist oder
(b) -(C₂-C₆-Alkylen)-R⁸ oder
(c) -(C₁-C₆-Alkylen)-R¹³;
R⁵ -CH₂OH oder CONR¹⁴R¹⁴ ist;
R⁶ und R⁷ jeweils unabhängig für H oder C₁-C₆-Alkyl stehen oder zusammen mit dem Stickstoffatom, an das sie gebunden sind, für Azetidinyl, Pyrrolidinyl oder Piperidinyl stehen, wobei Azetidinyl, Pyrrolidinyl und Piperidinyl gegebenenfalls durch C₁-C₆-Alkyl substituiert sind;
R⁸ für
(i) Azetidin-1-yl, Pyrrolidin-1-yl, Piperidin-1-yl, Morpholin-4-yl, Piperazin-1-yl, Homopiperidin-1-yl, Homopiperazin-1-yl oder Tetrahydroisochinolin-1-yl steht, wobei jeder Rest gegebenenfalls an einem Ringkohlenstoffatom durch C₁-C₆-Alkyl, C₃-C₈-Cycloalkyl, Phenyl, C₁-C₆-Alkoxy-(C₁-C₆)-Alkyl, R⁹R⁹N-(C₁-C₆)-Alkyl, Fluor-(C₁-C₆)-Alkyl, -CONR⁹R⁹, -COOR⁹ oder C₂-C₅-Alkanoyl substituiert ist und gegebenenfalls an einem Ringkohlenstoffatom, das einem Ringstickstoffatom nicht benachbart ist, durch Fluor-(C₁-C₆)-Alkoxy, Halogen, -OR⁹, Cyano, -S(O)ₘR¹⁰, -NR⁹R⁹, -SO₂NR⁹R⁹, -NR⁹COR¹⁰ oder -NR⁹SO₂R¹⁰ substituiert ist und das Piperazin-1-yl und Homopiperazin-1-yl gegebenenfalls an dem Ringstickstoffatom, das nicht an die C₂-C₆-Alkylengruppe gebunden ist, durch C₁-C₆-Alkyl, Phenyl, C₁-C₆-Alkoxy-(C₂-C₆)-Alkyl, R⁹R⁹N-(C₂-C₆)-Alkyl, Fluor-(C₁-C₆)-Alkyl, C₂-C₅-Alkanoyl, -COOR¹⁰, C₃-C₈-Cycloalkyl, -SO₂R¹⁰, -SO₂NR⁹R⁹ oder -CONR⁹R⁹ substituiert sind, oder
(ii) -NR¹¹R¹² steht;
R⁹ für H, C₁-C₆-Alkyl, C₃-C₈-Cycloalkyl oder Phenyl steht;
R¹⁰ für C₁-C₆-Alkyl, C₃-C₈-Cycloalkyl oder Phenyl steht;
R¹¹ für C₁-C₆-Alkyl, C₃-C₈-Cycloalkyl oder Benzyl steht;
R¹² C₁-C₆-Alkyl, C₃-C₈-Cycloalkyl, Phenyl, Benzyl, Fluor-(C₁-C₆)-Alkyl, -CONR⁹R⁹, -COOR¹⁰, -COR¹⁰, -SO₂R¹⁰ oder -SO₂NR⁹R⁹ bedeutet, wobei C₁-C₆-Alkyl gegebenenfalls durch Phenyl substituiert ist;
R¹³ für Phenyl, Pyridin-2-yl, Pyridin-3-yl oder Pyridin-4-yl steht, wobei jeder Rest gegebenenfalls durch C₁-C₆-Alkyl, C₁-C₆-Alkoxy, Halogen oder Cyano substituiert ist;
R¹⁴ für H oder C₁-C₆-Alkyl steht, das gegebenenfalls durch Cyclopropyl substituiert ist;
R¹⁵ Azetidin-3-yl, Pyrrolidin-3-yl, Piperidin-3-yl, Piperidin-4-yl, Homopiperidin-3-yl oder Homopiperidin-4-yl bedeutet, wobei jeder Rest gegebenenfalls durch R¹³, C₁-C₆-Alkyl, C₃-C₈-Cycloalkyl oder Benzyl substituiert ist;
m 0, 1 oder 2 bedeutet;
X für -CH₂- oder -CH₂CH₂- steht und
Y CO, CS, SO₂ oder C=N(CN) ist.

2. Verbindung der Formel (I) wie in Anspruch 1 definiert, worin
R¹ H, C₁-C₆-Alkyl oder Fluorenyl ist, wobei C₁-C₆-Alkyl gegebenenfalls durch 1 oder 2 Substituenten substituiert ist, die jeweils unabhängig ausgewählt sind aus Phenyl und Naphthyl, wobei Phenyl und Naphthyl gegebenenfalls durch C₁-C₆-Alkyl, C₁-C₆-Alkoxy, Halogen oder Cyano substituiert sind;
R² für H oder C₁-C₆-Alkyl steht;
entweder R³ und R⁴ zusammen mit dem Stickstoffatom, an das sie gebunden sind, für Azetidinyl, Pyrrolidinyl, Piperidinyl, Piperazinyl, Homopiperidinyl oder Homopiperazinyl stehen, wobei jeder Rest gegebenenfalls an einem Ringstickstoff- oder -kohlenstoffatom durch C₁-C₆-Alkyl oder C₃-C₈-Cycloalkyl substituiert ist und gegebenenfalls an einem Ringkohlenstoffatom, das einem Ringstickstoffatom nicht benachbart ist, durch -NR⁶R⁷ substituiert ist;
oder R³ für H, C₁-C₆-Alkyl, C₃-C₈-Cycloalkyl oder Benzyl steht und R⁴ für
(a) Azetidin-3-yl, Pyrrolidin-3-yl, Piperidin-3-yl, Piperidin-4-yl, Homopiperidin-3-yl oder Homopiperidin-4-yl steht, wobei jeder Rest gegebenenfalls durch C₁-C₆-Alkyl, C₃-C₈-Cycloalkyl oder Benzyl substituiert ist oder
(b) -(C₂-C₆-Alkylen)-R⁸ oder
(c) -(C₁-C₆-Alkylen)-R¹³; steht;
R⁵ -CH₂OH oder -CONR¹⁴R¹⁴ bedeutet;
R⁶ und R⁷ entweder jeweils unabhängig H oder C₁-C₆-Alkyl bedeuten oder zusammen mit dem Stickstoffatom, an das sie gebunden sind, für Azetidinyl, Pyrrolidinyl oder Piperidinyl stehen, wobei Azetidinyl, Pyrrolidinyl und Piperidinyl gegebenenfalls durch C₁-C₆-Alkyl substituiert sind;
R⁸ bedeutet
(i) Azetidin-1-yl, Pyrrolidin-1-yl, Piperidin-1-yl, Morpholin-4-yl, Piperazin-1-yl, Homopiperidin-1-yl, Homopiperazin-1-yl oder Tetrahydroisochinolin-1-yl, wobei jeder Rest gegebenenfalls an einem Ringkohlenstoffatom durch C₁-C₆-Alkyl, C₃-C₈-Cycloalkyl, Phenyl, C₁-C₆-Alkoxy-(C₁-C₆)-Alkyl, R⁹R⁹N-(C₁-C₆)-Alkyl, Fluor-(C₁-C₆)-Alkyl, -CONR⁹R⁹, -COOR⁹ oder C₂-C₅-Alkanoyl substituiert ist und gegebenenfalls an einem Ringkohlenstoffatom, das einem Ringstickstoffatom nicht benachbart ist, durch Fluor-(C₁-C₆)-Alkoxy, Halogen, -OR⁹, Cyano, -S(O)ₘR¹⁰, -NR⁹R⁹, -SO₂NR⁹R⁹, -NR⁹COR¹⁰ oder -NR⁹SO₂R¹⁰ substituiert ist und wobei Piperazin-1-yl und Homopiperazin-1-yl gegebenenfalls an dem Ringstickstoffatom, das nicht an die C₂-C₆-Alkylengruppe gebunden ist, durch C₁-C₆-Alkyl, Phenyl, C₁-C₆-Alkoxy-(C₂-C₆)-Alkyl, R⁹R⁹N-(C₂-C₆)-Alkyl, Fluor-(C₁-C₆)-Alkyl, C₂-C₅-Alkanoyl, -COOR¹⁰, C₃-C₈-Cycloalkyl, -SO₂R¹⁰, -SO₂NR⁹R⁹ oder -CONR⁹R⁹ substituiert sind, oder
(ii) -NR¹¹R¹²;
R⁹ für H, C₁-C₆-Alkyl, C₃-C₈-Cycloalkyl oder Phenyl steht;
R¹⁰ für C₁-C₆-Alkyl, C₃-C₈-Cycloalkyl oder Phenyl steht;
R¹¹ für H, C₁-C₆-Alkyl, C₃-C₈-Cycloalkyl oder Benzyl steht;
R¹² H, C₁-C₆-Alkyl, C₃-C₈-Cycloalkyl, Phenyl, Benzyl, Fluor-(C₁-C₆)-Alkyl, -CONR⁹R⁹, -COOR¹⁰, C₂-C₅-Alkanoyl oder -SO₂NR⁹R⁹ bedeutet;
R¹³ für Phenyl, Pyridin-2-yl, Pyridin-3-yl oder Pyridin-4-yl steht, wobei jeder Rest gegebenenfalls durch C₁-C₆-Alkyl, C₁-C₆-Alkoxy, Halogen oder Cyano substituiert ist;
R¹⁴ für H oder C₁-C₆-Alkyl steht, das gegebenenfalls durch Cyclopropyl substituiert ist;
m 0, 1 oder 2 bedeutet;
X für -CH₂- oder -CH₂CH₂- steht und
Y CO, CS, SO₂ oder C=N(CN) ist.

3. Verbindung nach Anspruch 1, worin R¹ für C₁-C₆-Alkyl steht, das gegebenenfalls durch 1 oder 2 Substituenten substituiert ist, die jeweils unabhängig ausgewählt sind aus Phenyl, Naphthyl und Fluorenyl, wobei Phenyl, Naphthyl und Fluorenyl gegebenenfalls durch C₁-C₆-Alkyl, C₁-C₆-Alkoxy, Halogen oder Cyano substituiert sind.

4. Verbindung nach Anspruch 3, worin R¹ 2,2-Diphenyleth-1-yl, 2,2-Di(4-chlorphenyl)eth-1-yl, 2,2-Di(3-chlorphenyl)eth-1-yl, 2,2-Di(4-methylphenyl)eth-1-yl, 2,2-Di(3-methylphenyl)eth-1-yl, Naphth-1-ylmethyl oder Fluoren-9-ylmethyl ist.

5. Verbindung nach einem der vorhergehenden Ansprüche, worin R² H oder C₁-C₄-Alkyl ist.

6. Verbindung nach Anspruch 5, worin R² H oder Methyl ist.

7. Verbindung nach einem der vorhergehenden Ansprüche, worin R³ H oder C₁-C₆-Alkyl ist.

8. Verbindung nach Anspruch 7, worin R³ H oder Methyl ist.

9. Verbindung nach Anspruch 1 oder einem der Ansprüche 3 bis 8, worin R⁴ bedeutet (a) C₁-C₄-Alkyl substituiert durch -R¹⁵, C₃-C₆-Cycloalkyl oder -R¹⁵; oder (b) -(C₂-C₄-Alkylen)-R⁸ oder (c) (C₁-C₄-Alkylen)-R¹³.

10. Verbindung nach Anspruch 9, worin R⁴ -CH₂R¹⁵, Cyclohexyl, -R¹⁵, -CH₂CH₂R⁸, -CH₂R¹³ oder -CH₂CH₂R¹³ ist.

11. Verbindung nach einem der vorhergehenden Ansprüche, worin R⁵ -CH₂OH oder -CONH(C₁-C₆-Alkyl) ist.

12. Verbindung nach Anspruch 11, worin R⁵ -CH₂OH oder -CONHCH₂CH₃ ist.

13. Verbindung nach Anspruch 1 oder einem der Ansprüche 3 bis 12, worin R⁸ bedeutet (i) Piperidin-1-yl oder 1,2,3,4-Tetrahydroisochinolin-2-yl, wobei jeder Rest gegebenenfalls an einem Ringkohlenstoffatom durch C₁-C₆-Alkyl, C₃-C₈-Cycloalkyl, Phenyl, C₁-C₆-Alkoxy-(C₁-C₆)-Alkyl, R⁹R⁹N-(C₁-C₆)-Alkyl, Fluor-(C₁-C₆)-Alkyl, -CONR⁹R⁹, -COOR⁹ oder C₂-C₅-Alkanoyl substituiert ist und gegebenenfalls an einem Ringkohlenstoffatom, das nicht einem Ringstickstoffatom benachbart ist, durch Fluor-(C₁-C₆)-Alkoxy, Halogen, -OR⁹, Cyano, -S(O)ₘR¹⁰, -NR⁹R⁹, -SO₂NR⁹R⁹, -NR⁹COR¹⁰ oder -NR⁹SO₂R¹⁰ substituiert ist oder (ii) -NR¹¹R¹².

14. Verbindung nach Anspruch 13, worin R⁸ Piperidin-1-yl, 4(2-Propyl)piperidin-1-yl, 2,2,6,6-Tetramethylpiperidin-1-yl, 1,2,3,4-Tetrahydroisochinoli-n-2-yl oder -NR¹¹R¹² ist.

15. Verbindung nach einem der vorhergehenden Ansprüche, worin R¹¹ C₁-C₆-Alkyl oder C₃-C₈-Cycloalkyl ist.

16. Verbindung nach Anspruch 15, worin R¹¹ -CH(CH₃)₂, -CH₂CH₂CH₂CH₃, -CH₂CH(CH₃)₂, -C(CH₃)₃, -CH(CH₂CH₃)₂, Cyclohexyl oder Cyclopentyl ist.

17. Verbindung nach Anspruch 1 oder einem der Ansprüche 3 bis 16, worin R¹² C₁-C₆-Alkyl, C₃-C₈-Cycloalkyl, -COR¹⁰ oder -SO₂R¹⁰ ist, wobei C₁-C₆-Alkyl gegebenenfalls durch Phenyl substituiert ist.

18. Verbindung nach Anspruch 17, worin R¹² -CH(CH₃)₂, -CH₂CH₂CH₂CH₃, -CH₂CH(CH₃)₂, -C(CH₃)₃, -CH(CH₂CH₃)₂, -C(CH₃)₂Ph, -SO₂Ph, -COPh, Cyclohexyl oder Cyclopentyl ist.

19. Verbindung nach einem der vorhergehenden Ansprüche, worin R¹³ Phenyl oder Pyridin-2-yl ist, wobei jeder Rest gegebenenfalls durch C₁-C₆-Alkyl, C₁-C₆-Alkoxy, Halogen oder Cyano substituiert ist.

20. Verbindung nach Anspruch 18, worin R¹³ Phenyl oder Pyridin-2-yl ist.

21. Verbindung nach Anspruch 1 oder einem der Ansprüche 3 bis 20, worin R¹⁵ Pyrrolidin-3-yl oder Piperidin-4-yl ist, wobei jeder Rest gegebenenfalls durch R¹³, C₁-C₆-Alkyl, C₃-C₈-Cycloalky oder Benzyl substituiert ist.

22. Verbindung nach Anspruch 21, worin R¹⁵ 1-Benzylpiperidin-4-yl, 1-(2-Pyridinyl)piperidin-4-yl oder 1-Benzylpyrrolidin-3-yl ist.

23. Verbindung nach einem der vorhergehenden Ansprüche, worin X -CH₂- ist.

24. Verbindung nach einem der vorhergehenden Ansprüche, worin Y CO oder C=N(CN) ist.

25. Verbindung nach Anspruch 1, ausgewählt aus der Gruppe, bestehend aus:
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(diisopropylamino)ethyl]harnstoff;
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(1-piperidinyl)ethyl]harnstoff;
(2S,3S,4R,5R)-5-{2-{[({[2-(Diisopropylamino)ethyl]amino}carbonyl)amino]methyl}-6-[(2,2-diphenylethyl)amino]-9H-purin-9-yl}-N-ethyl-3,4-dihydroxytetrahydro-2-furancarboxamid;
(2S,3S,4R,5R)-5-(6-[(2,2-Diphenylethyl)amino]-2-{[({[2-(1-piperidinyl)ethyl]amino}carbonyl)amino]methyl}-9H-purin-9-yl)-N-ethyl-3,4-dihydroxytetrahydro-2-furancarboxamid;
(2S,3S,4R,5R)-5-{2-{[((E)-(Cyanoimino){[2-(1-piperidinyl)ethyl]amino}methyl)amino]methyl}-6-[(2,2-diphenylethyl)amino]-9H-purin-9-yl}-N-ethyl-3,4-dihydroxytetrahydro-2-furancarboxamid;
(2S,3S,4R,5R)-5-{2-({[(Benzylamino)carbonyl]amino}methyl)-6-[ (2, 2-diphenylethyl)amino]-9H-purin-9-yl}-N-ethyl-3,4-dihydroxytetrahydro-2-furancarboxamid;
(2S,3S,4R,5R)-5-{2-({[(Cyclohexylamino)carbonyl]amino}methyl)-6-[(2,2-diphenylethyl)amino]-9H-purin-9-yl]-N-ethyl-3,4-dihydroxytetrahydro-2-furancarboxamid;
(2S,3S,4R,5R)-5-{2-({[({2-[Benzoyl(isopropyl)amino]ethyl}amino)carbonyl]amino}methyl)-6-[(2,2-diphenylethyl)amino]-9Hpurin-9-yl}-N-ethyl-3,4-dihydroxytetrahydro-2-furancarboxamid;
(2S,3S,4R,5R)-5-[6-[(2,2-Diphenylethyl)amino]-2-({[({2-[isopropyl(phenylsulfonyl)amino]ethyl}amino)carbonyl]amino}methyl)-9H-purin-9-yl]-N-ethyl-3,4-dihydroxytetrahydro-2-furancarboxamid;
N'-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N-methyl-N-[2-(2-pyridinyl)ethyl]harnstoff;
(2S,3S,4R,5R)-5-{2-[({[(1-Benzyl-4-piperidinyl)amino]carbonyl}amino)methyl]-6-[(2,2-diphenylethyl)amino}-9H-purin-9-yl}-N-ethyl-3,4-dihydroxytetrahydro-2-furancarboxamid;
(2S,3S,4R,5R)-5-[6-[(2,2-Diphenylethyl)amino]-2-({[({2-[1-ethylpropyl)(isobutyl)amino]ethyl}amino)carbonyl]amino}methyl)-9H-purin-9-yl]-N-ethyl-3,4-dihydroxytetrahydro-2-furancarboxamid;
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-{2-[(1-ethylpropyl)(isobutyl)amino]ethyl)harnstoff;
N-[2-(3,4-Dihydro-2(1H)-isochinolinyl)ethyl]-N'-({9-[2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
(2S,3S,4R,5R)-5-{2-{[({[2-(3,4-Dihydro-2(1H)-isochinolinyl)ethyl]amino}carbonyl)amino]methyl}-6-[(2,2-diphenylethyl)amino]-9H-purin-9-yl}-N-ethyl-3,4-diyhdroxytetrahydro-2-furancarboxamid;
(2S,3S,4R,5R)-5-{2-{[({[2-(Dibutylamino)ethyl]amino}carbonyl)amino]methyl}-6-[(2,2-diphenylethyl)amino]-9H-purin-9-yl}-N-ethyl-3,4-dihydroxytetrahydro-2-furancarboxamid; (2S,3S,4R,5R)-5-{2-({[({2-[Cyclopentyl(isopropyl)amino]ethyl}amino)carbonyl]amino}methyl)-6-[(2,2-diphenylethyl)amino]-9H-purin-9-yl}-N-ethyl-3,4-dihydroxytetrahydro-2-furancarboxamid;
N-{2-[Cyclopentyl(isopropyl)amino]ethyl}-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
(2S,3S,4R,5R)-5-(6-[(2,2-Diphenylethyl)amino]-2-{[({[1-(2-pyridinyl)-4-piperidinyl]amino}carbonyl)amino]methyl}-9Hpurin-9-yl)-N-ethyl-3,4-dihydroxytetrahydro-2-furancarboxamid;
(2S,3S,4R,5R)-5-(6-[(2,2-Diphenylethyl)amino]-2-{[methyl({[2-(1-piperidinyl)ethyl]amino}carbonyl)amino]methyl}-9H-purin-9-yl)-N-ethyl-3,4-dihydroxytetrahydro-2-furancarboxamid;
(2S,3S,4R,5R)-5-{2-({[({2-[tert-Butyl(cyclohexyl)amino]ethyl}amino)carbonyl]amino}methyl)-6-[(2,2-diphenylethyl)amino]-9H-purin-9-yl}-N-ethyl-3,4-dihydroxytetrahydro-2-furancarboxamid;
N-{2-[tert-Butyl(cyclohexyl)amino]ethyl}-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[1-(2-pyridinyl)-4-piperidinyl]harnstoff;
N-[(1-Benzyl-4-piperidinyl)methyl]-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
(2S,3S,4R,5R)-5-(6-[(2,2-Diphenylethyl)amino]-2-{[({[(1-benzyl-4-piperidinyl)methyl]amino}carbonyl)amino]methyl}-9*H*purin-9-yl)-*N*-ethyl-3,4-dihydroxytetrahydro-2-furancarboxamid;
(2S,3S,4R,5R)-5-[6-[(2,2-Diphenylethyl)amino]-2-({[({2-[isopropyl(1-methyl-1-phenylethyl)amino]ethyl}amino)carbonyl]amino}methyl)-9H-purin-9-yl]-N-ethyl-3,4-dihydroxytetrahydro-2-furancarboxamid;
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-{2-[isopropyl(1-methyl-1-phenylethyl)amino]ethyl}harnstoff;
N-[2-(Dicyclopentylamino)ethyl]-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
N-({9-[(2R,3R,4S,5R)-3,4-Dihydroxy-5-(hydroxymethyl)tetrahydro-2-furanyl]-6-[(9H-fluoren-9-ylmethyl)amino]-9H-purin-2-yl}methyl)-N'-[2-(diisopropylamino)ethyl]harnstoff;
N-({9-[(2R,3R,4S,5R)-3,4-Dihydroxy-5-(hydroxymethyl)tetrahydro-2-furanyl]-6-[(2,2-diphenylethyl)amino]-9H-purin-2-yl}methyl)-N'-[2-(2,2,6,6-tetramethyl-1-piperidinyl)ethyl]harnstoff;
(2S,3S,4R,5R)-5-(6-[(2,2-Diphenylethyl)amino]-2-{[({[2-(4-isopropyl-1-piperidinyl)ethyl]amino}carbonyl)amino]methyl}-9H-purin-9-yl)-N-ethyl-3,4-dihydroxytetrahydro-2-furancarboxamid;
(2S,3S,4R,5R)-5-(6-[(2,2-Diphenylethyl)amino]-2-{[({[2-(2,2,6,6-tetramethyl-1-piperidinyl)ethyl]amino}carbonyl)amino]methyl}-9H-purin-9-yl)-N-ethyl-3,4-dihydroxytetrahydro-2-furancarboxamid;
N-[(3R)-1-Benzylpyrrolidinyl]-N'-({9-[(2R,3R,4S,5R)-3,4-dihydroxy-5-(hydroxymethyl)tetrahydro-2-furanyl]-6-[(2,2-diphenylethyl)amino]-9H-purin-2-yl}methyl)harnstoff;
(2S,3S,4R,5R)-5-{2-{[({[(3R)-1-Benzylpyrrolidinyl]amino}carbonyl)amino]methyl}-6-[(2,2-diphenylethyl)amino]-9H-purin-9-yl}-N-ethyl-3,4-dihydroxytetrahydro-2-furancarboxamid;
(2S,3S,4R,5R)-5-(6-{[2,2-bis(4-Chlorphenyl)ethyl]amino}-2-{[({[2-(diisopropylamino)ethyl]amino}carbonyl)amino]methyl}-9H-purin-9-yl)-N-ethyl-3,4-dihydroxytetrahydro-2-furancarboxamid;
N-({6-{[2,2-bis(4-chlorphenyl)ethyl]amino}-9-[(2R,3R,4S,5R)-3,4-dihydroxy-5-(hydroxymethyl)tetrahydro-2-furanyl]-9Hpurin-2-yl}methyl)-N'-[2-(diisopropylamino)ethyl]harnstoff;
N-({6-{[2,2-bis(4-Methylphenyl)ethyl]amino}-9-[(2R,3R,4S,5R)-3,4-dihydraxy-5-(hydroxymethyl)tetrahydro-2-furanyl]-9Hpurin-2-yl}methyl)-N'-[2-(diisopropylamino)ethyl]harnstoff;
N-({6-{[2,2-bis(3-Chlorphenyl)ethyl]amino}-9-[(2R,3R,4S,5R)-3,4-dihydroxy-5-(hydroxymethyl)tetrahydro-2-furanyl]-9Hpurin-2-yl}methyl)-N'-[2-(diisopropylamino)ethyl]harnstoff;
N-({6-([2,2-bis(3-Methylphenyl}ethyl]amino}-9-[(2R,3R,4S,5R)-3,4-dihydroxy-5-(hydroxymethyl)tetrahydro-2-furanyl]-9Hpurin-2-yl}methyl)-N'-[2-(diisopropylamino)ethyl]harnstoff; und
(2S,3S,4R,5R)-5-{2-{[({[2-(Diisopropylamino)ethyl]amino}carbonyl)amino]methyl}-6-[(1-naphthylmethyl)amino]-9Hpurin-9-yl}-N-ethyl-3,4-dihydroxytetrahydro-2-furancarboxamid;
und pharmazeutisch annehmbare Salze und Solvate davon.

26. Pharmazeutische Zusammensetzung, die eine Verbindung der Formel (I) oder ein pharmazeutisch annehmbares Salz oder Solvat davon nach einem der vorhergehenden Ansprüche zusammen mit einem pharmazeutisch annehmbaren Exzipiens, Verdünnungsmittel oder Träger enthält.

27. Verbindung der Formel (I) oder ein pharmazeutisch annehmbares Salz, Solvat oder eine Zusammensetzung davon nach einem der Ansprüche 1 bis 25 bzw. 26 zur Verwendung als Arzneimittel.

28. Verwendung einer Verbindung der Formel (I) oder eines pharmazeutisch annehmbaren Salzes, Solvats oder einer Zusammensetzung davon nach einem der Ansprüche 1 bis 25 bzw. 26 zur Herstellung eines Medikaments zur Behandlung einer Krankheit, für die ein A2a-Rezeptoragonist indiziert ist.

29. Verwendung einer Verbindung der Formel (I) oder eines pharmazeutisch annehmbaren Salzes, Solvats oder einer Zusammensetzung davon nach einem der Ansprüche 1 bis 25 bzw. 26 zur Herstellung eines entzündungshemmenden Mittels.

30. Verwendung einer Verbindung der Formel (I) oder eines pharmazeutisch annehmbaren Salzes, Solvats oder einer Zusammensetzung davon nach einem der Ansprüche 1 bis 25 bzw. 26 zur Herstellung eines Medikaments zur Behandlung einer Atemwegserkrankung.

31. Verwendung nach Anspruch 30, worin die Krankheit ausgewählt ist aus der Gruppe, bestehend aus Schocklunge ("adult respiratory distress syndrom"; ARDS), Bronchitis, chronischer Bronchitis, chronischer obstruktiver pulmonaler Erkrankung, zystischer Fibrose, Asthma, Emphysem, Bronchiektasie, chronischer Sinusitis und Rhinitis.

32. Verwendung einer Verbindung der Formel (I) oder eines pharmazeutisch annehmbaren Salzes, Solvats oder einer Zusammensetzung davon nach einem der Ansprüche 1 bis 25 bzw. 26 zur Herstellung eines Medikaments zur Behandlung des septischen Schocks, der männlichen erektilen Dysfunktion, der männlichen Unfruchtbarkeit, der weiblichen Unfruchtbarkeit, Bluthochdruck, Schlaganfall, Epilepsie, cerebraler Ischämie, Erkrankung der peripheren Gefäße, postischämischer Reperfusionsverletzung, Diabetes, rheumatoider Arthritis, Multipler Sklerose, Psoriasis, allergischer Dermatitis, Ekzemen, ulcerativer Colitis, Crohn'scher Krankheit, entzündlicher Darmkrankheit, Heliobacter pylori-Gastritis, nicht-Heliobacter pylori-Gastritis, durch nicht-steroidale entzündungshemmende Arzneimittel hervorgerufener Schädigung des Gastrointestinaltrakts oder einer psychotischen Störung oder zur Wundheilung.

33. Verbindung der Formel (I) oder eines pharmazeutisch annehmbaren Salzes, Solvats oder einer Zusammensetzung davon nach einem der Ansprüche 1 bis 25 bzw. 26 zur Verwendung als A2a-Rezeptoragonist.

34. Verbindung der Formel (I) oder ein pharmazeutisch annehmbares Salzes, Solvats oder einer Zusammensetzung davon nach einem der Ansprüche 1 bis 25 bzw. 26 zur Verwendung als entzündungshemmendes Mittel.

35. Verbindung der Formel (I) oder ein pharmazeutisch annehmbares Salzes, Solvats oder einer Zusammensetzung davon nach einem der Ansprüche 1 bis 25 bzw. 26 zur Verwendung in der Behandlung einer Atemwegserkrankung.

36. Verbindung nach Anspruch 35, worin die Krankheit ausgewählt ist aus der Gruppe, bestehend aus Schocklunge (ARDS), Bronchitis, chronischer Bronchitis, chronischer obstruktiver pulmonaler Erkrankung, zystischer Fibrose, Asthma, Emphysem, Bronchiektasie, chronischer Sinusitis und Rhinitis.

37. Verbindung der Formel (I) oder ein pharmazeutisch annehmbares,Salz, Solvat oder eine Zusammensetzung davon nach einem der Ansprüche 1 bis 25 bzw. 26 zur Verwendung in der Behandlung des septischen Schocks, der männlichen erektilen Dysfunktion, der männlichen Unfruchtbarkeit (male factor infertility), der weiblichen Unfruchtbarkeit (female factor infertility), von Bluthochdruck, Schlaganfall, Epilepsie, cerebraler Ischämie, der Krankheit der peripheren Gefäße, von postischämischer Reperfusionsverletzung, Diabetes, rheumatoider Arthritis, Multipler Sklerose, Psoriasis, allergischer Dermatitis, Ekzemen, ulcerativer Colitis, Crohn'scher Krankheit, entzündlicher Darmkrankheit, Heliobacter pylori-Gastritis, nicht-Heliobacter pylori-Gastritis, durch nicht-steroidale entzündungshemmende Arzneimittel hervorgerufener Schädigung des Gastrointestinaltrakts oder einer psychotischen Störung oder zur Wundheilung.

38. Verfahren zur Herstellung einer Verbindung der Formel (I) nach Anspruch 1 oder eines pharmazeutisch annehmbaren Salzes oder Solvats davon, das einschließt
(a) Entschützen einer Verbindung der Formel 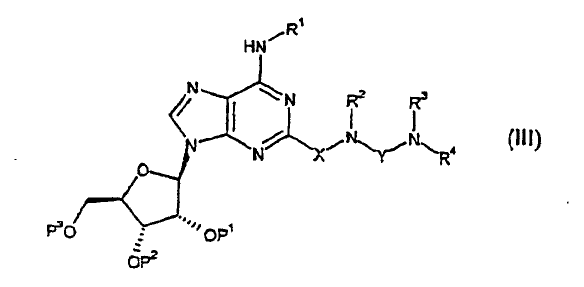 worin R¹, R², R³, R⁴, X und Y wie in Anspruch 1 definiert sind und entweder P¹, P² und P³ getrennt voneinander Schutzgruppen sind oder P¹ und P² zusammengenommen eine Schutzgruppe sind und P³ eine Schutzgruppe ist, wobei die Schutzgruppen zusammen oder aufeinander folgend entfernt werden; oder
(b) Entschützen einer Verbindung der Formel 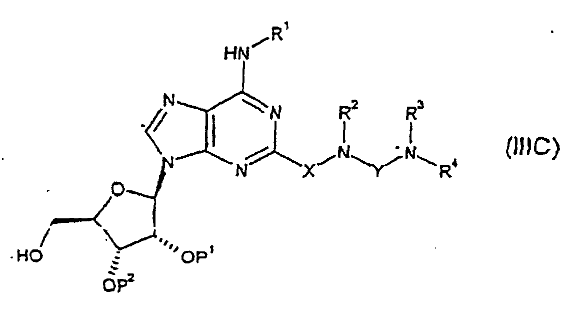 worin R¹, R², R³, R⁴, X und Y wie in Anspruch 1 definiert sind und entweder P¹ und P² getrennt voneinander Schutzgruppen sind oder P¹ und P² zusammengenommen eine Schutzgruppe sind und die Schutzgruppen P¹ und P² getrennt voneinander genommen entweder zusammen oder aufeinander folgend entfernt werden; oder
(c) Entschützen einer Verbindung der Formel 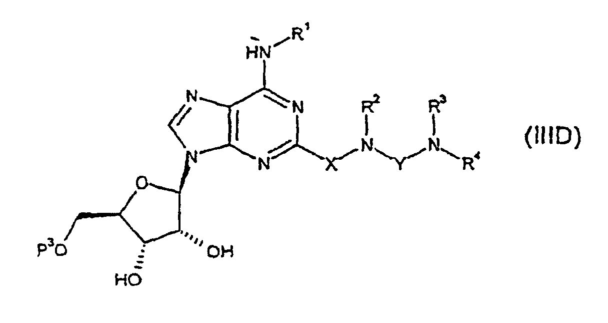 worin P³ eine Schutzgruppe ist und R¹, R², R³, R⁴, X und Y wie in Anspruch 1 definiert sind oder
(d) Entschützen einer Verbindung der Formel 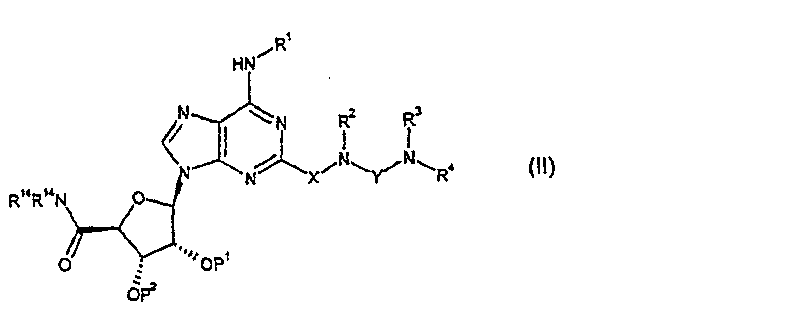
worin R¹, R², R³, R⁴, R¹⁴, X und Y wie in Anspruch 1 definiert sind und entweder P¹ und P², wenn getrennt genommen, Schutzgruppen sind oder P¹ und P², wenn zusammengenommen, eine Schutzgruppe sind, wobei die Schutzgruppen P¹ und P², wenn getrennt genommen, entweder zusammen oder aufeinander folgend entfernt werden;
wobei sich an jedes der Verfahren (a) bis (d) gegebenenfalls die Umwandlung der Verbindung der Formel (I) zu einem pharmazeutisch annehmbaren Salz davon anschließt.

39. Verfahren zur Herstellung einer wie in Anspruch 1 definierten Verbindung der Formel (I) oder eines pharmazeutisch annehmbaren Salzes oder Solvats davon, das die Reaktion einer Verbindung der Formel 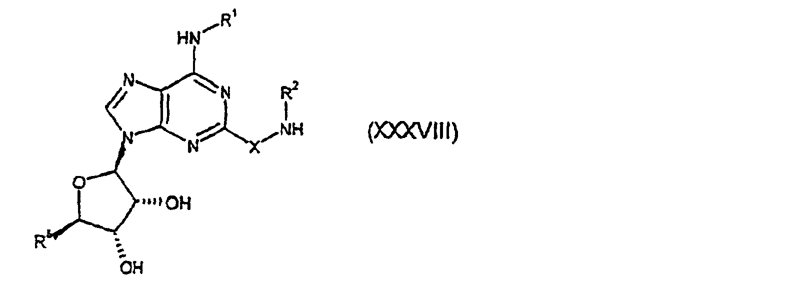 worin R¹, R², R⁵ und X wie in Anspruch 1 definiert sind, mit
(a) einer Verbindung der Formel 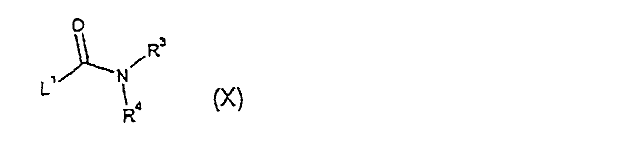 worin R³ und R⁴ wie in Anspruch 1 definiert sind und L¹ eine geeignete Abgangsgruppe, vorzugsweise Imidazol-1-yl, ist; oder
(b) einer Verbindung der Formel
R³R⁴NSO₂L⁹ (XVII)
worin R³ und R⁴ wie in Anspruch 1 definiert sind und L⁹ eine geeignete Abgangsgruppe, vorzugsweise Chlor, ist oder
(c) einer Verbindung der Formel 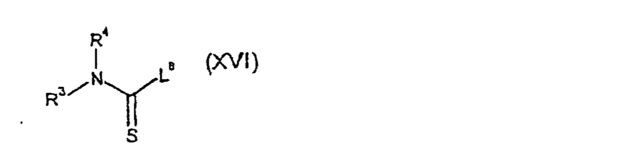 worin R³ und R⁴ wie in Anspruch 1 definiert sind und L⁸ eine geeignete Abgangsgruppe, vorzugsweise Methylthio oder Imidazol-1-yl, ist; oder
(d) einer Verbindung der Formel 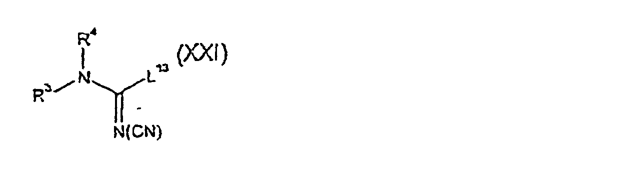
worin R³ und R⁴ wie in Anspruch 1 definiert sind und L¹³ eine geeignete Abgangsgruppe, vorzugsweise Methylhio, ist;
einschließt, wobei sich an das Verfahren gegebenenfalls die Umwandlung der Verbindung der Formel (I) in ein pharmazeutisch annehmbares Salz davon anschließt.

40. Ein Verfahren für die Herstellung einer wie in Anspruch 1 definierten Verbindung der Formel (I) oder eines pharmazeutisch annehmbaren Salzes oder Solvats davon, welches einschließt
(a) die Umsetzung einer Verbindung der Formel 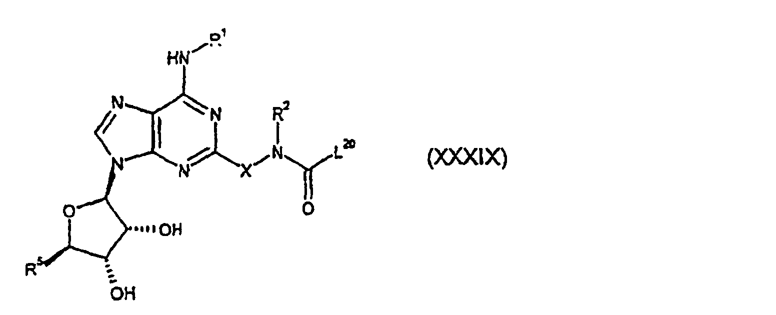 worin R¹, R², R⁵ und X wie in Anspruch 1 definiert sind und L²⁰ eine geeignete Abgangsgruppe, vorzugsweise Imidazol-1-yl, ist mit einer Verbindung der Formel
R³R⁴NH (XI)
worin R³ und R⁴ wie in Anspruch 1 definiert sind oder
(b) die Umsetzung einer Verbindung der Formel 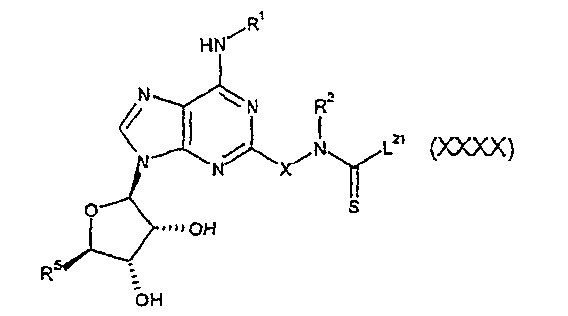 worin R¹, R², R⁵ und X wie in Anspruch 1 definiert sind und L²¹ eine geeignete Abgangsgruppe, vorzugsweise Methylthio oder Imidazol-1-yl, ist, mit einer Verbindung der Formel
R³R⁴NH (XI)
worin R³ und R⁴ wie in Anspruch i definiert sind, oder
(c) die Umsetzung einer Verbindung der Formel 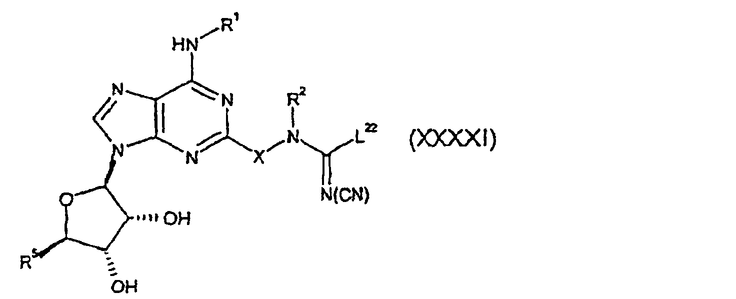
worin R¹, R², R⁵ und X wie in Anspruch 1 definiert sind und L²² eine geeignete Abgangsgruppe, vorzugsweise Methylthio, ist, mit einer Verbindung der Formel
R³R⁴NH (XI)
worin R³ und R⁴ wie in Anspruch 1 definiert sind,
wobei sich an jedes der Verfahren (a) bis (c) gegebenenfalls die Umwandlung der Verbindung der Formel (I) in ein pharmazeutisch annehmbares Salz davon anschließt.

41. Verfahren zur Herstellung einer wie in Anspruch 1 definierten Verbindung der Formel (I) oder eines pharmazeutisch annehmbaren Salzes oder Solvats davon, das die Acylierung oder Sulphonylierung einer Verbindung der Formel 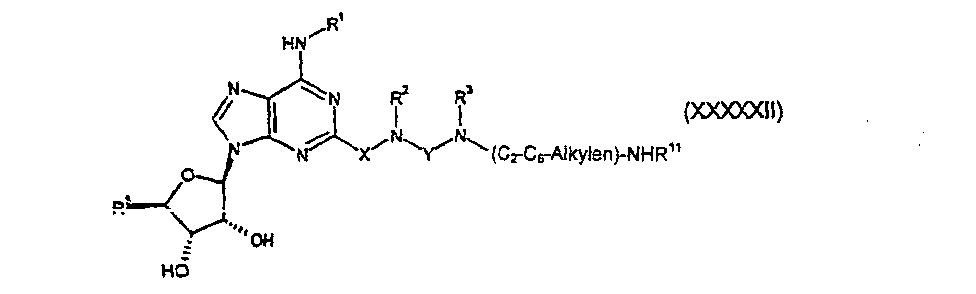 worin R¹, R², R³, R⁵, R¹¹, X und Y wie in Anspruch 1 definiert sind, einschließt,
wobei sich an das Verfahren gegebenenfalls die Umwandlung der Verbindung der Formel (I) in ein pharmazeutisch annehmbares Salz davon anschließt.

42. Eine Verbindung der Formel 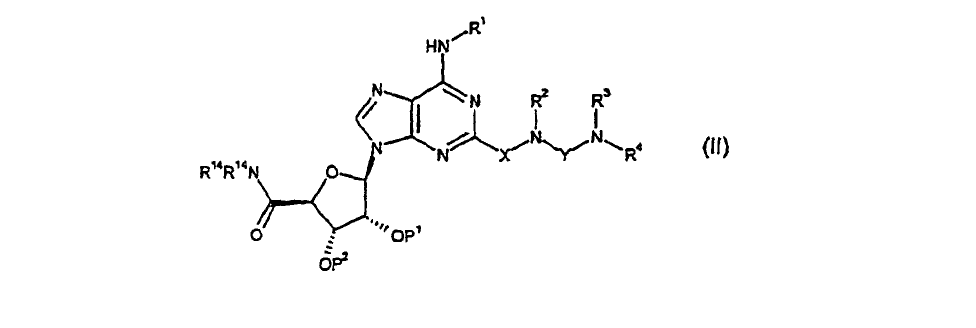 worin P¹ und P², wenn getrennt genommen, Schutzgruppen sind oder P¹ und P², wenn zusammengenommen, eine Schutzgruppe sind, oder 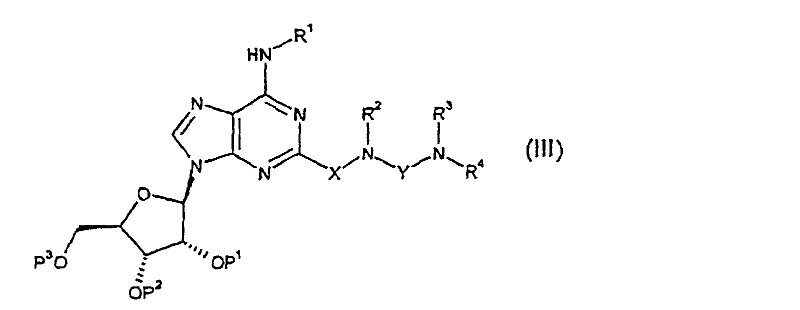 worin entweder P¹, P² und P³, wenn getrennt genommen, Schutzgruppen sind oder P¹ und P², wenn zusammengenommen, eine Schutzgruppe sind und P³ eine Schutzgruppe ist, oder 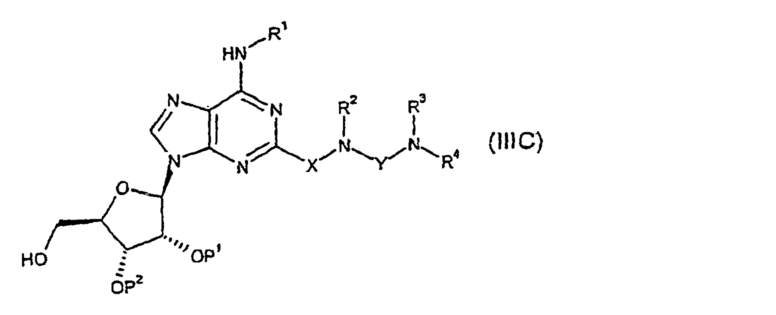 worin entweder P¹ und P², wenn getrennt genommen, Schutzgruppen sind, oder P¹ und P², wenn zusammengenommen, eine Schutzgruppe sind, oder 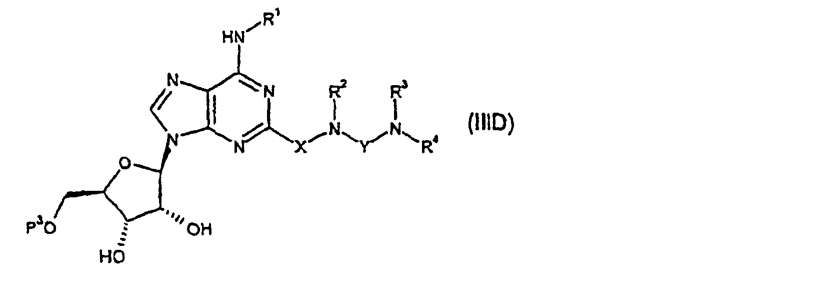 worin P³ eine Schutzgruppe ist, oder 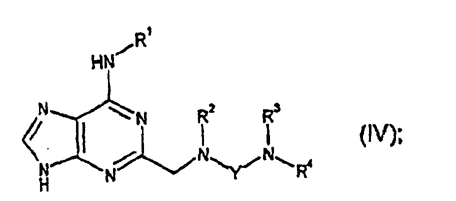 oder 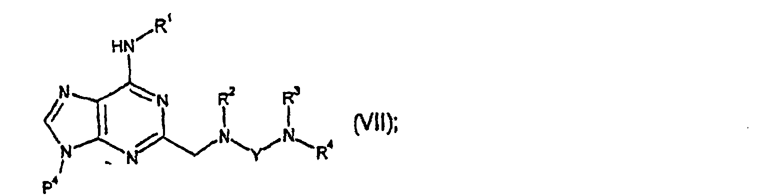 worin P⁴ eine Schutzgruppe ist, oder 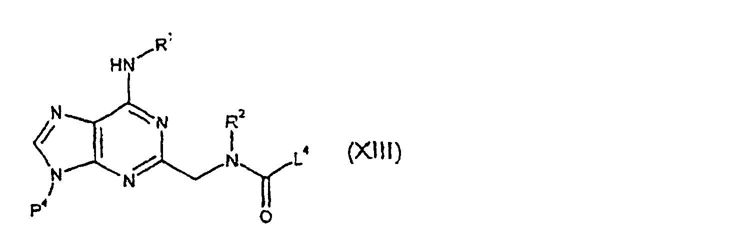 worin L⁴ eine geeignete Abgangsgruppe, vorzugsweise Imidazol-1-yl, ist und P⁴ für eine Schutzgruppe steht, oder 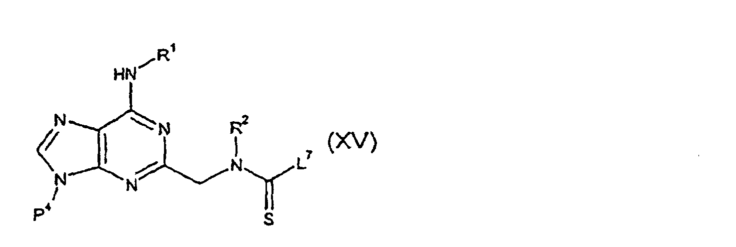 worin L⁷ eine geeignete Abgangsgruppe, vorzugsweise Methylthio oder Imidazol-1-yl, ist und P⁴ eine Schutzgruppe ist, oder 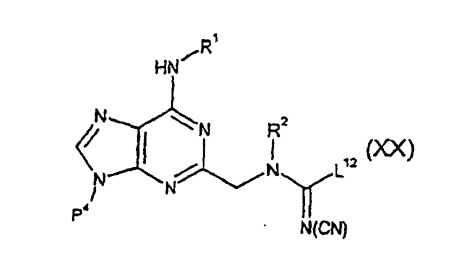 worin L¹² eine geeignete Abgangsgruppe, vorzugsweise Methylthio, ist und P⁴ eine Schutzgruppe ist, oder 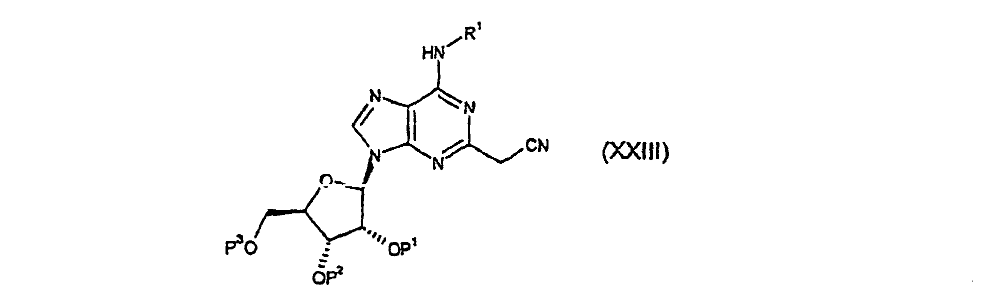 worin entweder P¹, P² und P³, wenn getrennt genommen, Schutzgruppen sind oder P¹ und P², wenn zusammengenommen, eine Schutzgruppe sind und P³ eine Schutzgruppe ist, oder 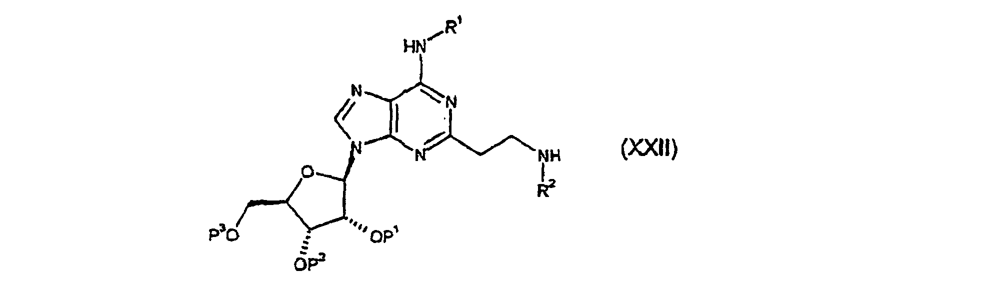 worin entweder P¹, P² und P³, wenn getrennt genommen, Schutzgruppen sind oder P¹ und P², wenn zusammengenommen, eine Schutzgruppe sind und P³ eine Schutzgruppe ist, oder 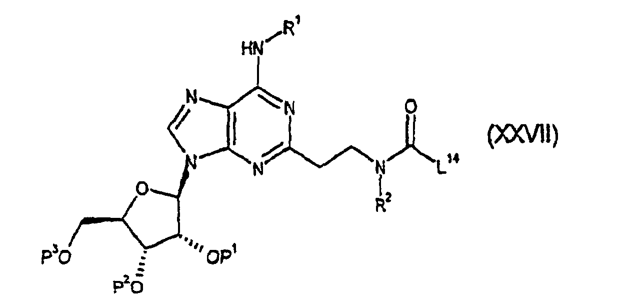 worin L¹⁴ eine geeignete Abgangsgruppe, vorzugsweise Imidazol-1-yl, ist und entweder P¹, P² und P³, wenn getrennt genommen, Schutzgruppen sind oder P¹ und P², wenn zusammengenommen, eine Schutzgruppe sind und P³ eine Schutzgruppe ist, oder 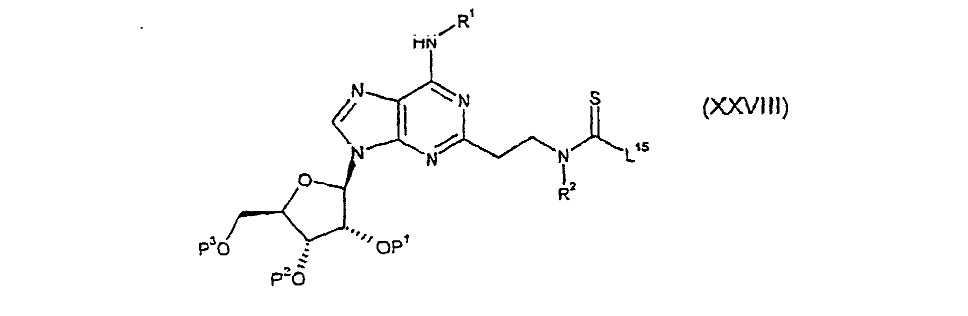 worin L¹⁵ eine geeignete Abgangsgruppe, vorzugsweise Methylthio oder Imidazol-1-yl, ist und entweder P¹, P² und P³, wenn getrennt voneinander genommen, Schutzgruppen sind oder P¹ und P², wenn zusammengenommen, eine Schutzgruppe sind und P³ eine Schutzgruppe ist, oder 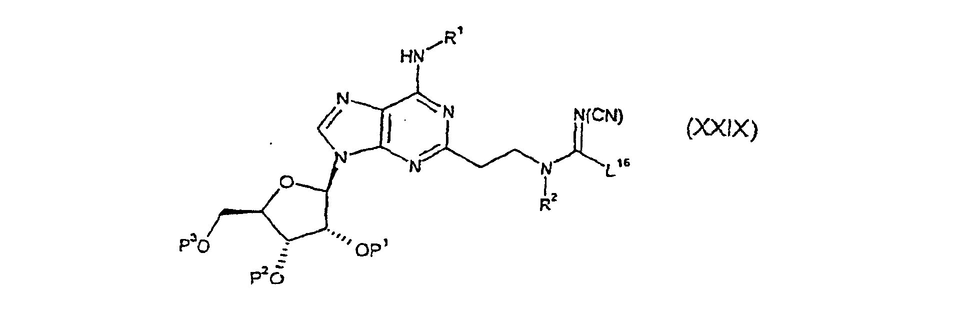 worin L¹⁶ eine geeignete Abgangsgruppe, vorzugsweise Methylthio, ist und entweder P¹, P² und P³, wenn getrennt genommen, Schutzgruppen sind oder P¹ und P², wenn zusammengenommen eine Schutzgruppe sind und P³ eine Schutzgruppe ist, oder 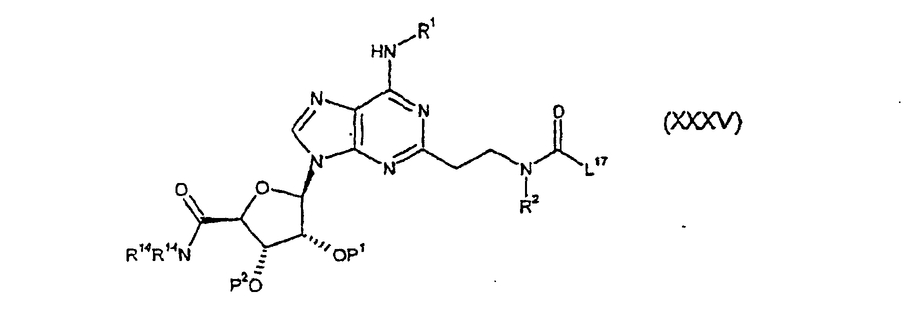 worin L¹⁷ eine geeignete Abgangsgruppe, worzugsweise Imidazol-1-yl, ist und entweder P¹ und P², wenn getrennt genommen, Schutzgruppen sind oder P¹ und P², wenn zusammengenommen, eine Schutzgruppe sind, oder 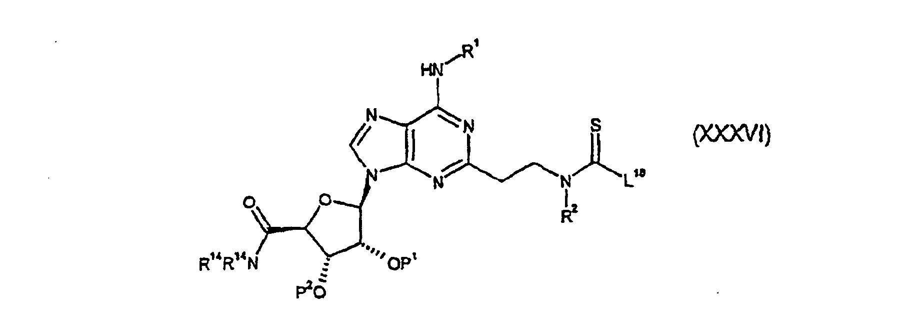 worin L¹⁸ eine geeignete Abgangsgruppe, vorzugsweise Imidazol-1-yl oder Methylthio, ist und entweder P¹ und P², wenn getrennt genommen, Schutzgruppen sind oder P¹ und P², wenn zusammengenommen, eine Schutzgruppe sind, oder 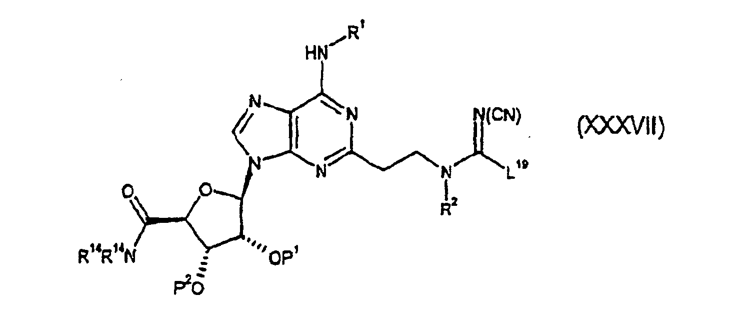 worin L¹⁹ eine geeignete Abgangsgruppe, vorzugsweise Methylthio, ist und entweder P¹ und P², wenn getrennt genommen, Schutzgruppen sind oder P¹ und P², wenn zusammengenommen, eine Schutzgruppe sind, oder 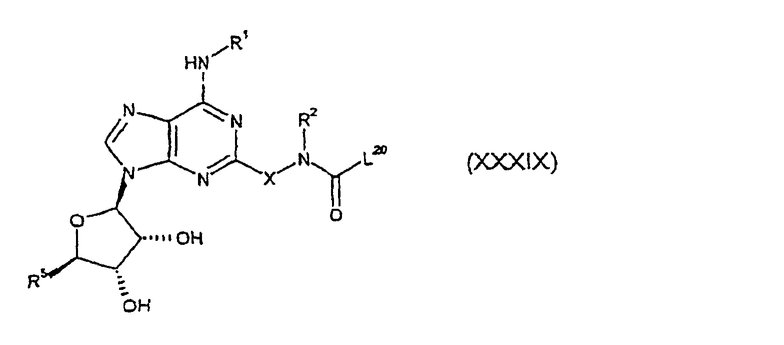 worin L²⁰ eine geeignete Abgangsgruppe, vorzugsweise Imidazol-1-yl, ist, oder 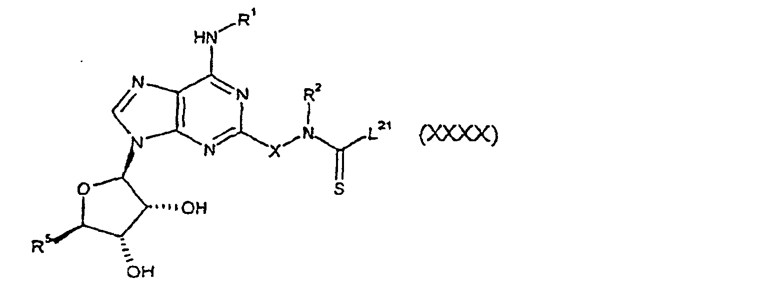 worin L²¹ eine geeignete Abgangsgruppe, vorzugsweise Methylthio oder Imidazol-1-yl, ist, oder 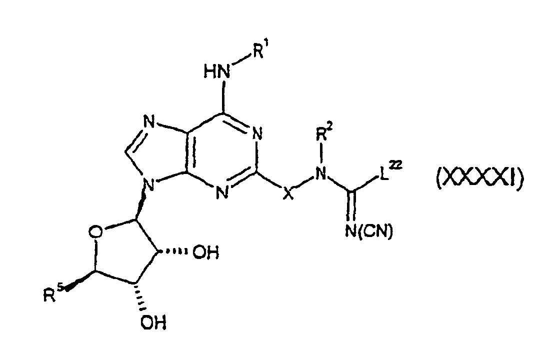 worin L²² eine geeignete Abgangsgruppe, vorzugsweise Methylthio, ist, oder 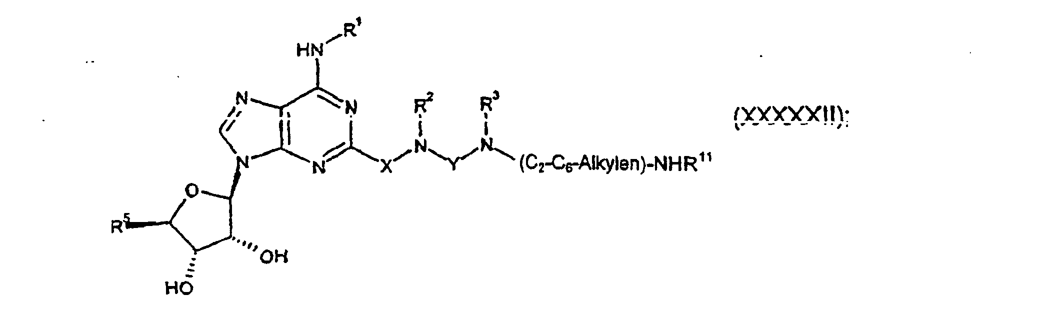 worin die Gruppen R¹, R², R³, R⁴, R⁵, R¹¹, R¹⁴, X und Y wie in Anspruch 1 definiert sind.

## Revendications

1. Composé de formule 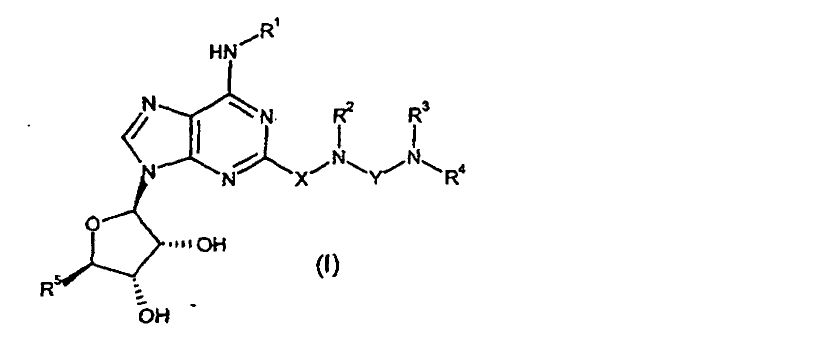 ou sel pharmaceutiquement acceptable ou solvate d'un tel composé, formule
dans laquelle
R¹ est (i) H, (ii) un groupe alkyle en C₁-C₆ portant éventuellement 1 ou 2 substituants choisis chacun indépendamment parmi les groupes phényle, naphtyle et fluorényle, lesdits groupes phényle, naphtyle et fluorényle étant éventuellement substitués par des groupes alkyle en C₁-C₆, alcoxy en C₁-C₆, halogéno ou cyano, ou (iii) le groupe fluorényle ;
R² est H ou un groupe alkyle en C₁-C₆ ;
soit R³ et R⁴, considérés ensemble avec l'atome d'azote auquel ils sont fixés, représentent un groupe azétidinyle, pyrrolidinyle, pipéridinyle, pipérazinyle, homopipéridinyle ou homopipérazinyle, chacun étant éventuellement substitué, sur un atome de carbone ou d'azote formant le cycle, par un groupe alkyle en C₁-C₆ ou cycloalkyle en C₃-C₈, et éventuellement substitué, sur un atome de carbone formant le cycle qui n'est pas adjacent à un atome d'azote formant le cycle, par -NR⁶R⁷ ou -OR⁹,
soit R³ est H, un groupe alkyle en C₁-C₆, cycloalkyle en C₃-C₈ ou benzyle, ledit groupe alkyle en C₁-C₆ étant éventuellement substitué par un groupe cycloalkyle en C₃-C₈, et R⁴ est
(a) un groupe alkyle en C₁-C₆, cycloalkyle en C₃-C₈ ou R¹⁵, ledit groupe alkyle en C₁-C₆ étant éventuellement substitué par R¹⁵, ou
(b) un groupe -[alkylène(C₂-C₆)]-R⁸, ou
(c) un groupe -[alkylène(C₁-C₆)]-R¹³ ;
R⁵ est -CH₂OH ou -CONR¹⁴R¹⁴ ;
R⁶ et R⁷ soit représentent chacun indépendamment H ou un groupe alkyle en C₁-C₆, soit, considérés ensemble avec l'atome d'azote auquel ils sont fixés, représentent un groupe azétidinyle, pyrrolidinyle ou pipéridinyle, lesdits groupes azétidinyle, pyrrolidinyle et pipéridinyle étant éventuellement substitués par des groupes alkyle en C₁-C₆ ;
R⁸ est
(i) un groupe azétidin-1-yle, pyrrolidin-1-yle, pipéridin-1-yle, morpholin-4-yle, pipérazin-1-yle, homopipéridin-1-yle, homopipérazin-1-yle ou tétra-hydro-isoquinolin-1-yle, chacun étant éventuellement substitué, sur un atome de carbone formant le cycle, par un groupe alkyle en C₁-C₆, cycloalkyle en C₃-C₈, phényle, alcoxy(C₁-C₆)-alkyle(C₁-C₆), R⁹R⁹-N-alkyle(C₁-C₆), fluoro-alkyle(C₁-C₆), -CONR⁹R⁹, -COOR⁹ ou un groupe alcanoyle en C₂-C₅, et éventuellement substitué, sur un atome de carbone formant le cycle qui n'est pas adjacent à un atome d'azote formant le cycle, par un atome d'halogène ou un groupe fluoro-alcoxy(C₁-C₆), -OR⁹, cyano, -S(O)ₘR¹⁰, -NR⁹R⁹, -SO₂NR⁹R⁹, -NR⁹COR¹⁰ ou -NR⁹SO₂R¹⁰, et lesdits groupes pipérazin-1-yle et homopipérazin-1-yle étant éventuellement substitués, sur l'atome d'azote formant le cycle qui n'est pas lié au groupe alkylène en C₂-C₆, par un groupe alkyle en C₁-C₆, phényle, alcoxy(C₁-C₆)-alkyle(C₂-C₆), R⁹R⁹N-alkyle(C₂-C₆), fluoro-alkyle(C₁-C₆), alcanoyle en C₂-C₅, -COOR¹⁰, cycloalkyle en C₃-C₈, -SO₂R¹⁰, -SO₂NR⁹R⁹ ou -CONR⁹R⁹, ou
(ii) -NR¹¹R¹² ;
R⁹ est H, un groupe alkyle en C₁-C₆, cycloalkyle en C₃-C₈ ou phényle ;
R¹⁰ est un groupe alkyle en C₁-C₆, cycloalkyle en C₃-C₈ ou phényle ;
R¹¹ est un groupe alkyle en C₁-C₆, cycloalkyle en C₃-C₈ ou benzyle ;
R¹² est un groupe alkyle en C₁-C₆, cycloalkyle en C₃-C₈, phényle, benzyle, fluoro-alkyle(C₁-C₆), -CONR⁹R⁹, -COOR¹⁰, -COR¹⁰, -SO₂R¹⁰ ou -SO₂NR⁹R⁹, ledit groupe alkyle en C₁-C₆ étant éventuellement substitué par un groupe phényle ;
R13 est un groupe phényle, pyridin-2-yle, pyridin-3-yle ou pyridin-4-yle, chacun étant éventuellement substitué par un atome d'halogène ou par un groupe alkyle en C₁-C₆, alcoxy en C₁-C₆ ou cyano ;
R14 est H ou un groupe alkyle en C₁-C₆ éventuellement substitué par le groupe cyclopropyle ;
R¹⁵ est un groupe azétidin-3-yle, pyrrolidin-3-yle, pipéridin-3-yle, pipéridin-4-yle, homopipéridin-3-yle ou homopipéridin-4-yle, chacun étant éventuellement substitué par R¹³, un groupe alkyle en C₁-C₆, cycloalkyle en C₃-C₈ ou benzyle ;
m est 0, 1 ou 2 ;
X est -CH₂- ou -CH₂CH₂- ; et
Y est CO, CS, SO₂ ou C=N(CN).

2. Composé de formule (I) tel que défini dans la revendication 1, dans lequel
R¹ est H, un groupe alkyle en C₁-C₆ ou fluorényle, ledit groupe alkyle en C₁-C₆ portant éventuellement 1 ou 2 substituants choisis chacun indépendamment parmi les groupes phényle et naphtyle, lesdits groupes phényle et naphtyle étant éventuellement substitués par des atomes d'halogène ou des groupes alkyle en C₁-C₆, alcoxy en C₁-C₆ ou cyano ;
R² est H ou un groupe alkyle en C₁-C₆ ;
soit R³ et R⁴, considérés ensemble avec l'atome d'azote auquel ils sont fixés, représentent un groupe azétidinyle, pyrrolidinyle, pipéridinyle, pipérazinyle, homopipéridinyle ou homopipérazinyle, chacun étant éventuellement substitué, sur un atome de carbone ou d'azote formant le cycle, par un groupe alkyle en C₁-C₆ ou cycloalkyle en C₃-C₈, et éventuellement substitué, sur un atome de carbone formant le cycle qui n'est pas adjacent à un atome d'azote formant le cycle, par -NR⁶R⁷,
soit R³ est H, un groupe alkyle en C₁-C₆, cycloalkyle en C₃-C₈ ou benzyle, et R⁴ est
(a) un groupe azétidin-3-yle, pyrrolidin-3-yle, pipéridin-3-yle, pipéridin-4-yle, homopipéridin-3-yle ou homopipérdin-4-yle, chacun étant éventuellement substitué par un groupe alkyle en C₁-C₆, cycloalkyle en C₃-C₈ ou benzyle, ou
(b) un groupe -[alkylène(C₂-C₆)]-R⁸, ou
(c) un groupe -[alkylène(C₁-C₆)]-R¹³ ;
R⁵ est CH₂OH ou -CONR¹⁴R¹⁴ ;
R6 et R7 soit représentent chacun indépendamment H ou un groupe alkyle en C₁-C₆, soit, considérés ensemble avec l'atome d'azote auquel ils sont fixés, représentent un groupe azétidinyle, pyrrolidinyle ou pipéridinyle, lesdits groupes azétidinyle, pyrrolidinyle et pipéridinyle étant éventuellement substitués par des groupes alkyle en C₁-C₆ ;
R⁸ est
(i) un groupe azétidin-1-yle, pyrrolidin-1-yle, pipéridin-1-yle, morpholin-4-yle, pipérazin-1-yle, homopipéridin-1-yle, homopipérazin-1-yle ou tétra-hydro-isoquinolin-1-yle, chacun étant éventuellement substitué, sur un atome de carbone formant le cycle, par un groupe alkyle en C₁-C₆, cycloalkyle en C₃-C₈, phényle, alcoxy(C₁-C₆)-alkyle(C₁-C₆), R⁹R⁹N-alkyle(C₁-C₆), fluoro-alkyle(C₁-C₆), -CONR⁹R⁹, -COOR⁹ ou un groupe alcanoyle en C₂-C₅, et éventuellement substitué, sur un atome de carbone formant le cycle qui n'est pas adjacent à un atome d'azote formant le cycle, par un atome d'halogène ou un groupe fluoro-alcoxy(C₁-C₆), -OR⁹, cyano, -S(O)ₘR¹⁰, -NR9R9, -SO₂NR⁹R⁹, -NR⁹COR¹⁰ ou -NR⁹SO₂R¹⁰, et lesdits groupes pipérazin-1-yle et homopipérazin-1-yle étant éventuellement substitués, sur l'atome d'azote formant le cycle qui n'est pas lié au groupe alkylène en C₂-C₆, par un groupe alkyle en C₁-C₆, phényle, alcoxy(C₁-C₆)-alkyle(C₂-C₆), R⁹R⁹N-alkyle(C₂-C₆), fluoro-alkyle(C₁-C₆), alcanoyle en C₂-C₅, -COOR¹⁰, cycloalkyle en C₃-C₈, -SO₂R¹⁰, -SO₂NR⁹R⁹ ou -CONR⁹R⁹, ou
(ii) -NR¹¹R¹² ;
R⁹ est H, un groupe alkyle en C₁-C₆, cycloalkyle en C₃-C₈ ou phényle ;
R¹⁰ est un groupe alkyle en C₁-C₆, cycloalkyle en C₃-C₈ ou phényle ;
R¹¹ est H, un groupe alkyle en C₁-C₆, cycloalkyle en C₃-C₈ ou benzyle ;
R¹² est H, un groupe alkyle en C₁-C₆, cycloalkyle en C₃-C₈, phényle, benzyle, fluoro-alkyle(C₁-C₆), -CONR⁹R⁹, -COOR¹⁰, alcanoyle en C₂-C₅ ou -SO₂NR⁹R⁹ ;
R¹³ est un groupe phényle, pyridin-2-yle, pyridin-3-yle ou pyridin-4-yle, chacun étant éventuellement substitué par un atome d'halogène ou par un groupe alkyle en C₁-C₆, alcoxy en C₁-C₆ ou cyano ;
R¹⁴ est H ou un groupe alkyle en C₁-C₆ éventuellement substitué par le groupe cyclopropyle ;
m est 0, 1 ou 2 ;
X est -CH₂- ou -CH₂CH₂- ; et
Y est CO, CS, SO₂ ou C=N(CN).

3. Composé selon la revendication 1, dans lequel R¹ est un groupe alkyle en C₁-C₆ portant éventuellement 1 ou 2 substituants choisis chacun indépendamment parmi les groupes phényle, naphtyle et fluorényle, lesdits groupes phényle, naphtyle et fluorényle étant éventuellement substitués par des atomes d'halogène ou par des groupes alkyle en C₁-C₆, alcoxy en C₁-C₆ ou cyano.

4. Composé selon la revendication 3, dans lequel R¹ est le groupe 2,2-diphényléth-1-yle, 2,2-di(4-chlorophényl)éth-1-yle, 2,2-di(3-chlorophényl)éth-1-yle, 2,2-di(4-méthylphényl)éth-1-yle, 2,2-di(3-méthylphényl)éth-1-yle, napht-1-ylméthyle ou fluorén-9-ylméthyle.

5. Composé selon l'une quelconque des revendications précédentes, dans lequel R² est H ou un groupe alkyle en C₁-C₄.

6. Composé selon la revendication 5, dans lequel R² est H ou le groupe méthyle.

7. Composé selon l'une quelconque des revendications précédentes, dans lequel R³ est H ou un groupe alkyle en C₁-C₆.

8. Composé selon la revendication 7, dans lequel R³ est H ou le groupe méthyle.

9. Composé selon la revendication 1 ou l'une quelconque des revendications 3 à 8, dans lequel R⁴ est (a) un groupe alkyle en C₁-C₄ substitué par -R¹⁵, cycloalkyle en C₃-C₆ ou -R¹⁵, ou (b) un groupe [alkylène(C₂-C₄)]-R⁸ ou (c) un groupe -[alkylène(C₁-C₄)]-R¹³.

10. Composé selon la revendication 9, dans lequel R⁴ est un groupe -CH₂R¹⁵, cyclohexyle, -R¹⁵, -CH₂CH₂R⁸, -CH₂R¹³ ou -CH₂CH₂R¹³.

11. Composé selon l'une quelconque des revendications précédentes, dans lequel R⁵ est un groupe -CH₂OH ou -CONH[alkyle(C₁-C₆)].

12. Composé selon la revendication 11, dans lequel R⁶ est un groupe -CH₂OH ou -CONHCH₂CH₃.

13. Composé selon la revendication 1 ou l'une quelconque des revendications 3 à 12, dans lequel R⁸ est (i) un groupe pipéridin-1-yle ou 1,2,3,4-tétrahydro-isoquinolin-2-yle, chacun étant éventuellement substitué, sur un atome de carbone formant le cycle, par un groupe alkyle en C₁-C₆, cycloalkyle en C₃-C₈, phényle, alcoxy(C₁-C₆)-alkyle(C₁-C₆), R⁹R⁹N-alkyle(C₁-C₆), fluoro-alkyle(C₁-C₆), -CONR⁹R⁹, -COOR⁹ ou alcanoyle en C₂-C₅, et éventuellement substitué, sur un atome de carbone formant le cycle qui n'est pas adjacent à un atome d'azote formant le cycle, par un atome d'halogène ou un groupe fluoro-alcoxy(C₁-C₆), -OR⁹, cyano, -S(O)ₘR¹⁰, -NR⁹R⁹, -SO₂NR⁹R⁹, -NR⁹COR¹⁰ ou -NR⁹SO₂R¹⁰, ou (ii) un groupe -NR¹¹R¹².

14. Composé selon la revendication 13, dans lequel R⁸ est un groupe pipéridin-1-yle, 4-(2-propyl)pipéridin-1-yle, 2,2,6,6-tétraméthylpipéridin-1-yle, 1,2,3,4-tétrahydro-isoquinolin-2-yle ou -NR¹¹R¹².

15. Composé selon l'une quelconque des revendications précédentes, dans lequel R¹¹ est un groupe alkyle en C₁-C₆ ou cycloalkyle en C₃-C₈.

16. Composé selon la revendication 15, dans lequel R¹¹ est un groupe -CH(CH₃)₂, -CH₂CH₂CH₂CH₃, -CH₂CH(CH₃)₂, -C(CH₃)₃, -CH(CH₂CH₃)₂, cyclohexyle ou cyclopentyle.

17. Composé selon la revendication 1 ou l'une quelconque des revendications 3 à 16, dans lequel R¹² est un groupe alkyle en C₁-C₆, cycloalkyle en C₃-C₈, -COR¹⁰ ou -SO₂R¹⁰, ledit groupe alkyle en C₁-C₆ étant éventuellement substitué par le groupe phényle.

18. Composé selon la revendication 17, dans lequel R¹² est un groupe -CH(CH₃)₂, -CH₂CH₂CH₂CH₃, -CH₂CH(CH₃)₂, -C(CH₃)₃, -CH(CH₂CH₃)₂, -C(CH₃)₂Ph, -SO₂Ph, -COPh, cyclohexyle ou cyclopentyle.

19. Composé selon l'une quelconque des revendications précédentes, dans lequel R¹³ est un groupe phényle ou pyridin-2-yle, chacun étant éventuellement substitué par un atome d'halogène ou par un groupe alkyle en C₁-C₆, alcoxy en C₁-C₆ ou cyano.

20. Composé selon la revendication 18, dans lequel R¹³ est le groupe phényle ou pyridin-2-yle.

21. Composé selon la revendication 1 ou l'une quelconque des revendications 3 à 20, dans lequel R¹⁵ est un groupe pyrrolidin-3-yle ou pipéridin-4-yle, chacun étant éventuellement substitué par R¹³, un groupe alkyle en C₁-C₆, cycloalkyle en C₃-C₈ ou benzyle.

22. Composé selon la revendication 21, dans lequel R¹⁵ est le groupe 1-benzylpipéridin-4-yle, 1-(2-pyridinyl)pipéridin-4-yle ou 1-benzylpyrrolidin-3-yle.

23. Composé selon l'une quelconque des revendications précédentes, dans lequel X est -CH₂-.

24. Composé selon l'une quelconque des revendications précédentes, dans lequel Y est CO ou C=N(CN).

25. Composé selon la revendication 1, qui est choisi dans l'ensemble constitué par les composés suivants :
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-[2-diisopropylamino)éthyl]urée,
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-[2-(1-pipéridinyl)éthyl]urée,
(2S,3S,4R,5R)-5-{2-{[({[2-(diisopropylamino)éthyl]amino}carbonyl)amino]-méthyl}-6-[(2,2-diphényléthyl)amino]-9H-purin-2-yl}-N-éthyl-3,4-dihydroxy-tétrahydro-2-furannecarboxamide,
(2S,3S,4R,5R)-5-(6-[(2,2-diphényléthyl)amino-2-{[({[2-(1-pipéridinyl)éthyl]-amino}carbonyl)amino]méthyl}-9H-purin-9-yl)-N-éthyl-3,4-dihydroxytétra-hydro-2-furannecarboxamide,
(2S,3S,4R,5R)-5-{2-{[((E)-(cyano-imino){[2-(1-pipéridinyl)éthyl]amino}-méthyl)amino]méthyl}-6-[(2,2-diphényléthyl)amino]-9H-purin-9-yl}-N-éthyl-3,4-dihydroxytétrahydro-2-furannecarboxamide,
(2S,3S,4R,5R)-5-{2-({[(benzylamino)carbonyl]amino}méthyl)-6-[(2,2-diphényléthyl)amino]-9H-purin-9-yl}-N-éthyl-3,4-dihydroxytétrahydro-2-furannecarboxamide,
(2S,3S,4R,5R)-5-{2-({[(cyclohexylamino)carbonyl]amino}méthyl)-6-[(2,2-diphényléthyl)amino]-9H-purin-9-yl}-N-éthyl-3,4-dihydroxytétrahydro-2-furannecarboxamide,
(2S,3S,4R,5R)-5-{2-({[({2-[benzoyl(isopropyl)amino]éthyl}amino)carbonyl]-amino}méthyl)-6-[(2,2-diphényléthyl)amino]-9H-purin-9-yl}-N-éthyl-3,4-dihydroxytétrahydro-2-furannecarboxamide,
(2S,3S,4R,5R)-5-[6-[(2,2-diphényléthyl)amino]-2-({[({2-[isopropyl(phénylsulfonyl)amino]éthyl}amino)carbonyl]amino}méthyl)-9H-purin-9-yl]-N-éthyl3,4-dihydroxytétrahydro-2-furannecarboxamide,
N'-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N-méthyl-N-[2-(2-pyridinyl)éthyl] urée,
(2S,3S,4R,5R)-5-{2-[({[(1-benzyl-4-pipéridinyl)amino]carbonyl}amino)méthyl]-6-[(2,2-diphényléthyl)amino]-9H-purin-9-yl}-N-éthyl-3,4-dihydroxytétrahydro-2-furannecarboxamide,
(2S,3S,4R,5R)-5-[6-[(2,2-diphényléthyl)amino]-2-({[({2-[(1-éthylpropyl)(isobutyl)amino]éthyl}amino)carbonyl]amino}méthyl)-9H-purin-9-yl]-N-éthyl-3,4-dihydroxytétrahydro-2-furannecarboxamide,
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-{2-[(1-éthylpropyl)-(isobutyl)amino]éthyl}urée,
N-[2-(3,4-dihydro-2(1H)-isoquinolinyl)éthyl]-N'-({9-[(2R,3R,4S,5R)-3,4-dihydroxy-5-(hydroxyméthyl)tétrahydro-2-furannyl]-6-[(2,2-diphényléthyl)-amino]-9H-purin-2-yl}méthyl)urée,
(2S,3S,4R,5R)-5-{2-{[({[2-(3,4-dihydro-2(1H)-isoquinolinyl)éthyl]amino}-carbonyl)amino]méthyl}-6-[(2,2-diphényléthyl)amino]-9H-purin-9-yl}-N-éthyl-3,4-dihydroxytétrahydro-2-furannecarboxamide,
(2S,3S,4R,5R)-5-{2-{[({[2-(dibutylamino)éthyl]amino}carbonyl)amino]-méthyl}-6-[(2,2-diphényléthyl)amino]-9H-purin-9-yl}-N-éthyl-3,4-dihydroxytétrahydro-2-furannecarboxamide,
(2S,3S,4R,5R)-5-{2-({[({2-[cyclopentyl(isopropyl)amino]éthyl}amino)-carbonyl]amino}méthyl)-6-[(2,2-diphényléthyl)amino]-9H-purin-9-yl}-N-éthyl-3,4-dihydroxytétrahydro-2-furannecarboxamide,
N-{2-[cyclopentyl(isopropyl)amino]éthyl}-N'-({9-[{2R,3R,4S,5R)-3,4-dihydroxy-5-(hydroxyméthyl)tétrahydro-2-furannyl]-6-[(2,2-diphényléthyl)amino]-9H-purin-2-yl}méthyl)urée,
(2S,3S,4R,5R)-5-(6-[(2,2-diphényléthyl)amino]-2-{[({[1-(2-pyridinyl)-4-pipéridinyl]amino}carbonyl)amino]méthyl}-9H-purin-9-yl}-N-éthyl-3,4-dihydroxytétrahydro-2-furannecarboxamide,
(2S,3S,4R,5R)-5-(6-[(2,2-diphényléthyl)amino]-2-{[méthyl-({[2-(1-pipéridinyl)-éthyl]amino}carbonyl)amino]méthyl}-9H-purin-9-yl)-N-éthyl-3,4-dihydroxytétrahydro-2-furannecarboxamide,
(2S,3S,4R,5R)-5-{2-({[({2-[tert-butyl(cyclohexyl)amino]éthyl}amino)carbonyl]-amino}méthyl)-6-[(2,2-diphényléthyl)amino]-9H-purin-9-yl}-N-éthyl-3,4-dihydroxytétrahydro-2-furannecarboxamide,
N-{2-[tert-butyl(cyclohexyl)amino]éthyl}-N'-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)urée,
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-[1-(2-pyridinyl)-4-pipéridinyl]urée,
N-[(1-benzyl-4-pipéridinyl)méthyl]-N'-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)urée,
(2*S*,3*S*,4*R*,5*R*)-5-(6-[(2,2-diphényléthyl)amino]-2-{[({[(1-benzyl-4-piperidinyl)méthyl]amino}carbonyl)amino]méthyl}-9*H*-purin-9-yl)-*N*-éthyl-3,4-dihydroxytétrahydro-2-furannecarboxamide;
(2S,3S,4R,5R)-5-[6-[(2,2-diphényléthyl)amino]-2-({[({2-[isopropyl-(1-méthyl-1-phényléthyl)amino]éthyl}amino)carbonyl]amino}méthyl)-9H-purin-9-yl]-N-éthyl-3,4-dihydroxytétrahydro-2-furannecarboxamide,
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-{2-[isopropyl-(1-méthyl-1-phényléthyl)amino]éthyl}urée,
N-[2-(dicyclopentylamino)éthyl]-N'-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)urée,
N-({9-[(2R,3R,4S,5R)-3,4-dihydroxy-5-(hydroxyméthyl)tétrahydro-2-furannyl]-6-[(9H-fluorén-9-ylméthyl)amino]-9H-purin-2-yl}méthyl)-N'-[2-(diisopropylamino)éthyl]urée,
N-({9-[(2R,3R,4S,5R)-3,4-dihydroxy-5-(hydroxyméthyl)tétrahydro-2-furannyl]-6-[(2,2-diphényléthyl)amino]-9H-purin-2-yl}méthyl)-N'-[2-(2,2,6,6-tétraméthyl-1-pipéridinyl)éthyl]urée,
(2S,3S,4R,5R)-5-(6-[(2,2-diphényléthyl)amino]-2-{[({[2-(4-isopropyl-1-pipéridinyl)éthyl]amino}carbonyl)amino]méthyl}-9H-purin-9-yl)-N-éthyl-3,4-dihydroxytétrahydro-2-furannecarboxamide,
(2S,3S,4R,5R)-5-(6-[(2,2-diphényléthyl)amino]-2-{[({[2-(2,2,6,6-tétraméthyl-1-pipéridinyl)éthyl]amino}carbonyl)amino]méthyl}-9H-purin-9-yl)-N-éthyl-3,4-dihydroxytétrahydro-2-furannecarboxamide,
N-[(3R)-1-benzypyrrolidinyl]-N'-({9-[(2R,3R,4S,5R)-3,4-dihydroxy-5-(hydroxyméthyl)tétrahydro-2-furannyl]-6-[(2,2-diphényléthyl)amino]-9H-purin-2-yl}-méthyl)urée,
(2S,3S,4R,5R)-5-{2-{[({[(3R)-1-benzylpyrrolidinyl]amino}carbonyl)amino]-méthyl}-6-[(2,2-diphényléthyl)amino]-9H-purin-9-yl)-N-éthyl-3,4-dihydroxytétrahydro-2-furannecarboxamide,
(2S,3S,4R,5R)-5-(6-{[2,2-bis(4-chlorophényl)éthyl]amino}-2-{[({[2-(diisopropylamino)éthyl]amino}carbonyl)amino]méthyl}-9H-purin-9-yl)-N-éthyl-3,4-dihydroxytétrahydro-2-furannecarboxamide,
N-({6-{[2,2-bis(4-chlorophényl)éthyl]amino}-9-[(2R,3R,4S,5R)-3,4-dihydroxy-5-(hydroxyméthyl)tétrahydro-2-furannyl]-9H-purin-2-yl}méthyl)-N'-[2-(diisopropylamino)éthyl]urée,
N-({6-{[2,2-bis(4-méthylphényl)éthyl]amino}-9-[(2R,3R,4S,5R)-3,4-dihydroxy-5-(hydroxyméthyl)tétrahydro-2-furannyl]-9H-purin-2-yl}méthyl)-N'-[2-(diisopropylamino)éthyl]urée,
N-({6-{[2,2-bis(3-chlorophényl)éthyl]amino}-9-[(2R,3R,4S,5R)-3,4-dihydroxy-5-(hydroxyméthyl)tétrahydro-2-furannyl]-9H-purin-2-yl}méthyl)-N'-[2-(diisopropylamino)éthyl]urée
N-({6-{[2,2-bis(3-méthylphényl)éthyl]amino}-9-[(2R,3R,4S,5R)-3,4-dihydroxy-5-(hydroxyméthyl)tétrahydro-2-furannyl]-9H-purin-2-yl}méthyl)-N'-[2-(diisopropylamino)éthyl]urée et
(2S,3S,4R,5R)-5-{2-{[({[2-(diisopropylamino)éthyl]amino}carbonyl)amino]-méthyl}-6-[(1-naphtylméthyl)amino]-9H-purin-9-yl}-N-éthyl-3,4-dihydroxytétrahydro-2-furannecarboxamide ;
et leurs sels pharmaceutiquement acceptables et solvates.

26. Composition pharmaceutique comprenant un composé de formule (I) ou un sel pharmaceutiquement acceptable ou solvate d'un tel composé, tels que définis dans l'une quelconque des revendications précédentes, conjointement avec un excipient, diluant ou véhicule pharmaceutiquement acceptable.

27. Composé de formule (I) ou sel pharmaceutiquement acceptable, solvate ou composition d'un tel composé, tels que définis dans l'une quelconque des revendications 1 à 25 et 26, respectivement, pour utilisation en tant que médicament.

28. Utilisation d'un composé de formule (I) ou d'un sel pharmaceutiquement acceptable, solvate ou composition d'un tel composé, tels que définis dans l'une quelconque des revendications 1 à 25 et 26, respectivement, pour la fabrication d'un médicament destiné à traiter une maladie pour laquelle est indiqué un agoniste de récepteur A2a.

29. Utilisation d'un composé de formule (I) ou d'un sel pharmaceutiquement acceptable, solvate ou composition d'un tel composé, tels que définis dans l'une quelconque des revendications 1 à 25 et 26, respectivement, pour la fabrication d'un agent anti-inflammatoire.

30. Utilisation d'un composé de formule (I) ou d'un sel pharmaceutiquement acceptable, solvate ou composition d'un tel composé, tels que définis dans l'une quelconque des revendications 1 à 25 et 26, respectivement, pour la fabrication d'un médicament destiné au traitement d'une maladie respiratoire.

31. Utilisation selon la revendication 30, dans laquelle la maladie est choisie dans l'ensemble constitué par le syndrome de détresse respiratoire de l'adulte (SDRA), la bronchite, la bronchite chronique, la bronchopneumopathie chronique obstructive, la fibrose kystique, l'asthme, l'emphysème, la bronchectasie, la sinusite chronique et la rhinite.

32. Utilisation d'un composé de formule (I) ou d'un sel pharmaceutiquement acceptable, d'un solvate ou d'une composition d'un tel composé, tels que définis dans l'une quelconque des revendications 1 à 25 et 26, respectivement, pour la fabrication d'un médicament destiné au traitement du choc septique, du dysfonctionnement érectile masculin, de la stérilité factorielle masculine, de la stérilité factorielle féminine, de l'hypertension, de l'accident vasculaire cérébral, de l'épilepsie, de l'ischémie cérébrale, d'une maladie vasculaire périphérique, d'une lésion de reperfusion post-ischémique, du diabète, de la polyarthrite rhumatoïde, de la sclérose en plaques, du psoriasis, de la dermatite allergique, de l'eczéma, de la colite ulcéreuse, de la maladie de Crohn, de la maladie intestinale inflammatoire, de la gastrite à *Helicobacter pylori*, de la gastrite non à *Helicobacter pylori*, d'un endommagement du tractus gastro-intestinal dû à un anti-inflammatoire non stéroïdien ou d'un trouble psychotique, ou pour la cicatrisation.

33. Composé de formule (I) ou sel pharmaceutiquement acceptable, solvate ou composition d'un tel composé, tels que définis dans l'une quelconque des revendications 1 à 25 et 26, respectivement, pour utilisation en tant qu'agoniste de récepteur A2a.

34. Composé de formule (I) ou sel pharmaceutiquement acceptable, solvate ou composition d'un tel composé, tels que définis dans l'une quelconque des revendications 1 à 25 et 26, respectivement, pour utilisation en tant qu'agent anti-inflammatoire.

35. Composé de formule (I) ou sel pharmaceutiquement acceptable, solvate ou composition d'un tel composé, tels que définis dans l'une quelconque des revendications 1 à 25 et 26, respectivement, pour utilisation dans le traitement d'une maladie respiratoire.

36. Composé selon la revendication 35, pour lequel la maladie est choisie dans l'ensemble constitué par le syndrome de détresse respiratoire de l'adulte (SDRA), la bronchite, la bronchite chronique, la bronchopneumopathie chronique obstructive, la fibrose kystique, l'asthme, l'emphysème, la bronchectasie, la sinusite chronique et la rhinite.

37. Composé de formule (I) ou sel pharmaceutiquement acceptable, solvate ou composition d'un tel composé, tels que définis dans l'une quelconque des revendications 1 à 25 et 26, respectivement, pour utilisation dans le traitement du choc septique, du dysfonctionnement érectile masculin, de la stérilité factorielle masculine, de la stérilité factorielle féminine, de l'hypertension, de l'accident vasculaire cérébral, de l'épilepsie, de l'ischémie cérébrale, d'une maladie vasculaire périphérique, d'une lésion de reperfusion post-ischémique, du diabète, de la polyarthrite rhumatoïde, de la sclérose en plaques, du psoriasis, de la dermatite allergique, de l'eczéma, de la colite ulcéreuse, de la maladie de Crohn, de la maladie intestinale inflammatoire, de la gastrite à *Helicobacter pylori*, de la gastrite non à *Helicobacter pylori*, d'un endommagement du tractus gastro-intestinal dû à un anti-inflammatoire non stéroïdien ou d'un trouble psychotique, ou pour la cicatrisation.

38. Procédé pour la préparation d'un composé de formule (I) tel que défini dans la revendication 1, ou d'un sel pharmaceutiquement acceptable ou solvate d'un tel composé, comprenant
(a) la déprotection d'un composé de formule 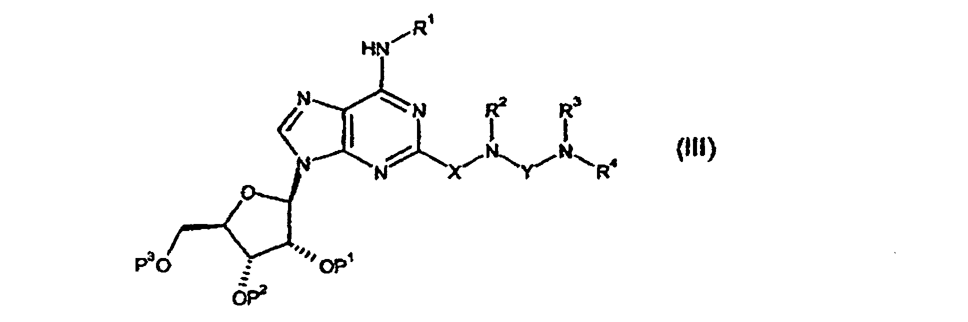 dans laquelle R¹, R², R³, R⁴, X et Y sont tels que définis dans la revendication 1, et soit P¹, P² et P³ considérés séparément sont des groupes protecteurs, soit P¹ et P² considérés ensemble représentent un groupe protecteur et P³ est un groupe protecteur, les groupes protecteurs étant éliminés ensemble ou successivement ; ou
(b) la déprotection d'un composé de formule 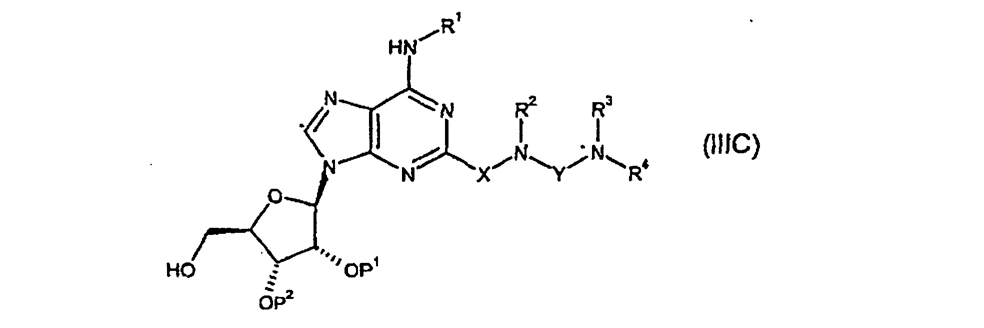 dans laquelle R¹, R², R³, R⁴, X et Y sont tels que définis dans la revendication 1, et soit P¹ et P² considérés séparément sont des groupes protecteurs, soit P¹ et P² considérés ensemble représentent un groupe protecteur, les groupes protecteurs P¹ et P² considérés séparément étant éliminés soit ensemble, soit successivement ; ou
(c) la déprotection d'un composé de formule 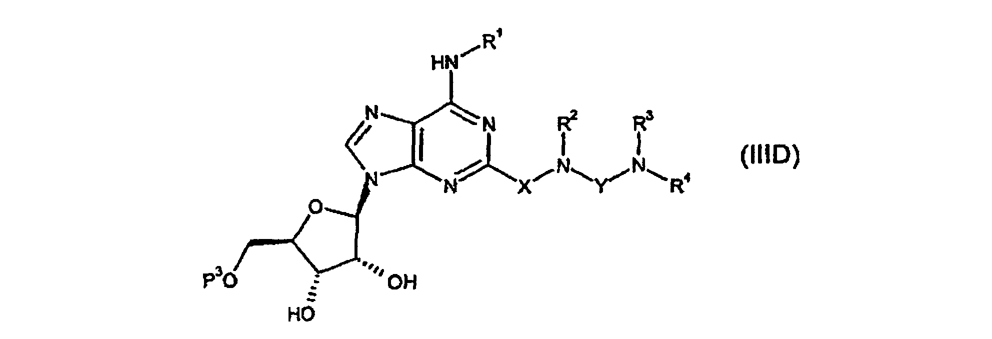 dans laquelle P³ est un groupe protecteur et R¹, R², R³, R⁴, X et Y sont tels que définis dans la revendication 1 ; ou
(d) la déprotection d'un composé de formule 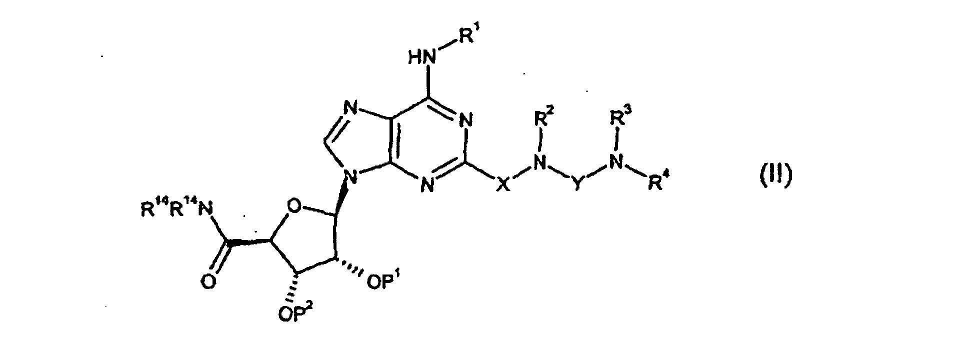
dans laquelle R¹, R², R³, R⁴, R¹⁴, X et Y sont tels que définis dans la revendication 1, et soit P¹ et P² considérés séparément sont des groupes protecteurs, soit P¹ et P² considérés ensemble représentent un groupe protecteur, les groupes protecteurs P¹ et P² considérés séparément étant éliminés soit ensemble, soit successivement ;
l'un quelconque desdits processus (a) à (d) étant éventuellement suivi de la conversion du composé de formule (I) en un sel pharmaceutiquement acceptable.

39. Procédé pour la préparation d'un composé de formule (I) tel que défini dans la revendication 1, ou d'un sel pharmaceutiquement acceptable ou un solvate d'un tel composé, comprenant la réaction d'un composé de formule 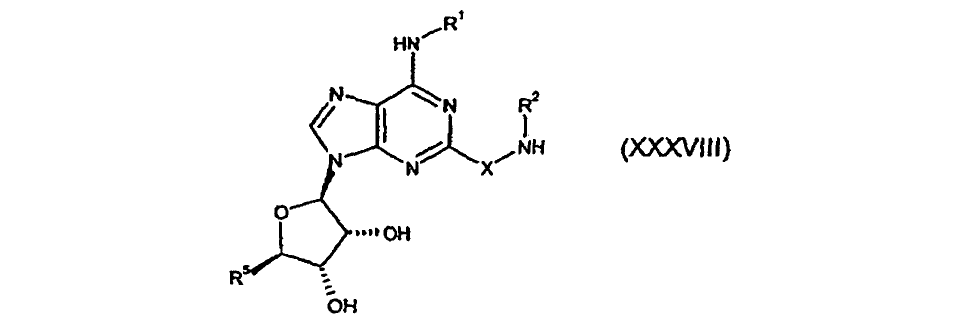 dans laquelle R¹, R², R⁵ et X sont tels que définis dans la revendication 1, avec
(a) un composé de formule 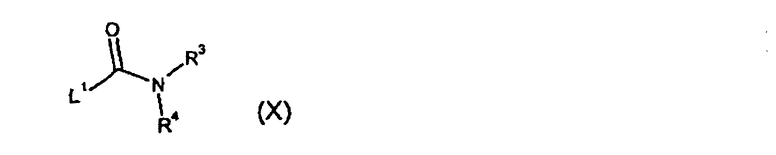 dans laquelle R³ et R⁴ sont tels que définis dans la revendication 1 et L¹ est un groupe partant convenable, de préférence le groupe imidazol-1-yle ; ou
(b) un composé de formule
R³R⁴NSO₂L⁹ (XVII)
dans laquelle R³ et R⁴ sont tels que définis dans la revendication 1 et L⁹ est un groupe partant convenable, de préférence un atome de chlore ; ou
(c) un composé de formule 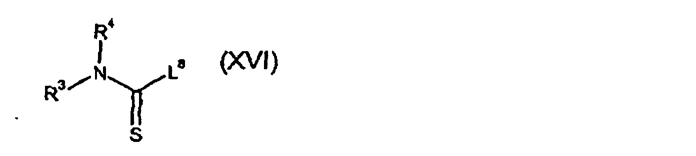 dans laquelle R³ et R⁴ sont tels que définis dans la revendication 1 et L⁸ est un groupe partant convenable, de préférence le groupe méthylthio ou imidazol-1-yle ; ou
(d) un composé de formule 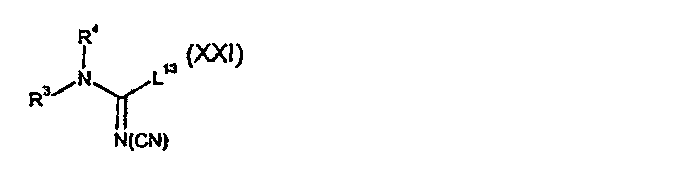
dans laquelle R³ et R⁴ sont tels que définis dans la revendication 1 et L¹³ est un groupe partant convenable, de préférence le groupe méthylthio ;
ledit procédé étant éventuellement suivi de la conversion du composé de formule (I) en un sel pharmaceutiquement acceptable d'un tel composé.

40. Procédé pour la préparation d'un composé de formule (I) tel que défini dans la revendication 1, ou d'un sel pharmaceutiquement acceptable ou d'un solvate d'un tel composé, comprenant
(a) la réaction d'un composé de formule 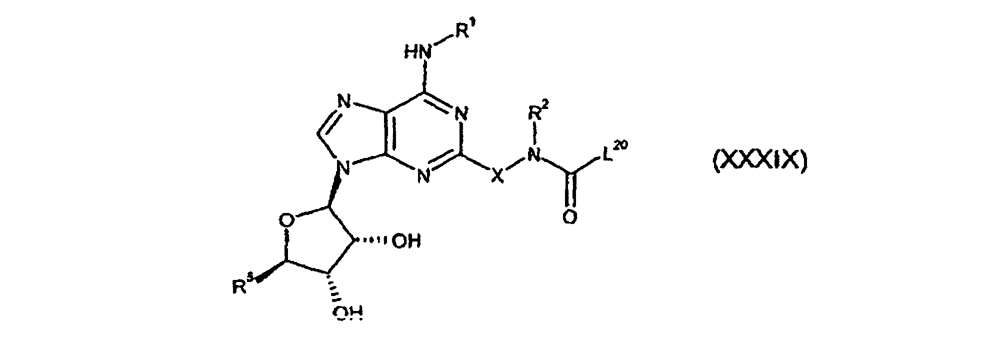 dans laquelle R¹, R², R⁵ et X sont tels que définis dans la revendication 1 et L²⁰ est un groupe partant convenable, de préférence le groupe imidazol-1-yle ; avec un composé de formule
R³R⁴NH (XI)
dans laquelle R³ et R⁴ sont tels que définis dans la revendication 1 ; ou
(b) la réaction d'un composé de formule 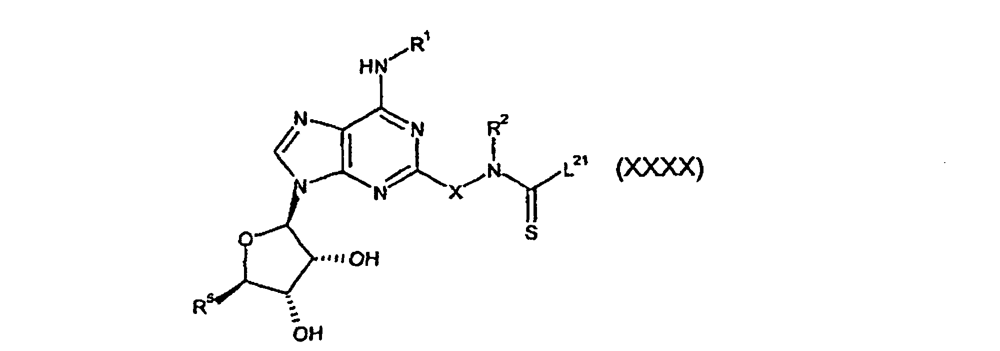 dans laquelle R¹, R², R⁵ et X sont tels que définis dans la revendication 1 et L²¹ est un groupe partant convenable, de préférence le groupe méthylthio ou imidazol-1-yle ; avec un composé de formule
R³R⁴NH (XI)
dans laquelle R³ et R⁴ sont tels que définis dans la revendication 1 ; ou
(c) la réaction d'un composé de formule 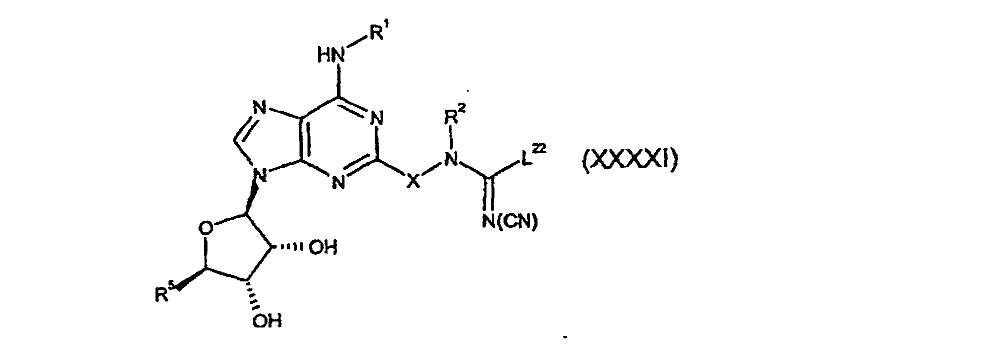
dans laquelle R¹, R², R⁵ et X sont tels que définis dans la revendication 1 et L²² est un groupe partant convenable, de préférence le groupe méthylthio ; avec un composé de formule
R³R⁴NH (XI)
dans laquelle R³ et R⁴ sont tels que définis dans la revendication 1 ; l'un quelconque desdits processus (a) à (c) étant éventuellement suivi de la conversion du composé de formule (I) en un sel pharmaceutiquement acceptable.

41. Procédé pour la préparation d'un composé de formule (I) tel que défini dans la revendication 1, ou d'un sel pharmaceutiquement acceptable ou d'un solvate d'un tel composé, comprenant l'acylation ou la sulfonylation d'un composé de formule 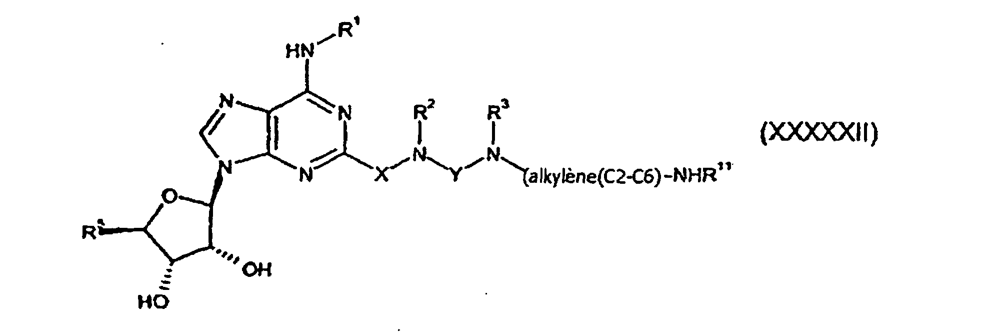 dans laquelle R¹, R², R⁵, R¹¹, X et Y sont tels que définis dans la revendication 1 ;
ledit procédé étant éventuellement suivi de la conversion du composé de formule (I) en un sel pharmaceutiquement acceptable d'un tel composé.

42. Composé de formule 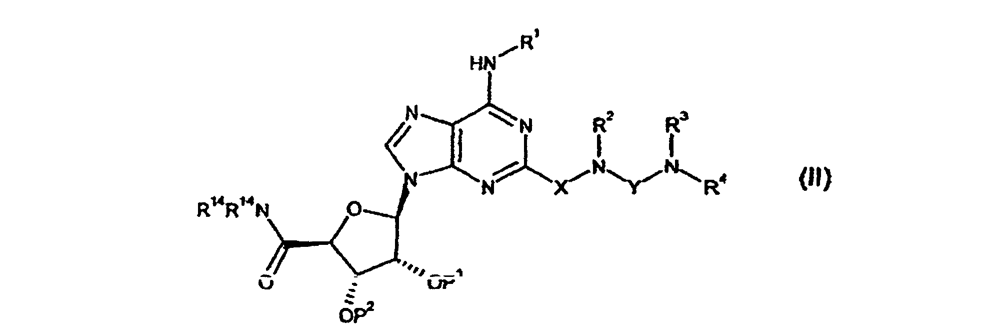 dans laquelle P¹ et P² considérés séparément représentent des groupes protecteurs, ou P¹ et P² considérés ensemble représentent un groupe protecteur ; ou de formule 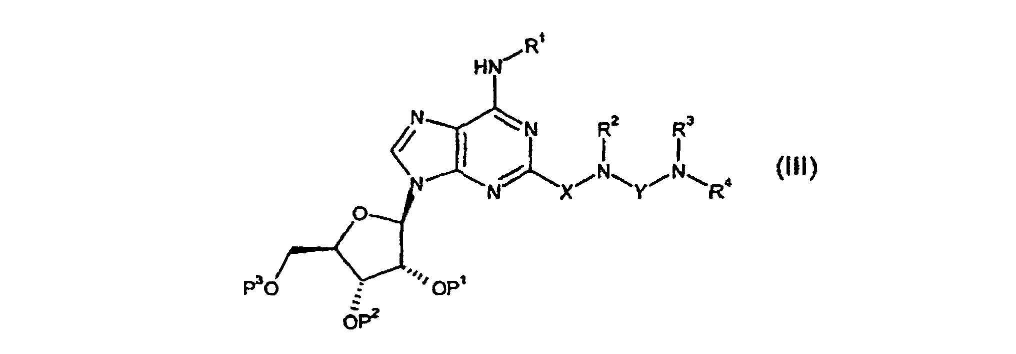 dans laquelle soit P¹, P² et P³ considérés séparément représentent des groupes protecteurs, soit P¹ et P² considérés ensemble représentent un groupe protecteur et P³ est un groupe protecteur ; ou de formule 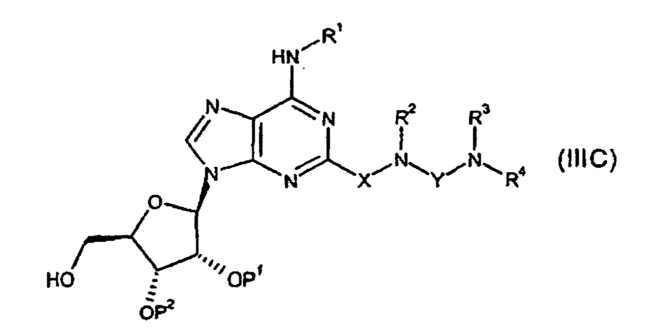 dans laquelle soit P¹ et P² considérés séparément représentent des groupes protecteurs, soit P¹ et P² considérés ensemble représentent un groupe protecteur ; ou de formule 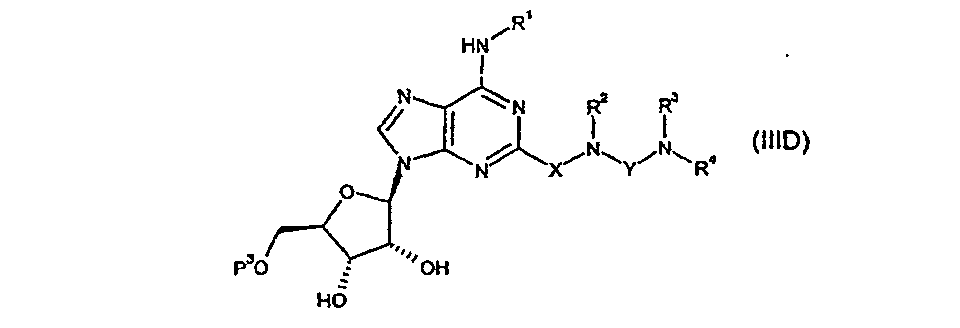 dans laquelle P³ est un groupe protecteur ; ou de formule 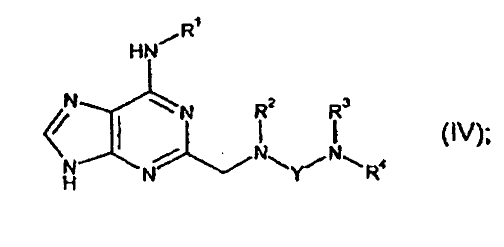 ou 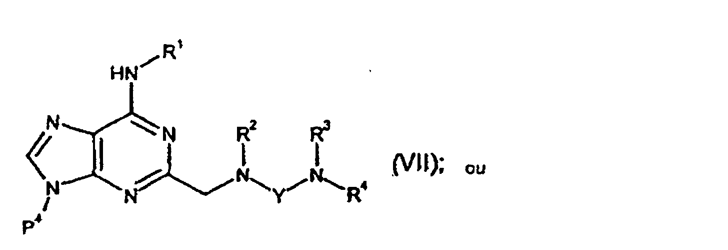 P⁴ étant un groupe protecteur ; ou de formule 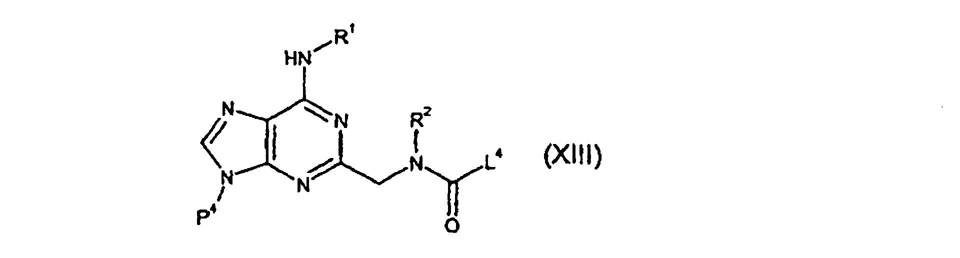 dans laquelle L⁴ est un groupe partant convenable, de préférence le groupe imidazol-1-yle, et P⁴ est un groupe protecteur ; ou de formule 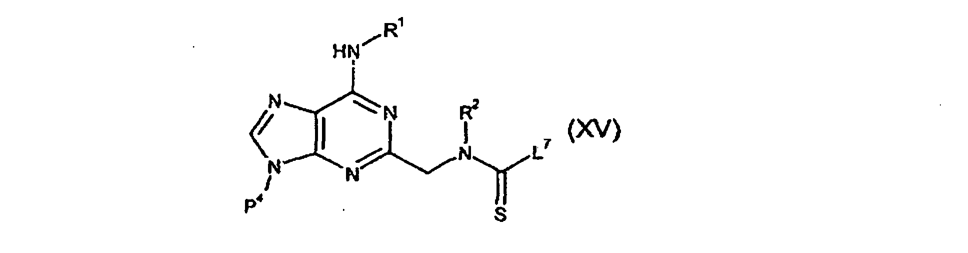 dans laquelle L⁷ est un groupe partant convenable, de préférence le groupe méthylthio ou imidazol-1-yle, et P⁴ est un groupe protecteur ; ou de formule 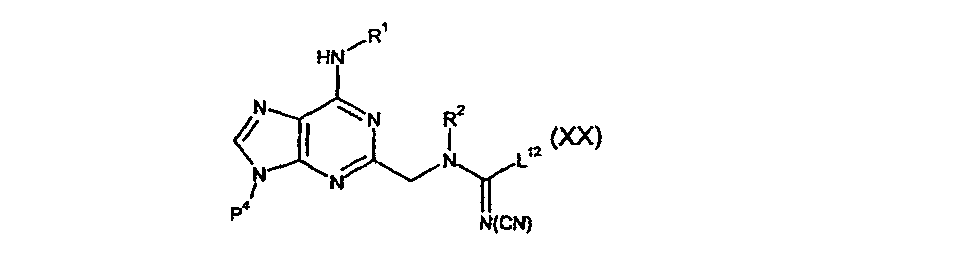 dans laquelle L¹² est un groupe partant convenable, de préférence méthylthio, et P⁴ est un groupe protecteur ; ou de formule 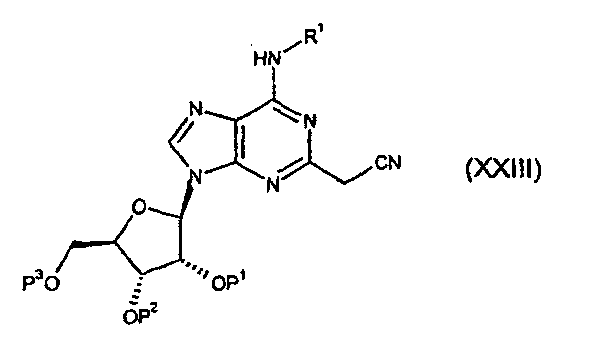 dans laquelle soit P¹, P² et P³, considérés séparément représentent des groupes protecteurs, soit P¹ et P² considérés ensemble représentent un groupe protecteur et P³ est un groupe protecteur ; ou de formule 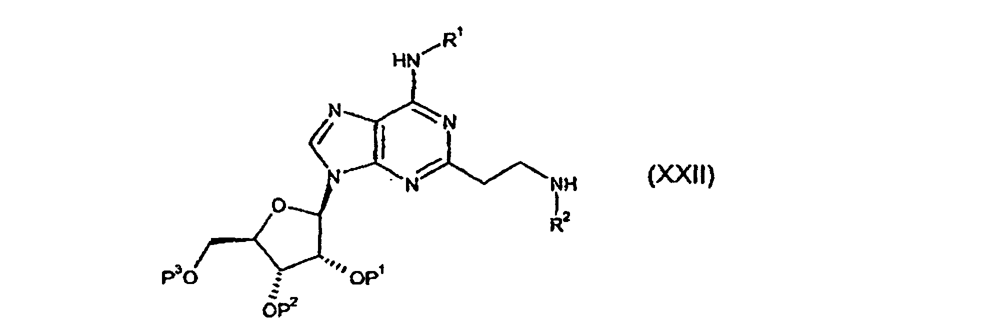 dans laquelle soit P¹, P² et P³ considérés séparément représentent des groupes protecteurs, soit P¹ et P² considérés ensemble représentent un groupe protecteur et P³ est un groupe protecteur ; ou de formule 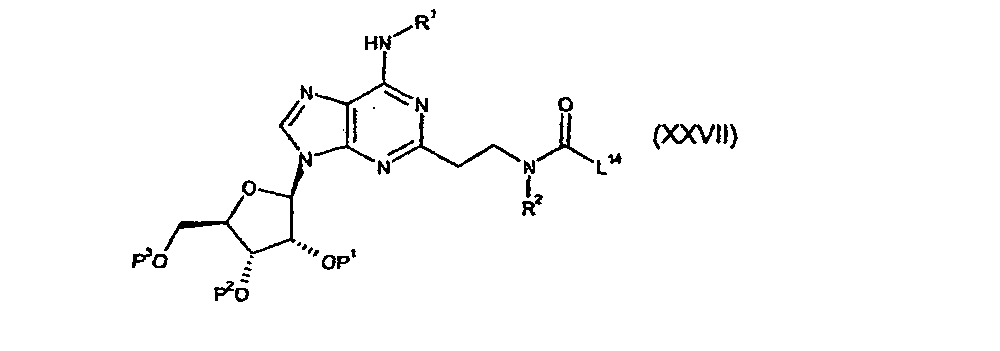 dans laquelle L¹⁴ est un groupe partant convenable, de préférence le groupe imidazol-1-yle, et soit P¹, P² et P³ considérés séparément représentent des groupes protecteurs, soit P¹ et P² considérés ensemble représentent un groupe protecteur et P³ est un groupe protecteur ; ou de formule 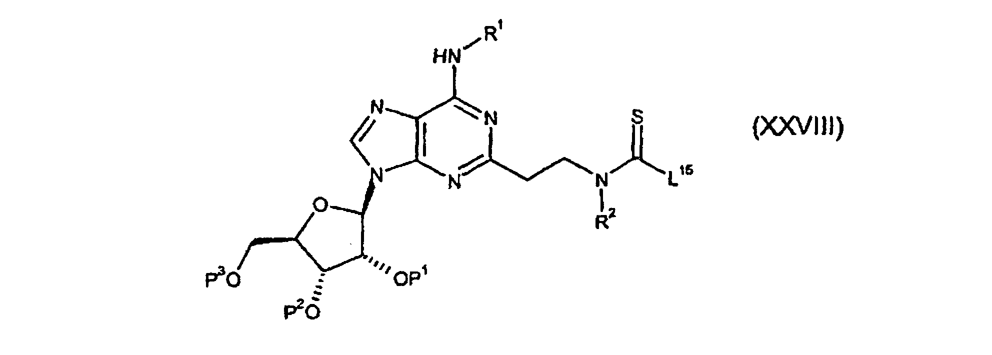 dans laquelle L¹⁵ est un groupe partant convenable, de préférence méthylthio
ou imidazol-1-yle, et soit P¹, P² et P³ considérés séparément représentent des groupes protecteurs, soit P¹ et P² considérés ensemble représentent un groupe protecteur et P³ est un groupe protecteur ; ou de formule 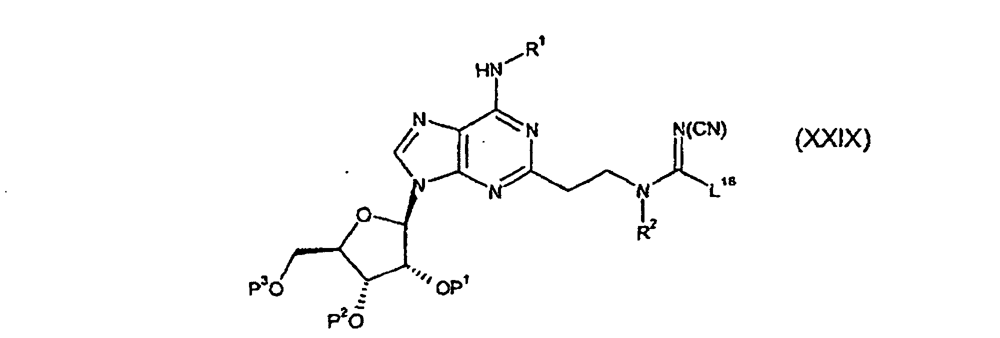 dans laquelle L¹⁶ est un groupe partant convenable, de préférence le groupe méthylthio, et soit P¹, P² et P³ considérés séparément représentent des groupes protecteurs, soit P¹ et P² considérés ensemble représentent un groupe protecteur et P³ est un groupe protecteur ; ou de formule 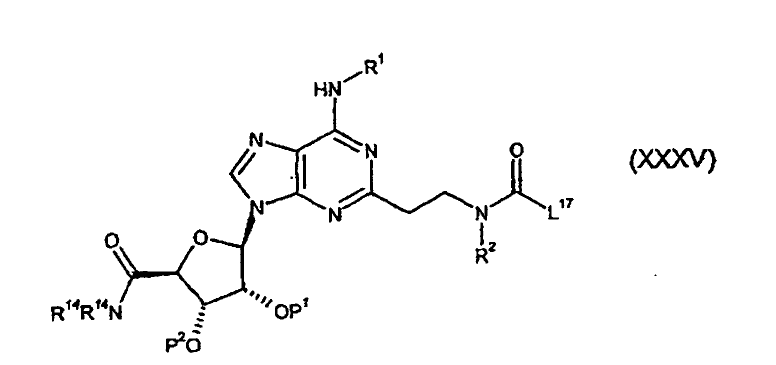 dans laquelle L¹⁷ est un groupe partant convenable, de préférence le groupe imidazol-1-yle, et soit P¹ et P² considérés séparément représentent des groupes protecteurs, soit P¹ et P² considérés ensemble représentent un groupe protecteur ; ou de formule 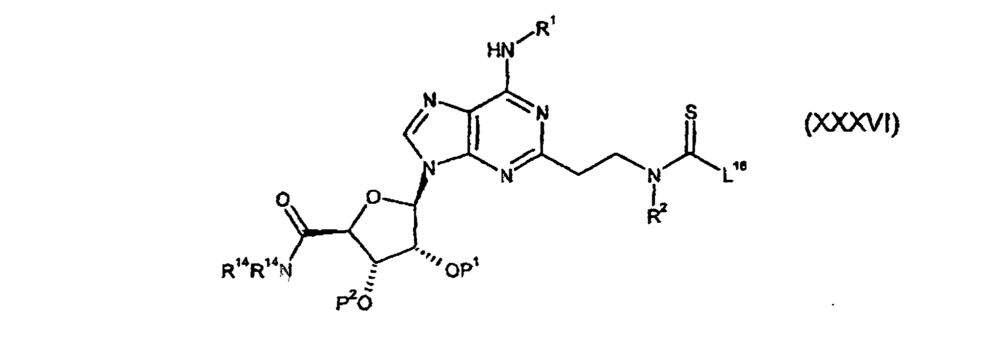 dans laquelle L¹⁸ est un groupe partant convenable, de préférence imidazol-1-yle ou méthylthio, et soit P¹ et P² considérés séparément représentent des groupes protecteurs, soit P¹ et P² considérés ensemble représentent un groupe protecteur ; ou de formule 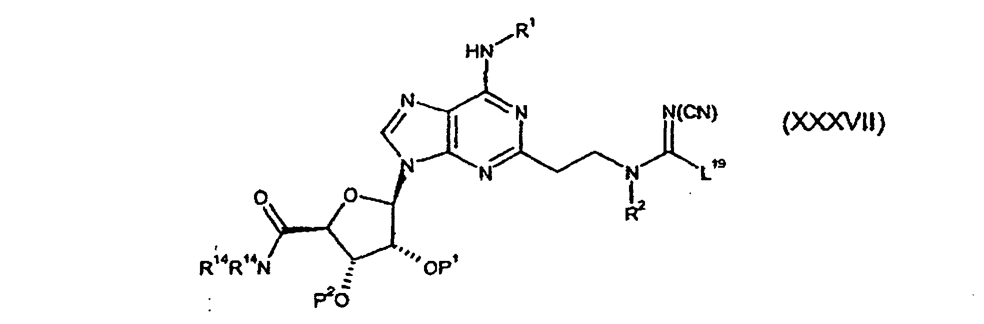 dans laquelle L¹⁹ est un groupe partant convenable, de préférence le groupe méthylthio, et soit P¹ et P² considérés séparément représentent des groupes protecteurs, soit P¹ et P² considérés ensemble représentent un groupe protecteur ; ou de formule 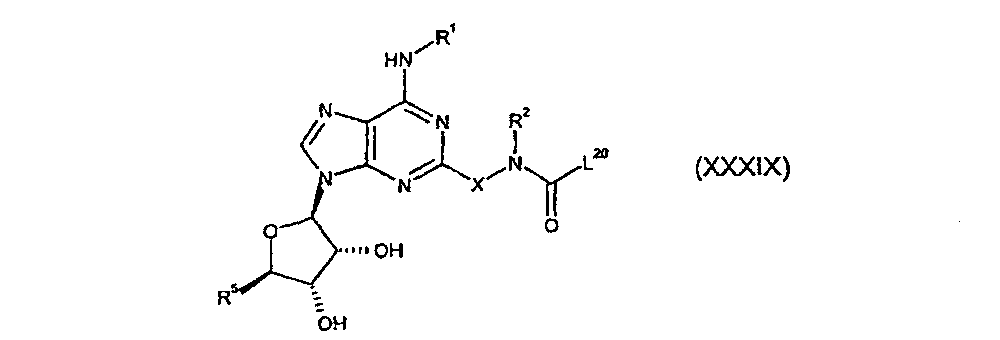 dans laquelle L²⁰ est un groupe partant convenable, de préférence le groupe imidazol-1-yle ; ou de formule 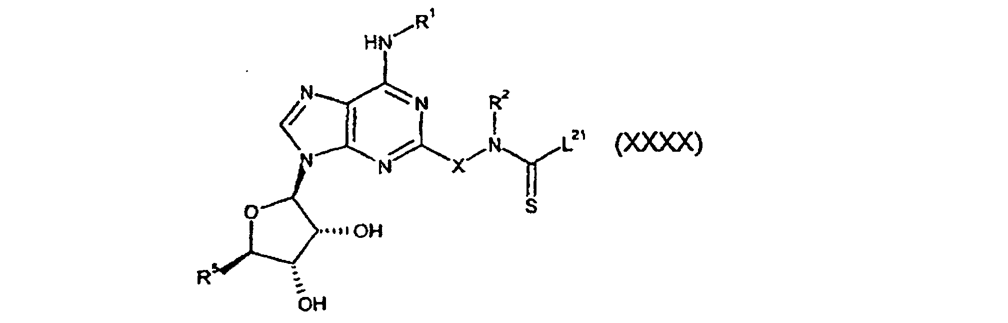 dans laquelle L²¹ est un groupe partant convenable, de préférence méthylthio ou imidazol-1-yle ; ou de formule 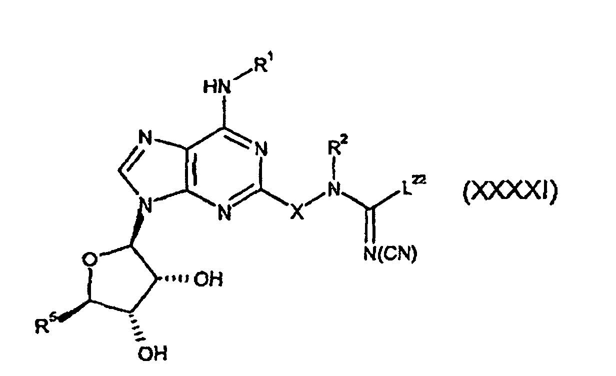 dans laquelle L²² est un groupe partant convenable, de préférence le groupe méthylthio ; ou de formule 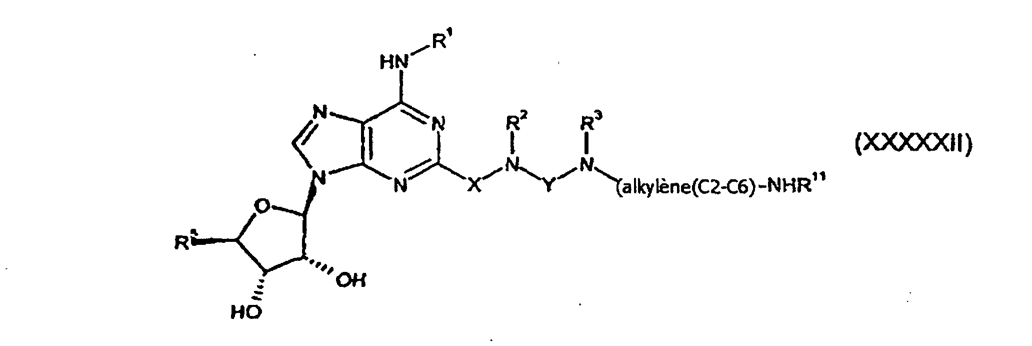 les groupes R¹, R², R³, R⁴, R⁵, R¹¹, R¹⁴, X et Y étant tels que définis dans la revendication 1.
